# EUROPEAN PATENT SPECIFICATION

(11) **EP 3 914 590 B1**
(45) Date of publication and mention of the grant of the patent: **28.09.2022**
(21) Application number: 20700833.5
(22) Date of filing: 20.01.2020
(51) Int. Cl.: C07D 401/14, C07D 407/14, C07D 409/14, C07D 417/14, C07D 471/10, A61P 3/00, A61P 9/00, A61P 17/00, A61P 27/00, A61P 29/00, A61P 33/00, A61P 35/00, A61K 31/454, A61K 31/404, A61K 31/405

(54) **HETEROCYCLIC DERIVATIVES**
HETEROCYCLISCHE DERIVATE
DÉRIVÉS HÉTÉROCYCLIQUES

(30) Priority: 22.01.2019 EP 19152928
(43) Date of publication of application: 01.12.2021
(73) Proprietor: Merck Patent GmbH, 64293 Darmstadt (DE)
(72) Inventor: HEINRICH, Timo, 63579 Freigericht (DE); SCHLESIGER, Sarah, 64293 Darmstadt (DE)
(86) International application number: PCT/EP2020/051210
(87) International publication number: WO 2020/152067

(56) References cited:
- WO-A1-2016/020031
- US-A1- 2013 296 274
- US-A1- 2015 031 670
- US-A1- 2018 228 907

## Description

### BACKGROUND OF THE INVENTION

The invention had the object of finding novel compounds having valuable properties, in particular those which can be used for the preparation of medicaments.

The present invention relates to E3 ligase binding compounds which degrade target proteins, preferably MetAP-2.

The compounds of this invention are heterocyclic derivatives and are useful in treating diseases such as of tumours, tumour metastases, proliferative diseases of the mesangial cells, haemangioma, proliferative retinopathy, rheumatoid arthritis, atherosclerotic neovascularisation, psoriasis, ocular neovascularisation, osteoporosis, diabetes and obesity, lymphoid leukaemia, lymphoma, malaria and prostate hypertrophy. The present invention also provides methods for preparing these compounds and pharmaceutical compositions comprising these compounds.

Small molecule degraders are increasingly utilized as tools to examine the functional roles of proteins and emerged as a novel therapeutic modality. Operating at the post-translational level, these molecules provide the potential for differentiated biological responses in comparison to classical inhibitors and expand the repertoire of methods for protein knock down beyond genetic approaches (e.g.: knock-out, siRNA).

Degrader molecules provide an example of a chemical genetic technique capable of more generally targeting the proteome. These chimeric molecules are designed to induce the degradation of their target proteins via the ubiquitin proteasome system (UPS), thereby eliminating pre-existing proteins. The UPS is the major intracellular pathway for protein degradation in which a series of enzymes known as E1s (ubiquitin activating enzymes), E2s (ubiquitin conjugating enzymes) and E3s (ubiquitin ligases) carry out covalent linkage of the 9kDa, 76 amino acid protein ubiquitin to a target protein. Subsequent enzymatic reactions result in the formation of a polyubiquitin chain, which targets the protein for degradation by the 26S proteasome.

Bifunctional degraders comprise an E3 ligase-binding motif that is linked to a target protein binding moiety. Consequently, these molecules hijack the cell's own degradation machinery by recruiting an E3 ligase in vicinity of the target protein. The spatial proximity enables ubiquitination of the protein and subsequent recognition and depletion by the UPS through the formation of a stable ternary complex.

Specificity for a particular target protein is associated with the E3 ligase (Li W, et al. PLoS One. 2008; 3:e1487) that facilitates the final step of ubiquitin attachment to the target protein. While the first generation degraders were successfully developed using peptides as an E3 ubiquitin ligase-recognizing motif, they were either not cell-permeable or made cell-permeable by adding a cell-permeating motif such as the TAT peptide (Sakamoto KM, et al Proc Natl Acad Sci U S A. 2001; 98:8554-8559; Zhang D, et al. Bioorg Med Chem Lett. 2004; 14:645-648; Schneekloth JS Jr. et al. J Am Chem Soc. 2004; 126:3748-3754.). The poor cell permeability of the first generation of bifunctional degraders was significantly improved by the discovery of small molecules that bind to E3 ligases such as the Von Hippel Lindau (VHL) ligand binding to VHL ligase (Buckley et al, J. Am. Chem. Soc., 2012, 134 (10), pp 4465-4468) or thalidomide derivatives binding to the CRBN or Cereblon E3 Ligase (Winter et al, Science 19 Jun 2015: Vol. 348, Issue 6241, pp. 1376-1381). Having all small molecule degraders in hand enabled scientists to optimize those tool compounds into relevant therapeutic compounds.

So far, androgen receptor (AR) and estrogen receptor (ER) targeting degraders have been developed into clinical candidates, demonstrating the potential applications of these molecules in the treatment of prostate and breast cancers (Rodriguez-Gonzalez A, et al. Oncogene. 2008; 27:7201-7211; Cyrus K, et al. Chembiochem. 2010; 11:1531-1534).

Next to peptidic degraders targeting methionine aminopeptidase-2 (MetAP-2) (Sakamoto KM, et al. Proc Natl Acad Sci U S A. 2001; 98:8554-8559; WO2002020740) a range of other bifunctional degraders have been developed targeting proteins ranging from kinases, signaling proteins as well as cytosolic proteins and membrane receptors (examples in Ottis et al ACS Chem. Biol., 2017, 12 (4), pp 892-898).

The synthesis of degrader compounds that mediate the degradation of MetAP-2 by recruiting VHL E3 ligase is reported in (WO2002020740). This approach is focused on MetAP-2 ligands that bind the target protein in a covalent fashion and a peptidic sequence targetic VHL. A covalent mode of action drastically impairs the putative catalytic function of degrader molecules.

In this invention, the target protein MetAP-2 is bound by reversible binding ligands described for example in WO2013/149704. The E3 ligase is recognized by known motives as described for example in WO2018/033556 or CN107540608.

It has been found that the compounds according to the invention and salts thereof have very valuable pharmacological properties while being well tolerated.

Surprisingly, we found that compounds of the invention degrade MetAP-2 in a dose dependent manner in disease relevant cell lines.

Compounds of the invention inhibit the MetAP-2 enzyme with nanomolar concentration and inhibit HUVEC cell proliferation with micromolar IC50s as given in the attached table.

This inhibition of MetAP-2 in the enzymatic as well as the cellular assays is comparable to the MetAP-2 inhibition demonstrated with compounds from the Merck applications WO 2012/048775, WO 2013/149704, WO 2016/020031. Accordingly, compounds presently claimed are useful for the treatment of diseases as decribed in WO 2012/048775, WO 2013/149704, WO 2016/020031.

The present invention specifically relates to compounds of the formula I which degrade the target protein MetAP-2, to compositions which comprise these compounds, and to processes for the use thereof for the treatment of diseases and complaints.

The host or patient can belong to any mammalian species, for example a primate species, particularly humans; rodents, including mice, rats and hamsters; rabbits; horses, cows, dogs, cats, etc. Animal models are of interest for experimental investigations, providing a model for treatment of human disease.

The susceptibility of a particular cell to treatment with the compounds according to the invention can be determined by in vitro tests. Typically, a culture of the cell is combined with a compound according to the invention at various concentrations for a period of time which is sufficient to allow active agents such as anti IgM to induce a cellular response such as expression of a surface marker, usually between about one hour and one week. In vitro testing can be carried out using cultivated cells from blood or from a biopsy sample. The amount of surface marker expressed is assessed by flow cytometry using specific antibodies recognising the marker.

The dose varies depending on the specific compound used, the specific disease, the patient status, etc. A therapeutic dose is typically sufficient considerably to reduce the undesired cell population in the target tissue while the viability of the patient is maintained. The treatment is generally continued until a considerable reduction has occurred, for example an at least about 50% reduction in the cell burden, and may be continued until essentially no more undesired cells are detected in the body.

### PRIOR ART

Other heterocyclic amide derivatives as inhibitors of the activity of target proteins are disclosed in WO 2018/033556 A1.

Cyclic amides are described as MetAP-2 inhibitors in WO 2012/048775 A1, WO 2013/149704 A1 and WO 2016/020031 A1.

### SUMMARY OF THE INVENTION

The invention relates to compounds of the formula I

Q¹-Q²-Q³

in which
- Q¹: denotes 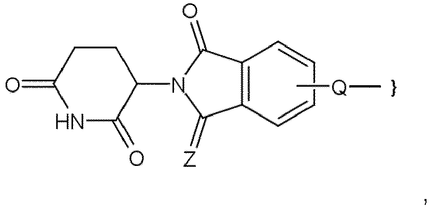 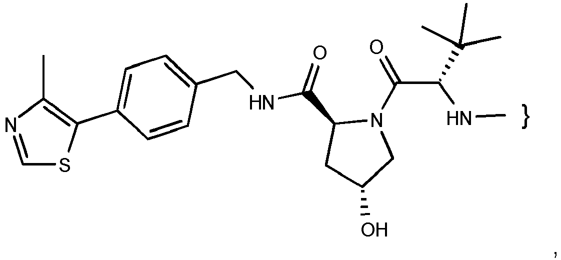 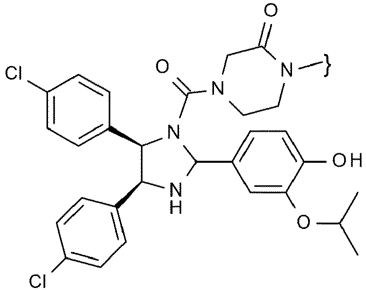
or 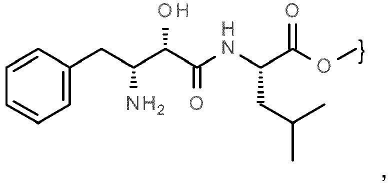
- Z: denotes O or CH₂,
- Q: denotes O, NH, NCH₃ or CH₂,
- Q²: denotes unbranched alkylene having 4-25 C atoms, in which 1-8 non-adjacent CH₂ groups may be replaced by O, CONH and/or NHCO and in which one CH₂ group may be replaced by

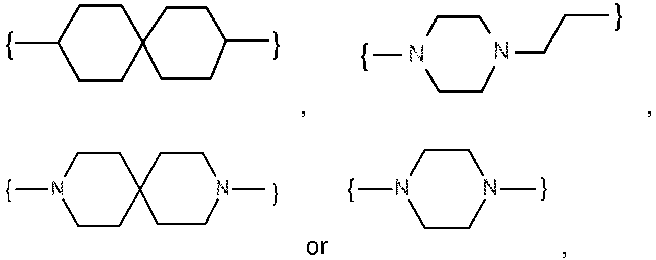

- Q³: denotes

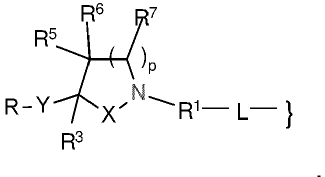

- L: denotes NR⁴CO, CONR⁴, NH, O, CO, S, SO₂, SO(=NH), NHCONH, SO₂NH or NHSO₂,
- R: denotes NR²R⁴, Alk, C(=CH₂)[C(R⁴)₂]ₙAr², Het², O[C(R⁴)₂]ₙAr² or OA,
- X: denotes CO or CH₂,
- Y: denotes CO or CH₂,
- R¹: denotes (CH₂)n, [C(R⁴)₂]nAr¹-, (CH₂)ₙHet-, (CH₂)ₙCyc-, [C(R⁴)₂]ₙCONHAr¹-, [C(R⁴)₂]ₙNA-, O[C(R⁴)₂]ₙAr¹- or [C(R⁴)₂]ₙCOO(CH₂)nAr¹-,
- R²: wherein substituent L directly is connected to Ar¹, Het or Cyc, denotes H, [C(R⁴)₂]ₙAr², (CH₂)ₙCOHet¹, (CH₂)ₙCOAr², (CH₂)ₘNA₂, (CH₂)ₙCyc or (CH₂)ₙHet¹,
- R³: denotes OH or OCOA,
- R⁴: denotes H or alkyl having 1, 2, 3 or 4 C-atoms,
- R² and R⁴: together also denote alkylene having 2, 3, 4 or 5 C-atoms, where a CH₂ group may also be replaced by N(CH₂)ₘOH or SO₂,
- R⁵, R⁶: each, independently of one another H, F or A,
- R⁵ and R⁶: together also denote alkylene having 2, 3, 4 or 5 C-atoms, where a CH₂ group may also be replaced by NCOA or O,
- R⁷: denotes H, Hal or A,
- Ar¹: denotes phenyl which is unsubstituted or mono-, di- tri-, tetra- or pentasubstituted by Hal, OH, OA, CONH₂, CONHA, CONA₂, NHSO₂A, CONHCyc, NHSO₂Cyc, CONHAr², Het¹, COHet¹ and/or NASO₂A,
- Ar²: denotes phenyl which is unsubstituted or mono-, di-, tri-, tetra- or pentasubstituted by Hal, A, CONH₂, and/or OAr³,
- Ar³: denotes phenyl which is unsubstituted or monosubstituted by NH₂,
- Het: denotes a mono- or bicyclic saturated, unsaturated or aromatic heterocycle having 1 to 4 N, and/or O and/or S atoms which is unsubstituted or mono-, di- or trisubstituted by Hal, A, OA, CN, NH₂, NHA, NA2, NO₂, CN, COOH, COOA, (CH2)nCONH2, (CH2)nCONHA, (CH2)nCONA2, NHCOA, COA, CHO, Het¹, SO₂A, SO₂NH₂, SO₂NHA, SO₂NA₂, CONHNH₂, CONHAr³, =O and/or Ar³,
- Het¹: denotes pyridazinyl, pyrazolyl, pyridyl, piperazinyl, morpholinyl, pyrimidinyl, furyl, thienyl, imidazolyl, pyrrolyl, oxazolyl, oxadiazolyl, isoxazolyl, thiazolyl, triazolyl, tetrazolyl, thiadiazole, piperidin-1-yl, pyrrolidin-1-yl, tetrahydropyranyl, 1,2-oxazinan-2-yl, 1,2,5-oxadiazinan-2-yl, 1,3-oxazinan-3-yl or hexahydropyrimidinyl, each of which is unsubstituted or mono-, di- or trisubstituted by A and/or OA,
- Het²: denotes isoindolyl,

- A: denotes unbranched or branched alkyl having 1-10 C atoms, in which 1-7 H atoms may be replaced by F, Cl, Br, OH, CHO, COA, COOA, CN, CONA₂, CONHA and/or CONH₂, and/or in which one or two non-adjacent CH and/or CH₂ groups may be replaced by O, or Cyc,
- Alk: denotes alkenyl having 2, 3, 4, 5 or 6 C atoms
- Cyc: denotes cyclic alkyl having 3-7 C atoms which is unsubstituted or mono-, di- or trisubstituted by NHCOA, NHSO₂,OH, OA, A, NH₂, NHA, NA₂, COOA, COOH and/or CONHA,
- Hal: denotes F, Cl, Br or I,
- m: denotes 1, 2, 3 or 4,
- n: denotes 0, 1, 2, 3 or 4,
- p: denotes 1, 2 or 3,
and pharmaceutically acceptable salts, tautomers and stereoisomers thereof, including mixtures thereof in all ratios.

The invention also relates to the optically active forms (stereoisomers), the enantiomers, the racemates, the diastereomers and the hydrates and solvates of these compounds.

Moreover, the invention relates to pharmaceutically acceptable derivatives of compounds of formula I.

The term solvates of the compounds is taken to mean adductions of inert solvent molecules onto the compounds which form owing to their mutual attractive force. Solvates are, for example, mono- or dihydrates or alkoxides.

It is understood, that the invention also relates to the solvates of the salts. The term pharmaceutically acceptable derivatives is taken to mean, for example, the salts of the compounds according to the invention and also so-called prodrug compounds.

As used herein and unless otherwise indicated, the term "prodrug" means a derivative of a compound of formula I that can hydrolyze, oxidize, or otherwise react under biological conditions (in vitro or in vivo) to provide an active compound, particularly a compound of formula I. Examples of prodrugs include, but are not limited to, derivatives and metabolites of a compound of formula I that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. In certain embodiments, prodrugs of compounds with carboxyl functional groups are the lower alkyl esters of the carboxylic acid. The carboxylate esters are conveniently formed by esterifying any of the carboxylic acid moieties present on the molecule. Prodrugs can typically be prepared using well- known methods.

The expression "effective amount" denotes the amount of a medicament or of a pharmaceutical active ingredient which causes in a tissue, system, animal or human a biological or medical response which is sought or desired, for example, by a researcher or physician.

In addition, the expression "therapeutically effective amount" denotes an amount which, compared with a corresponding subject who has not received this amount, has the following consequence:
improved treatment, healing, prevention or elimination of a disease, syndrome, condition, complaint, disorder or side-effects or also the reduction in the advance of a disease, complaint or disorder.

The expression "therapeutically effective amount" also encompasses the amounts which are effective for increasing normal physiological function.

The invention also relates to the use of mixtures of the compounds of the formula I, for example mixtures of two diastereomers, for example in the ratio 1:1, 1:2, 1:3, 1:4, 1:5, 1:10, 1:100 or 1:1000.

These are particularly preferably mixtures of stereoisomeric compounds.

"Tautomers" refers to isomeric forms of a compound that are in equilibrium with each other. The concentrations of the isomeric forms will depend on the environment the compound is found in and may be different depending upon, for example, whether the compound is a solid or is in an organic or aqueous solution.

The invention relates to the compounds of the formula I and salts thereof and to a process for the preparation of compounds of the formula I, wherein L denotes CONR⁴, and pharmaceutically acceptable salts, solvates, tautomers and stereoisomers thereof,
characterised in that a compound of formula II 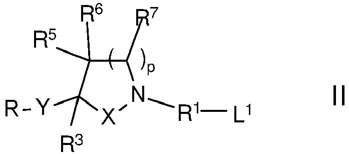
in which X, R, R¹, R³, R⁵, R⁶, R⁷ and p have the meanings indicated in Claim 1,
and L¹ denotes Cl, Br, I or a free or reactively functionally modified OH group,
is reacted with a compound of the formula III

   Q¹-Q²-NH₂ III
in which Q¹ and Q² have the meanings indicated in Claim 1,
and/or
a base or acid of the formula I is converted into one of its salts.

Above and below, the radicals R¹, R², R³ have the meanings indicated for the formula I, unless explicitely stated otherwise.

A denotes alkyl, this is unbranched (linear) or branched, and has 1, 2, 3, 4, 5, 6, 7 or 8 C atoms. A preferably denotes methyl, ethyl, propyl, isopropyl, butyl, isobutyl, sec-butyl or tert-butyl, furthermore also pentyl, 1-, 2- or 3-methylbutyl, 1,1- , 1,2- or 2,2-dimethylpropyl, 1-ethylpropyl, hexyl, 1- , 2- , 3- or 4-methylpentyl, 1,1- , 1,2- , 1,3- , 2,2- , 2,3- or 3,3-dimethylbutyl, 1- or 2-ethylbutyl, 1-ethyl-1-methylpropyl, 1-ethyl-2-methylpropyl, 1,1,2- or 1,2,2-trimethylpropyl, furthermore preferably, for example, trifluoromethyl. A very particularly preferably denotes alkyl having 2, 3, 4, 5 or 6 C atoms, preferably ethyl, propyl, isopropyl, butyl, isobutyl, sec-butyl, tert-butyl, pentyl, hexyl, trifluoromethyl, pentafluoroethyl or 1,1,1-trifluoroethyl. Moreover, A denotes preferably CH₂OCH₃, CH₂CH₂OH or CH₂CH₂OCH₃. Cyclic alkyl preferably denotes cyclopropyl, cyclobutyl, cyclopentyl, cyclohexyl or cycloheptyl.

R preferably denotes NR²R⁴, furthermore Alk, C(=CH₂)[C(R⁴)₂]ₙAr² or Het².

R particularly preferably denotes NR²R⁴, very particularly preferably NHCH₂Ar².

X preferably denotes CO, furthermore CH₂.

Y preferably denotes CO, furthermore CH₂.

R¹ preferably denotes (CH₂)n, [C(R⁴)₂]nAr¹-, (CH₂)ₙHet- or (CH₂)ₙCyc-, furthermore [C(R ⁴)₂]ₙCONHAr¹or [C(R⁴)₂]nNA-.

Substituent L is directly connected to Ar¹, Het or Cyc and not to the (CH₂)ₙ or [C(R⁴)₂]ₙ moiety.

R¹ particularly preferably denotes o-, m- or p-phenylen, indole-diyl or benzimidazole-diyl.

R² preferably denotes [C(R⁴)₂]ₙAr², (CH₂)ₙCyc or (CH₂)ₙHet¹.

R³ preferably denotes OH.

R⁴ preferably denotes H, methyl, ethyl or propyl, very particularly preferably H or methyl.

R⁵, R⁶ preferably denote H.

R⁷ preferably denotes H, F or CH₃.

Ar¹ preferably denotes phenyl, o-, m- or p-fluorophenyl, o-, m- or p-bromophenyl, o-, m- or p-chlorophenyl, o-, m- or p-hydroxyphenyl, o-, m- or p-methoxyphenyl, o-, m- or p-aminocarbonylphenyl, further preferably 2,3-, 2,4-, 2,5-, 2,6-, 3,4- or 3,5-difluorophenyl, 2,3-, 2,4-, 2,5-, 2,6-, 3,4- or 3,5-dichlorophenyl, 2,3-, 2,4-, 2,5-, 2,6-, 3,4- or 3,5-dibromophenyl, 2,3,4-, 2,3,5-, 2,3,6-, 2,4,6- or 3,4,5-trichlorophenyl, p-iodophenyl, 4-fluoro-3-chlorophenyl, 2-fluoro-4-bromophenyl or 2,5-difluoro-4-bromophenyl.

Ar¹ preferably denotes phenyl.

Ar² preferably denotes phenyl, o-, m- or p-tolyl, o-, m- or p-ethylphenyl, o-, m- or p-propylphenyl, o-, m- or p-isopropylphenyl, o-, m- or p-tert-butylphenyl, o-, m- or p-trifluoromethylphenyl, o-, m- or p-fluorophenyl, o-, m- or p-bromophenyl, o-, m- or p-chlorophenyl, o-, m- or p-aminocarbonylphenyl, further preferably 2,3-, 2,4-, 2,5-, 2,6-, 3,4- or 3,5-difluorophenyl, 2,3-,

2,4-, 2,5-, 2,6-, 3,4- or 3,5-dichlorophenyl, 2,3-, 2,4-, 2,5-, 2,6-, 3,4- or 3,5-dibromophenyl, 2,3,4-, 2,3,5-, 2,3,6-, 2,4,6- or 3,4,5-trichlorophenyl, p-iodophenyl, 4-fluoro-3-chlorophenyl, 2-fluoro-4-bromophenyl, 2,5-difluoro-4-bromophenyl or 2,5-dimethyl-4-chlorophenyl.

Ar² preferably denotes phenyl which is unsubstituted or mono-, di-, tri- or tetra-substituted by Hal.

Ar² furthermore particularly preferably denotes phenyl which is mono- or disubstituted by Hal.

Irrespective of further substitutions, Het preferably denotes 2- or 3-furyl, 2- or 3-thienyl, 1-, 2- or 3-pyrrolyl, 1-, 2, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, furthermore preferably 1,2,3-triazol-1-, -4- or -5-yl, 1,2,4-triazol-1-, -3- or 5-yl, 1- or 5-tetrazolyl, 1,2,3-oxadiazol-4- or -5-yl, 1,2,4-oxadiazol-3- or -5-yl, 1,3,4-thia-diazol-2- or -5-yl, 1,2,4-thiadiazol-3- or -5-yl, 1,2,3-thiadiazol-4- or -5-yl, 3- or 4-pyridazinyl, pyrazinyl, 1-, 2-, 3-, 4-, 5-, 6- or 7-indolyl, 4- or 5-isoindolyl, 1-, 2-, 4- or 5-benzimidazolyl, 1-, 2-, 3-, 4-, 5-, 6- or 7-indazolyl, 1-, 3-, 4-, 5-, 6- or 7-benzopyrazolyl, 2-, 4-, 5-, 6- or 7-benzoxazolyl, 3-, 4-, 5-, 6- or 7- benzisoxazolyl, 2-, 4-, 5-, 6- or 7-benzothiazolyl, 2-, 4-, 5-, 6- or 7-benzisothiazolyl, 4-, 5-, 6- or 7-benz-2,1,3-oxadiazolyl, 2-, 3-, 4-, 5-, 6-, 7- or 8-quinolyl, 1-, 3-, 4-, 5-, 6-, 7- or 8-isoquinolyl, 3-, 4-, 5-, 6-, 7- or 8-cinnolinyl, 2-, 4-, 5-, 6-, 7- or 8-quinazolinyl, 5- or 6-quinoxalinyl, 2-, 3-, 5-, 6-, 7- or 8-2H-benzo-1,4-oxazinyl, further preferably 1,3-benzodioxol-5-yl, 1,4-benzodioxan-6-yl, 2,1,3-benzothiadiazol-4- or -5-yl or 2,1,3-benzoxadiazol-5-yl.

The heterocyclic radicals may also be partially or fully hydrogenated. Unsubstituted Het can thus also denote, for example, 2,3-dihydro-2-, -3-, -4-or -5-furyl, 2,5-dihydro-2-, -3-, -4- or 5-furyl, tetrahydro-2- or -3-furyl, 1,3-di-oxolan-4-yl, tetrahydro-2- or -3-thienyl, 2,3-dihydro-1-, -2-, -3-, -4- or -5-pyrrolyl, 2,5-dihydro-1-, -2-, -3-, -4- or -5-pyrrolyl, 1-, 2- or 3-pyrrolidinyl, tetra-hydro-1-, -2- or -4-imidazolyl, 2,3-dihydro-1-, -2-, -3-, -4- or -5-pyrazolyl, tetra-hydro-1-, -3- or -4-pyrazolyl, 1,4-dihydro-1-, -2-, -3- or -4-pyridyl, 1,2,3,4-tetrahydro-1-, -2-, -3-, -4-, -5- or -6-pyridyl, 1-, 2-, 3- or 4-piperidinyl, 2-, 3- or 4-morpholinyl, tetrahydro-2-, -3- or -4-pyranyl, 1,4-dioxanyl, 1,3-dioxan-2-, -4-or -5-yl, hexahydro-1-, -3- or -4-pyridazinyl, hexahydro-1-, -2-, -4- or -5-pyrimidinyl, 1-, 2- or 3-piperazinyl, 1,2,3,4-tetrahydro-1-, -2-, -3-, -4-, -5-, -6-, -7-or -8-quinolyl, 1,2,3,4-tetrahydro-1-, -2-, -3-, -4-, -5-, -6-, -7- or -8-isoquinolyl, 2-, 3-, 5-, 6-, 7- or 8- 3,4-dihydro-2H-benzo-1,4-oxazinyl, further preferably 2,3-methylenedioxyphenyl, 3,4-methylenedioxyphenyl, 2,3-ethylenedioxyphenyl, 3,4-ethylenedioxyphenyl, 3,4-(difluoromethylenedioxy)phenyl, 2,3-dihydrobenzofuran-5- or 6-yl, 2,3-(2-oxomethylenedioxy)phenyl or also 3,4-dihydro-2H-1,5-benzodioxepin-6- or -7-yl, furthermore preferably 2,3-dihydro-benzofuranyl or 2,3-dihydro-2-oxofuranyl.

Het furthermore preferably denotes pyrazinyl, pyrazolyl, benzimidazolyl, pyridyl, indolyl, dihydroindolyl, benzofuranyl, tetrahydropyranyl, dihydroquinolinyl, dihydroisoquinolinyl, tetrahydroquinolinyl, tetrahydroisoquinolinyl, indazolyl, imidazolyl, pyrrolyl, oxazolyl, oxadiazolyl, isoxazolyl, benzothiazolyl, piperidin-1-yl, pyrrolidin-1-yl, 3,4-dihydro-2H-pyrido[3,2-b]-1,4-oxazinyl, 3,4-dihydro-2H-benzo-1,4-oxazinyl, benzofuranyl, azetidinyl, 3-azabicylo[3.2.0]hexyl, pyrrolo[2,3-b]pyridinyl, tetrahydrofuranyl, tetrahydro-1,8-naphthyridinyl, 2,3-dihydrobenzoisothiazolyl, 1,2,3,4-tetrahydrobenzothiazinyl or hexahydro-benzo-1,3-dioxolyl, each of which is unsubstituted or mono-, di- or trisubstituted by Hal, A, OA, CN, NH₂, NHA, NA₂, NO₂, CN, COOH, COOA, (CH2)nCONH2, (CH2)nCONHA, (CH2)nCONA2, NHCOA, COA, CHO, Het¹, SO₂A, SO₂NH₂, SO₂NHA,SO₂NA₂, CONHNH₂, CONHAr³, =O and/or Ar³.

Het furthermore preferably denotes benzimidazolyl or indolyl, each of which is unsubstituted or monosubstituted by Hal.

Het¹ preferably denotes pyridazinyl, pyrazolyl, pyridyl, piperazinyl, morpholinyl, pyrimidinyl, furyl, thienyl, imidazolyl, pyrrolyl, oxazolyl, oxadiazolyl, isoxazolyl, thiazolyl, triazolyl, tetrazolyl, thiadiazole, piperidin-1-yl, pyrrolidin-1-yl, tetrahydropyranyl, 1,2-oxazinan-2-yl, 1,2,5-oxadiazinan-2-yl, 1,3-oxazinan-3-yl or hexahydropyrimidinyl, each of which is unsubstituted or monosubstituted by A and/or OA.

Het¹ furthermore preferably denotes pyridyl, pyrimidinyl, furyl, thienyl, imidazolyl, pyrrolyl, oxazolyl, isoxazolyl, thiazolyl, triazolyl or tetrazolyl.

Het¹ furthermore particularly preferably denotes pyridyl, furyl, thienyl, imidazolyl or pyrrolyl.

Q2 preferably denotes CH₂OCH₂CH₂OCH₂CH₂O, (CH₂)₂O(CH₂)₂O(CH₂)₂O(CH₂)₂O(CH₂)₂O(CH₂)₂, OCH₂CH₂CH₂OCH₂ CH2CH2, (CH2)5, (CH2)6, (CH2)7, (CH2)8, (CH₂)₃O(CH₂)₄, (CH₂)₂O(CH₂)₄O, wherein one CH₂ group may be replaced by a group such as

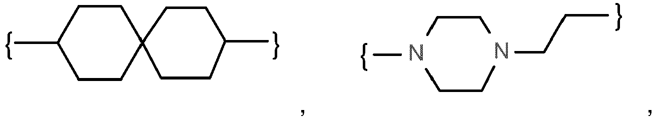

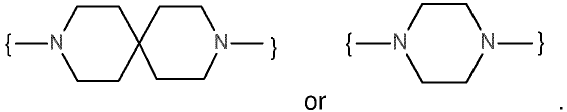

Throughout the invention, all radicals which occur more than once may be identical or different, i.e. are independent of one another.

The compounds of the formula I may have one or more chiral centres and can therefore occur in various stereoisomeric forms. The formula I encompasses all these forms.

Accordingly, the invention relates, in particular, to the compounds of the formula I in which at least one of the said radicals has one of the preferred meanings indicated above. Some preferred groups of compounds may be expressed by the following sub-formulae Ia to le, which conform to the formula I and in which the radicals not designated in greater detail have the meaning indicated for the formula I, but in which
- in Ia Het: denotes pyrazinyl, pyrazolyl, benzimidazolyl, pyridyl, thienyl, furanyl, indolyl, dihydroindolyl, benzofuranyl, tetrahydropyranyl, dihydroquinolinyl, dihydroisoquinolinyl, tetrahydroquinolinyl, tetra-hydroisoquinolinyl, indazolyl, imidazolyl, pyrrolyl, oxazolyl, oxadiazolyl, isoxazolyl, benzothiazolyl, piperidin-1-yl, pyrrolidin-1-yl, 3,4-dihydro-2H-pyrido[3,2-b]-1,4-oxazinyl, 3,4-dihydro-2H-benzo-1,4-oxazinyl, benzofuranyl, azetidinyl, 1H-pyrrolo[2,3-b]pyridinyl, 2H-chromenyl, 3-azabicylo[3.2.0]hexyl, pyrrolo[2,3-b]pyridinyl, tetrahydrofuranyl, tetrahydro-1,8-naphthyridinyl 2,3-dihydro-benzoisothiazolyl, 1,2,3,4-tetrahydrobenzothiazinyl or hexahydrobenzo-1,3-dioxolyl, each of which is unsubstituted or mono-, di- or trisubstituted by Hal, A, OA, CN, NH₂, NHA, NA₂, NO₂, CN, COOH, COOA, (CH₂)nCONH₂, (CH₂)nCONHA, (CH₂)ₙCONA₂, NHCOA, COA, CHO, Het¹, SO₂A, SO₂NH₂, SO₂NHA,SO₂NA₂, CONHNH₂, CONHAr³, =O and/or Aᵣ³;
- in Ib Het: denotes benzimidazolyl or indolyl, each of which is unsubstituted or monosubstituted by Hal;
- in Ic Q¹: denotes 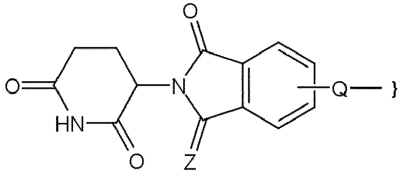
or 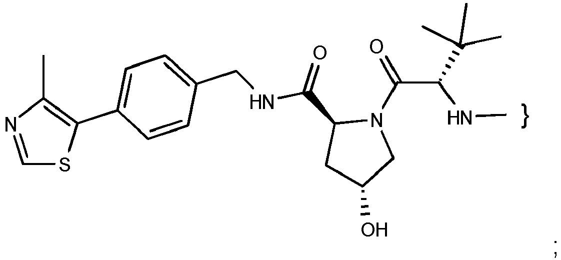
- in Id R: denotes NR²R⁴;
- in le R¹: denotes (CH₂)ₙ, [C(R⁴)₂]ₙAr¹- or (CH₂)ₙHet-;
- in If Ar²: denotes phenyl which is unsubstituted or mono-, di-, tri- or tetra-substituted by Hal;
- in Ig R²: denotes [C(R⁴)₂]ₙAr², (CH₂)ₙCyc or (CH₂)ₙHet¹;
- in Ih A: denotes unbranched or branched alkyl having 1-6 C atoms, in which 1-5 H atoms may be replaced by F, Cl and/or OH;
- in li Q¹: denotes 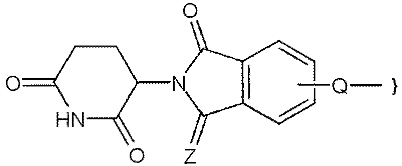
or 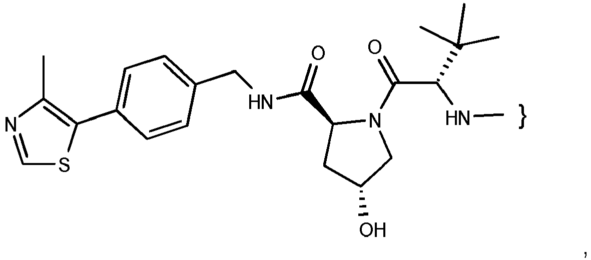
- Z: denotes O or CH₂,
- Q: denotes O, NH, NCH₃ or CH₂,
- Q²: denotes unbranched alkylene having 4-25 C atoms, in which 1-8 non-adjacent CH₂ groups may be replaced by O, CONH and/or NHCO and in which one CH₂ group may be replaced by

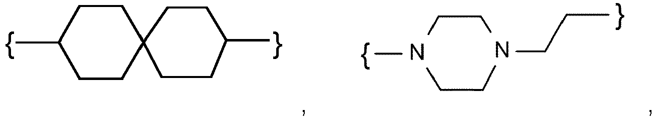

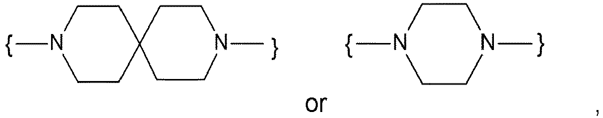

- Q³: denotes

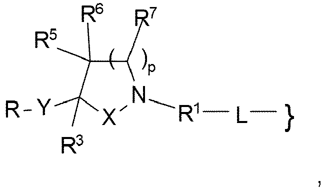

- L: denotes NR⁴CO, CONR⁴, NH, O, CO, S, SO₂, SO(=NH), NHCONH, SO₂NH or NHSO₂,
- R: denotes NR²R⁴,
- X: denotes CO or CH₂,
- Y: denotes CO or CH₂,
- R¹: denotes (CH₂)n, [C(R⁴)₂]ₙAr¹- or (CH₂)ₙHet-, wherein substituent L directly is connected to Ar¹, Het or Cyc,
- R²: denotes [C(R⁴)₂]ₙAr², (CH₂)ₙCyc or (CH₂)ₙHet¹,
- R³: denotes OH,
- R⁴: denotes H or alkyl having 1, 2, 3 or 4 C-atoms,
- R⁵, R⁶: denote H,
- R⁷: denotes H, Hal or A,
- Ar¹: denotes phenyl,
- Ar²: denotes phenyl which is unsubstituted or mono-, di-, tri- or tetra-substituted by Hal,
- Het: denotes pyrazinyl, pyrazolyl, benzimidazolyl, pyridyl, thienyl, furanyl, indolyl, dihydroindolyl, benzofuranyl, tetrahydropyranyl, dihydroquinolinyl, dihydroisoquinolinyl, tetrahydroquinolinyl, tetrahydroisoquinolinyl, indazolyl, imidazolyl, pyrrolyl, oxazolyl, oxadiazolyl, isoxazolyl, benzothiazolyl, piperidin-1-yl, pyrrolidin-1-yl, 3,4-dihydro-2H-pyrido[3,2-b]-1,4-oxazinyl, 3,4-dihydro-2H-benzo-1,4-oxazinyl, benzofuranyl, azetidinyl, 1H-pyrrolo[2,3-b]-pyridinyl, 2H-chromenyl, 3-azabicylo[3.2.0]hexyl, pyrrolo[2,3-b]pyridinyl, tetrahydrofuranyl, tetrahydro-1,8-naphthyridinyl 2,3-dihydro-benzoisothiazolyl, 1,2,3,4-tetrahydrobenzothiazinyl or hexahydrobenzo-1,3-dioxolyl, each of which is unsubstituted or mono-, di- or trisubstituted by Hal, A, OA, CN, NH₂, NHA, NA₂, NO₂, CN, COOH, COOA, (CH2)nCONH2, (CH2)nCONHA, (CH2)nCONA2, NHCOA, COA, CHO, Het¹, SO₂A, SO₂NH₂, SO₂NHA, SO₂NA₂, CONHNH₂, CONHAr³, =O and/or Ar³,
- Het¹: denotes pyridyl, furyl, thienyl, imidazolyl or pyrrolyl,
- A: denotes unbranched or branched alkyl having 1-6 C atoms, in which 1-5 H atoms may be replaced by F, Cl and/or OH,
- Cyc: denotes cyclic alkyl having 3-7 C atoms,
- Hal: denotes F, Cl, Br or I,
- n: denotes 0, 1, 2, 3 or 4,
- p: denotes 1, 2 or 3;
and pharmaceutically acceptable salts, tautomers and stereoisomers thereof, including mixtures thereof in all ratios.

The compounds of the formula I and also the starting materials for their preparation are, in addition, prepared by methods known per se, as described in the literature (for example in the standard works, such as Houben-Weyl, Methoden der organischen Chemie [Methods of Organic Chemistry], Georg-Thieme-Verlag, Stuttgart), to be precise under reaction conditions which are known and suitable for the said reactions. Use can also be made here of variants known per se which are not mentioned here in greater detail.

Compounds of the formula I can preferably be obtained by reacting compounds of the formula II with a compound of the formula III.

The compounds of the formula II and of the formula III are generally known. If they are novel, however, they can be prepared by methods known per se.

In the compounds of the formula II, L preferably denotes Cl, Br, I or a free or a reactively modified OH group, such as, for example, an activated ester, an imidazolide or alkylsulfonyloxy having 1-6 C atoms (preferably methylsulfonyloxy or trifluoromethylsulfonyloxy) or arylsulfonyloxy having 6-10 C atoms (preferably phenyl- or p-tolylsulfonyloxy).

The reaction preferably succeeds in the presence of a dehydrating agent, such as, for example, a carbodiimide, such as N,N'-dicyclohexylcarbodiimide ("DCCI"), 1,1'-carbonyldiimidazole or N-3-dimethylaminopropyl-N'-ethylcarbodiimide ("DAPECI"), furthermore propanephosphonic anhydride T3P (cf. Angew. Chem. 92, 129 (1980)), diphenylphosphoryl azide or 2-ethoxy-N-ethoxycarbonyl-1,2-dihydroquinoline, optionally in the presence of N-hydroxybenzotriaole; 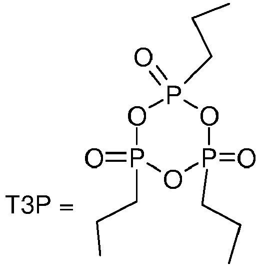 Moreover, preferably preferred is HATU [*O*-(7-Azabenzotriazol-1-yl)-*N*, *N*, *N'*, *N'*--tetramethyluronium-hexafluorphosphat]

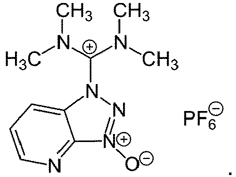

The reaction is carried out in an inert solvent and is generally carried out in the presence of an acid-binding agent, preferably an organic base, such as DIPEA, 4-methylmorpholine, triethylamine, dimethylaniline, pyridine or quinoline.

The addition of an alkali or alkaline-earth metal hydroxide, carbonate or bicarbonate or another salt of a weak acid of the alkali or alkaline-earth metals, preferably of potassium, sodium, calcium or caesium, may also be favourable.

Depending on the conditions used, the reaction time is between a few minutes and 14 days, the reaction temperature is between about -15°and 150°, normally between 0°and 120°, particularly pr eferably between 20° and 40°C.

Suitable inert solvents are, for example, hydrocarbons, such as hexane, petroleum ether, benzene, toluene or xylene; chlorinated hydrocarbons, such as trichloroethylene, 1,2-dichloroethane, carbon tetrachloride, chloroform or dichloromethane; alcohols, such as methanol, ethanol, isopropanol, n-propanol, n-butanol or tert-butanol; ethers, such as diethyl ether, diisopropyl ether, tetrahydrofuran (THF) or dioxane; glycol ethers, such as ethylene glycol monomethyl or monoethyl ether, ethylene glycol dimethyl ether (diglyme); ketones, such as acetone or butanone; amides, such as acetamide, dimethylacetamide or dimethylformamide (DMF); nitriles, such as acetonitrile; sulfoxides, such as dimethyl sulfoxide (DMSO); carbon disulfide; carboxylic acids, such as formic acid or acetic acid; nitro compounds, such as nitromethane or nitrobenzene; esters, such as ethyl acetate, or mixtures of the said solvents.

Particular preference is given to glycol ethers, such as ethylene glycol monomethyl ether, THF, dichloromethane and/or DMF.

### Pharmaceutical salts and other forms

The said compounds according to the invention can be used in their final non-salt form. On the other hand, the present invention also encompasses the use of these compounds in the form of their pharmaceutically acceptable salts, which can be derived from various organic and inorganic acids and bases by procedures known in the art. Pharmaceutically acceptable salt forms of the compounds of the formula I are for the most part prepared by conventional methods. If the compound of the formula I contains a carboxyl group, one of its suitable salts can be formed by reacting the compound with a suitable base to give the corresponding base-addition salt. Such bases are, for example, alkali metal hydroxides, including potassium hydroxide, sodium hydroxide and lithium hydroxide; alkaline earth metal hydroxides, such as barium hydroxide and calcium hydroxide; alkali metal alkoxides, for example potassium ethoxide and sodium propoxide; and various organic bases, such as piperidine, diethanolamine and N-methylglutamine. The aluminium salts of the compounds of the formula I are likewise included. In the case of certain compounds of the formula I, acid-addition salts can be formed by treating these compounds with pharmaceutically acceptable organic and inorganic acids, for example hydrogen halides, such as hydrogen chloride, hydrogen bromide or hydrogen iodide, other mineral acids and corresponding salts thereof, such as sulfate, nitrate or phosphate and the like, and alkyl- and monoarylsulfonates, such as ethanesulfonate, toluenesulfonate and benzenesulfonate, and other organic acids and corresponding salts thereof, such as acetate, trifluoroacetate, tartrate, maleate, succinate, citrate, benzoate, salicylate, ascorbate and the like. Accordingly, pharmaceutically acceptable acid-addition salts of the compounds of the formula I include the following: acetate, adipate, alginate, arginate, aspartate, benzoate, benzenesulfonate (besylate), bisulfate, bisulfite, bromide, butyrate, camphorate, camphorsulfonate, caprylate, chloride, chlorobenzoate, citrate, cyclopentanepropionate, digluconate, dihydrogenphosphate, dinitrobenzoate, dodecylsulfate, ethanesulfonate, fumarate, formate, galacterate (from mucic acid), galacturonate, glucoheptanoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, isobutyrate, lactate, lactobionate, malate, maleate, malonate, mandelate, metaphosphate, methanesulfonate, methylbenzoate, monohydrogenphosphate, 2-naphthalenesulfonate, nicotinate, nitrate, oxalate, oleate, palmoate, pectinate, persulfate, phenylacetate, 3-phenylpropionate, phosphate, phosphonate, phthalate, but this does not represent a restriction.

Furthermore, the base salts of the compounds according to the invention include aluminium, ammonium, calcium, copper, iron(III), iron(II), lithium, magnesium, manganese(III), manganese(II), potassium, sodium and zinc salts, but this is not intended to represent a restriction. Of the above-mentioned salts, preference is given to ammonium; the alkali metal salts sodium and potassium, and the alkaline earth metal salts calcium and magnesium. Salts of the compounds of the formula I which are derived from pharmaceutically acceptable organic non-toxic bases include salts of primary, secondary and tertiary amines, substituted amines, also including naturally occurring substituted amines, cyclic amines, and basic ion exchanger resins, for example arginine, betaine, caffeine, chloroprocaine, choline, N,N'-dibenzylethylenediamine (benzathine), dicyclohexylamine, diethanolamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lidocaine, lysine, meglumine, N-methyl-D-glucamine, morpholine, piperazine, piperidine, polyamine resins, procaine, purines, theobromine, triethanolamine, triethylamine, trimethylamine, tripropylamine and tris-(hydroxymethyl)methylamine (tromethamine), but this is not intended to represent a restriction.

Compounds of the present invention which contain basic nitrogen-containing groups can be quaternised using agents such as (C₁-C₄)alkyl halides, for example methyl, ethyl, isopropyl and tert-butyl chloride, bromide and iodide; di(C₁-C₄)alkyl sulfates, for example dimethyl, diethyl and diamyl sulfate; (C₁₀-C₁₈)alkyl halides, for example decyl, dodecyl, lauryl, myristyl and stearyl chloride, bromide and iodide; and aryl(C₁-C₄)alkyl halides, for example benzyl chloride and phenethyl bromide. Both water- and oil-soluble compounds according to the invention can be prepared using such salts.

The above-mentioned pharmaceutical salts which are preferred include acetate, trifluoroacetate, besylate, citrate, fumarate, gluconate, hemisuccinate, hippurate, hydrochloride, hydrobromide, isethionate, mandelate, meglumine, nitrate, oleate, phosphonate, pivalate, sodium phosphate, stearate, sulfate, sulfosalicylate, tartrate, thiomalate, tosylate and tromethamine, but this is not intended to represent a restriction.

Particular preference is given to hydrochloride, dihydrochloride, hydrobromide, maleate, mesylate, phosphate, sulfate and succinate.

The acid-addition salts of basic compounds of the formula I are prepared by bringing the free base form into contact with a sufficient amount of the desired acid, causing the formation of the salt in a conventional manner. The free base can be regenerated by bringing the salt form into contact with a base and isolating the free base in a conventional manner. The free base forms differ in a certain respect from the corresponding salt forms thereof with respect to certain physical properties, such as solubility in polar solvents; for the purposes of the invention, however, the salts otherwise correspond to the respective free base forms thereof.

As mentioned, the pharmaceutically acceptable base-addition salts of the compounds of the formula I are formed with metals or amines, such as alkali metals and alkaline earth metals or organic amines. Preferred metals are sodium, potassium, magnesium and calcium. Preferred organic amines are N,N'-dibenzylethylenediamine, chloroprocaine, choline, diethanolamine, ethylenediamine, N-methyl-D-glucamine and procaine.

The base-addition salts of acidic compounds according to the invention are prepared by bringing the free acid form into contact with a sufficient amount of the desired base, causing the formation of the salt in a conventional manner. The free acid can be regenerated by bringing the salt form into contact with an acid and isolating the free acid in a conventional manner. The free acid forms differ in a certain respect from the corresponding salt forms thereof with respect to certain physical properties, such as solubility in polar solvents; for the purposes of the invention, however, the salts otherwise correspond to the respective free acid forms thereof.

If a compound according to the invention contains more than one group which is capable of forming pharmaceutically acceptable salts of this type, the invention also encompasses multiple salts. Typical multiple salt forms include, for example, bitartrate, diacetate, difumarate, dimeglumine, diphosphate, disodium and trihydrochloride, but this is not intended to represent a restriction.

With regard to that stated above, it can be seen that the expression "pharmaceutically acceptable salt" in the present connection is taken to mean an active ingredient which comprises a compound of the formula I in the form of one of its salts, in particular if this salt form imparts improved pharmacokinetic properties on the active ingredient compared with the free form of the active ingredient or any other salt form of the active ingredient used earlier. The pharmaceutically acceptable salt form of the active ingredient can also provide this active ingredient for the first time with a desired pharmacokinetic property which it did not have earlier and can even have a positive influence on the pharmacodynamics of this active ingredient with respect to its therapeutic efficacy in the body.

### Isotopes

There is furthermore intended that a compound of the formula I includes isotope-labelled forms thereof. An isotope-labelled form of a compound of the formula I is identical to this compound apart from the fact that one or more atoms of the compound have been replaced by an atom or atoms having an atomic mass or mass number which differs from the atomic mass or mass number of the atom which usually occurs naturally. Examples of isotopes which are readily commercially available and which can be incorporated into a compound of the formula I by well-known methods include isotopes of hydrogen, carbon, nitrogen, oxygen, phosphorus, fluorine and chlorine, for example ²H, ³H, ¹³C, ¹⁴C, ¹⁵N, ¹⁸O, ¹⁷O, ³¹P, ³²P, ³⁵S, ¹⁸F and ³⁶Cl, respectively. A compound of the formula I, a prodrug, thereof or a pharmaceutically acceptable salt of either which contains one or more of the above-mentioned isotopes and/or other iso-topes of other atoms is intended to be part of the present invention. An isotope-labelled compound of the formula I can be used in a number of beneficial ways. For example, an isotope-labelled compound of the formula I into which, for example, a radioisotope, such as ³H or ¹⁴C, has been incorporated is suitable for medicament and/or substrate tissue distribution assays. These radioisotopes, i.e. tritium (³H) and carbon-14 (¹⁴C), are particularly preferred owing to simple preparation and excellent detectability. Incorporation of heavier isotopes, for example deuterium (²H), into a compound of the formula I has therapeutic advantages owing to the higher metabolic stability of this isotope-labelled compound. Higher metabolic stability translates directly into an increased in vivo half-life or lower dosages, which under most circumstances would represent a preferred embodiment of the present invention. An isotope-labelled compound of the formula I can usually be prepared by carrying out the procedures disclosed in the synthesis schemes and the related description, in the example part and in the preparation part in the present text, replacing a non-isotope-labelled reactant by a readily available isotope-labelled reactant.

Deuterium (²H) can also be incorporated into a compound of the formula I for the purpose in order to manipulate the oxidative metabolism of the compound by way of the primary kinetic isotope effect. The primary kinetic isotope effect is a change of the rate for a chemical reaction that results from exchange of isotopic nuclei, which in turn is caused by the change in ground state energies necessary for covalent bond formation after this isotopic exchange. Exchange of a heavier isotope usually results in a lowering of the ground state energy for a chemical bond and thus cause a reduction in the rate in rate-limiting bond breakage. If the bond breakage occurs in or in the vicinity of a saddle-point region along the coordinate of a multi-product reaction, the product distribution ratios can be altered substantially. For explanation: if deuterium is bonded to a carbon atom at a non-exchangeable position, rate differences of k_{M}/k_{D} = 2-7 are typical. If this rate difference is successfully applied to a compound of the formula I that is susceptible to oxidation, the profile of this compound in vivo can be drastically modified and result in improved pharmacokinetic properties.

When discovering and developing therapeutic agents, the person skilled in the art attempts to optimise pharmacokinetic parameters while retaining desirable in vitro properties. It is reasonable to assume that many compounds with poor pharmacokinetic profiles are susceptible to oxidative metabolism. In vitro liver microsomal assays currently available provide valuable information on the course of oxidative metabolism of this type, which in turn permits the rational design of deuterated compounds of the formula I with improved stability through resistance to such oxidative meta-bolism. Significant improvements in the pharmacokinetic profiles of compounds of the formula I are thereby obtained, and can be expressed quantitatively in terms of increases in the in vivo half-life (t1/2), concen-tra-tion at maximum therapeutic effect (Cₘₐₓ), area under the dose response curve (AUC), and F; and in terms of reduced clearance, dose and materi-als costs.

The following is intended to illustrate the above: a compound of the formula I which has multiple potential sites of attack for oxidative metabolism, for example benzylic hydrogen atoms and hydrogen atoms bonded to a nitrogen atom, is prepared as a series of analogues in which various combinations of hydrogen atoms are replaced by deuterium atoms, so that some, most or all of these hydrogen atoms have been replaced by deuterium atoms. Half-life determinations enable favourable and accurate determination of the extent of the extent to which the improve-ment in resistance to oxidative metabolism has improved. In this way, it is deter-mined that the half-life of the parent compound can be extended by up to 100% as the result of deuterium-hydrogen exchange of this type.

Deuterium-hydrogen exchange in a compound of the formula I can also be used to achieve a favourable modification of the metabolite spectrum of the starting compound in order to diminish or eliminate undesired toxic metabolites. For example, if a toxic metabolite arises through oxidative carbon-hydrogen (C-H) bond cleavage, it can reasonably be assumed that the deuterated analogue will greatly diminish or eliminate production of the unwanted metabolite, even if the particular oxidation is not a ratedetermining step. Further information on the state of the art with respect to deuterium-hydrogen exchange may be found, for example in Hanzlik et al., J. Org. Chem. 55, 3992-3997, 1990, Reider et al., J. Org. Chem. 52, 3326-3334, 1987, Foster, Adv. Drug Res. 14, 1-40, 1985, Gillette et al, Biochemistry 33(10) 2927-2937, 1994, and Jarman et al. Carcinogenesis 16(4), 683-688, 1993.

The invention furthermore relates to medicaments comprising at least one compound of the formula I and/or pharmaceutically acceptable derivatives, solvates and stereoisomers thereof, including mixtures thereof in all ratios, and optionally excipients and/or adjuvants.

Pharmaceutical formulations can be administered in the form of dosage units which comprise a predetermined amount of active ingredient per dosage unit. Such a unit can comprise, for example, 0.5 mg to 1 g, preferably 1 mg to 700 mg, particularly preferably 5 mg to 100 mg, of a compound according to the invention, depending on the condition treated, the method of administration and the age, weight and condition of the patient, or pharmaceutical formulations can be administered in the form of dosage units which comprise a predetermined amount of active ingredient per dosage unit. Preferred dosage unit formulations are those which comprise a daily dose or part-dose, as indicated above, or a corresponding fraction thereof of an active ingredient. Furthermore, pharmaceutical formulations of this type can be prepared using a process which is generally known in the pharmaceutical art.

Pharmaceutical formulations can be adapted for administration via any desired suitable method, for example by oral (including buccal or sublingual), rectal, nasal, topical (including buccal, sublingual or transdermal), vaginal or parenteral (including subcutaneous, intramuscular, intravenous or intradermal) methods. Such formulations can be prepared using all processes known in the pharmaceutical art by, for example, combining the active ingredient with the excipient(s) or adjuvant(s).

Pharmaceutical formulations adapted for oral administration can be administered as separate units, such as, for example, capsules or tablets; powders or granules; solutions or suspensions in aqueous or non-aqueous liquids; edible foams or foam foods; or oil-in-water liquid emulsions or water-in-oil liquid emulsions.

Thus, for example, in the case of oral administration in the form of a tablet or capsule, the active-ingredient component can be combined with an oral, non-toxic and pharmaceutically acceptable inert excipient, such as, for example, ethanol, glycerol, water and the like. Powders are prepared by comminuting the compound to a suitable fine size and mixing it with a pharmaceutical excipient comminuted in a similar manner, such as, for example, an edible carbohydrate, such as, for example, starch or mannitol. A flavour, preservative, dispersant and dye may likewise be present.

Capsules are produced by preparing a powder mixture as described above and filling shaped gelatine shells therewith. Glidants and lubricants, such as, for example, highly disperse silicic acid, talc, magnesium stearate, calcium stearate or polyethylene glycol in solid form, can be added to the powder mixture before the filling operation. A disintegrant or solubiliser, such as, for example, agar-agar, calcium carbonate or sodium carbonate, may likewise be added in order to improve the availability of the medicament after the capsule has been taken.

In addition, if desired or necessary, suitable binders, lubricants and disintegrants as well as dyes can likewise be incorporated into the mixture. Suitable binders include starch, gelatine, natural sugars, such as, for example, glucose or beta-lactose, sweeteners made from maize, natural and synthetic rubber, such as, for example, acacia, tragacanth or sodium alginate, carboxymethylcellulose, polyethylene glycol, waxes, and the like. The lubricants used in these dosage forms include sodium oleate, sodium stearate, magnesium stearate, sodium benzoate, sodium acetate, sodium chloride and the like. The disintegrants include, without being restricted thereto, starch, methylcellulose, agar, bentonite, xanthan gum and the like. The tablets are formulated by, for example, preparing a powder mixture, granulating or dry-pressing the mixture, adding a lubricant and a disintegrant and pressing the entire mixture to give tablets. A powder mixture is prepared by mixing the compound comminuted in a suitable manner with a diluent or a base, as described above, and optionally with a binder, such as, for example, carboxymethylcellulose, an alginate, gelatine or polyvinylpyrrolidone, a dissolution retardant, such as, for example, paraffin, an absorption accelerator, such as, for example, a quaternary salt, and/or an absorbant, such as, for example, bentonite, kaolin or dicalcium phosphate. The powder mixture can be granulated by wetting it with a binder, such as, for example, syrup, starch paste, acadia mucilage or solutions of cellulose or polymer materials and pressing it through a sieve. As an alternative to granulation, the powder mixture can be run through a tabletting machine, giving lumps of non-uniform shape, which are broken up to form granules. The granules can be lubricated by addition of stearic acid, a stearate salt, talc or mineral oil in order to prevent sticking to the tablet casting moulds. The lubricated mixture is then pressed to give tablets. The compounds according to the invention can also be combined with a free-flowing inert excipient and then pressed directly to give tablets without carrying out the granulation or dry-pressing steps. A transparent or opaque protective layer consisting of a shellac sealing layer, a layer of sugar or polymer material and a gloss layer of wax may be present. Dyes can be added to these coatings in order to be able to differentiate between different dosage units.

Oral liquids, such as, for example, solution, syrups and elixirs, can be prepared in the form of dosage units so that a given quantity comprises a prespecified amount of the compound. Syrups can be prepared by dissolving the compound in an aqueous solution with a suitable flavour, while elixirs are prepared using a non-toxic alcoholic vehicle. Suspensions can be formulated by dispersion of the compound in a non-toxic vehicle. Solubilisers and emulsifiers, such as, for example, ethoxylated isostearyl alcohols and polyoxyethylene sorbitol ethers, preservatives, flavour additives, such as, for example, peppermint oil or natural sweeteners or saccharin, or other artificial sweeteners and the like, can likewise be added.

The dosage unit formulations for oral administration can, if desired, be encapsulated in microcapsules. The formulation can also be prepared in such a way that the release is extended or retarded, such as, for example, by coating or embedding of particulate material in polymers, wax and the like.

The compounds of the formula I and pharmaceutically salts, tautomers and stereoisomers thereof can also be administered in the form of liposome delivery systems, such as, for example, small unilamellar vesicles, large unilamellar vesicles and multilamellar vesicles. Liposomes can be formed from various phospholipids, such as, for example, cholesterol, stearylamine or phosphatidylcholines.

The compounds of the formula I and the salts, tautomers and stereoisomers thereof can also be delivered using monoclonal antibodies as individual carriers to which the compound molecules are coupled. The compounds can also be coupled to soluble polymers as targeted medicament carriers. Such polymers may encompass polyvinylpyrrolidone, pyran copolymer, polyhydroxypropylmethacrylamidophenol, polyhydroxy-ethylaspartamidophenol or polyethylene oxide polylysine, substituted by palmitoyl radicals. The compounds may furthermore be coupled to a class of biodegradable polymers which are suitable for achieving controlled release of a medicament, for example polylactic acid, poly-epsilon-caprolactone, polyhydroxybutyric acid, polyorthoesters, polyacetals, polydihy-droxypyrans, polycyanoacrylates and crosslinked or amphipathic block copolymers of hydrogels.

Pharmaceutical formulations adapted for transdermal administration can be administered as independent plasters for extended, close contact with the epidermis of the recipient. Thus, for example, the active ingredient can be delivered from the plaster by iontophoresis.

Pharmaceutical compounds adapted for topical administration can be formulated as ointments, creams, suspensions, lotions, powders, solutions, pastes, gels, sprays, aerosols or oils.

For the treatment of the eye or other external tissue, for example mouth and skin, the formulations are preferably applied as topical ointment or cream. In the case of formulation to give an ointment, the active ingredient can be employed either with a paraffinic or a water-miscible cream base. Alternatively, the active ingredient can be formulated to give a cream with an oil-in-water cream base or a water-in-oil base.

Pharmaceutical formulations adapted for topical application to the eye include eye drops, in which the active ingredient is dissolved or suspended in a suitable carrier, in particular an aqueous solvent.

Pharmaceutical formulations adapted for topical application in the mouth encompass lozenges, pastilles and mouthwashes.

Pharmaceutical formulations adapted for rectal administration can be administered in the form of suppositories or enemas.

Pharmaceutical formulations adapted for nasal administration in which the carrier substance is a solid comprise a coarse powder having a particle size, for example, in the range 20-500 microns, which is administered in the manner in which snuff is taken, i.e. by rapid inhalation via the nasal passages from a container containing the powder held close to the nose. Suitable formulations for administration as nasal spray or nose drops with a liquid as carrier substance encompass active-ingredient solutions in water or oil.

Pharmaceutical formulations adapted for administration by inhalation encompass finely particulate dusts or mists, which can be generated by various types of pressurised dispensers with aerosols, nebulisers or insufflators.

Pharmaceutical formulations adapted for vaginal administration can be administered as pessaries, tampons, creams, gels, pastes, foams or spray formulations.

Pharmaceutical formulations adapted for parenteral administration include aqueous and non-aqueous sterile injection solutions comprising antioxidants, buffers, bacteriostatics and solutes, by means of which the formulation is rendered isotonic with the blood of the recipient to be treated; and aqueous and non-aqueous sterile suspensions, which may comprise suspension media and thickeners. The formulations can be administered in single-dose or multidose containers, for example sealed ampoules and vials, and stored in freeze-dried (lyophilised) state, so that only the addition of the sterile carrier liquid, for example water for injection purposes, immediately before use is necessary. Injection solutions and suspensions prepared in accordance with the recipe can be prepared from sterile powders, granules and tablets.

It goes without saying that, in addition to the above particularly mentioned constituents, the formulations may also comprise other agents usual in the art with respect to the particular type of formulation; thus, for example, formulations which are suitable for oral administration may comprise flavours.

A therapeutically effective amount of a compound of the formula I depends on a number of factors, including, for example, the age and weight of the animal, the precise condition that requires treatment, and its severity, the nature of the formulation and the method of administration, and is ultimately determined by the treating doctor or vet. However, an effective amount of a compound according to the invention is generally in the range from 0.1 to 100 mg/kg of body weight of the recipient (mammal) per day and particularly typically in the range from 1 to 10 mg/kg of body weight per day. Thus, the actual amount per day for an adult mammal weighing 70 kg is usually between 70 and 700 mg, where this amount can be administered as a single dose per day or usually in a series of part-doses (such as, for example, two, three, four, five or six) per day, so that the total daily dose is the same. An effective amount of a salt or solvate or of a physiologically functional derivative thereof can be determined as the fraction of the effective amount of the compound according to the invention *per se.* It can be assumed that similar doses are suitable for the treatment of other conditions mentioned above.

A combined treatment of this type can be achieved with the aid of simultaneous, consecutive or separate dispensing of the individual components of the treatment. Combination products of this type employ the compounds according to the invention.

The invention furthermore relates to medicaments comprising at least one compound of the formula I and/or pharmaceutically acceptable salts, tautomers and stereoisomers thereof, including mixtures thereof in all ratios, and at least one further medicament active ingredient.

The invention also relates to a set (kit) consisting of separate packs of
(a) an effective amount of a compound of the formula I and/or pharmaceutically acceptable salts, tautomers and stereoisomers thereof, including mixtures thereof in all ratios,
   and
(b) an effective amount of a further medicament active ingredient.

The set comprises suitable containers, such as boxes, individual bottles, bags or ampoules. The set may, for example, comprise separate ampoules, each containing an effective amount of a compound of the formula I and/or pharmaceutically acceptable salts, tautomers and stereoisomers thereof, including mixtures thereof in all ratios,
and an effective amount of a further medicament active ingredient in dissolved or lyophilised form.

"Treating" as used herein, means an alleviation, in whole or in part, of symptoms associated with a disorder or disease, or slowing, or halting of further progression or worsening of those symptoms, or prevention or prophylaxis of the disease or disorder in a subject at risk for developing the disease or disorder.

The term "effective amount" in connection with a compound of formula (I) can mean an amount capable of alleviating, in whole or in part, symptoms associated with a disorder or disease, or slowing or halting further progression or worsening of those symptoms, or preventing or providing prophylaxis for the disease or disorder in a subject having or at risk for developing a disease disclosed herein, such as inflammatory conditions, immunological conditions, cancer or metabolic conditions.

### USE

The present invention specifically relates to compounds of the formula I and pharmaceutically acceptable salts, tautomers and stereoisomers thereof, including mixtures thereof in all ratios,
for the use for the treatment of diseases in which the degradation and/or modulation of MetAP-2 plays a role.

The present invention specifically relates to compounds of the formula I and pharmaceutically acceptable salts, tautomers and stereoisomers thereof, including mixtures thereof in all ratios, for the use for the degradation and/or modulation of MetAP-2.

The present invention specifically relates to compounds of the formula I and pharmaceutically acceptable salts, tautomers and stereoisomers thereof, including mixtures thereof in all ratios, for use for the treatment and control of diseases.

These diseases include the proliferation of tumour cells, pathological neovascularisation (or angiogenesis), which promotes the growth of solid tumours, neovascularisation in the eye (diabetic retinopathy, age-induced macular degeneration and the like) and inflammation (psoriasis, rheumatoid arthritis and the like), and proliferative diseases of the mesangial cells.

The invention relates to compounds for use of the formula I according to claim 1 and pharmaceutically acceptable salts, solvates, tautomers and stereoisomers thereof, including mixtures thereof in all ratios, for the treatment and/or prevention of tumours, tumour metastases, proliferative diseases of the mesangial cells, haemangioma, proliferative retinopathy, rheumatoid arthritis, atherosclerotic neovascularisation, psoriasis, ocular neovascularisation, osteoporosis, diabetes and obesity, lymphoid leukaemia, lymphoma, malaria and prostate hypertrophy.

The invention relates to compounds for use where the tumour disease is selected from the group
of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx, of the lung, of the skin, monocytic leukaemia, lung adenocarcinoma, small-cell lung carcinoma, pancreatic cancer, glioblastoma, breast carcinoma, acute myeloid leukaemia, chronic myeloid leukaemia, acute lymphatic leukaemia, chronic lymphatic leukaemia, Hodgkin's lymphoma, non-Hodgkin's lymphoma.

The present invention encompasses the use of the compounds of the formula I and/or physiologically acceptable salts and solvates thereof for the preparation of a medicament for the treatment or prevention of tumours, tumour diseases and/or tumour metastases.

The tumour disease is preferably selected from the group
tumour of the squamous epithelium, the bladder, the stomach, the kidneys, of head and neck, the oesophagus, the cervix, the thyroid, the intestine, the liver, the brain, the prostate, the urogenital tract, the lymphatic system, the stomach, the larynx, the lung, the skin, monocytic leukaemia, lung adenocarcinoma, small-cell lung carcinoma, pancreatic cancer, glioblastoma, breast carcinoma, acute myeloid leukaemia, chronic myeloid leukaemia, acute lymphatic leukaemia, chronic lymphatic leukaemia, Hodgkin's lymphoma, non-Hodgkin's lymphoma.

Likewise encompassed is the use of the compounds according to Claim 1 according to the invention and/or physiologically acceptable salts and solvates thereof for the preparation of a medicament for the treatment of osteoporosis, diabetes and obesity.

Likewise encompassed is the use of the compounds according to Claim 1 according to the invention and/or physiologically acceptable salts and solvates thereof for the preparation of a medicament for the treatment or prevention of a disease in which angiogenesis is involved.

A disease of this type in which angiogenesis is involved is an eye disease, such as retina vascularisation, diabetic retinopathy, age-induced macular degeneration and the like.

The angiogenic disease is preferably selected from the group diabetic retinopathy, arthritis, cancer, psoriasis, Kaposi's sarcoma, haemangioma, myocardial angiogenesis, atherosclerotic plaque neovascularisation, angiogenic eye diseases, choroidal neovascularisation, retrolental fibroplasia, macular degeneration, corneal transplant rejection, rubeosis iridis, neuroscular glaucoma, Oster Webber syndrome.

The proliferative disease of the mesangial cells is preferably selected from the group
glomerulonephritis, diabetic nephropathy, malignant nephrosclerosis, thrombotic microangiopathy syndrome, transplant rejection, glomerulopathy.

The use of compounds of the formula I and/or physiologically acceptable salts and solvates thereof for the preparation of a medicament for the treatment or prevention of inflammatory diseases likewise falls within the scope of the present invention. Examples of such inflammatory diseases include rheumatoid arthritis, psoriasis, contact dermatitis, delayed hypersensitivity reaction and the like.

The inflammatory disease is preferably selected from the group inflammatory bowel disease, arthritis, atherosclersosis, asthma, allergies, inflammatory kidney diseases, multiple sclerosis, chronic obstructive pulmonary disease, inflammatory skin diseases, pardontal diseases, psoriasis, T-cell-promoted immune disease.

The inflammatory bowel disease is preferably selected from the group ulcerative colitis, Crohn's disease, non-specific colitis.

The T-cell-promoted immune disease is preferably selected from the group allergic encephalomyelitis, allergic neuritis, transplant rejection, graft-versus-host reaction, myocarditis, thyroiditis, nephritis, systemic lupus erythematosus, insulin-dependent diabetes mellitus.

The arthritis disease is preferably selected from the group
rheumatoid arthritis, osteoarthritis, Caplan's syndrome, Felty's syndrome, Sjogren's syndrome, spondylitis ankylosans, Still's disease, chondrocalcinosis, metabolic arthritis, rheumatic fever, Reiter's disease, Wissler's syndrome.

The inflammatory kidney disease is preferably selected from the group glomerulonephritis, glomerular injury, nephrotic syndrome, interstitial nephritis, lupus nephritis, Goodpasture's syndrome, Wegener's granulomatosis, renal vasculitis, IgA nephropathy, idiopatic glomerular disease.

The inflammatory skin disease is preferably selected from the group psoriasis, atopic dermatitis, contact sensitivity, acne.

Likewise encompassed is the use of the compounds of the formula I and/or physiologically acceptable salts and solvates thereof for the preparation of a medicament for the treatment or prevention of a disease or condition in a mammal, in which to this method a therapeutically effective amount of a compound according to the invention is administered to a sick mammal in need of such treatment. The therapeutic amount varies according to the specific disease and can be determined by the person skilled in the art without undue effort.

The present invention also encompasses the use compounds of the formula I and/or physiologically acceptable salts and solvates thereof for the preparation of a medicament for the treatment or prevention of retinal vascularisation.

Likewise encompassed is the use of the compounds of the formula I and/or physiologically acceptable salts thereof for the preparation of a medicament for the treatment and/or combating of a tumour-induced disease in a mammal, in which to this method a therapeutically effective amount of a compound according to the invention is administered to a sick mammal in need of such treatment. The therapeutic amount varies according to the specific disease and can be determined by the person skilled in the art without undue effort.

The disclosed compounds of the formula I can be administered in combination with other known therapeutic agents, including anticancer agents. As used here, the term "anticancer agent" relates to any agent which is administered to a patient with cancer for the purposes of treating the cancer.

The anti-cancer treatment defined above may be applied as a monotherapy or may involve, in addition to the herein disclosed compounds of formula I, conventional surgery or radiotherapy or medicinal therapy. Such medicinal therapy, e.g. a chemotherapy or a targeted therapy, may include one or more, but preferably one, of the following anti-tumor agents:
Alkylating agents
   such as altretamine, bendamustine, busulfan, carmustine, chlorambucil, chlormethine, cyclophosphamide, dacarbazine, ifosfamide, improsulfan, tosilate, lomustine, melphalan, mitobronitol, mitolactol, nimustine, ranimustine, temozolomide, thiotepa, treosulfan, mechloretamine, carboquone; apaziquone, fotemustine, glufosfamide, palifosfamide, pipobroman, trofosfamide, uramustine, TH-302⁴, VAL-083⁴;
Platinum Compounds
   such as carboplatin, cisplatin, eptaplatin, miriplatine hydrate, oxaliplatin, lobaplatin, nedaplatin, picoplatin, satraplatin;
   lobaplatin, nedaplatin, picoplatin, satraplatin;
DNA altering agents
   such as amrubicin, bisantrene, decitabine, mitoxantrone, procarbazine, trabectedin, clofarabine;
   amsacrine, brostallicin, pixantrone, laromustine^{1,3};
Topoisomerase Inhibitors
   such as etoposide, irinotecan, razoxane, sobuzoxane, teniposide, topotecan; amonafide, belotecan, elliptinium acetate, voreloxin;
Microtubule modifiers
   such as cabazitaxel, docetaxel, eribulin, ixabepilone, paclitaxel, vinblastine, vincristine, vinorelbine, vindesine, vinflunine;
   fosbretabulin, tesetaxel;
Antimetabolites
   such as asparaginase³, azacitidine, calcium levofolinate, capecitabine, cladribine, cytarabine, enocitabine, floxuridine, fludarabine, fluorouracil, gemcitabine, mercaptopurine, methotrexate, nelarabine, pemetrexed, pralatrexate, azathioprine, thioguanine, carmofur;
   doxifluridine, elacytarabine, raltitrexed, sapacitabine, tegafur^{2,3}, trimetrexate;
Anticancer antibiotics
   such as bleomycin, dactinomycin, doxorubicin, epirubicin, idarubicin, levamisole, miltefosine, mitomycin C, romidepsin, streptozocin, valrubicin, zinostatin, zorubicin, daunurobicin, plicamycin;
   aclarubicin, peplomycin, pirarubicin;
Hormones/Antagonists
   such as abarelix, abiraterone, bicalutamide, buserelin, calusterone, chlorotrianisene, degarelix, dexamethasone, estradiol, fluocortolone fluoxymesterone, flutamide, fulvestrant, goserelin, histrelin, leuprorelin, megestrol, mitotane, nafarelin, nandrolone, nilutamide, octreotide, prednisolone, raloxifene, tamoxifen, thyrotropin alfa, toremifene, trilostane, triptorelin, diethylstilbestrol;
   acolbifene, danazol, deslorelin, epitiostanol, orteronel, enzalutamide¹³;
Aromatase inhibitors
   such as aminoglutethimide, anastrozole, exemestane, fadrozole, letrozole, testolactone;
   formestane;
Small molecule kinase inhibitors
   such as crizotinib, dasatinib, erlotinib, imatinib, lapatinib, nilotinib, pazopanib, regorafenib, ruxolitinib, sorafenib, sunitinib, vandetanib, vemurafenib, bosutinib, gefitinib, axitinib;
   afatinib, alisertib, dabrafenib, dacomitinib, dinaciclib, dovitinib, enzastaurin, nintedanib, lenvatinib, linifanib, linsitinib, masitinib, midostaurin, motesanib, neratinib, orantinib, perifosine, ponatinib, radotinib, rigosertib, tipifarnib, tivantinib, tivozanib, trametinib, pimasertib, brivanib alaninate, cediranib, apatinib⁴, cabozantinib S-malate^{1,3}, ibrutinib^{1,3}, icotinib⁴, buparlisib², cipatinib⁴, cobimetinib^{1,3}, idelalisib^{1,3}, fedratinib¹, XL-647⁴;
Photosensitizers
   such as methoxsalen³;
   porfimer sodium, talaporfin, temoporfin;
Antibodies
   such as avelumab, alemtuzumab, besilesomab, brentuximab vedotin, cetuximab, denosumab, ipilimumab, ofatumumab, panitumumab, rituximab, tositumomab, trastuzumab, bevacizumab, pertuzumab^{2,3};
   catumaxomab, elotuzumab, epratuzumab, farletuzumab, mogamulizumab, necitumumab, nimotuzumab, obinutuzumab, ocaratuzumab, oregovomab, ramucirumab, rilotumumab, siltuximab, tocilizumab, zalutumumab, zanolimumab, matuzumab, dalotuzumab^{1,2,3}, onartuzumab^{1,3}, racotumomab¹, tabalumab^{1,3} , EMD-525797⁴, nivolumab^{1,3};
Cytokines
   such as aldesleukin, interferon alfa², interferon alfa2a³, interferon alfa2b²³; celmoleukin, tasonermin, teceleukin, oprelvekin^{1,3}, recombinant interferon beta-1a⁴;
Drug Conjugates
   such as denileukin diftitox, ibritumomab tiuxetan, iobenguane 1123, prednimustine, trastuzumab emtansine, estramustine, gemtuzumab, ozogamicin, aflibercept;
   cintredekin besudotox, edotreotide, inotuzumab ozogamicin, naptumomab estafenatox, oportuzumab monatox, technetium (99mTc) arcitumomab^{1,3}, vintafolide^{1,3};
Vaccines
   such as sipuleucel3; vitespen³, emepepimut-S³, oncoVAX⁴, rindopepimut³, troVax⁴, MGN-1601⁴, MGN-1703⁴;
Miscellaneous
   alitretinoin, bexarotene, bortezomib, everolimus, ibandronic acid, imiquimod, lenalidomide, lentinan, metirosine, mifamurtide, pamidronic acid, pegaspargase, pentostatin, sipuleucel3, sizofiran, tamibarotene, temsirolimus, thalidomide, tretinoin, vismodegib, zoledronic acid, vorinostat;
   celecoxib, cilengitide, entinostat, etanidazole, ganetespib, idronoxil, iniparib, ixazomib, lonidamine, nimorazole, panobinostat, peretinoin, plitidepsin, pomalidomide, procodazol, ridaforolimus, tasquinimod, telotristat, thymalfasin, tirapazamine, tosedostat, trabedersen, ubenimex, valspodar, gendicine⁴, picibanil⁴, reolysin⁴, retaspimycin hydrochloride¹³, trebananib^{2,3}, virulizin⁴, carfilzomib^{1,3}, endostatin⁴, immucothel⁴, belinostat³, MGN-1703⁴;
   ¹**Prop.** INN (Proposed International Nonproprietary Name)
   ² **Rec.** INN (Recommended International Nonproprietary Names)
   ³ **USAN** (United States Adopted Name)
   ⁴ **no INN.**

The following abbreviations refer respectively to the definitions below:
aq (aqueous), h (hour), g (gram), L (liter), mg (milligram), MHz (Megahertz), min. (minute), mm (millimeter), mmol (millimole), mM (millimolar), m.p. (melting point), eq (equivalent), mL (milliliter), L (microliter), ACN (acetonitrile), AcOH (acetic acid), CDCl₃ (deuterated chloroform), CD₃OD (deuterated methanol), CH₃CN (acetonitrile), c-hex (cyclohexane), DCC (dicyclohexyl carbodiimide), DCM (dichloromethane), DIC (diisopropyl carbodiimide), DIEA (diisopropylethyl-amine), DMF (dimethylformamide), DMSO (dimethylsulfoxide), DMSO-d₆ (deuterated dimethylsulfoxide), EDC (1-(3-dimethyl-amino-propyl)-3-ethylcarbodiimide), ESI (Electro-spray ionization), EtOAc (ethyl acetate), Et₂O (diethyl ether), EtOH (ethanol), HATU (dimethylamino-([1,2,3]triazolo[4,5-b]pyridin-3-yloxy)-methylene]-dimethylammonium hexafluorophosphate), HPLC (High Performance Liquid Chromatography), i-PrOH (2-propanol), K₂CO₃ (potassium carbonate), LC (Liquid Chromatography), MeOH (methanol), MgSO₄ (magnesium sulfate), MS (mass spectrometry), MTBE (Methyl tert-butyl ether), NaHCO₃ (sodium bicarbonate), NaBH₄ (sodium borohydride), NMM (N-methyl morpholine), NMR (Nuclear Magnetic Resonance), PyBOP (benzotriazole-1-yl-oxy-tris-pyrrolidino-phosphonium hexafluorophosphate), RT (room temperature), Rt (retention time), SPE (solid phase extraction), TBTU (2-(1-H-benzotriazole-1-yl)-1,1,3,3-tetramethyluromium tetrafluoro borate), TEA (triethylamine), TFA (trifluoroacetic acid), THF (tetrahydrofuran), TLC (Thin Layer Chromatography), UV (Ultraviolet).

Above and below, all temperatures are indicated in°C. In the following examples, "conventional work-up" means: water is added if necessary, the pH is adjusted, if necessary, to values between 2 and 10, depending on the constitution of the end product, the mixture is extracted with ethyl acetate or dichloromethane, the phases are separated, the organic phase is dried over sodium sulfate and evaporated, and the residue is purified by chromatography on silica gel and/or by crystallisation. Rf values on silica gel; eluent: ethyl acetate/methanol 9:1.

¹H NMR was recorded on Bruker DPX-300, DRX-400, AVII-400 or on a 500 MHz spectrometer, using residual signal of deuterated solvent as internal reference. Chemical shifts (δ) are reported in ppm relative to the residual solvent signal (δ = 2.49 ppm for ¹H NMR in DMSO-d₆). ¹H NMR data are reported as follows: chemical shift (multiplicity, coupling constants, and number of hydrogens). Multiplicity is abbreviated as follows: s (singlet), d (doublet), t (triplet), q (quartet), m (multiplet), br (broad).

### Biochemical activity testing of MetAP-2

MetAP-2 activity was determined by an enzyme-coupled assay using the tripeptide Met-Ala-Ser (MAS) as substrate and recombinant human MetAP-2 (His-Tev- MetAP-2, prepared at Merck). The released methionine is converted by L-amino acid oxidase (AAO) to oxidized Methionine and hydrogen peroxide is released. In a second step horse radish peroxidase catalyzes the oxidation of the leuko dye dianisidine to oxidized dianisidine using hydrogen peroxide as co-substrate. The produced dianisidine ox was detected photometrically as increase in absorbance at 450 nm. Met-AP2 activity was determined in a kinetic measurement mode. The release of one molecule methionine corresponds to the production of one molecule dianisidine ox. The MetAP2 enzymatic activity is directly corresponding to the increase in absorbance per time.

In detail, the assay was performed in 384 well microtiter plate (Greiner 78110 MTP, transparent) in a total reaction volume of 50 µl at 22°C. 0.35 µg of N-terminal His tag human rec MetAP2 (prepared in house, AA 2-478, final concentration (fc) 123 nM), 1 unit horse radish Peroxidase (Roche, Mannheim), 0.02 unit L-amino acid oxidase (Merck, Darmstadt), 0.6 mM dianisidine (Merck, Darmstadt, dissolved in 50 mM HCI, 10% DMSO) were incubated in the absence or presence of the test compound (10 dilution concentrations) in 100 mM Hepes, 50 mM NaCl, 50 µM MnCl₂ at pH 7.0 for 15 min at 22 °C. The reaction was started by the addit ion of 500µM (fc) MAS peptide (Merck, Darmstadt). After mixing the first absorbance measurement was performed on an Envision multimode reader (Perkin-Elmer, Waltham) at wavelength of 450 nm. The reaction was incubated at 22°C for additional 45 min and the second absorbance measurement was performed. The increase of absorbance per time was determined. The control value used was the inhibitor- free reaction with 0.5 % DMSO (fc). As pharmacological inhibitor control Fumagillin (Merck, Darmstadt) in a final concentration of 5 µM was used. The inhibitory values (IC₅₀) were determined using the program ASSAY ANALYZER@ from GeneData (Basel, Switzerland).

### HUVEC proliferation assay

Proliferation of HUVEC primary endothelial cells was used as cell-based mechanistical assay.²⁵ Applying the CyQUANT^{®} Direct Cell Proliferation Assay (Invitrogen C35011) which is based on a cell-permeant fluorescent DNA-binding dye, DNA content is used as a direct measure for cell number. Pooled HUVEC cells (Promocell C-12203) are cultivated in the medium supplied by Promocell (Cat No C-22020) for maximum 4 passages. For the assay 500 cells/well are seeded into black 384-well culture plates with clear bottom in 70 µl culture medium and incubated for 6 hours at 37°C, 5 % CO₂. 10 µl prediluted test compounds are added and cells incubated for 3 days at 37°C, 5 % CO ₂ before measurement of DNA content. CyQUANT detection reagent is prepared according to the manufacturers protocol, 20µl/well added and incubated at 37°C, 5 % CO2 for at least 1h befo re measurement of fluorescence at Envision multimode reader (Perkin-Elmer, Waltham) with excitation 480 nm and emission 535 nm (bottom read mode). The assay is performed as dose response with 10 compound dilutions. Inhibitory values (IC₅₀) were determined using the program ASSAY ANALYZER^{®} from GeneData (Basel, Switzerland).

In addition, the degradation of MetAP-2 in HCT116 cells could be demonstrated. See scans below. This degradation suggests a prolonged/sustained effect as the target protein MetAP-2 is not only inhibited but even degraded/removed from the cell by the compounds and accordingly, the treatment of the indications should be even more efficacious.

The initial MetAP-2 degradation results were obtained after 24 h incubation of the compounds in three different concentrations (0.1; 1; 10 µM), cell lysis, SDS-PAGE separation, blotting and antibody aided detection.

The picture below indicates how the two compounds "A1" and "A2" degrade MetAP-2 over the time.

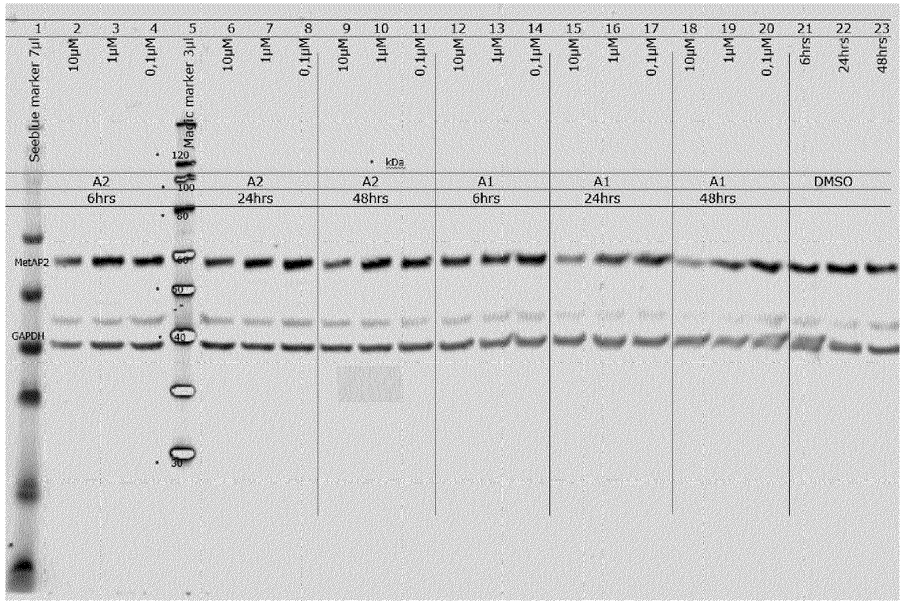

The following tables contain a quantification of these results.

In summary:
"A2" showed activity at 10 µM (40% reduction of MetAP-2 protein after 24h and 20% reduction after 48h)
"A1" is more active: At 10µM: 10% reduction after 6h, 70% reduction after 24h and again 70% after 48h. At 1µM: 15% reduction after 6h, 40% reduction after 24 h. After 48 h, any reduction could be detected. At 0,1 µM: 30% reduction after 24 h.

Comparison of MetAP2 protein levels in HTC116 cells after compound treatments at different concentrations and time points. As control, DMSO was used and its values set as "1". MetAp2 values <1 means degradation.

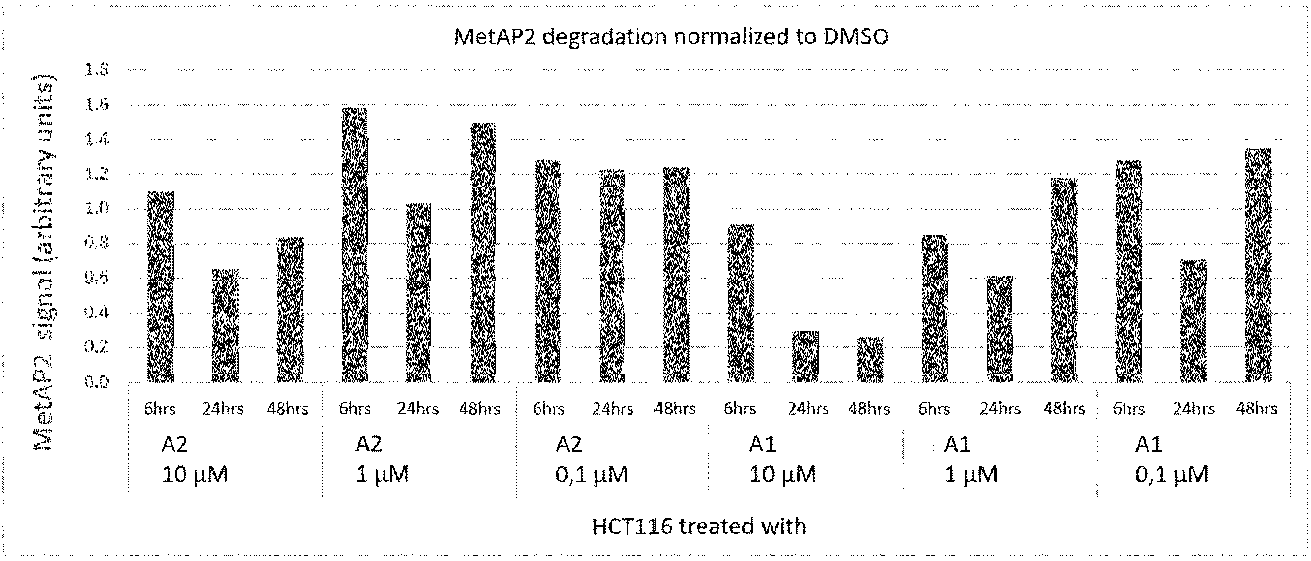

| | **Sample** | | **Signal MetAp2/GAPDH normalized to untreated (DMSO)** |
|---|---|---|---|
| 10µM | A2 | 6hrs | 1,1 |
| | | 24hrs | 0,6 |
| | | 48hrs | 0,8 |
| 1µM | A2 | 6hrs | 1,6 |
| | | 24hrs | 1,0 |
| | | 48hrs | 1,5 |
| 0,1µM | A21 | 6hrs | 1,3 |
| | | 24hrs | 1,2 |
| | | 48hrs | 1,2 |
| 10µM | A1 | 6hrs | 0,9 |
| | | 24hrs | 0,3 |
| | | 48hrs | 0,3 |
| 1µM | A1 | 6hrs | 0,9 |
| | | 24hrs | 0,6 |
| | | 48hrs | 1,2 |
| 0,1µM | A1 | 6hrs | 1,3 |
| | | 24hrs | 0,7 |
| | | 48hrs | 1,3 |

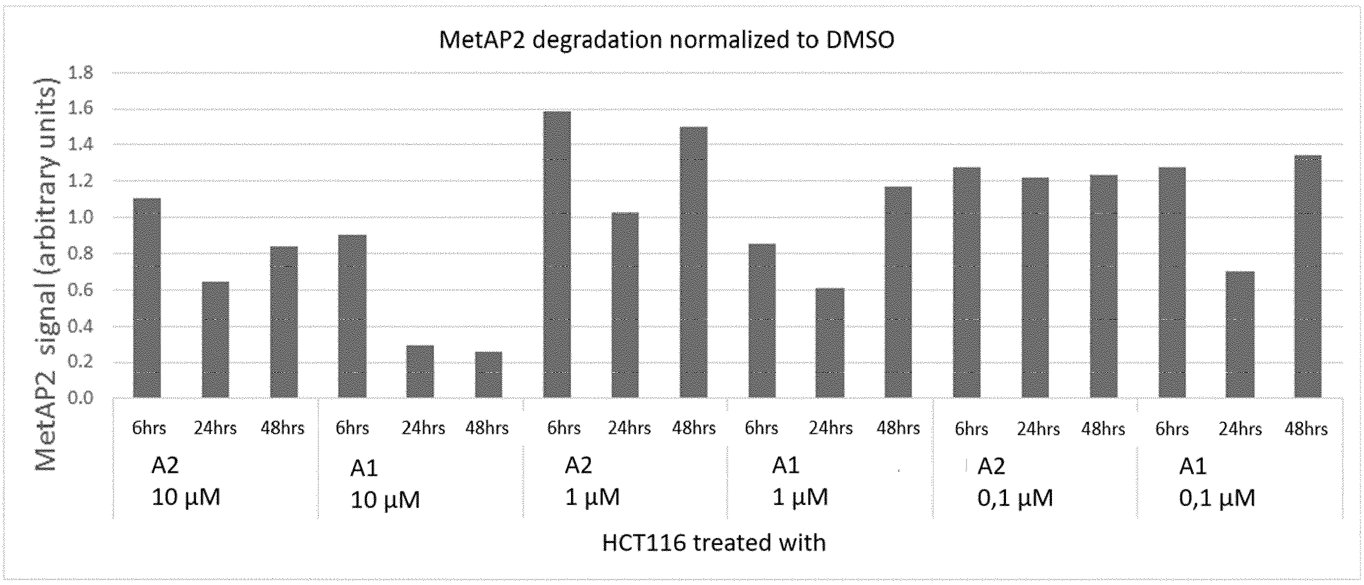

| **Sample** | | | **Signal MetAp2/GAPDH normalized to untreated (DMSO)** |
|---|---|---|---|
| 10µM | A2 | 6hrs | 1,1 |
| | | 24hrs | 0,6 |
| | | 48hrs | 0,8 |
| 10µM | A1 | 6hrs | 0,9 |
| | | 24hrs | 0,3 |
| | | 48hrs | 0,3 |
| 1µM | A2 | 6hrs | 1,6 |
| | | 24hrs | 1,0 |
| | | 48hrs | 1,5 |
| 1µM | A1 | 6hrs | 0,9 |
| | | 24hrs | 0,6 |
| | | 48hrs | 1,2 |
| 0,1µM | A2 | 6hrs | 1,3 |
| | | 24hrs | 1,2 |
| | | 48hrs | 1,2 |
| 0,1µM | A1 | 6hrs | 1,3 |
| | | 24hrs | 0,7 |
| | | 48hrs | 1,3 |

The preferred examples shown below degrade MetAP-2 in HCT116 cells in a dose and time dependent manner.

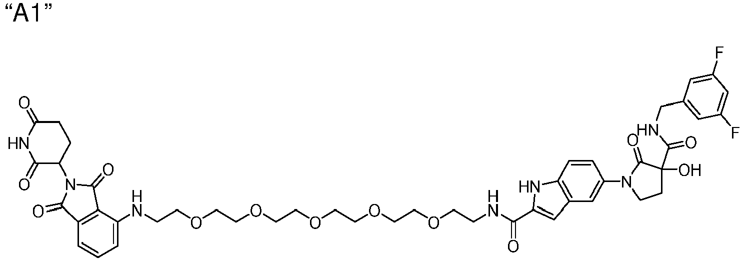

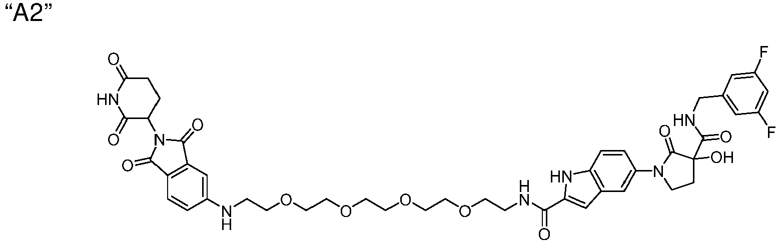

### 1. Exemplary Synthesis of "A1":

5-[3-[(3,5-difluorophenyl)methylcarbamoyl]-3-hydroxy-2-oxo-pyrrolidin-1-yl]-N-[2-[2-[2-[2-[2-[2-[[2-(2,6-dioxo-3-piperidyl)-1,3-dioxo-isoindolin-4-yl]amino]ethoxy]ethoxy]ethoxy]ethoxy]ethoxy]ethyl]-1H-indole-2-carboxamide

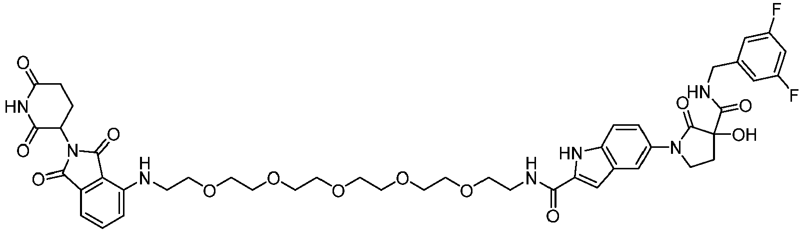

The building blocks were prepared as known from the literature and linked in an amide forming reaction as shown below:

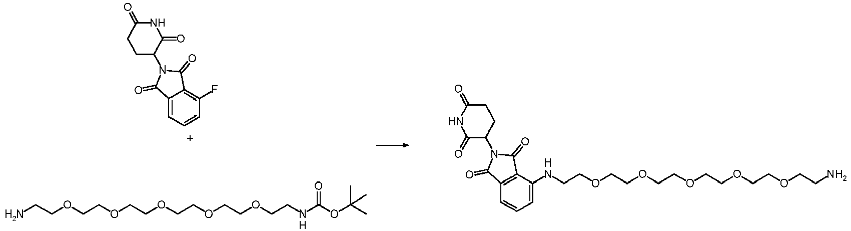

To a stirred solution of commercially available 2-(2,6-dioxopiperidin-3-yl)-4-fluoro-2,3-dihydro-1H-isoindole-1,3-dione (200 mg; 0.72 mmol) in N,N-Dimethylformamide (1.5 ml; 18 mmol) was added commercially available tert-butyl N-(17-amino-3,6,9,12,15-pentaoxaheptadecan-1-yl)carbamate (275 mg; 0,72 mmol) and Sodium carbonate anhydrous (153 mg; 1.45 mmol). The reaction mixture was heated to 90 °C for 14 h. Afte r cooling to room temperature the reaction mixture was evaporated, dissolved in DMSO and purified by reverse phase chromatography. Fractions containing the product were evaporated. The compound tert-butyl N-(17-{[2-(2,6-dioxopiperidin-3-yl)-1,3-dioxo-2,3-dihydro-1H-isoindol-4-yl]amino}-3,6,9,12,15-pentaoxahepta-decan-1-yl)carbamate was dissolved in dichlormethane (4 ml) and treated with trifluoroacetic acid (1 ml) for 3 h. The reaction was evaporated to dryness, co-evaporated twice with toluene and used directly in the next reaction without further purification.

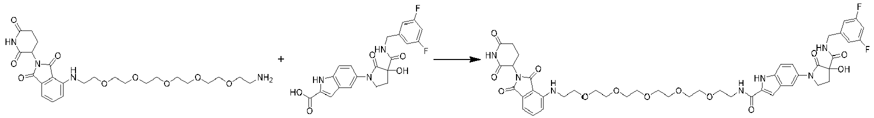

### Procedure:

5-(3-{[(3,5-difluorophenyl)methyl]carbamoyl}-3-hydroxy-2-oxopyrrolidin-1-yl)-1H-indole-2-carboxylic acid (30 mg; 0.07 mmol), 5-(15-amino-4,7,10,13-tetraoxa-1-azapentadecan-1-yl)-2-(2,6-dioxopiperidin-3-yl)-2,3-dihydro-1H-isoindole-1,3-dione (40.5 mg; 0.07 mmol), HATU (31.9 mg; 0.08 mmol) and 4-methylmorpholine (31µL; 0.28 mmol) were dissolved in *N*,*N*-dimethylformamide (5.4 mL) and stirred for 14 h at room temperature.

After completion of the reaction indicated by LC-MS, the mixture was evaporated to dryness, re-dissolved in DMSO and purified by preparative HPLC. All fractions were checked by UPLC. yielding a pure product fraction 25.1 mg and an impure fraction. The impure fraction was dissolved in DCM/MeOH, absorbed on Isolute^{®} and purified by column chromatography using DCM to DCM/20%MeOH as eluents. Pure fractions were combined.

The product was lyophilized from water/MeCN.

### Analytical Data:

Yield: 42.2 mg (66%)
appearance: white solid
LC-MS: RT: 1.46 min; Area: 100%, m/z 452.8= [M+2H]^{2+;} m/z 904.8= [M+H]⁺ NMR:
¹H NMR (700 MHz, DMSO-d₆) δ 11.62 (d, J = 2.3 Hz, 1H), 11.05 (s, 1H), 8.69 (t, J = 6.4 Hz, 1H), 8.55 (t, J = 5.7 Hz, 1H), 7.78 (d, J = 2.0 Hz, 1H), 7.61 - 7.51 (m, 2H), 7.43 (d, J = 8.8 Hz, 1H), 7.17 - 7.10 (m, 2H), 7.06 (tt, J = 9.4, 2.4 Hz, 1H), 7.00 (qd, J = 6.4, 3.1 Hz, 4H), 6.88 (dd, J = 8.5, 2.2 Hz, 1H), 6.70 (s, 1H), 5.03 (dd, J = 12.8, 5.5 Hz, 1H), 4.41 (dd, J = 15.8, 6.8 Hz, 1H), 4.27 (dd, J = 15.8, 6.0 Hz, 1H), 3.89 (ddt, J = 9.2, 5.8, 3.4 Hz, 2H), 3.59 - 3.45 (m, 18H), 3.44 (q, J = 5.9 Hz, 2H), 3.36 - 3.33 (m, 2H), 2.87 (m, 1H), 2.65 - 2.51 (m, 4H), 2.17 - 2.10 (m, 1H), 1.99 (dtd, J = 13.1, 5.4, 2.4 Hz, 1H).

The following compounds are prepared analogously:
5-[3-[(3,5-difluorophenyl)methylcarbamoyl]-3-hydroxy-2-oxo-pyrrolidin-1-yl]-N-[2-[2-[2-[2-[2-[[2-(2,6-dioxo-3-piperidyl)-1,3-dioxo-isoindolin-5-yl]amino]-ethoxy]ethoxy]ethoxy]ethoxy]ethyl]-1H-indole-2-carboxamide ("A2") 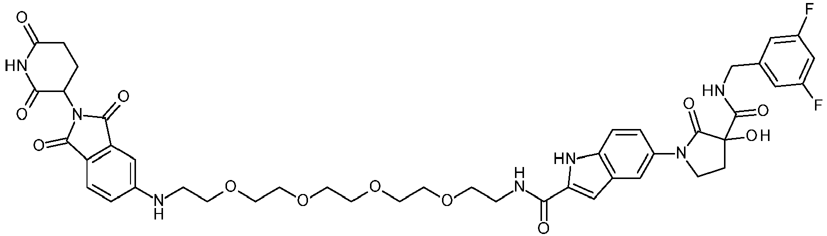
LC-MS: RT: 1,46 min, Area: 100%, m/z 452.8= [M+2H]²⁺, m/z 904.8= [M+H]⁺. ¹H NMR (700 MHz, DMSO-de) δ 11.62 (d, J = 2.3 Hz, 1H), 11.05 (s, 1H), 8.69 (t, J = 6.4 Hz, 1H), 8.55 (t, J = 5.7 Hz, 1H), 7.78 (d, J = 2.0 Hz, 1H), 7.61 - 7.51 (m, 2H), 7.43 (d, J = 8.8 Hz, 1H), 7.16
- 7.11 (m, 2H), 7.06 (tt, J = 9.4, 2.4 Hz, 1H), 7.00 (qd, J = 6.4, 3.1 Hz, 3H), 6.88 (dd, J = 8.5, 2.2 Hz, 1H), 6.70 (s, 1H), 5.03 (dd, J = 12.8, 5.5 Hz, 1H), 4.41 (dd, J = 15.8, 6.8 Hz, 1H), 4.27 (dd, J = 15.8, 6.0 Hz, 1H), 3.89 (ddt, J = 9.2, 5.8, 3.4 Hz, 2H), 3.59 - 3.46 (m, 14H), 3.44 (q, J = 5.9 Hz, 2H), 3.36-3.32 (m, 3H), 2.87 (m, 1H), 2.65 - 2.52 (m, 4H), 2.20 - 2.07 (m, 1H), 1.99 (dtd, J = 13.1, 5.4, 2.4 Hz, 1H).
(3RS)-N-[(3,5-difluorophenyl)methyl]-3-hydroxy-1-{2-[(5-{[(2S)-1-[(2S,4R)-4-hydroxy-2-({[4-(4-methyl-1,3-thiazol-5-yl)phenyl]methyl}carbamoyl)pyrrolidin-1-yl]-3,3-dimethyl-1-oxobutan-2-yl]carbamoyl}pentyl)carbamoyl]-1H-indol-5-yl}-2-oxopyrrolidine-3-carboxamide ("A3") 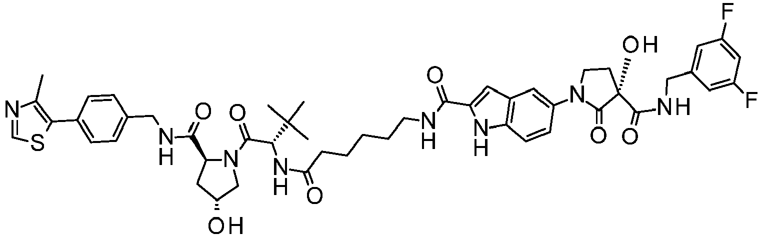
LC-MS: RT: 1,51 min, Area: 96%, m/z 638.3 = fragment.
¹H NMR (700 MHz, DMSO-de) δ 11.56 (d, J = 2.1 Hz, 1H), 8.98 (s, 1H), 8.66 (t, J = 6.4 Hz, 1H), 8.52 (t, J = 6.0 Hz, 1H), 8.43 (t, J = 5.8 Hz, 1H), 7.81 (d, J = 9.4 Hz, 1H), 7.77 (d, J = 2.0 Hz, 1H), 7.52 (dd, J = 9.0, 2.1 Hz, 1H), 7.44 - 7.40 (m, 3H), 7.38 (d, J = 8.2 Hz, 2H), 7.10 (d, J = 2.0 Hz, 1H), 7.05 (tt, J = 9.3, 2.6 Hz, 1H), 7.00 (h, J = 4.7 Hz, 2H), 4.54 (d, J = 9.4 Hz, 1H), 4.42 (dt, J = 13.0, 7.0 Hz, 3H), 4.35 (dq, J = 6.5, 3.5, 3.0 Hz, 1H), 4.27 (dd, J = 15.8, 6.1 Hz, 1H), 4.22 (dd, J = 15.9, 5.6 Hz, 1H), 3.91 - 3.87 (m, 2H), 3.69 - 3.62 (m, 2H), 3.26 (q, J = 7.1 Hz, 2H), 2.62 (ddd, J = 11.8, 6.6, 4.8 Hz, 1H), 2.27 (dt, J = 14.6, 7.6 Hz, 1H), 2.14 (ddd, J = 14.7, 9.9, 5.9 Hz, 2H), 2.03 (ddd, J = 11.2, 7.9, 2.6 Hz, 1H), 1.90 (ddd, J = 12.8, 8.5, 4.7 Hz, 1H), 1.59 - 1.47 (m, 4H), 1.35 - 1.26 (m, 2H), 0.93 (s, 9H).
5-[3-[(3,5-difluorophenyl)methylcarbamoyl]-3-hydroxy-2-oxo-pyrrolidin-1-yl]-N-[5-[[2-(2,6-dioxo-3-piperidyl)-1,3-dioxo-isoindolin-5-yl]amino]pentyl]-1H-indole-2-carboxamide ("A4") 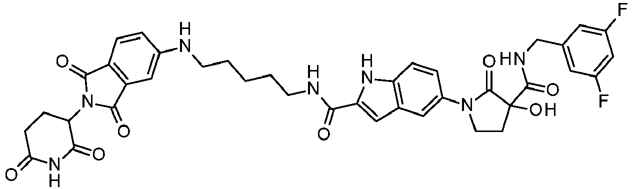
LC-MS: RT: 1,53 min, Area: 97%, m/z 770.4.
¹H NMR (500 MHz, DMSO-de) δ 11.56 (d, J = 2.2 Hz, 1H), 11.02 (s, 1H), 8.66 (t, J = 6.4 Hz, 1H), 8.46 (t, J = 5.8 Hz, 1H), 7.77 (d, J = 2.0 Hz, 1H), 7.55 (d, J = 8.4 Hz, 1H), 7.52 (dd, J = 8.9, 2.1 Hz, 1H), 7.43 (d, J = 8.9 Hz, 1H), 7.12 - 7.02 (m, 2H), 6.95 (d, J = 2.1 Hz, 1H), 6.85 (dd, J = 8.4, 2.1 Hz, 1H), 5.02 (dd, J = 12.7, 5.4 Hz, 1H), 4.42 (dd, J = 15.8, 6.8 Hz, 1H), 4.27 (dd, J = 15.8, 6.0 Hz, 1H), 3.93 - 3.86 (m, 2H), 3.31 (q, J = 6.7 Hz, 2H), 3.17 (d, J = 7.2 Hz, 2H), 2.87 (ddd, J = 16.6, 13.7, 5.3 Hz, 1H), 2.66 - 2.57 (m, 2H), 2.54 (s, 4H), 2.14 (dt, J = 12.8, 7.5 Hz, 1H), 1.99 (ddd, J = 12.6, 5.6, 3.2 Hz, 1H), 1.62 (dp, J = 15.0, 7.3 Hz, 4H), 1.45 (dt, J = 13.8, 7.1 Hz, 2H).
5-[3-[(3,5-difluorophenyl)methylcarbamoyl]-3-hydroxy-2-oxo-pyrrolidin-1-yl]-N-[2-[2-[2-[[2-(2,6-dioxo-3-piperidyl)-1,3-dioxo-isoindolin-4-yl]amino]ethoxy]-ethoxy]ethyl]-1H-indole-2-carboxamide ("A5") 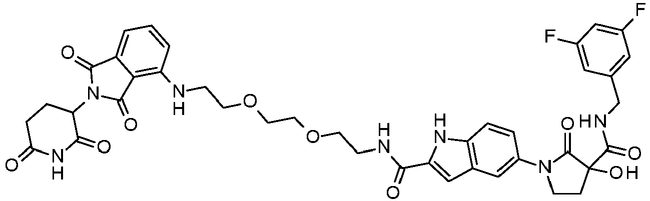
LC-MS: RT: 1,49 min, Area: 98%, m/z 816,4.
¹H NMR (500 MHz, DMSO-de) δ 11.58 (d, J = 2.2 Hz, 1H), 11.06 (s, 1H), 8.66 (t, J = 6.4 Hz, 1H), 8.50 (t, J = 5.7 Hz, 1H), 7.76 (d, J = 2.0 Hz, 1H), 7.57 - 7.51 (m, 2H), 7.42 (d, J = 8.9 Hz, 1H), 7.11 (d, J = 2.2 Hz, 1H), 7.10 - 7.04 (m, 2H), 7.01 (qd, J = 6.9, 3.6 Hz, 4H), 6.67 (s, 1H), 6.58 (t, J = 5.8 Hz, 1H), 5.04 (dd, J = 12.8, 5.4 Hz, 1H), 4.42 (dd, J = 15.8, 6.8 Hz, 1H), 4.27 (dd, J = 15.8, 6.0 Hz, 1H), 3.89 (dd, J = 8.3, 5.5 Hz, 2H), 3.67 - 3.49 (m, 8H), 3.43 (dq, J = 11.1, 5.6 Hz, 4H), 2.87 (ddd, J = 16.8, 13.7, 6.1 Hz, 1H), 2.66 - 2.53 (m, 4H), 2.14 (dt, J = 12.9, 7.5 Hz, 1H), 2.08 -1.94 (m, 1H), 1.24 (d, J = 3.4 Hz, 1H).
5-[3-[(3,5-difluorophenyl)methylcarbamoyl]-3-hydroxy-2-oxo-pyrrolidin-1-yl]-N-[2-[2-[2-[[(1S)-1-[(2S,4R)-4-hydroxy-2-[[4-(4-methylthiazol-5-yl)phenyl]methylcarbamoyl]pyrrolidine-1-carbonyl]-2,2-dimethyl-propyl]amino]-2-oxo-ethoxy]ethoxy]ethyl]-1H-indole-2-carboxamide ("A6") 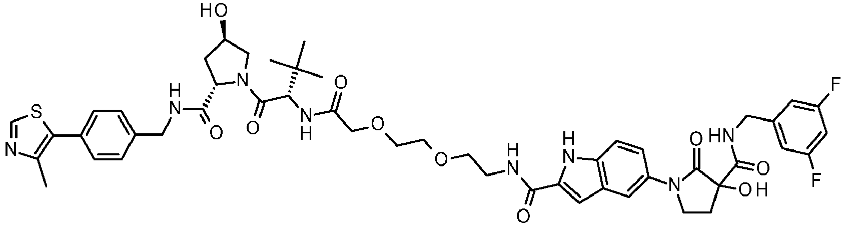
LC-MS: RT: 1,48 min, Area: 91%, m/z 670.3 = fragment.
¹H NMR (500 MHz, DMSO-de) δ 11.58 (d, J = 2.2 Hz, 1H), 8.95 (s, 1H), 8.66 (t, J = 6.5 Hz, 1H), 8.53 (dt, J = 9.8, 5.9 Hz, 2H), 7.77 (t, J = 1.4 Hz, 1H), 7.54 (dt, J = 8.9, 1.8 Hz, 1H), 7.43 (dd, J = 9.2, 5.3 Hz, 2H), 7.38 (s, 4H), 7.12 (d, J = 2.5 Hz, 1H), 7.09 - 6.97 (m, 3H), 4.57 (d, J = 9.6 Hz, 1H), 4.50 - 4.32 (m, 6H), 4.26 (dt, J = 15.7, 6.4 Hz, 2H), 3.98 (d, J = 1.3 Hz, 2H), 3.92 - 3.85 (m,
2H), 3.71 - 3.54 (m, 8H), 3.47 (q, J = 5.6 Hz, 2H), 2.66 - 2.57 (m, 1H), 2.43 (s, 3H), 2.18 - 2.10 (m, 1H), 2.10 - 2.03 (m, 1H), 1.91 (ddd, J = 13.0, 8.7, 4.6 Hz, 1H), 0.94 (s, 9H).
5-[3-[(3,5-difluorophenyl)methylcarbamoyl]-3-hydroxy-2-oxo-pyrrolidin-1-yl]-N-[2-[2-[2-[2-[2-[[(1S)-1-[(2S,4R)-4-hydroxy-2-[[4-(4-methylthiazol-5-yl)phenyl]-methylcarbamoyl]pyrrolidine-1-carbonyl]-2,2-dimethyl-propyl]amino]-2-oxo-ethoxy]ethoxy]ethoxy]ethoxy]ethyl]-1H-indole-2-carboxamide ("A7") 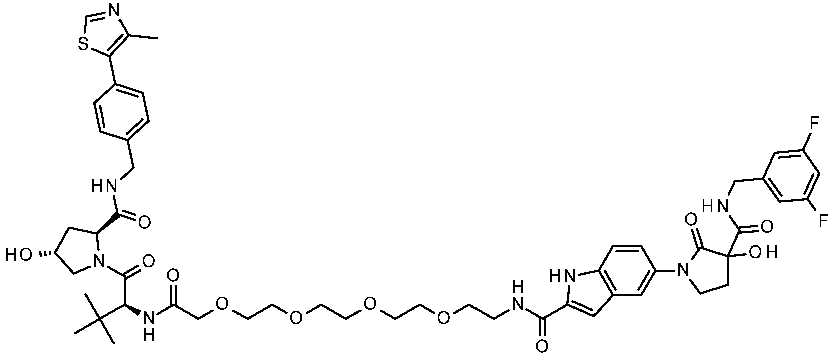
LC-MS: RT: 1,49 min, Area: 98%, m/z 538.4 = [M+2H]²⁺, m/z 1075.4 = [M+H]⁺. ¹H NMR (700 MHz, DMSO-de) δ 11.62 (d, J = 2.1 Hz, 1H), 8.98 (s, 1H), 8.69 (t, J = 6.4 Hz, 1H), 8.57 (m, 2H), 7.77 (d, J = 2.1 Hz, 1H), 7.54 (dd, J = 9.0, 2.1 Hz, 1H), 7.48 - 7.33 (m, 6H), 7.13 (d, J = 2.2 Hz, 1H), 7.06 (tt, J = 9.3, 2.4 Hz, 1H), 7.00 (h, J = 4.3 Hz, 2H), 6.70 (s, 1H), 5.14 (s, 1H), 4.56 (d, J = 9.6 Hz, 1H), 4.46 - 4.33 (m, 4H), 4.26 (ddd, J = 16.1, 11.0, 5.8 Hz, 2H), 3.95 (s, 2H), 3.90 (tq, J = 9.1, 4.7, 4.0 Hz, 2H), 3.67 (dd, J = 10.6, 4.0 Hz, 1H), 3.63 - 3.48 (m, 14H), 3.43 (q, J = 5.9 Hz, 2H), 2.65 - 2.58 (m, 1H), 2.44 (s, 3H), 2.14 (dt, J = 12.7, 7.5 Hz, 1H), 2.06 (ddd, J = 9.1, 4.4, 2.2 Hz, 1H), 1.90 (ddd, J = 13.1, 8.9, 4.6 Hz, 1H), 1.29 - 1.17 (m, 1H), 0.94 (s, 9H).
5-[3-[(3,5-difluorophenyl)methylcarbamoyl]-3-hydroxy-2-oxo-pyrrolidin-1-yl]-N-[2-[2-[2-[2-[2-[2-[[(1S)-1-[(2S,4R)-4-hydroxy-2-[[4-(4-methylthiazol-5-yl)phenyl]-methylcarbamoyl]pyrrolidine-1-carbonyl]-2,2-dimethyl-propyl]amino]-2-oxo-ethoxy]ethoxy]ethoxy]ethoxy]ethoxy]ethyl]-1H-indole-2-carboxamide ("A8") 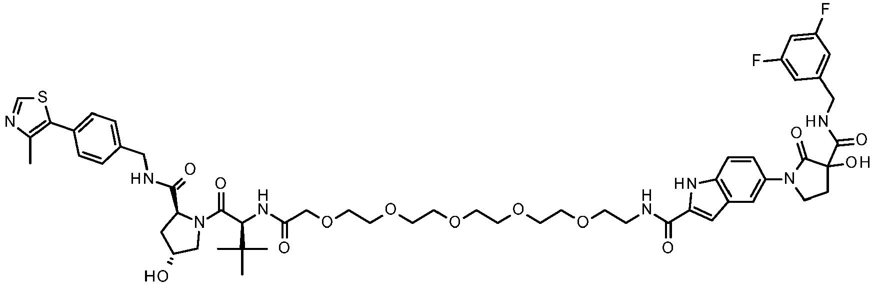
LC-MS: RT: 1,49 min, Area: 100%, m/z 560.4 = [M+2H]²⁺, m/z 1119.4 = [M+H]⁺.
¹H NMR (700 MHz, DMSO-de) δ 11.62 (d, J = 2.1 Hz, 1H), 8.98 (s, 1H), 8.69 (t, J = 6.5 Hz, 1H), 8.57 (m, 2H), 7.77 (d, J = 2.0 Hz, 1H), 7.54 (dd, J = 8.9, 2.1 Hz, 1H), 7.45 - 7.37 (m, 6H), 7.13 (d, J = 2.1 Hz, 1H), 7.06 (tt, J = 9.3, 2.4 Hz, 1H), 7.00 (h, J = 4.3 Hz, 2H), 4.56 (d, J = 9.6 Hz, 1H), 4.41 (m, 3H), 4.35 (tt, J = 4.4, 2.1 Hz, 1H), 4.26 (m, 3H), 3.96 (m, 2H), 3.90 (m, 3H), 3.67 (dd, J = 10.6, 4.0 Hz, 1H), 3.62 - 3.58 (m, 3H), 3.56 - 3.42 (m, 18H), 2.66 - 2.58 (m, 1H), 2.44 (s, 3H), 2.14 (m, 1H), 2.05 (ddt, J = 12.0, 7.6, 1.9 Hz, 1H), 1.90 (ddd, J = 13.0, 8.8, 4.5 Hz, 1H), 0.94 (s, 9H).
5-[3-[(3,5-difluorophenyl)methylcarbamoyl]-3-hydroxy-2-oxo-pyrrolidin-1-yl]-N-[2-[2-[2-[2-[2-[2-[[2-(2,6-dioxo-3-piperidyl)-1,3-dioxo-isoindolin-4-yl]-methyl-amino]ethoxy]ethoxy]ethoxy]ethoxy]ethoxy]ethyl]-1H-indole-2-carboxamide ("A9") 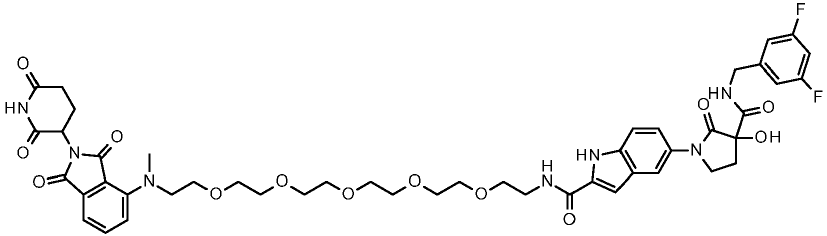
5-[3-[(3,5-difluorophenyl)methylcarbamoyl]-3-hydroxy-2-oxo-pyrrolidin-1-yl]-N-[2-[2-[2-[2-[2-[2-[2-(2,6-dioxo-3-piperidyl)-1,3-dioxo-isoindolin-4-yl]oxyethoxy]-ethoxy]ethoxy]ethoxy]ethoxy]ethyl]-1H-indole-2-carboxamide ("A10") 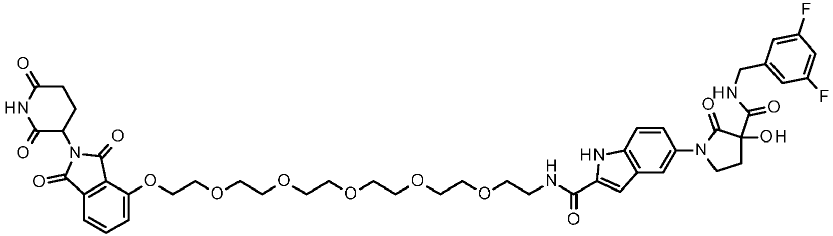
5-[3-[(3,5-difluorophenyl)methylcarbamoyl]-3-hydroxy-2-oxo-pyrrolidin-1-yl]-N-[2-[2-[2-[2-[2-[2-[2-(2,6-dioxo-3-piperidyl)-1,3-dioxo-isoindolin-4-yl]ethoxy]-ethoxy]ethoxy]ethoxy]ethoxy]ethyl]-1H-indole-2-carboxamide ("A11") 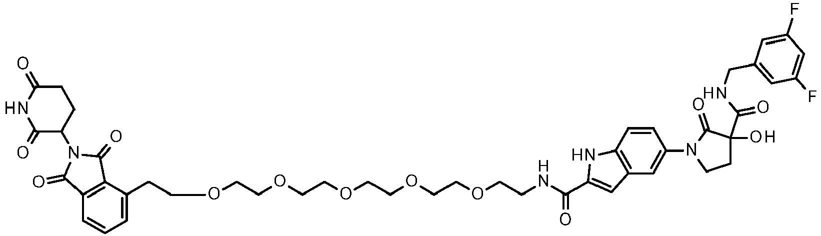
5-[3-[(3,5-difluorophenyl)methylcarbamoyl]-3-hydroxy-2-oxo-pyrrolidin-1-yl]-N-[2-[2-[2-[2-[2-[2-[2-(2,6-dioxo-3-piperidyl)-1,3-dioxo-isoindolin-4-yl]oxyethoxy]-ethoxy]ethoxy]ethoxy]ethoxy]ethyl]-N-methyl-1H-indole-2-carboxamide ("A12") 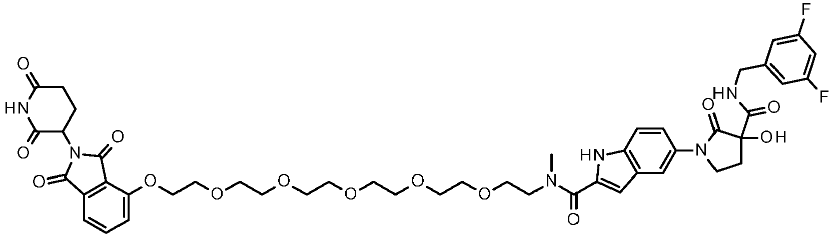
5-[3-[(3,5-difluorophenyl)methylcarbamoyl]-3-hydroxy-2-oxo-pyrrolidin-1-yl]-N-[5-[2-[2-[2-[2-[2-(2,6-dioxo-3-piperidyl)-1,3-dioxo-isoindolin-4-yl]oxyethoxy]-ethoxy]ethoxy]ethoxy]pentyl]-1H-indole-2-carboxamide ("A13") 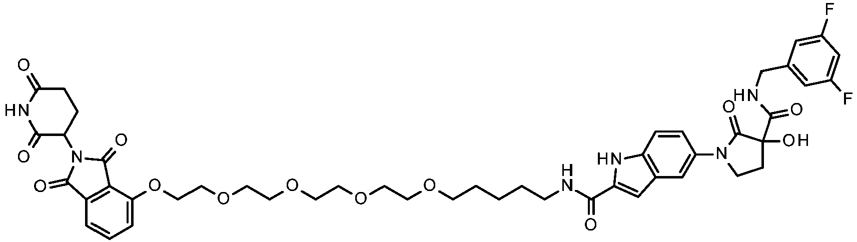
5-[3-[(3,5-difluorophenyl)methylcarbamoyl]-3-hydroxy-2-oxo-pyrrolidin-1-yl]-N-[8-[2-[2-[2-[2-(2,6-dioxo-3-piperidyl)-1,3-dioxo-isoindolin-4-yl]oxyethoxy]-ethoxy]ethoxy]octyl]-1H-indole-2-carboxamide ("A14") 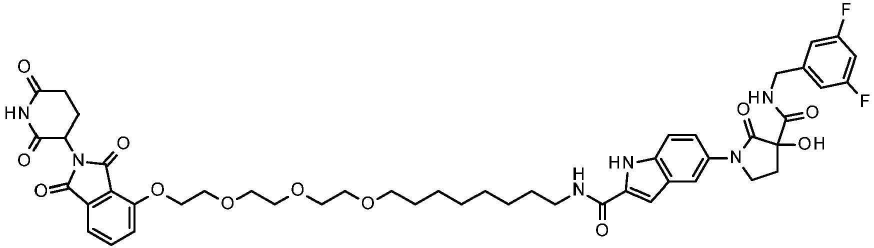
5-[3-[(3,5-difluorophenyl)methylcarbamoyl]-3-hydroxy-2-oxo-pyrrolidin-1-yl]-N-[2-[2-[2-[2-[5-[[2-(2,6-dioxo-3-piperidyl)-1,3-dioxo-isoindolin-4-yl]amino]-pentoxy]ethoxy]ethoxy]ethoxy]ethyl]-1H-indole-2-carboxamide ("A15") 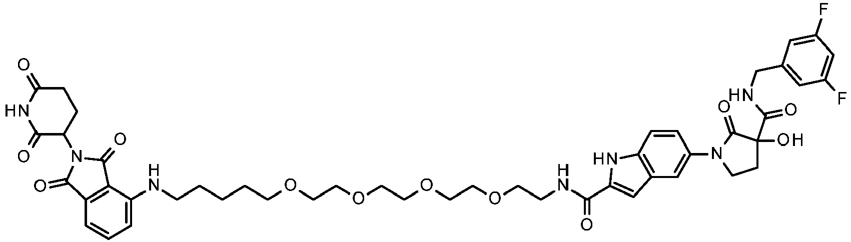
5-[3-[(3,5-difluorophenyl)methylcarbamoyl]-3-hydroxy-2-oxo-pyrrolidin-1-yl]-N-[2-[2-[2-[8-[[2-(2,6-dioxo-3-piperidyl)-1,3-dioxo-isoindolin-4-yl]amino]octoxy]-ethoxy]ethoxy]ethyl]-1H-indole-2-carboxamide ("A16") 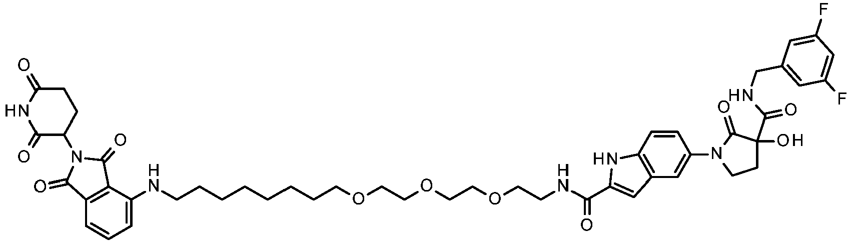
5-[3-[(3,5-difluorophenyl)methylcarbamoyl]-3-hydroxy-2-oxo-pyrrolidin-1-yl]-N-[2-[2-[2-[2-[2-[2-[[2-(2,6-dioxo-3-piperidyl)-1,3-dioxo-isoindolin-4-yl]amino]-ethoxy]ethoxy]ethoxy]ethoxy]ethoxy]ethyl]-1H-benzimidazole-2-carboxamide ("A17") 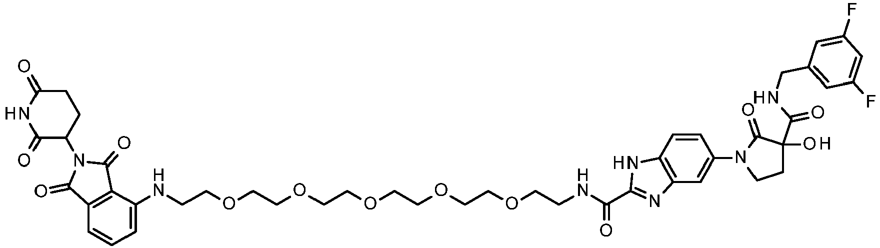
N-[(3,5-difluorophenyl)methyl]-1-[4-[2-[2-[2-[2-[2-[2-[[2-(2,6-dioxo-3-piperidyl)-1,3-dioxo-isoindolin-4-yl]amino]ethoxy]ethoxy]ethoxy]ethoxy]ethoxy]ethylcarbamoyl]phenyl]-3-hydroxy-2-oxo-pyrrolidine-3-carboxamide ("A18") 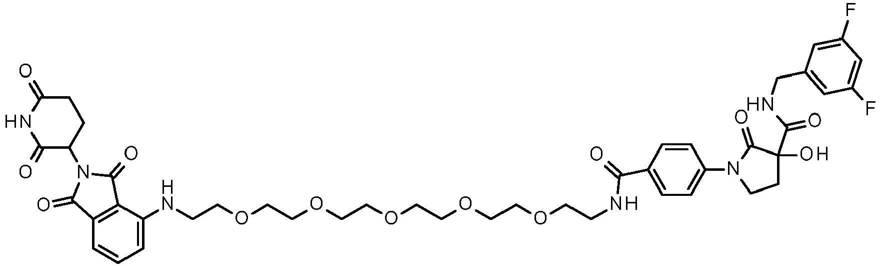
5-[3-[(3-chloro-5-fluoro-phenyl)methylcarbamoyl]-3-hydroxy-2-oxo-pyrrolidin-1-yl]-N-[2-[2-[2-[2-[2-[2-[[2-(2,6-dioxo-3-piperidyl)-1,3-dioxo-isoindolin-4-yl]amino]ethoxy]ethoxy]ethoxy]ethoxy]ethoxy]ethyl]-1H-indole-2-carboxamide ("A19") 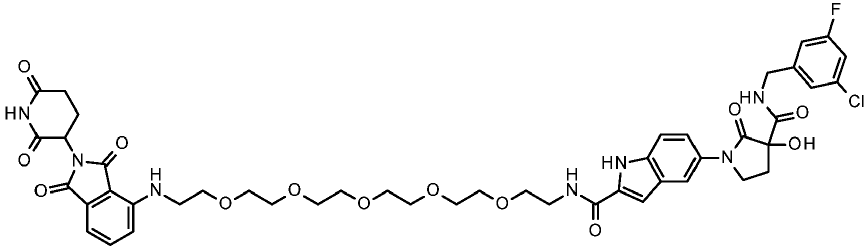
5-[3-[(3-chloro-5-fluoro-phenyl)methylcarbamoyl]-3-hydroxy-5-methyl-2-oxopyrrolidin-1-yl]-N-[2-[2-[2-[2-[2-[2-[[2-(2,6-dioxo-3-piperidyl)-1,3-dioxo-isoindolin-4-yl]amino]ethoxy]ethoxy]ethoxy]ethoxy]ethoxy]ethyl]-1H-indole-2-carboxamide ("A20") 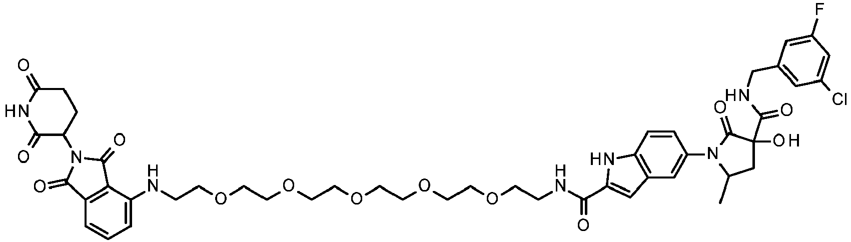
5-[3-[(3,5-difluorophenyl)methylcarbamoyl]-3,4-dihydroxy-2-oxo-pyrrolidin-1-yl]-N-[2-[2-[2-[2-[2-[2-[[2-(2,6-dioxo-3-piperidyl)-1,3-dioxo-isoindolin-4-yl]amino]ethoxy]ethoxy]ethoxy]ethoxy]ethoxy]ethyl]-1H-indole-2-carboxamide ("A21") 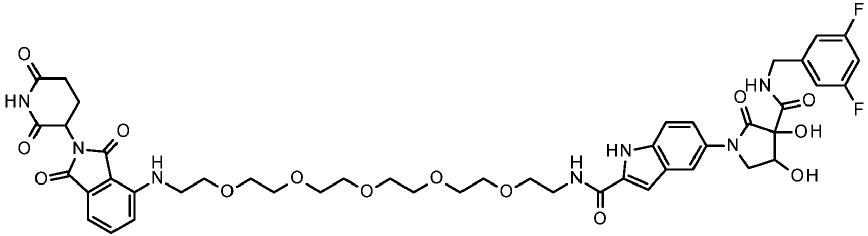
5-[3-[(3-chloro-5-fluoro-phenyl)methylcarbamoyl]-5-fluoro-3-hydroxy-2-oxopyrrolidin-1-yl]-N-[2-[2-[2-[2-[2-[2-[[2-(2,6-dioxo-3-piperidyl)-1,3-dioxo-isoindolin-4-yl]amino]ethoxy]ethoxy]ethoxy]ethoxy]ethoxy]ethyl]-1H-indole-2-carboxamide ("A22") 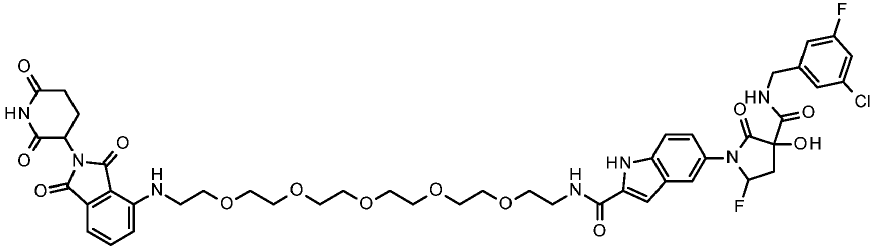
5-[3-[(3-chloro-5-fluoro-phenyl)methylcarbamoyl]-3-hydroxy-2-oxo-1-piperidyl]-N-[2-[2-[2-[2-[2-[2-[[2-(2,6-dioxo-3-piperidyl)-1,3-dioxo-isoindolin-4-yl]amino]-ethoxy]ethoxy]ethoxy]ethoxy]ethoxy]ethyl]-1H-indole-2-carboxamide ("A23") 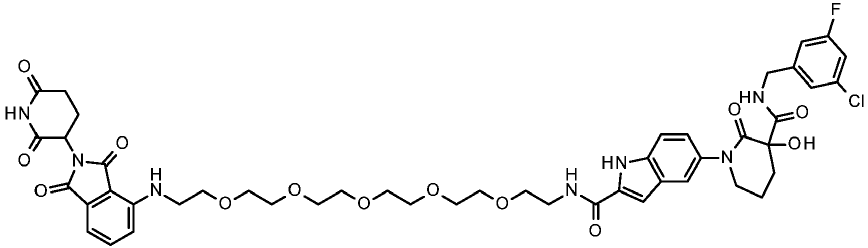
5-[3-[2-(3,5-difluorophenyl)ethylcarbamoyl]-3-hydroxy-2-oxo-pyrrolidin-1-yl]-N-[2-[2-[2-[2-[2-[2-[[2-(2,6-dioxo-3-piperidyl)-1,3-dioxo-isoindolin-4-yl]amino]-ethoxy]ethoxy]ethoxy]ethoxy]ethoxy]ethyl]-1H-indole-2-carboxamide ("A24") 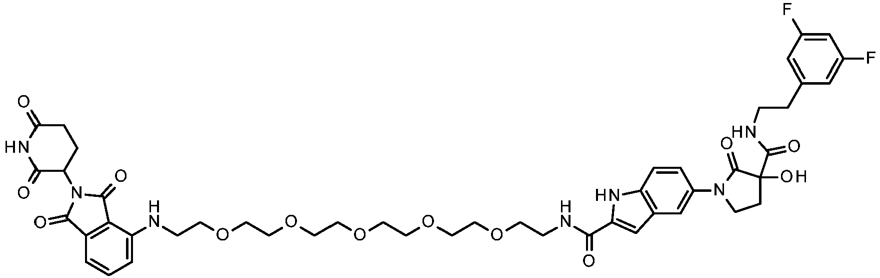
N-[2-[2-[2-[2-[2-[2-[[2-(2,6-dioxo-3-piperidyl)-1,3-dioxo-isoindolin-4-yl]amino]-ethoxy]ethoxy]ethoxy]ethoxy]ethoxy]ethyl]-5-[3-hydroxy-2-oxo-3-[2-(2-thienyl)-ethylcarbamoyl]pyrrolidin-1-yl]-1H-indole-2-carboxamide ("A25") 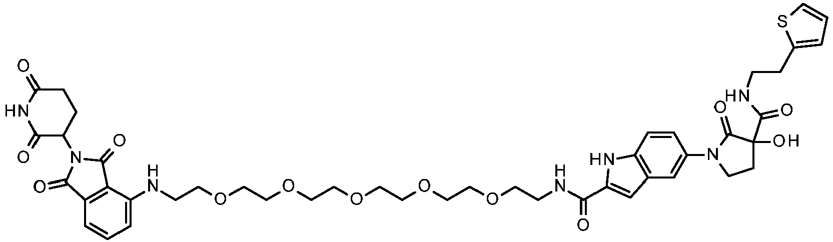
N-[2-[2-[2-[2-[2-[2-[[2-(2,6-dioxo-3-piperidyl)-1,3-dioxo-isoindolin-4-yl]amino]ethoxy]ethoxy]ethoxy]ethoxy]ethoxy]ethyl]-5-[3-[2-(2-furyl)ethylcarbamoyl]-3-hydroxy-2-oxo-pyrrolidin-1-yl]-1H-indole-2-carboxamide ("A26") 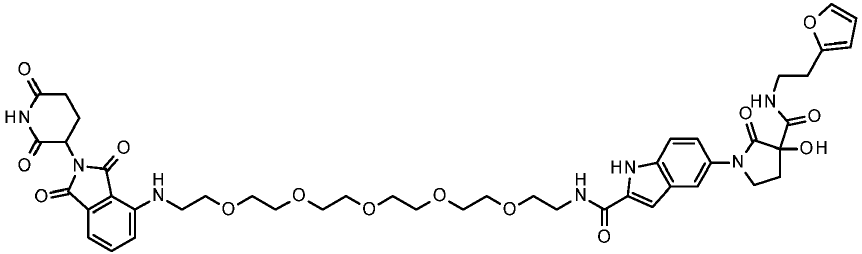
5-[3-(cyclohexylmethylcarbamoyl)-3-hydroxy-2-oxo-pyrrolidin-1-yl]-N-[2-[2-[2-[2-[2-[2-[[2-(2,6-dioxo-3-piperidyl)-1,3-dioxo-isoindolin-4-yl]amino]ethoxy]-ethoxy]ethoxy]ethoxy]ethoxy]ethyl]-1H-indole-2-carboxamide ("A27") 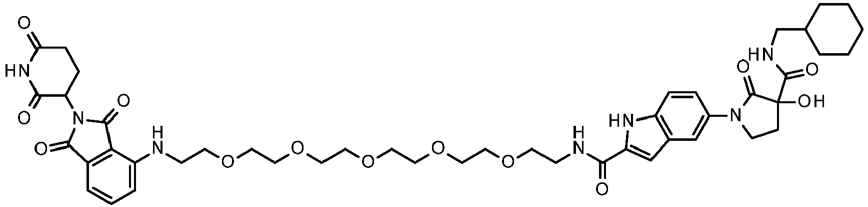
N-[(3,5-difluorophenyl)methyl]-1-[6-[2-[2-[2-[2-[2-[2-[[2-(2,6-dioxo-3-piperidyl)-1,3-dioxo-isoindolin-4-yl]amino]ethoxy]ethoxy]ethoxy]ethoxy]ethoxy]-ethylamino]-6-oxo-hexyl]-3-hydroxy-2-oxo-pyrrolidine-3-carboxamide ("A28") 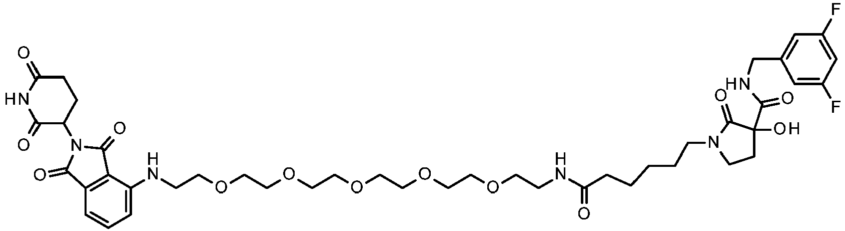
N-[(3,5-difluorophenyl)methyl]-1-[2-[4-[2-[2-[2-[2-[2-[[2-(2,6-dioxo-3-piperidyl)-1,3-dioxo-isoindolin-4-yl]amino]ethoxy]ethoxy]ethoxy]ethoxy]ethyl]piperazine-1-carbonyl]-1H-indol-5-yl]-3-hydroxy-2-oxo-pyrrolidine-3-carboxamide ("A29") 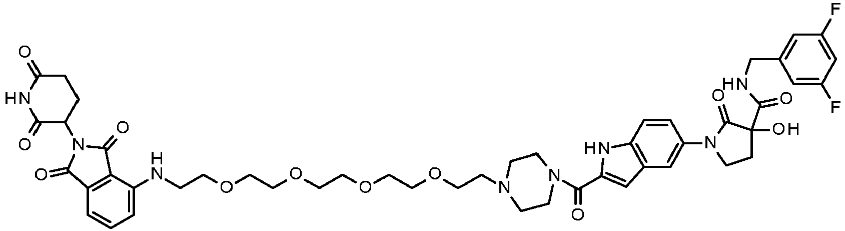
N-[(3,5-difluorophenyl)methyl]-1-[2-[9-[2-[2-[2-[2-[[2-(2,6-dioxo-3-piperidyl)-1 ,3-dioxo-isoindolin-4-yl]amino]ethoxy]ethoxy]ethoxy]ethyl]-3,9-diazaspiro[5.5]undecane-3-carbonyl]-1H-indol-5-yl]-3-hydroxy-2-oxo-pyrrolidine-3-carboxamide ("A30") 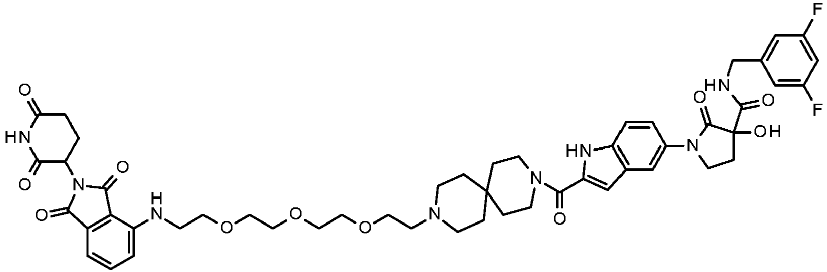
5-[3-[(3,5-difluorophenyl)methylcarbamoyl]-3-hydroxy-2-oxo-pyrrolidin-1-yl]-N-[14-[[2-(2,6-dioxo-3-piperidyl)-1 ,3-dioxo-isoindolin-5-yl]amino]tetradecyl]-1H-indole-2-carboxamide ("A31") 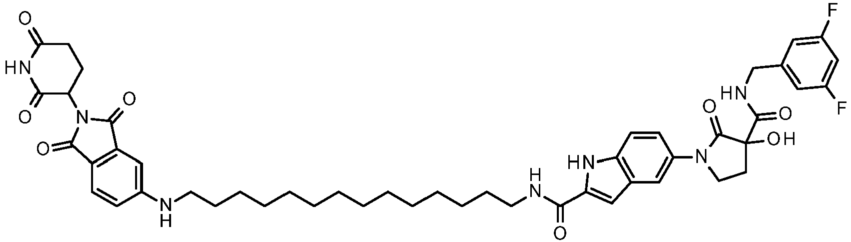
5-[3-[(3,5-difluorophenyl)methylcarbamoyl]-3-hydroxy-2-oxo-pyrrolidin-1-yl]-N-[3-[3-[3-[[2-(2,6-dioxo-3-piperidyl)-1,3-dioxo-isoindolin-5-yl]amino]propoxy]-propoxy]propoxymethyl]-1H-indole-2-carboxamide ("A32") 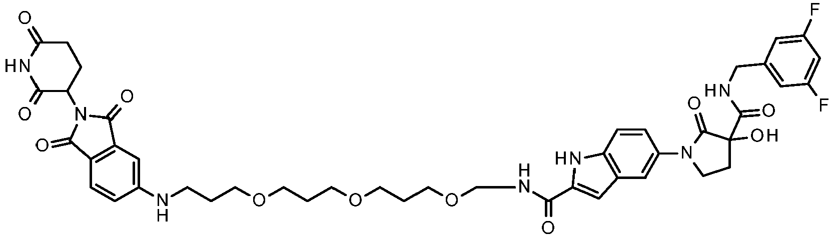
5-[3-[(3,5-difluorophenyl)methylcarbamoyl]-3-hydroxy-2-oxo-pyrrolidin-1-yl]-N-[4-[3-[4-[[2-(2,6-dioxo-3-piperidyl)-1,3-dioxo-isoindolin-5-yl]amino]butoxy]-propoxy]butyl]-1H-indole-2-carboxamide ("A33") 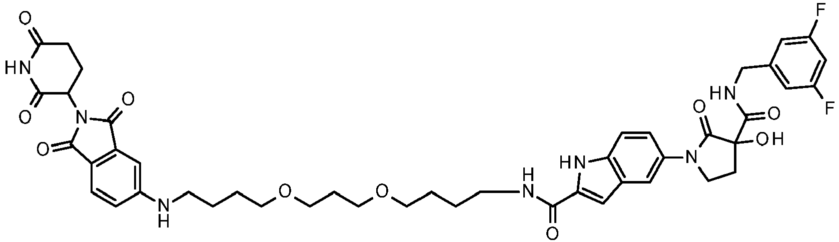
5-[3-[(3,5-difluorophenyl)methylcarbamoyl]-3-hydroxy-2-oxo-pyrrolidin-1-yl]-N-[2-[2-[2-[2-[[2-[2-(2,6-dioxo-3-piperidyl)-1,3-dioxo-isoindolin-5-yl]oxyacetyl]-amino]ethoxy]ethoxy]ethoxy]ethyl]-1H-indole-2-carboxamide ("A34") 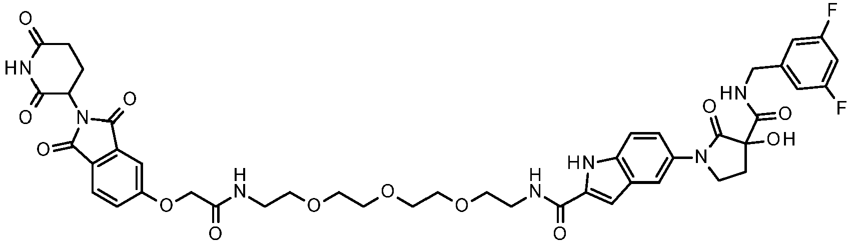
N-[(3,5-difluorophenyl)methyl]-1-[2-[2-[2-[2-[2-[2-[2-[[2-(2,6-dioxo-3-piperidyl)-1,3-dioxo-isoindolin-4-yl]amino]ethoxy]ethoxy]ethoxy]ethoxy]ethoxy]ethylsulfamoyl]-1H-indol-5-yl]-3-hydroxy-2-oxo-pyrrolidine-3-carboxamide ("A35") 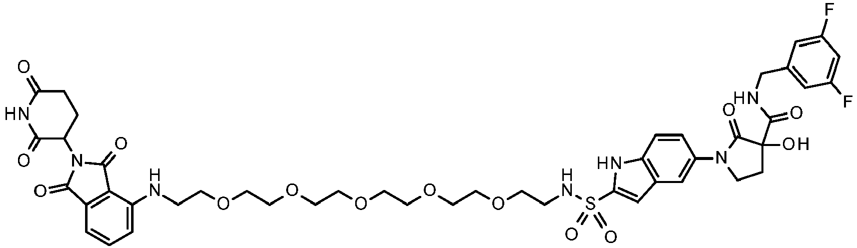
2-[2-[2-[2-[2-[2-[[2-(2,6-dioxo-3-piperidyl)-1,3-dioxo-isoindolin-4-yl]amino]-ethoxy]ethoxy]ethoxy]ethoxy]ethoxy]ethyl5-[3-[(3,5-difluorophenyl)methylcarbamoyl]-3-hydroxy-2-oxo-pyrrolidin-1-yl]-1H-indole-2-sulfonate ("A36") 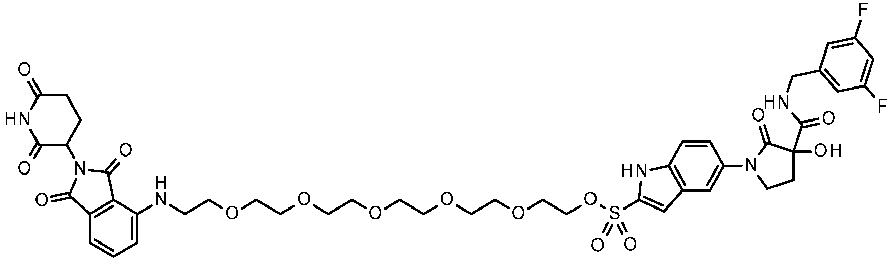
N-[(3,5-difluorophenyl)methyl]-1-[2-[2-[2-[2-[2-[2-[2-[[2-(2,6-dioxo-3-piperidyl)-1,3-dioxo-isoindolin-4-yl]amino]ethoxy]ethoxy]ethoxy]ethoxy]ethoxy]ethoxy-sulfonimidoyl]-1H-indol-5-yl]-3-hydroxy-2-oxo-pyrrolidine-3-carboxamide "A37" 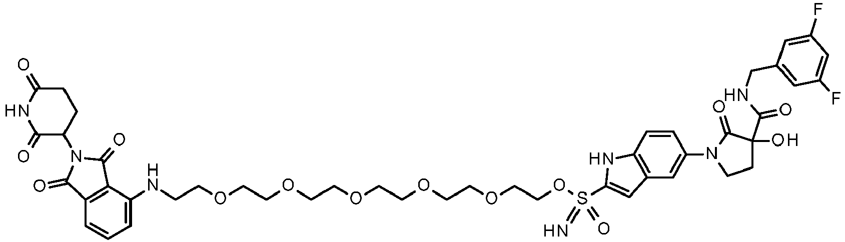
N-[(3,5-difluorophenyl)methyl]-1-[4-[2-[2-[2-[2-[2-[2-[[2-(2,6-dioxo-3-piperidyl)-1,3-dioxo-isoindolin-4-yl]amino]ethoxy]ethoxy]ethoxy]ethoxy]ethoxy]ethyl-carbamoylamino]phenyl]-3-hydroxy-2-oxo-pyrrolidine-3-carboxamide ("A38") 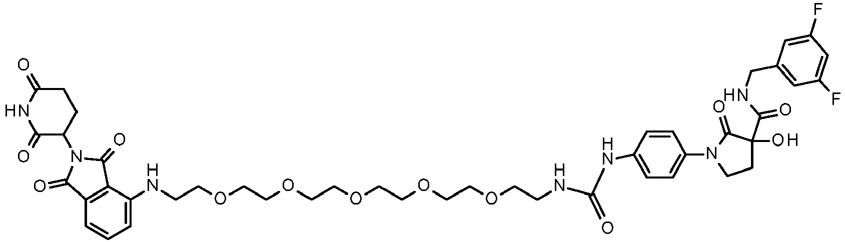
2-[2-[2-[2-[2-[2-[[2-(2,6-dioxo-3-piperidyl)-1,3-dioxo-isoindolin-4-yl]amino]-ethoxy]ethoxy]ethoxy]ethoxy]ethoxy]ethyl N-[4-[3-[(3,5-difluorophenyl)-methylcarbamoyl]-3-hydroxy-2-oxo-pyrrolidin-1-yl]phenyl]carbamate ("A39") 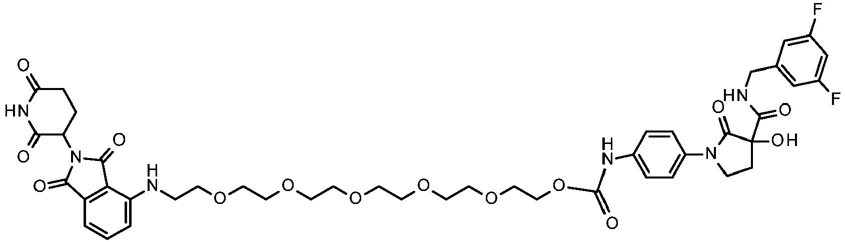
N-[(3,5-difluorophenyl)methyl]-1-[4-[2-[2-[2-[2-[2-[2-[2-[[2-(2,6-dioxo-3-piperidyl)-1,3-dioxo-isoindolin-4-yl]amino]ethoxy]ethoxy]ethoxy]ethoxy]-ethoxy]ethoxy]ethylsulfanyl]phenyl]-3-hydroxy-2-oxo-pyrrolidine-3-carboxamide ("A40") 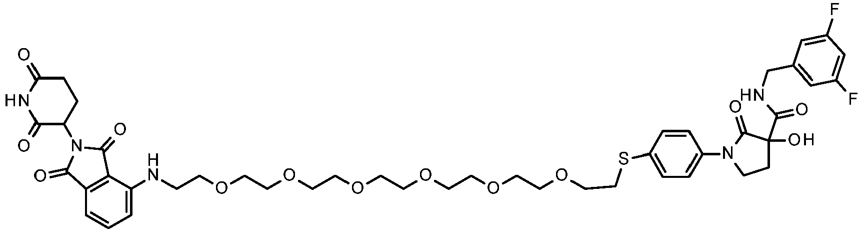
N-[(3,5-difluorophenyl)methyl]-1-[4-[2-[2-[2-[2-[2-[2-[2-[[2-(2,6-dioxo-3-piperidyl)-1,3-dioxo-isoindolin-4-yl]amino]ethoxy]ethoxy]ethoxy]ethoxy]-ethoxy]ethoxy]ethoxy]phenyl]-3-hydroxy-2-oxo-pyrrolidine-3-carboxamide ("A41") 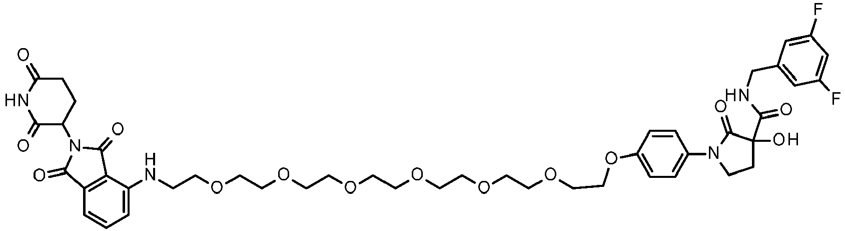
**5-[(3S)-3-{[(3,5-difluorophenyl)methyl]carbamoyl}-3-hydroxy-2-oxopyrrolidin-1-yl]-N-(2-{2-[2-(2-{[2-(2,6-dioxopiperidin-3-yl)-1,3-dioxo-2,3-dihydro-1H-isoindol-5-yl]amino}ethoxy)ethoxy]ethoxy}ethyl)-1H-indole-2-carboxamide ("A42")**

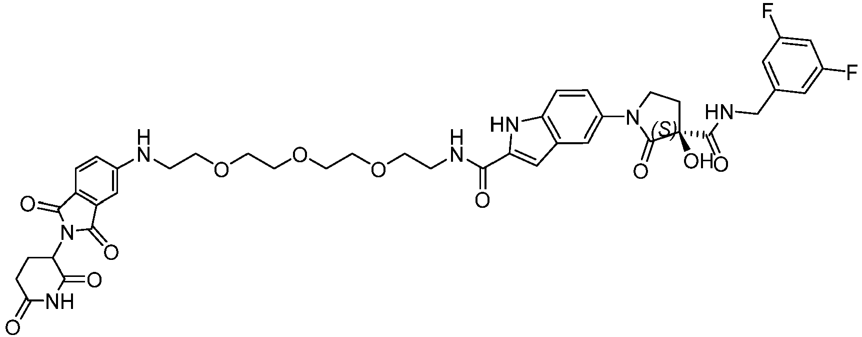

### Method 1:

MS instrument type: SHIMADZU LCMS-2020; column: Kinetex EVO C18 30*2.1mm,5um; mobile phase A: 0.0375% TFA in water (v/v), B: 0.01875% TFA in Acetonitrile (v/v); gradient: 0.0 min 5% B→ 0.80 min 95% B→ 1.20 min 95% B→ 1.21 min 5% B→ 1.55 min 5% B; flow rate: 1.5 (mL/min); oven temperature: 50 °C; UV detection: 220 nm & 254 nm.

### Method 2:

HPLC instrument type: SHIMADZU LC-20AB; column: Kinetex C18 LC Column 4.6X50mm,5um; mobile phase A: 0.0375% TFA in water (v/v), B: 0.01875% TFA in Acetonitrile (v/v); gradient: 0.0 min 10% B→ 2.40 min 80% B→ 3.70 min 80% B→ 3.71 min 10% B→ 4.00 min 10% B; oven temperature: 50 °C; UV detection: PDA(220nm&215nm&254nm).

### General procedure for preparation of intermediate 3

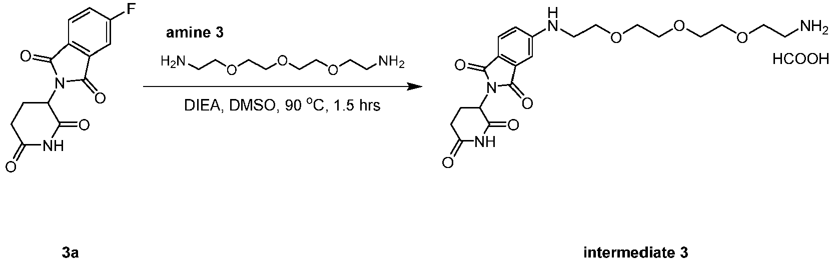

A solution of compound **3a** (70 mg, 253 umol), **amine 3** (80 mg, 416 umol) and DIEA (222 mg, 1.72 mmol, 300 uL) in DMSO (3 mL) was stirred at 90 °C under N₂ for 1.5 hrs. LCMS (Rt = 0.687 min, MS + 1 = 449.2) showed MS of **intermediate 3** was detected. The mixture was cooled to 25 - 30 °C, pH value of the mixture was adjusted to 6 - 7 with AcOH. The mixture was purified by Prep-HPLC (column: Phenomenex Synergi C18 150*25*10m; mobile phase: [water (0.225%FA) - ACN]; B%: 0% - 30%, 10 min), the fraction was concentrated under reduced pressure to give **intermediate 3** (50 mg, 96.4 umol, 12.7% yield, 95.3% purity, FA) as brown oil, which was confirmed by LCMS (Rt = 0.698 min, MS + 1 = 449.3).

**LCMS:** (Method 1), Rt = 0.687 min, MS + 1 = 449.2

### General procedure for preparation of the title compound "A42"

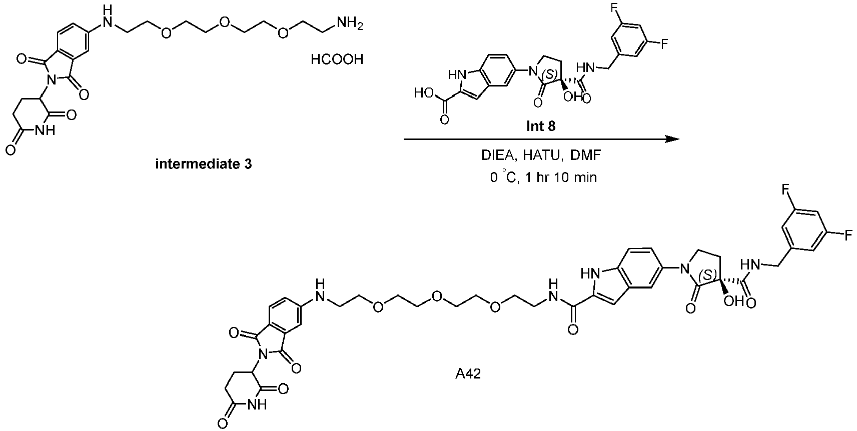

To a solution of **Int 8** (35 mg, 81.5 umol) in DMF (2 mL) was successively added DIEA (30 mg, 232 umol) and HATU (35 mg, 92.0 umol) at 0 °C under N₂, the mixture was stirred at 0 °C for 10 min, then a solution of **intermediate** 3 (40 mg, 80.8 umol, 1.00 eq, FA) in DMF (2.00 mL) was added into the mixture at 0 °C, it was stirred for 1 hr under N ₂ at 0 °C. The mixture was poured into icy water (20.0 mL), extracted with ethyl acetate (10.0 mL x 2), the organic phase was separated and washed with brine (20.0 mL x 2), dried with Na₂SO₄, filtered, the filtrate was concentrated under reduced pressure to give brown oil. It was purified by Prep-HPLC (column: Phenomenex Synergi C18 75*30*3um;mobile phase: [water(0.225%FA)-ACN];B%: 31%-51%,8min), solvent of the fraction was removed by lyophilization to **A42** (23 mg, 24.52 umol, 30.32% yield) as yellow solid, which was confirmed by ¹H-NMR, LCMS and HPLC.

**¹H NMR:** 400 MHz, DMSO-*d*₆;

11.62 (s, 1H), 11.05 (s, 1H), 8.70 ( t, *J* = 6.4 Hz, 1H), 8.60-8.52 (m, 1H), 8.42 (s, 1H), 7.78 (s, 1H), 7.58-7.50 (m, 2H), 7.42 (d, *J* = 8.9 Hz, 1H), 7.18-7.10 (m, 2H), 7.09-6.96 (m, 4H), 6.87 (d, *J =* 8.4 Hz, 1H), 6.72 (s, 1H), 5.02 (d, *J* = 8.8 Hz, 1H), 4.41 (d, *J* = 7.6 Hz, 1H), 4.26 (d, *J* = 8.8 Hz, 1H), 3.89 (t, *J* = 7.2 Hz, 2H), 3.59-3.49 (m, 15H), 2.92-2.81 (m, 1H), 2.65-2.58 (m, 3H), 2.19-2.10 (m, 1H), 2.03-1.95 (m, 1H), 1.23 (s, 1H)

**LCMS:** Rt = 0.720 min, MS + 1 = 860.2;

**HPLC:** Rt = 1.860 min

### 5-[(3S)-3-{[(3,5-difluorophenyl)methyl]carbamoyl}-3-hydroxy-2-oxopyrrolidin-1-yl]-N-{15-[2-(2,6-dioxopiperidin-3-yl)-1,3-dioxo-2,3-dihydro-1H-isoindol-5-yl]-3,6,9,12-tetraoxapentadec-14-yn-1-yl}-1H-indole-2-carboxamide ("A43")

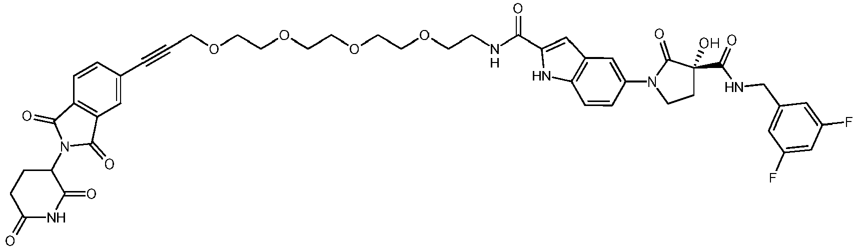

### LC-MS or HPLC Method:

### Method 1:

MS instrument type: SHIMADZU LCMS-2020, Column: Kinetex EVO C18 30*2.1mm, 5um, mobile phase A: 0.0375% TFA in water (v/v), B: 0.01875% TFA in Acetonitrile (v/v), gradient: 0.0 min 0% B→ 0.8 min 95% B→1.2 min 95% B→1.21 min 5% B→1.55 min 5% B, flow rate: 1.5 mL/min, oven temperature: 50 °C; UV detection: 220 nm & 254 nm.

### Method 2:

MS instrument type: SHIMADZU LCMS-2020, Column: Kinetex EVO C18 30*2.1mm, 5um, mobile phase A: 0.0375% TFA in water (v/v), B: 0.01875% TFA in acetonitrile (v/v), gradient: 0.0 min 0% B→-3.0 min 95% B→3.5 min95% B→3.51 min 5% B→-4.0 min 5% B, flow rate: 0.8 mL/min, oven temperature: 50 °C; UV detection: 220 nm & 254 nm.

### General procedure for preparation of compound 9c

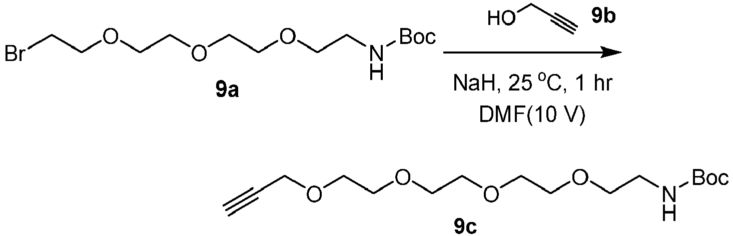

To a solution of Compound **9b** (52 mg, 927 umol) in DMF (4 mL) was added NaH (70 mg, 1.75 mmol, 60% purity) at 20-25 °C unde r N₂ and stirred for 30 mins, then Compound **9a** (300 mg, 842 umol, 1.00 eq) was added under N₂ at 20-25 °C, after addition, the mixture solution was stirred at 20-25 °C for 1 hr. TLC (Petroleum ether: Ethyl acetate = 1:1) showed the Compound **9a** was consumed up (Rf = 0.60), a new spot was detected (Rf = 0.40). The reaction solution was poured into water (10 mL), then it was extracted with ethyl acetate (10 mL*2), the combined organic layer was washed with brine (20 mL), then it was dried over Na₂SO₄, filtrated and concentrated under reduce pressure to afford the crude product. The crude product was purified by silica gel column chromatography (SiO₂, Petroleum ether: ethyl acetate = 20:1-10:1-3:1, the spot (Rf = 0.40) was collected). Compound **9c** (200 mg, 603 umol, 71.7% yield) was obtained as a colorless oil, which was confirmed by ¹H-NMR.

**¹H NMR:** 400 MHz, CDCl₃

δ 5.09 (s, 1H), 4.23 (d, *J* = 2.4 Hz, 2H), 3.61-3.76 (m, 12H), 3.56 (t, *J* = 5.2 Hz, 2H) 3.34 (d, *J* = 5.2 Hz, 2H), 2.50-2.53 (m, 1H), 1.47 (s, 9H).

### General procedure for preparation of compound 9d

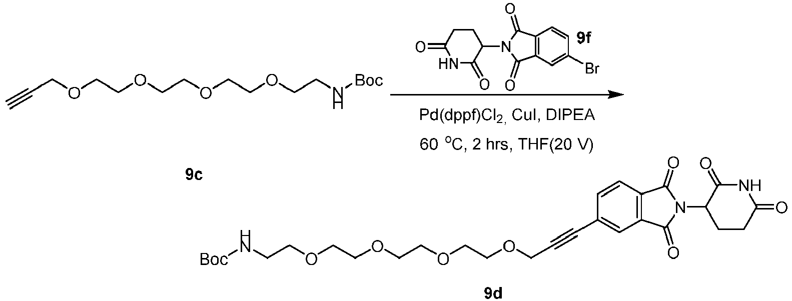

To a solution of Compound **9f** (187 mg, 554 umol) in THF (5 mL) was added Cul (15 mg, 78.7 umol) and DIPEA (585 mg, 4.53 mmol) at 20-25 °C under N₂, then Pd(dppf)Cl₂•CH₂Cl₂ (38 mg, 46.5 umol) and Compound **9c** (150 mg, 452 umol) was added to the solution at 20-25 °C und er N₂, then it was stirred at 60-65 °C for 2 hrs. LCMS showed the MS of Comp ound **9d** was detected (RT = 0.903 min M/Z+1 = 588), TLC (Petroleum ether: Ethyl acetate = 0:1) showed the Compound **9c** (Rf = 0.67) was consumed up, a new spot was detected (Rf = 0.30). The reaction solution was cooled to 20-30 °C, then it was quenched with acetic acid to pH = 3-4 and poured into water (10 mL), then extracted with ethyl acetate (10 mL*2), the combined organic layer was washed with brine (5 mL) dried over Na₂SO₄, filtrated and concentrated under reduce pressure to afford the crude product. Then it was purified by silica gel column chromatography (SiO₂, Petroleum ether: ethyl acetate = 10:1-5:1-0:1, the spot (Rf = 0.30) was collected). Compound **9d** (90 mg, 145 umol, 32.1% yield) was obtained as a colorless oil, which was confirmed by LCMS, RT = 0.897 min, M/Z+1 - 100 = 488), ¹H-NMR.

**¹H NMR:** 400 MHz, CDCl₃

δ 7.94 (s, 1H), 7.81-7.88 (m, 1H), 7.70-7.79 (m, 1H), 7.26-7.48 (m, 1H), 4.91 (dd, *J* = 12.4, 5.2 Hz, 1H), 4.94-5.00 (m, 1H), 4.40 (s, 1H), 4.21 (s, 1H), 4.13 (s, 1H), 3.61-3.75 (m, 12H), 3.55 (t, *J* = 5.2 Hz, 2H), 3.26-3.36 (m, 2H), 2.71-2.95 (m, 2H), 2.13-2.23 (m, 1H), 1.37 (s, 9H).

**LCMS:** (Method 1), RT = 0.903 min, M/Z+1 = 588

### General procedure for preparation of compound 9e

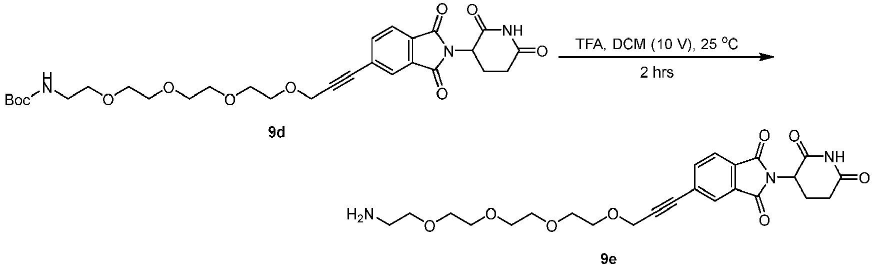

To a solution of Compound **9d** (90 mg, 153 umol) in DCM (3 mL) was added drop wise TFA (174 mg, 1.53 mmol) at 20-25 °C, afte r addition, the mixture was stirred at 20-25 °C for 2 hrs. TLC (Petroleum ether: Ethyl acetate = 0: 1) showed the Compound **9d** was consumed up (Rf = 0.60), a new spot was detected (Rf = 0.00). The reaction solution was concentrated under reduce pressure at 30 °C to afford Compound **9e** (90 mg, 110 umol, 72.3% yield, TFA) as a brown oil, which was confirmed by LCMS, RT = 0.746min, M/Z+1 = 488).

**LCMS:** (Method 1), RT = 0.746min, M/Z+1 = 488

### General procedure for preparation of title compound "A43"

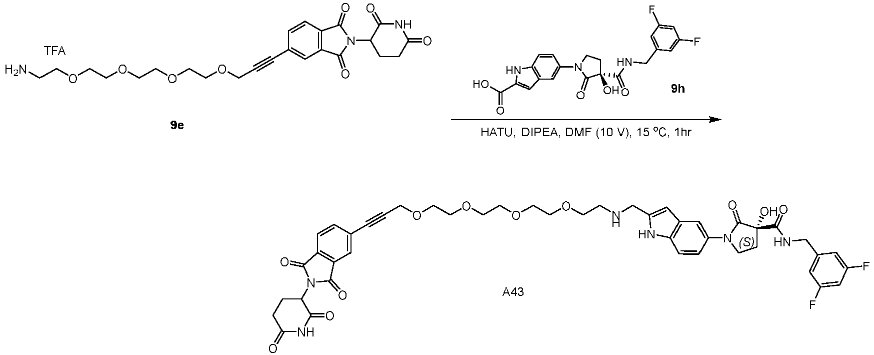

To a solution of Compound **9h** (25 mg, 58.2 umol) in DMF (3 mL) was added DIPEA (38 mg, 294 umol) and HATU (34 mg, 89.4 umol) at 10-15 °C under N₂, then Compound **9e** (42 mg, 69.8 umol, TFA) dissolved in DMF (1 mL) was added to the solution at 10-15 °C, after addition, it was stirred at 10-15 °C for 1 hrs. The LCMS showed the MS of Compound 9 was detected (RT = 0.917 min, M/Z+1 = 899). The reaction solution was quenched with AcOH (2 mL), then it was poured into water (10 mL) and extracted with ethyl acetate (10 mL*2), the combined organic layer was washed with brine (20 mL), then dried over Na₂SO₄, filtrated and concentrated under reduce pressure to afford the crude product. The crude was purified by pre-HPLC (column: Phenomenex Gemini-NX C18 75*30 mm*3um; mobile phase: [water (0.1% TFA)-ACN]; B%: 35% -45%, 7 min), the solvent was concentrated under reduce pressure to remove the ACN, then it was extracted with ethyl acetate (10 mL*3) washed with brine (10 mL), then dried over Na₂SO₄, filtrated and concentrated under reduce pressure to afford the title compound (33.7 mg, 35.6 umol, 61.1% yield) as a white solid, which was confirmed by LCMS, RT = 2.199 min, M/Z+1 = 899, ¹H-NMR, HPLC (RT = 1.856 min).

**¹H NMR:** 400 MHz, DMSO-*d*₆

δ 11.62 (s, 1H), 11.14 (s, 1H), 8.48-8.77 (m, 2H), 7.88-8.03 (m, 3H), 7.78 (s, 1H), 7.40-7.59 (m, 2H), 7.00- 7.25 (m, 4H), 6.71 (s, 1H), 5.15-5.28 (m, 1H), 4.37-4.54 (m, 2H), 4.24-4.35 (m, 1H), 3.90 (t, *J* = 6.8 Hz, 3H), 3.63-3.71 (m, 2H), 3.48-3.60 (m, 14H), 3.40 - 3.48 (m, 2H), 2.82-2.99 (m, 1H), 2.59-2.66 (m, 2H), 1.96-2.23 (m, 2H).

**LCMS:** (Method 2), RT = 2.199 min, M/Z+1 = 899

**HPLC:** (Method 2), RT = 1.856 min

### 5-[(3S)-3-{[(3,5-difluorophenyl)methyl]carbamoyl}-3-hydroxy-2-oxopyrrolidin-1-yl]-N-(14-{[2-(2,6-dioxopiperidin-3-yl)-1,3-dioxo-2,3-dihydro-1H-isoindol-4-yl]amino}-3,6,9,12-tetraoxatetradecan-1-yl)-1H-indole-2-carboxamide ("A44")

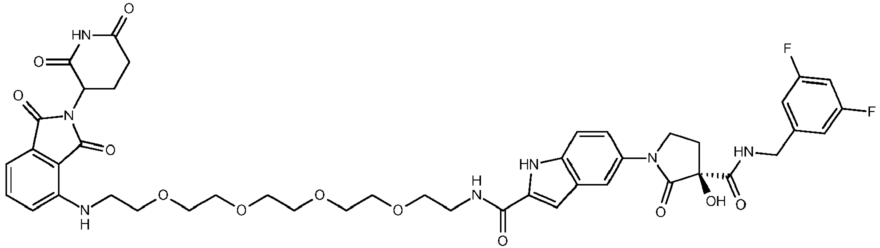

### LC-MS or HPLC Method:

### Method 1:

MS instrument type: SHIMADZU LCMS-2020; column: Kinetex EVO C18 30*2.1mm,5um; mobile phase A: 0.0375% TFA in water (v/v), B: 0.01875% TFA in Acetonitrile (v/v); gradient: 0.0 min 5% B→ 0.80 min 95% B→ 1.20 min 95% B→ 1.21 min 5% B→ 1.55 min 5% B; flow rate: 1.5 mL/min; oven temperature: 50 °C; UV detection: 220 nm & 254 nm.

### Method 2:

HPLC instrument type: SHIMADZU LC-20AB; column: Kinetex C18 LC Column 4.6X50mm,5um; mobile phase A: 0.0375% TFA in water (v/v), B: 0.01875% TFA in Acetonitrile (v/v); gradient: 0.0 min 10% B→ 2.40 min 80% B→ 3.70 min 80% B→ 3.71 min 10% B→ 4.00 min 10% B; oven temperature: 50 °C; UV detection: PDA(220nm&215nm&254nm).

### General procedure for preparation of compound 4a

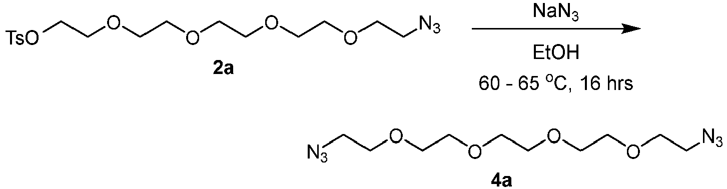

To a solution of compound **2a** (8.00 g, 19.2 mmol) in EtOH (60.0 mL) was added NaN₃ (2.49 g, 38.3 mmol). The mixture was stirred at 60-65 °C for 16 hrs under N₂. TLC (Petroleum ether/Ethyl acetate = 1/2) showed compound **2a** (Rf = 0.75) was consumed up and a new spot (Rf = 0.80) was formed. The reaction mixture was poured into saturated NaHCO₃ aqueous solution (150 mL), then extracted with MTBE (30.0 mL x 5). The organic layer was used directly for next step.

### General procedure for preparation of amine 11

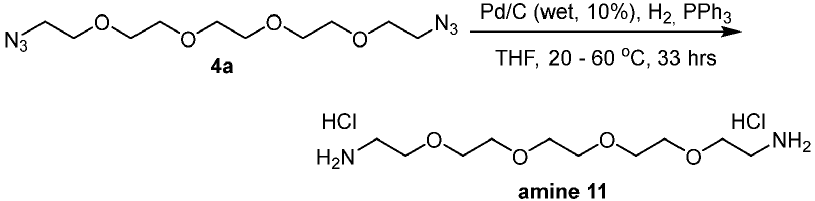

A mixture of compound **4a** (2.76 g, MTBE solution, 9.57 mmol, 75.0 mL), Pd/C (200 mg, wet, 10% purity) in THF (75 mL) was degassed and purged with H₂ (15 psi) for 3 times at 20-25 °C, then the mixture was stirred for 21 hrs while warming to 55-60 °C under H ₂ (15 psi). 1H NMR showed compound **4a** was remained. To the mixture was added PPh₃ (5.50 g, 21.0 mmol, 2.19 eq), the mixture was kept stirring at 35-40 °C for 12 hrs. ¹H-NMR showed compound **4a** was consumed up and the compound **amine 11** was formed. The reaction mixture was filtered over diatomite, the filtrate was concentrated under reduced pressure to give brown residue. Then HCI aqueous solution (1 M, 25 mL) was added into the residue, the mixture was washed with ethyl acetate (40 mL x 2). The aqueous layer was separated and lyophilized to give compound **amine 11** (1.30 g, 4.20 mmol, 2HCl) as yellow solid, which was confirmed by ¹H-NMR.

**¹H NMR:** 400 MHz, DMSO-*d*₆;

δ 8.00 (s, 6H) 3.60-3.65 (m, 4H) 3.51-3.60 (m, 12H) 2.92-3.01 (m, 4H)

### General procedure for preparation of intermediate 11

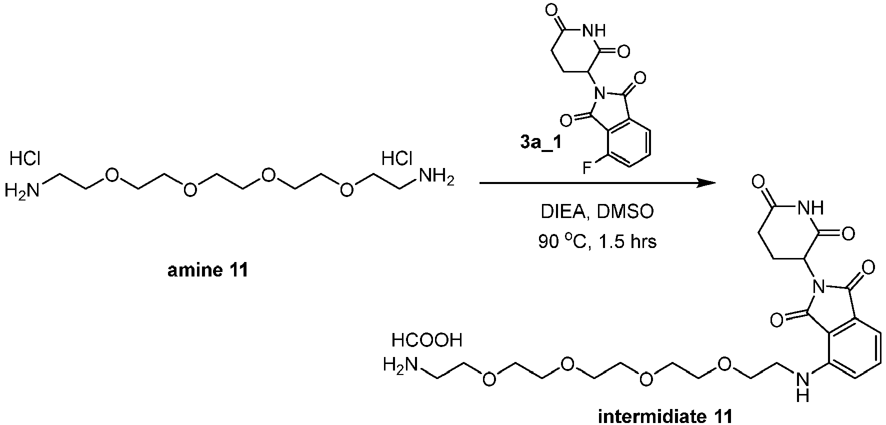

A solution of compound **amine 11** (100 mg, 323 umol, 2HCl), DIEA (222 mg, 1.72 mmol, 300 uL) and compound 3a_1 (80 mg, 289 umol) in DMSO (5.00 mL) was stirred at 90 °C under N ₂ for 1.5 hrs. LCMS showed MS of compound **intermediate 11** (Rt = 0.748 min, MS + 1 = 493.3) was detected. The mixture was cooled to 25-30 °C and pH value was adjusted to 5-6 with AcOH, then purified by prep-HPLC (column: Phenomenex Synergi C18 150 * 25 * 10µm; mobile phase: [water (0.225% FA)-ACN]; B%: 7%-37%,10 min, MS+1 = 493.3). The fraction was concentrated under reduced pressure to give compound **intermediate 11** (45 mg) as brown oil, which was confirmed by next step.

**LCMS:** (Method 1), Rt = 0.748 min, MS+1 = 493.3 ;

### General procedure for preparation of the title compound "A44"

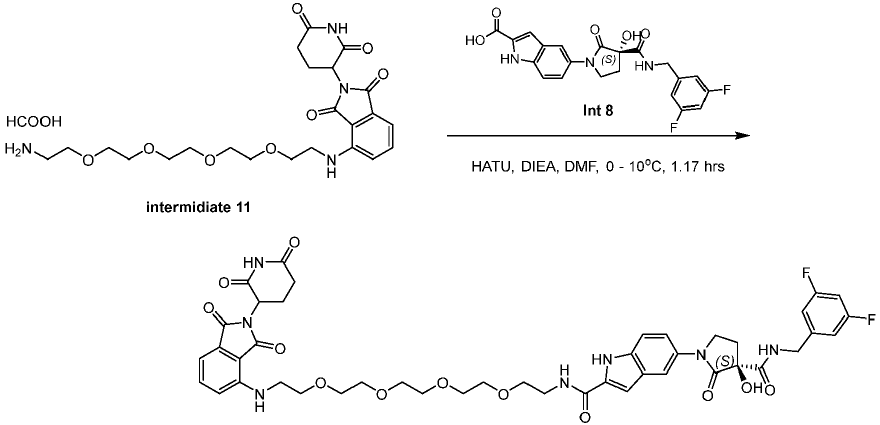

To a solution of compound **Int 8** (36 mg, 83.8 umol) in DMF (1.00 mL) was successively added DIEA (35 mg, 271 umol) and HATU (40 mg, 105 umol) at 0-10 °C under N ₂, the mixture was stirred at 0-10 °C for 10 mins, t hen a solution of compound **intermediate 11** (45 mg, 83.6 umol, FA) in DMF (1.00 mL) was added into the mixture at 0-10 °C, it was stirred for 1 hr under N₂ at 0-10 °C. LCMS showed MS of **A44** (Rt = 0.878 min, MS + 1 = 904.4) was detected. The mixture was poured into icy water (20 mL), extracted with ethyl acetate (10.0 mL x 2), the organic phase was separated and washed with brine (20.0 mL x 2), dried with Na₂SO₄, filtered, the filtrate was concentrated under reduced pressure to give brown oil. It was purified by prep-HPLC (column: Phenomenex Synergi C18 150 * 25 * 10 µm; mobile phase: [water (0.225%FA)-ACN]; B%: 29% -59%,10 min), the fraction was concentrated under reduced pressure to give **A44** (25.88 mg, 99.5% purity) as yellow solid, which was confirmed by ¹H-NMR, ¹⁹F-NMR, LCMS, (Rt = 0.884 min, MS + 1 = 904.4) and HPLC (Rt = 1.974 min).

**¹H NMR:** 400 MHz, DMSO-*d*₆;

δ 11.6 (s, 1H), 10.9-11.3 (m, 1H), 8.69 (t, *J* = 6.0 Hz, 1H), 8.55 (t, *J* = 5.2 Hz, 1H), 7.78 (s, 1H), 7.50-7.61 (m, 2H), 7.42 (d, *J* = 8.8 Hz, 1H), 6.93-7.24 (m, 6H), 6.72 (s, 1H), 6.58 (t, *J* = 4.8 Hz, 1H), 5.00-5.10 (m, 1H), 4.37-4.45 (m, 1H), 4.22-4.30 (m, 1H), 3.89 (t, *J* = 6.4 Hz, 2H), 3.46-3.58 (m, 18H), 2.81-2.93 (m, 2H), 2.58 (d, *J* = 15.2 Hz, 3H), 2.09-2.17 (m, 1H), 1.97-2.07 (m, 1H), 1.23 (s, 1H).

**¹⁹F NMR:** 400 MHz, DMSO-*d*₆;

δ-110 .29

**LCMS:** (Method 1), Rt = 0.878 min, MS + 1 = 904.4;

**HPLC:** (Method 2), Rt = 1.974 min;

### 5-[(3S)-3-{[(3,5-difluorophenyl)methyl]carbamoyl}-3-hydroxy-2-oxopyrrolidin-1-yl]-N-{2-[2-(2,6-dioxopiperidin-3-yl)-1,3-dioxo-2,3-dihydro-1H-isoindol-4-yl]-5,8,11,14,17-pentaoxa-2-azanonadecan-19-yl}-N-methyl-1H-indole-2-carboxamide ("A45")

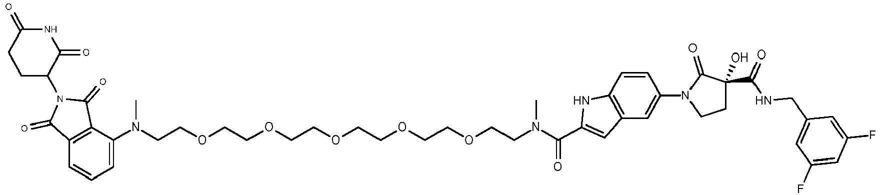

### LC-MS or HPLC Method:

### Method 1:

MS instrument type: SHIMADZU LCMS-2020; column: Kinetex EVO C18 30*2.1mm,5um; mobile phase A: 0.0375% TFA in water (v/v), B: 0.01875% TFA in Acetonitrile (v/v); gradient: 0.0 min 5% B→ 0.80 min 95% B→ 1.20 min 95% B→ 1.21 min 5% B→ 1.55 min 5% B; flow rate: 1.5 mL/min; oven temperature: 50 °C; UV detection: 220 nm & 254 nm.

### Method 2:

HPLC instrument type: SHIMADZU LC-20AB; column: Kinetex C18 LC Column 4.6X50mm,5um; mobile phase A: 0.0375% TFA in water (v/v), B: 0.01875% TFA in Acetonitrile (v/v); gradient: 0.0 min 10% B→ 4.20 min 80% B→ 5.30 min 80% B→ 5.31 min 10% B→ 6.00 min 10% B; oven temperature: 50 °C; UV detection: PDA(220nm&215nm&254nm).

### General procedure for preparation of compound 12b

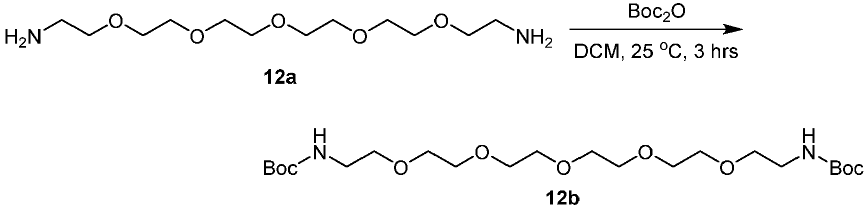

To a solution of compound **12a** (500 mg, 1.78 mmol) in DCM (10 mL) was added Boc₂O (807 mg, 3.70 mmol, 850 uL) at 0-10 °C, the mixtu re was stirred for 3 hrs while warming to 25 °C under N ₂. TLC (Petroleum ether/Ethyl acetate = 0/1) showed compound **12a** (Rf = 0.00) was consumed up and a new spot (Rf = 0.30) was formed. The mixture was poured into water (50 mL), it was extracted with ethyl acetate (20 mL x 3), the organic phase was washed with brine (50 mL x 4), then concentrated under reduced pressure to give brown oil, it was used directly for next step.

### General procedure for preparation of compound 12c

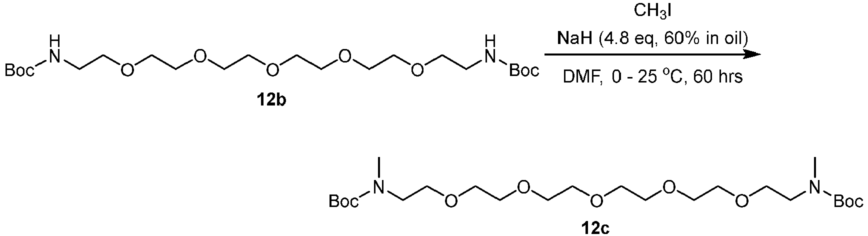

To a solution of compound **12b** (1.00 g, 2.08 mmol) in DMF (10 mL) was added NaH (200 mg, 5.00 mmol, 60% purity) at 0-10 °C under N₂, it was stirred for 1 hr at 0-10 °C under N ₂, then CH₃I (650 mg, 4.58 mmol, 285 uL, 2.20 eq) was added into the mixture, it was stirred for 10 hrs under N₂ while warming to 25 °C. TLC (Petroleum ether/Ethyl aceta te = 0/1) showed compound **12b** (Rf = 0.30) was remain and a new spot (Rf = 0.40) was formed. Then the mixture was cooled to 0-10 °C, NaH (200 mg , 5.00 mmol, 60% purity, 2.40 eq) was added into the mixture under N₂, it was stirred for 1 hr under N₂ at 0-10 °C, then CH ₃I (1.50 g, 10.6 mmol, 660 uL, 5.08 eq) was added into the mixture at 0-10 °C under N ₂, the mixture was stirred for 48 hrs under N₂ while warming to 25 °C. TLC (Petroleum ether/Ethy I acetate = 0/1) showed compound **12b** (Rf = 0.30) was consumed up and a new spot (Rf = 0.40) was formed. LCMS (Rt = 1.183 min, MS + 23 = 531.4) showed MS of compound **12c** was detected. The mixture was poured into water (30 mL), extracted with ethyl acetate (20 mL x 3), the organic phase was washed with brine (50 mL x 2), then separated and dried with Na₂SO₄, filtered, the filtrate was concentrated under reduced pressure to give brown oil. It was purified by column chromatography (SiO₂, Petroleum ether/Ethyl acetate, 1/0-0/1, Rf = 0.40) to give compound **12c** (400 mg, 786 umol) as a colourless oil, which was confirmed by ¹H-NMR.

**¹H NMR:** 400 MHz, CDCl₃;

δ 3.48-3.68 (m, 20H), 3.37 (d, *J* = 2.8 Hz, 4H), 2.89 (s, 6H), 1.43 (s, 18H)

**LCMS:** (Method 1), Rt = 1.183 min, MS + 23 = 531.4;

### General procedure for preparation of compound amine 12

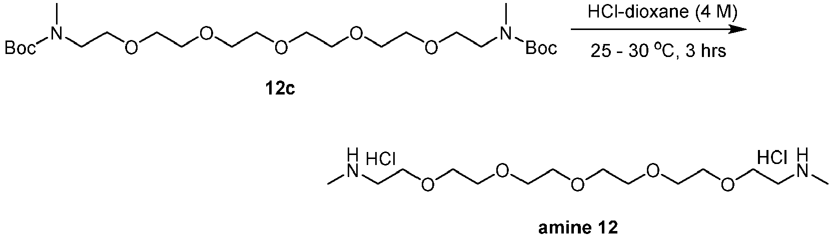

To HCl/dioxane (4 M, 20.0 mL) was added a solution of compound **12c** (400 mg, 786 umol) in dioxane (10 mL) at 25-30 °C under N₂, the mixture was stirred at 25-30 °C under N ₂ for 3 hrs. 300 µL reaction mixture was taken and concentrated under reduced pressure to give brown oil, it was sent for monitoring by ¹H-NMR. ¹H-NMR showed compound **amine 12** was formed. The mixture was concentrated under reduced pressure to give compound **amine 12** as brown oil, it was used directly for next step.

**¹H NMR:** 400 MHz, CDCl₃;

δ 3.84-3.96 (m, 4H), 3.69 (s, 10H), 3.67 (s, 6H), 3.20 (s, 4H), 2.78 (t, *J* = 5.6 Hz, 5H), 2.56 (s, 5H).

### General procedure for preparation of compound intermediate 12

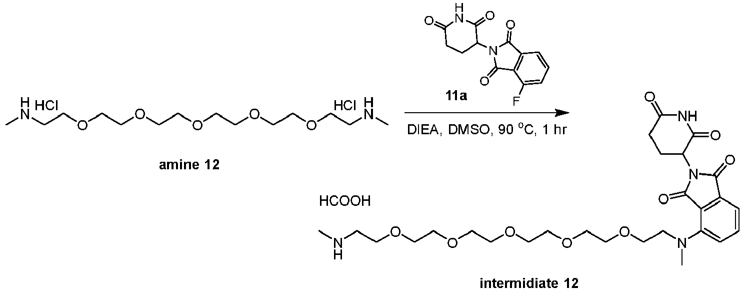

A solution of compound **11a** (50 mg, 181 umol), compound **amine 12** (200 mg, 524 umol, 2HCl) and DIEA (230 mg, 1.78 mmol, 310 uL) in DMSO (3 mL) was stirred at 90 °C for 1 hr under N ₂. LCMS (Rt = 0.770 min, MS + 1 = 565.3) showed MS of compound **intermediate 12** was detected. The mixture was cooled to 25-30 °C, pH value of the mixture was adj usted to 5-6 with AcOH, then it was purified by Prep-HPLC (column: Phenomenex Synergi C18 150 * 25 * 10µm; mobile phase: [water (0.225% FA) - ACN]; B%: 3%-33%, 10min). The reaction was concentrated under reduced pressure to give compound **intermediate 12** (50 mg, 81.9 umol, 45.2% yield, FA) as yellow oil, which was used directly for next step.

**LCMS:** (Method 1), Rt = 0.770 min, MS + 1 = 565.3;

### General procedure for preparation of the title compound "A45"

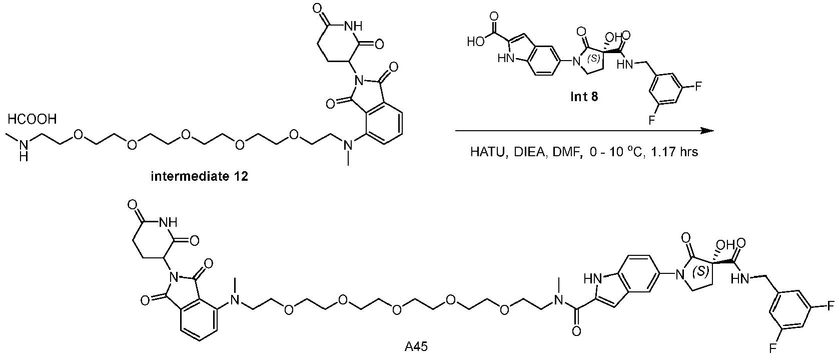

To a solution of compound **Int 8** (30 mg, 70.0 umol) in DMF (1.00 mL) was successively added DIEA (37 mg, 287 umol, 50 uL) and HATU (40 mg, 105 umol) at 0-10 °C under N ₂, the mixture was stirred at 0-10 °C for 10 mins, t hen a solution of compound **intermediate 12** (45 mg, 73.7 umol, FA) in DMF (1.00 mL) was added into the mixture at 0-10 °C, it was stirred for 1 hr under N₂ at 0-10 °C. LCMS (Rt = 0.897 min, MS + 1 = 976.7) showe d MS of **A45** was detected. The mixture was poured into icy water (10 mL), extracted with ethyl acetate (20 mL x 3), the organic phase was washed with brine (20 mL x 3), dried with Na₂SO₄, filtered, the filtrate was concentrated under reduced pressure to give brown oil. It was purified by Prep-HPLC (column: Phenomenex Synergi C18 150 * 25 * 10 µm; mobile phase: [water (0.225%FA)-ACN]; B%: 30% - 60%, 10 min), the fraction was concentrated under reduced pressure to give **A45** (30.51 mg, 31.2 umol, 42.4% yield, 99.9% purity) as yellow solid, which was confirmed by ¹H-NMR, ¹⁹F-NMR, LCMS (Rt = 0.901 min, MS + 1 = 976.5) and HPLC (Rt = 2.761 min).

**¹H NMR:** 400 MHz, DMSO-*d*₆;

δ 11.6 (s, 1H), 11.1 (s, 1H), 8.70 (t, *J* = 6.4 Hz, 1H), 7.78 (s, 1H), 7.52-7.61 (m, 2H), 7.42 (d, *J* = 9.2 Hz, 1H), 7.19-7.30 (m, 2H), 6.95-7.11 (m, 3H), 6.89 (s, 1H), 6.72 (s, 1H), 5.08 (dd, *J* = 13.2, 5.2 Hz, 1H), 4.41 (dd, *J* = 15.6, 6.8 Hz, 1H), 4.21-4.29 (m, 1H), 3.89 (t, *J* = 6.8 Hz, 3H), 3.64 (dd, *J* = 7.6, 4.0 Hz, 10H), 3.53 (d, *J* = 2.8 Hz, 3H), 3.47 (s, 2H), 3.41-3.43 (m, 4H), 3.31 (s, 4H), 3.03 (s, 3H), 2.81-2.95 (m, 3H), 2.58- 2.65 (m, 4H), 2.10-2.18 (m, 1H), 1.96-2.06 (m, 1H).

**¹⁹F NMR:** 400 MHz, DMSO-*d*₆;

δ -110.29

**LCMS:** (Method 1), Rt = 0.897 min, MS + 1 = 976.7;

**HPLC:** (Method 2), Rt = 2.761 min.

### 5-[(3S)-3-{[(3,5-difluorophenyl)methyl]carbamoyl}-3-hydroxy-2-oxopyrrolidin-1-yl]-N-{18-[2-(2,6-dioxopiperidin-3-yl)-1,3-dioxo-2,3-dihydro-1H-isoindol-4-yl]-3,6,9,12,15-pentaoxaoctadec-17-yn-1-yl}-1H-indole-2-carboxamide ("A46")

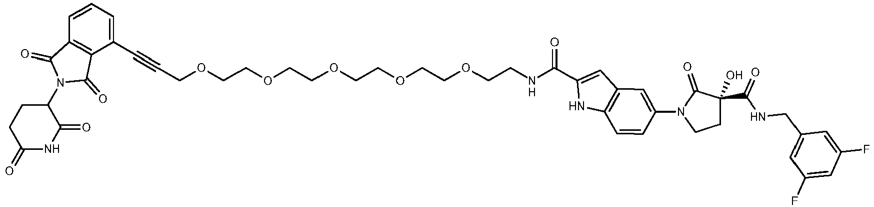

### LC-MS or HPLC Method:

### Method 1:

MS instrument type: SHIMADZU LCMS-2020; column: Kinetex EVO C18 30*2.1mm,5um; mobile phase A: 0.0375% TFA in water (v/v), B: 0.01875% TFA in Acetonitrile (v/v); gradient: 0.0 min 5% B→ 0.80 min 95% B→ 1.20 min 95% B→ 1.21 min 5% B→ 1.55 min 5% B; flow rate: 1.5 (mL/min); oven temperature: 50 °C; UV detection: 220 nm & 254 nm.

### Method 2:

HPLC instrument type: SHIMADZU LC-20AB; column: Kinetex C18 LC Column 4.6X50mm,5um; mobile phase A: 0.0375% TFA in water (v/v), B: 0.01875% TFA in Acetonitrile (v/v); gradient: 0.0 min 10% B→ 4.20 min 80% B→ 5.30 min 80% B→ 5.31 min 10% B→ 6.00 min 10% B; oven temperature: 50 °C; UV detection: PDA(220nm&215nm&254nm).

### General procedure for preparation of compound 14c

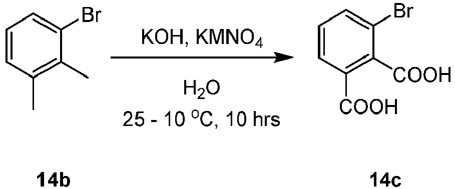

To a solution of compound **14b** (10.0 g, 54.0 mmol, 7.30 mL) and KOH (2.39 g, 36.2 mmol, 85.0% purity) in H₂O (500 mL) was dropwise added a solution of KMnO₄ (40.0 g, 252 mmol) in H₂O (100 mL) at 25-30 °C over 0.5 hr, the mixture was stirred at 100 °C for 9.5 hrs. 0.5 mL mixture was taken and was added into 3 mL water, EtOH (1 mL) was added into the solution, pH value of the mixture was adjusted to 1 with 1 M HCI aqueous solution, extracted with ethyl acetate (1 mL), the organic phase was concentrated under reduced pressure to give white solid, it was sent for monitoring by HNMR and LCMS, ¹H-NMR showed compound **14b** was consumed up and compound **14c** was formed, LCMS showed no MS of compound **14c** was detected. The mixture was cooled to 30 °C, EtOH (200 mL) was dropwise add ed into the mixture at 30 - 40 °C under N ₂, it was stirred for further 1 hr, then the mixture was extracted with ethyl acetate (400 mL x 2), the organic phase was washed with brine (400 mL x 3), dried with Na₂SO₄, filtered, the filtrate was concentrated under reduced pressure to give compound **14c** as white solid, which was confirmed by ¹H-NMR.

**¹H NMR:** 400 MHz, DMSO-*d*₆;

δ 8.00 (dd, J = 7.6, 1.2 Hz, 1H), 7.76 (dd, J = 8.0, 1.2 Hz, 1H), 7.32 (t, *J* = 8.0 Hz, 1H)

### General procedure for preparation of compound 14d

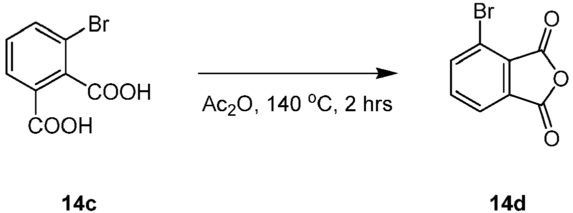

A solution of compound **14c** (9.50 g, 38.8 mmol) in AC₂O (80 mL) was stirred at 140 °C for 2 hrs under N ₂. 0.2 mL mixture was taken and sent for monitoring by ¹H-NMR, ¹H-NMR showed compound **14c** was consumed up and compound **14d** was formed. The mixture was concentrated under reduced pressure to give compound **14d** (8g, crude) as brown solid, which was confirmed by ¹H-NMR.

**¹H NMR:** 400 MHz, DMSO-*d*₆;

δ 8.18 (d, *J* = 8.0 Hz, 1H), 8.06 (d, *J* = 7.6 Hz, 1H), 7.78-7.90 (m, 1H)

### General procedure for preparation of compound 14a

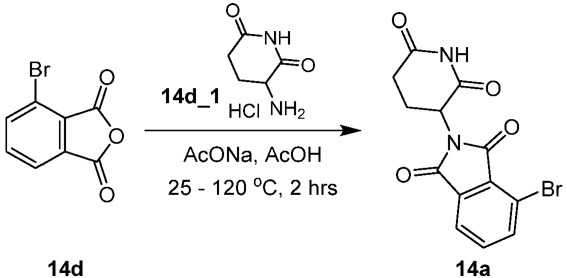

To a solution of compound **14d** (650 mg, 2.86 mmol) and compound **14d_1** (630 mg, 3.83 mmol, HCl) in AcOH (15.0 mL) was added AcONa (380 mg, 4.63 mmol) at 25-30 °C, the mixture was stirred at 120 °C for 2 hrs under N ₂. 0.3 mL mixture was taken and concentrated under reduced pressure to give gray solid, it was sent for monitoring by NMR, ¹H-NMR showed the compound **14a** was formed. The mixture was cooled to 25-30 °C, t hen filtered, the filter cake was collected as gray solid. The solid was poured into 0.05 M HCI aqueous solution (50 mL) and stirred for 0.5 hrs, then filtered, the filter cake was washed with water (20 mL), collected and dried under reduced pressure (-0.09 MPa) at 60 °C to give compound **14a** (800 mg, 2.37 mmol, 82.9% yield) as gray solid, which was confirmed by ¹H-NMR.

**¹H NMR:** 400 MHz, DMSO-*d*₆;

δ 11.15 (s, 1H), 8.06 (d, *J* = 8.0 Hz, 1H), 7.93 (d, *J* = 7.2 Hz, 1H), 7.72-7.82 (m, 1H), 5.17 (dd, *J* = 12.8, 5.4 Hz, 1H), 2.80-2.97 (m, 1H), 2.52 -2.70 (m, 2H), 1.96 - 2.15 (m, 1H).

### General procedure for preparation of compound 14a_1

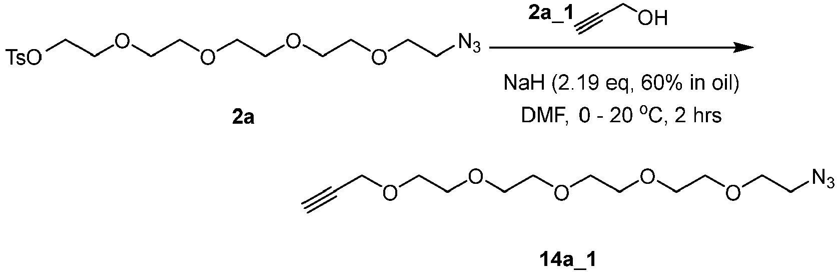

To a solution of compound **2a_1** (1.50 g, 26.7 mmol, 1.58 mL) in DMF (100 mL) was adedd NaH (2.10 g, 52.5 mmol, 60% purity) in batches at 0-10 °C under N₂, it was stirred for 0.5 hr at 0-10 °C under N ₂, compound **2a** (10.0 g, 23.9 mmol) was dropwise added into the mixture at 0-10 °C under N ₂, the mixture was stirred for 1.5 hrs while warming to 15-20 °C under N ₂. LCMS showed no MS of compound ***14a_1*** was detected. 0.5 mL mixture was taken and poured into icy-water (2 mL), extracted with ethyl acetate (2 mL), the organic phase was separated and concentrated under reduced pressure to give colorless oil, ¹H-NMR showed compound **2a** was consumed up and compound **14a_1** was formed. The mixture was poured into water (200 mL), extracted with ethyl acetate (100 mL x 2), the organic phase was washed with brine (100 mL x 3), dried with Na₂SO₄, filtered, the filtrate was concentrated unde reduced pressure to give compound **14a_1** (7.60 g, crude) as brown oil, it was used directly for next step.

**¹H NMR:** 400 MHz, CDCl₃;

δ 4.16 (d, *J* = 2.4 Hz, 2H), 3.59-3.67 (m, 18H),3.35 (t, *J* = 5.2 Hz, 2H), 2.41 (t, *J* = 2.4 Hz, 1H)

### General procedure for preparation of compound 14a_2

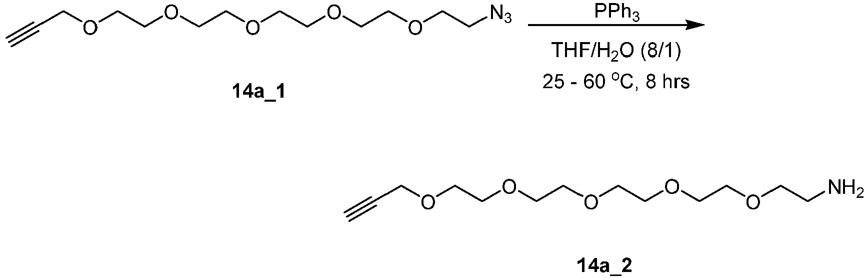

To a solution of compound **14a_1** (1.00 g, 3.32 mmol) in a mixed solvent of THF (16 mL) and H₂O (2 mL) was added PPh₃ (1.74 g, 6.64 mmol) at 25 °C, the mixture was stirred at 60 °C for 8 hrs under N ₂. LCMS (Rt = 1.146 min, MS + 1 = 263.2) showed MS of compound **14a_2** was detected. The mixture was poured into water (20 mL), pH value of the mixture was adjusted to 2-3 with 1 M HCI aqueous solution, it was extracted with ethyl acetate (20 mL x 3), the aqueous solution was separated and pH value was adjusted to 10-11 with saturated Na₂CO₃ aqueous solution. It was concentrated under reduced pressure to give compound **14a_2** (1 g, crude) as brown oil, which was confirmed by ¹H-NMR.

**¹H NMR:** 400 MHz, CDCl₃;

δ 4.89 (s, 2H), 4.13-4.26 (m, 2H), 3.61-3.69 (m, 16H), 3.49 (t, *J* = 5.2 Hz, 2H), 2.77-2.92 (m, 2H), 2.42 (t, *J* = 2.4 Hz, 1H)

**LCMS:** (Method 1), Rt = 1.146 min, MS + 1 = 263.2;

### General procedure for preparation of compound 14a_3

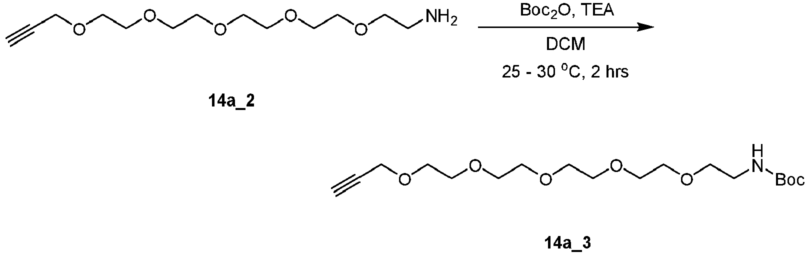

To a solution of **14a_2** (1 g, 3.63 mmol) in DCM (5.00 mL) was successively added TEA (1.10 g, 10.9 mmol, 1.52 mL) and a solution of Boc₂O (950 mg, 4.35 mmol, 1 mL) in DCM (5 mL) at 25-30 °C, the mix ture was stirred at 25-30 °C for 2 hrs under N ₂. 0.5 mL mixture was taken and poured into water (2 mL), extracted with ethyl acetate (1 mL), the organic phase was separated, and washed with brine (2 mL x 3), then separated and concentrated under reduced pressure to give brown oil, ¹H-NMR showed compound **14a_3** was formed. The mixture was poured into water (20 mL), extracted with ethyl acetate (40 mL), the organic phase was separated and washed with brine (30 mL x 3), dried with Na₂SO₄, filtered, the filtrate was concentrated under reduced pressure to give brown oil. TLC (Petroleum ether/Ethyl acetate = 0/1, Rf = 0.70). The oil was purified by column chromatography (SiO₂, Petroleum ether/Ethyl acetate, 1/0-0/1, Rf = 0.70) to give compound **14a_3** (750 mg, 2.00 mmol) as colourless oil, which was confirmed by ¹H-NMR.

**¹H NMR:** 400 MHz, CDCl₃;

δ 5.09 (s, 1H), 4.18 (d, *J* = 2.4 Hz, 2H), 3.59-3.68 (m, 16H), 3.51 (t, *J* = 5.07 Hz, 2H), 3.21-3.35 (m, 2H), 2.41 (t, *J* = 2.4 Hz, 1H), 1.42 (s, 9H).

### General procedure for preparation of compound 14a_4

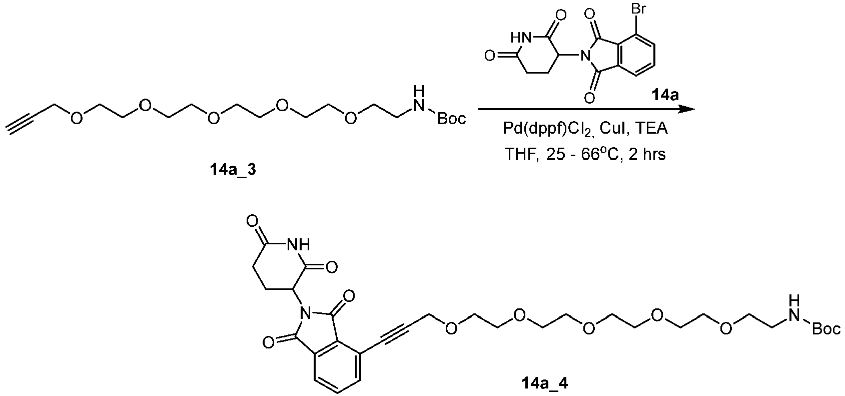

To a solution of compound **14a** (400 mg, 1.19 mmol) and compound **14a_3** (500 mg, 1.33 mmol) in THF (10.0 mL) was successively added DIEA (1.48 g, 11.5 mmol, 2 mL), Cul (50 mg, 262 umol, 0.22 eq) and Pd(dppf)Cl₂ (100 mg, 136 umol) at 25-30 °C, the mixture was stirred at 6 6 °C under N ₂ for 2 hrs. LCMS (Rt = 0.878 min, MS -100 + 1 = 532.3) showed MS of compound **14a_4** was detected. The mixture was cooled to 25-30 °C, then poured into water (30 mL), extracted with ethyl acetate (30 mL x 3), the organic phase was washed with brine (30 mL x 3), then separated and dried with Na₂SO₄, filtered, the filtrate was concentrated under reduced pressure to give brown oil. The oil was purified by Prep-HPLC (reversed phase chromatography ACN (0% - 50%)/H₂O (formic acid 0.1%)), the fraction was concentrated under reduced pressure to give compound **14a_4** (120 mg, 143 umol, 12.1% yield) as brown oil, which was confirmed by LCMS (Rt = 0.878 min, MS - 100 + 1 = 532.3).

**LCMS:** (Method 1), Rt = 0.878 min, MS-100+1 = 532.3;

### General procedure for preparation of compound 14a_5

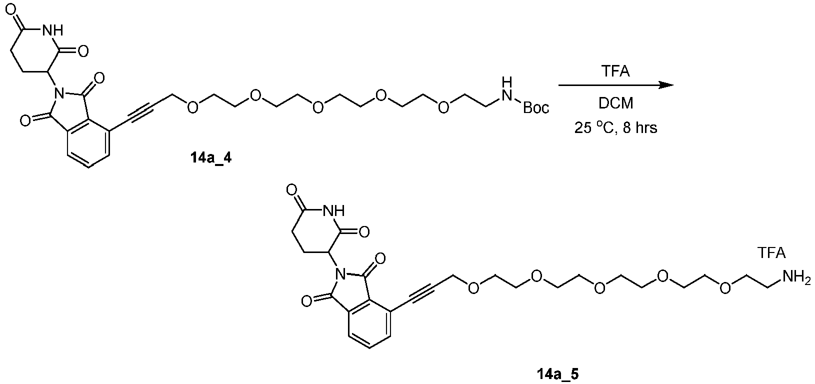

To a solution of compound **14a_4** (120 mg, 143 umol) in DCM (10 mL) was added TFA (1.54 g, 13.5 mmol, 1 mL) at 25 °C, the m ixture was stirred at 25 °C under N ₂ for 8 hrs. LCMS (Rt = 0.737 min, MS1 = 532.3) showed MS of compound **14a_5** was detected. The mixture was concentrated under reduced pressure to give brown oil. The oil was purified by Prep- HPLC (column: Phenomenex luna C18 150*25mm* 10um; mobile phase: [water (0.1% TFA) - ACN]; B%: 12% - 42%, 9 min), the fraction was concentrated under reduced pressure to give compound **14a_5** (50 mg, 77.5 umol, 53.9% yield, TFA) as brown oil, which was confirmed by LCMS (Rt = 0.761 min, MS+1 = 532.2).

**LCMS:** Rt = 0.737 min, MS+1 = 532.3;

### General procedure for preparation of the title compound "A46"

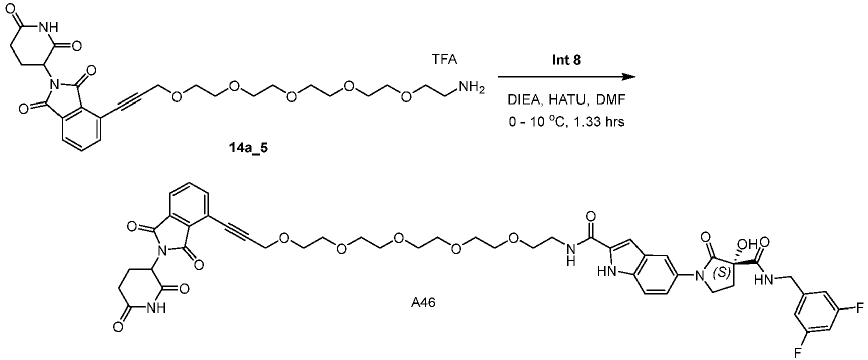

To a solution of compound **Int 8** (30 mg, 69.9 umol) in DMF (2 mL) was successively added DIEA (58 mg, 449 umol, 80 uL) and HATU (46 mg, 121 umol) at 0-10 °C under N ₂, it was stirred at 0-10 °C for 20 min, a solution of compound **14a_5** (50 mg, 85 umol, FA) in DMF (1.00 mL) was added into the mixture at 0-10 °C under N ₂, it was stirred for further 1 hr at 0-10 °C under N₂. LCMS showed the MS value of **the title compound** (Rt = 0.872 min, MS+1 = 943.4) was detected. The mixture was poured into icy water (20 mL), extracted with ethyl acetate (15 mL x 2), the organic phase was washed with brine (30 mL x 3), then separated and dried with Na₂SO₄, filtered, the filtrate was concentrated under reduced pressure to give brown oil. The oil was purified by Prep-HPLC (column: Phenomenex Synergi C18 150*25*10 µm; mobile phase: [water (0.225%FA)-ACN]; B%: 28%- 58%, 10 min), the fraction was concentrated under reduced pressure to give **A46** (13.19 mg, 14.0 µmol, 16.2% yield, 100% purity) as light yellow solid, which was confirmed by ¹H-NMR, ¹⁹F-NMR, LCMS (Rt = 0.885 min, MS+1 = 943.4) and HPLC (Rt = 2.671 min).

**¹H NMR:** 400 MHz, DMSO-*d*₆;

δ 11.6 (s, 1H), 11.1 (s, 1H), 8.69 (t, *J* = 6.4 Hz, 1H), 8.55 (t, *J* = 5.2 Hz, 1H), 7.80-7.97 (m, 3H), 7.77 (s, 1H), 7.48-7.59 (m, 1H), 7.42 (d, *J* = 9.2 Hz, 1H), 6.92-7.17 (m, 4H), 6.71 (s, 1H), 5.00-5.25 (m, 1H), 4.34- 4.56 (m, 3H), 4.20-4.33 (m, 1H), 3.89 (t, *J* = 6.8 Hz, 2H), 3.67-3.76 (m, 2H), 3.43-3.60 (m, 18H), 2.85- 2.97 (m, 1H), 2.56-2.68 (m, 3H), 2.11-2.20 (m, 1H), 2.04 (s, 1H).

**¹⁹F NMR:** 400 MHz, DMSO-*d*₆;

δ -110.29

**LCMS:** (Method 1), Rt = 0.872 min, MS + 1 = 943.4

**HPLC:** (Method 2), Rt = 2.671 min.

### 5-[(3S)-3-{[(3,5-difluorophenyl)methyl]carbamoyl}-3-hydroxy-2-oxopyrrolidin-1-yl]-N-{2-[2-(2,6-dioxopiperidin-3-yl)-1,3-dioxo-2,3-dihydro-1H-isoindol-5-yl]-5,8,11,14-tetraoxa-2-azahexadecan-16-yl}-N-methyl-1H-indole-2-carboxamide ("A47")

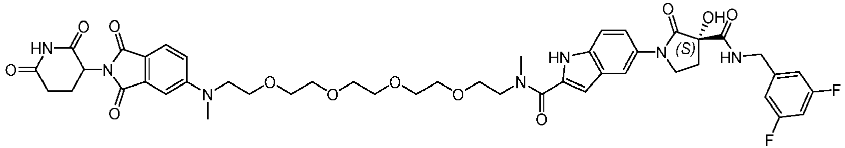

### General procedure for preparation of compound 4f;

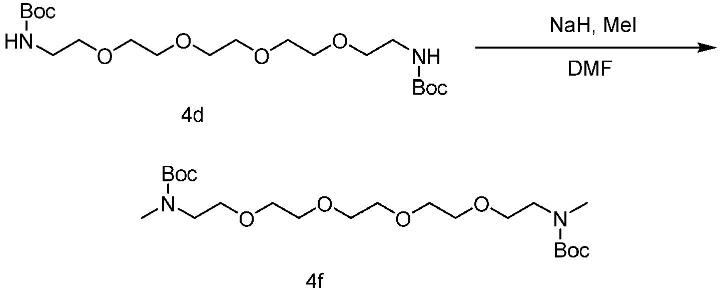

To a solution of compound **4d** (500 mg, 1.15 mmol) in DMF (10 mL) was added NaH (230 mg, 5.75 mmol, 60% purity) at 0-10 °C under N₂, the mixture was stirred for 1 hr at 0-10 °C under N ₂, then CH₃l (600 mg, 4.23 mmol, 263 uL) was drop-wise added into the mixture at 0-10 °C, the mixture was kept stirring for 11 hours under N₂ while warming to 25 °C. The mixture was poured into saturated NH₄Cl aqueous solution (30 mL), extracted with ethyl acetate (15 mL x 2), the combined organic phase was washed with brine (30 mL x3), the organic phase was separated and dried with Na₂SO₄, then filtered, the filtrate was concentrated under reduced pressure to give brown oil. The oil was purified by column chromatography (SiO₂, Petroleum ether/Ethyl acetate = 1/0 - 1/1, Rf = 0.50). Compound **4f** (500 mg, 1.08 mmol, 94% yield) was obtained as yellow oil.

**¹H NMR:** 400 MHz, CDCl₃;

3.67-3.54 (m, 16H), 3.39 (d, *J* = 2.2 Hz, 4H), 2.91 (s, 6H), 1.46 (s, 18H)

### General procedure for preparation of amine 4

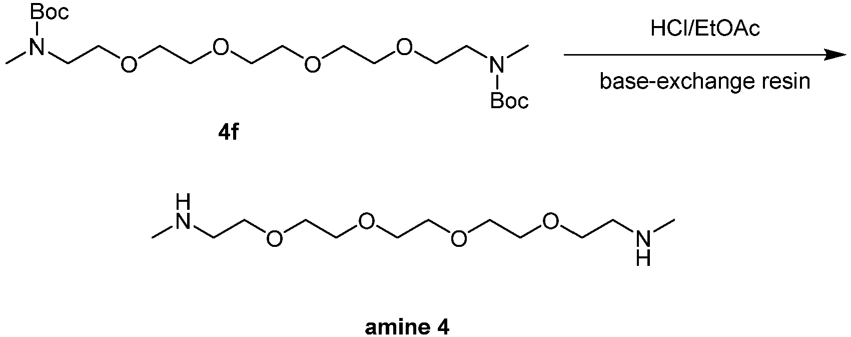

To a solution of compound **4f** (500 mg, 1.08 mmol) in EtOAc (5 mL) was added HCl/EtOAc (4 M, 5 mL) at 0-10 °C under N ₂, the mixture was stirred for 1 hr at 20 °C under N ₂, TLC (Petrleum ether/Ethyl acetate = 1/2) the compound **4f** (R_{f} = 0.4) was consumed up and a new spot (Rf = 0) was formed. The mixture was concentrated, and diluted with MeOH (20 mL), the solution was alkalized by base-exchange resin, filtered and the filtrate was concentrated. Compound **amine 4** (280 mg, crude) was obtained as yellow oil and confirmed with ¹H-NMR.

**¹H NMR:** 400 MHz, MeOH;

δ 3.74 - 3.80 (m, 4 H), 3.68 (d, 10 H), 3.16 - 3.26 (m, 4 H), 2.74 (s, 6 H)

### General procedure for preparation of intermediate 4

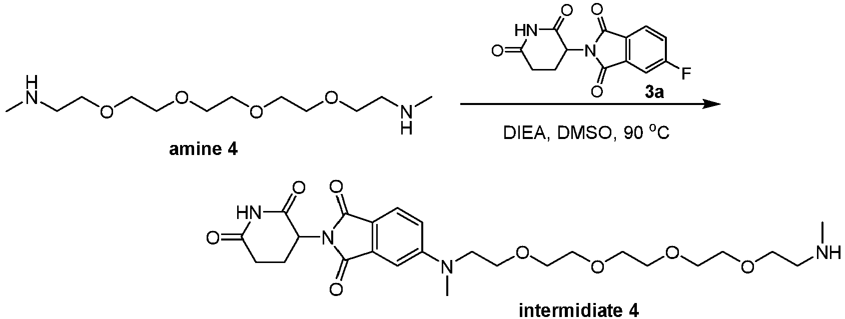

To a solution of **compound 3a** (250 mg, 905 umol) and **amine 4** (250 mg, 946 umol) in DMSO (3 mL) was added DIEA (371 mg, 2.87 mmol, 0.5 mL), and the mixture was stirred at 90 °C for 1 hour. T he reaction was diluted with AcOH (1 mL). The mixture was purified with pre-HPLC (column: Phenomenex Synergi C18 150*25*10 um; mobile phase: [water (0.225% FA)-ACN]; B%: 5%-35%, 10 min). **Intermediate 4** (130 mg, 229.44 umol, 25% yield, FA) was obtained as yellow gum.

**LCMS:** RT = 0.725 min, m/z (M + 1) = 521.2

**¹H NMR:** 400 MHz, DMSO;

δ 8.35 (s, 1H), 7.63 (d, *J* = 8.4 Hz, 1H), 7.11 (d, *J* = 2.0 Hz, 1H), 7.02-6.99 (m, 1H), 5.08-5.03 (m, 1H), 3.71-3.68 (m, 2H), 3.63-3.60 (m, 2H), 3.55 (t, *J* = 5.6 Hz, 2H), 3.50-3.48 (m, 12H), 3.09 (s, 3H), 2.93-2.84 (m, 3H), 2.60-2.54 (m, 2H), 2.42 (s, 3H), 2.01-1.99 (m, 1H).

### General procedure for preparation of "A47"

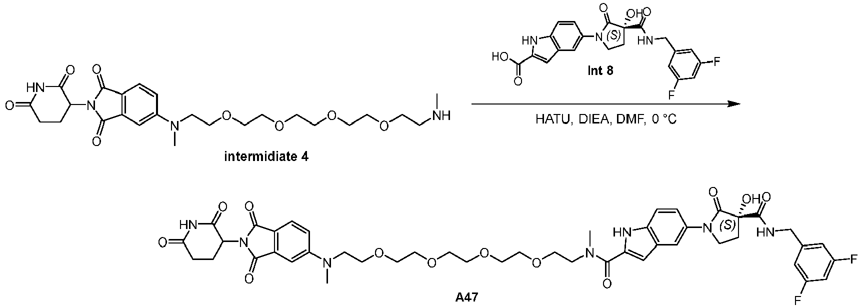

To a solution of **Int 8** (40 mg, 93.2 µmol) in DMF (1 mL) was successively added DIEA (35 mg, 271 µmol) and HATU (38 mg, 99.9 µmol) at 0 °C under N₂, the mixture was stirred at 0 °C for 10 min, then a solution of **intermediate 4** (50 mg, 88.3 µmol) in DMF (1 mL) was added into the mixture at 0 °C, it was stirred for 1 hr under N ₂ at 0 °C. The reaction mixture was poured into ice water (30 mL) and extracted with EtOAc (20 mL*3). The combined organic layer was washed with brine (20 mL), dried over Na₂SO₄, filtered and concentrated. The mixture was purified by Prep-HPLC (column: 3_Phenomenex Luna C18 75*30 mm*3 um; mobile phase: [water (0.225% FA)-ACN]; B%: 32%-62%, 7 min). **"A47"** (30.46 mg, 30.9 umol, 35.1% yield, 99.3% purity, FA salt) was obtained as yellow solid.

**¹H NMR:** 400 MHz, DMSO-*d*₆;

δ 11.56 (s, 1H), 11.18-10.85 (m, 1H), 8.69 (t, *J* = 6.3 Hz, 1H), 7.78 (s, 1H), 7.66-7.51 (m, 2H), 7.42 (d, J = 8.8 Hz, 1H), 7.12-6.95 (m, 5H), 6.89 (s, 1H), 6.72 (s, 1H), 5.07-5.02 (m, 1H), 4.44-4.39 (m, 1H), 4.29-4.23 (m, 1H), 3.89 (s, 2H), 3.80-3.46 (m, 23H), 3.05 (s, 3H), 2.94-2.85 (m, 1H), 2.67 (s, 3H), 2.22-2.12 (m, 1H), 2.03-1.97 (m, 1H).

**LCMS:** RT = 0.908 min, m/z (M + 1) = 932.4;

**HPLC:** RT = 1.948 min.

### (3S)-N-[(3,5-difluorophenyl)methyl]-1-{2-[4-(2-{2-[2-(2-{[2-(2,6-dioxopiperidin-3-yl)-1,3-dioxo-2,3-dihydro-1H-isoindol-5-yl]amino}ethoxy)ethoxy]ethoxy}ethyl)piperazine-1-carbonyl]-1H-indol-5-yl}-3-hydroxy-2-oxopyrrolidine-3-carboxamide ("A48")

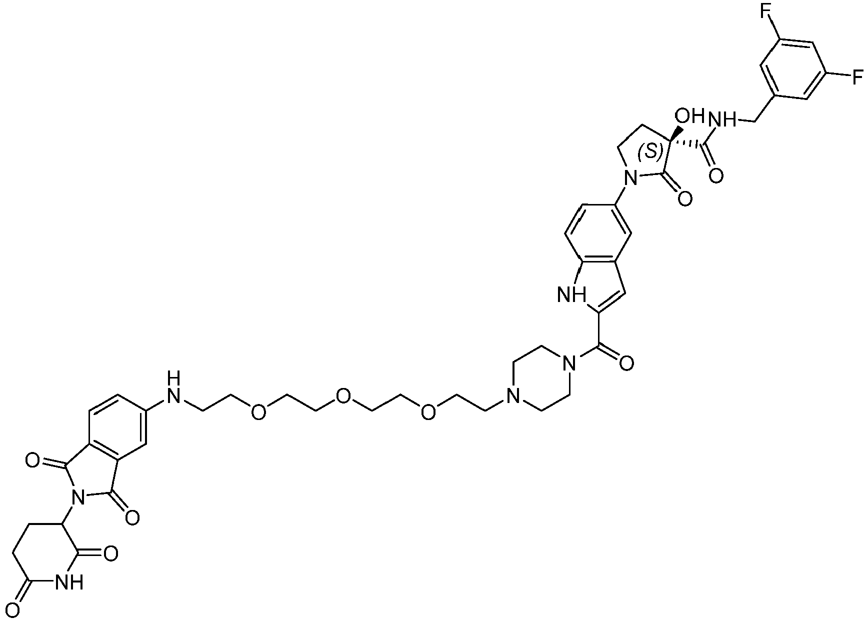

### General procedure for preparation of compound 5b -

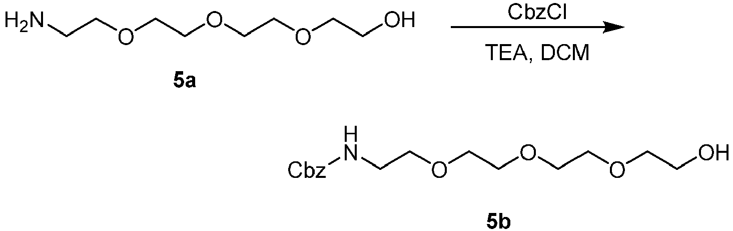

A solution of compound **5a** (5 g, 25.9 mmol) and TEA (2.88 g, 28.5 mmol) in DCM (150 mL) was treated with CbzCl (4.80 g, 28.1 mmol) in one portion and the mixture was stirred at 20 °C for 10 hours. The mixture was concentrated and purified by column chromatography (SiO₂, Petroleum ether/Ethyl acetate = 2/1 to 0/1), (Petroleum ether/Ethyl acetate = 0/1, Rf = 0.4). Compound **5a** (2.50 g, 7.21 mmol, 94.4% purity) was obtained as colorless oil.

**LCMS:** RT = 0.775 min, m/z (M + 23) = 550.3

### General procedure for preparation of compound 5c

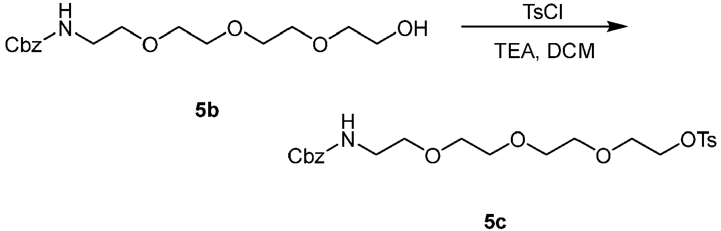

To a solution of compound **5b** (2.50 g, 7.64 mmol) and TEA (2.18 g, 21.5 mmol) in DCM (40 mL) was added 4-methylbenzenesulfonyl chloride (2.20 g, 11.5 mmol) at 20 °C, the mixture was stirred at 40 °C for 10 hours under N₂. The mixture was concentrated. The residue was purified by column chromatography (SiO₂, Petroleum ether/Ethyl acetate = 5/1 to 1/2), (Petroleum ether/Ethyl acetate = 1/2, Rf = 0.5). Compound **5c** (2.00 g, 4.15 mmol, 54% yield) was obtained as yellow oil).

**¹H NMR:** 400 MHz, CDCl₃;

δ 7.79 (d, *J* = 8.2 Hz, 2H), 7.36-7.31 (m, 7H), 5.32 (s, 1H), 5.09 (s, 2H), 4.15-4.11 (m, 2H), 3.66-3.63 (m, 2H), 3.60-3.54 (m, 10H), 3.40-3.36 (m, 2H), 2.44 (s, 3H)

### General procedure for preparation of compound 5d

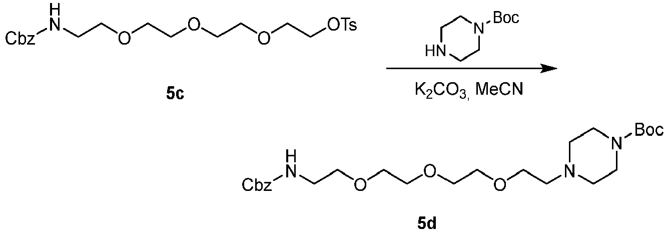

To a solution of compound **5c** (1.50 g, 3.11 mmol) and tert-butyl piperazine-1-carboxylate (600 mg, 3.22 mmol) in MeCN (10 mL) was added Kl (1.03 g, 6.23 mmol) and K₂CO₃ (1.29 g, 9.34 mmol), the mixture was stirred at 70 °C under N ₂ for 12 hours. The mixture was filtered and the filtrate was concentrated. The residue was purified by Reversed-phase chromatographic column (0.1% NH₃.H₂O condition). Compound **5d** (500 mg, 32% yield) was obtained as yellow oil.

**LCMS:** RT = 0.989 min, m/z(M + 1) = 496.5

**¹H NMR:** 400 MHz, CDCl₃;

7.37-7.27 (m, 5H), 5.58 (s, 1H), 5.10 (s, 2H), 3.65-3.55 (m, 12H), 3.44-3.38 (m, 6H), 2.56 (t, *J* = 5.6 Hz, 2H), 2.42 (d, *J* = 4.4 Hz, 4H), 1.46 (s, 9H)

### General procedure for preparation of amine 5-

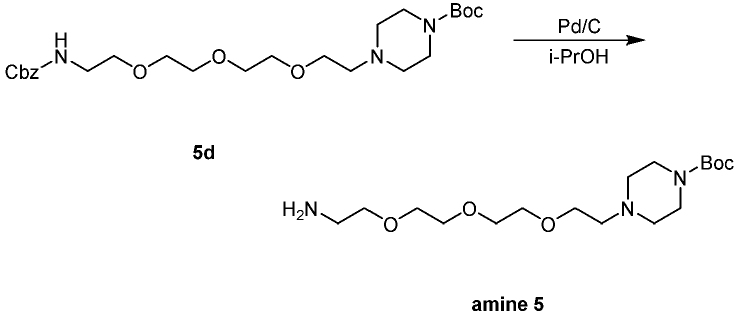

To a solution of compound **5d** (500 mg, 1.01 mmol) in i-PrOH (20 mL) was added Pd/C (100 mg, 10% purity) under N₂, the suspension under vacuum and purge with H₂ three times, then stirred the mixture under H₂ (15 psi) at 25 °C for 10 hours. The mixture was filtered, and the filtrate was concentrated. Compound **amine 5** (360 mg, crude) was obtained as yellow oil.

**¹H NMR:** 400 MHz, CDCl₃;

δ 3.67-3.1 (m, 10H), 3.51 (t, *J* = 5.2 Hz, 2H), 3.44-3.42 (m, 4H), 2.86 (t, *J* = 5.2 Hz, 2H), 2.59 (t, J= 5.8 Hz, 2H), 2.45-2.43 (m, 4H), 1.45 (s, 9H)

### General procedure for preparation of 1;

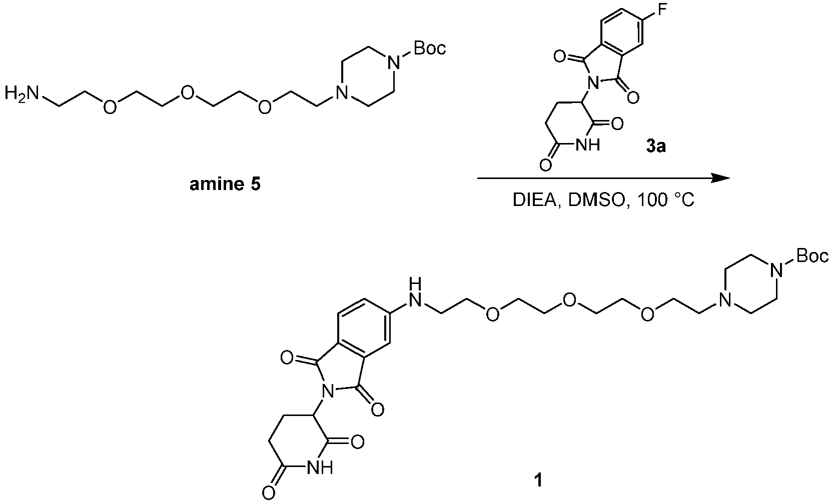

To a solution of compound **3a** (150 mg, 543 µmol) and DIEA (281 mg, 2.17 mmol, 378 µL) in DMSO (3 mL) was added **amine** 5 (350 mg, 968 µmol) at 100 °C, and the mixture was stirred at 100 °C for 8 hours. LCMS showed desired mass (RT = 0.758 min, m/z= 618.3) was detected. The reaction mixture was purified with pre-HPLC (column: Phenomenex luna C 18 150 * 40 mm * 15 um; mobile phase: [water (0.225% FA)-A CN]; B%: 9%-39%, 10 min). Compound 1 (85 mg, 121 µmol, 22% yield, 94.35% purity, FA) was obtained as yellow gum and confirmed with LCMS.

**LCMS:** RT = 0.762 min, m/z (M + 1) = 618.3

### General procedure for preparation of 2

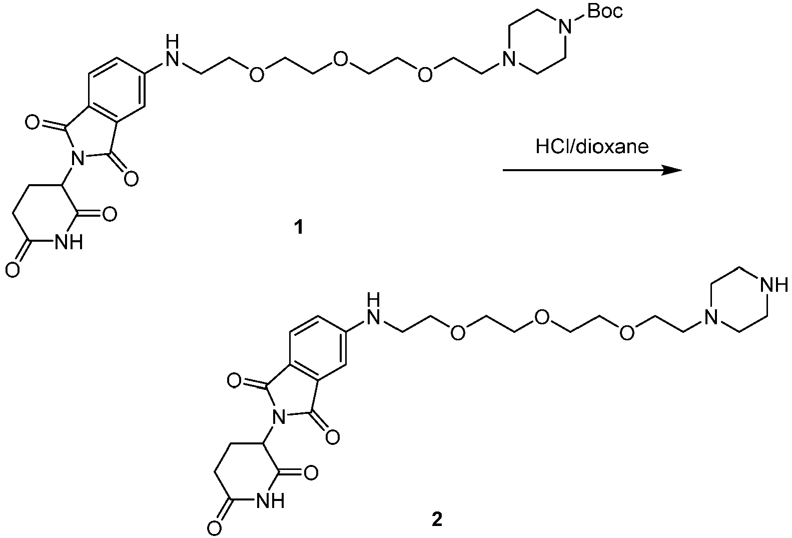

To a solution of compound **1** (80 mg, 130 µmol) in dioxane (2 mL) was added HCl/dioxane (4 M, 2 mL) at 0 °C, then the mix ture was stirred at 20 °C for 10 mins. The reaction mixture was concentra ted. Compound **2** (76 mg, crude, 2HCl) was obtained as yellow gum.

**LCMS:** RT = 0.650 min, m/z (M + 1) = 518.3

### General procedure for preparation of Compound "A48"

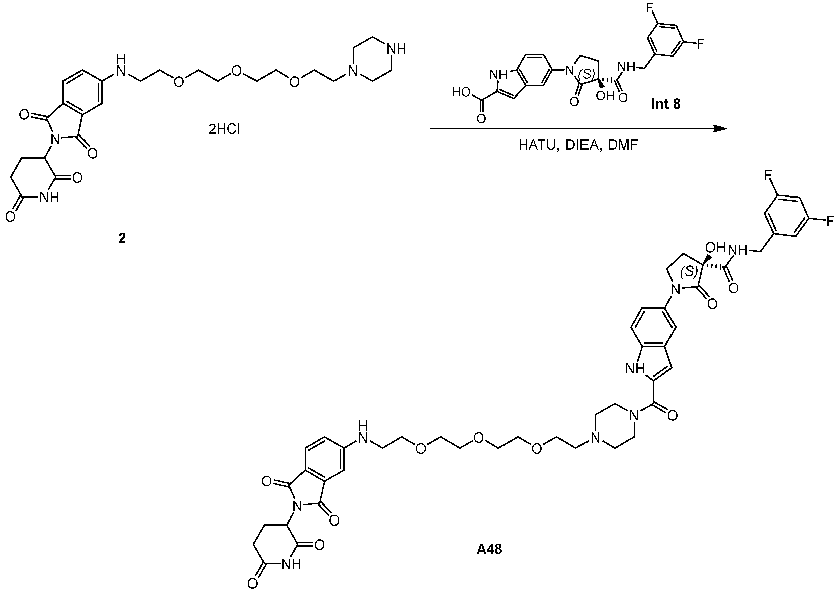

To a solution of **Int 8** (52 mg, 120 µmol) in DMF (1 mL) was successively added DIEA (23 mg, 181 µmol, 31.6 µL) and HATU (59 mg, 155 µmol) at 0 °C under N ₂, the mixture was stirred at 0 °C for 10 min, then a solution of compound **2** (76 mg, 129 µmol, 2HCl) in DMF (1 mL) was added into the mixture at 0 °C, it was stirred for 1 hr under N ₂ at 0 °C. The reaction mixture was poured into ice water (30 mL) and extracted with EtOAc (20 mL*3). The combined organic layer was washed with brine (20 mL), dried over Na₂SO₄, filtered and concentrated. The mixture was purified by Prep-HPLC (column: Phenomenex Luna C 18 150 * 25 mm * 10 um; mobile phase: [water (0.225% FA)-ACN]; B%: 15%-45%, 10 min). "A48" (35.04 mg, 35.3 µmol, 27% yield, 98% purity, FA) was obtained as yellow solid.

**¹H NMR:** 400 MHz, DMSO-*d*₆;

δ 11.61 (s, 1H), 11.05 (s, 1H), 8.69 (t, *J* = 6.4 Hz, 1H), 8.24 (s, 1H), 7.78 (s, 1H), 7.56-7.54 (m, 2H), 7.42 (d, *J* = 8.8 Hz, 1H), 7.14 (t, *J* = 5.6 Hz, 1H), 7.08-6.99 (m, 4H), 6.89 (d, *J =* 8.6 Hz, 1H), 6.78 (s, 1H), 5.05-5.00 (m, 1H), 4.44-4.39 (m, 1H), 4.29-4.24 (m, 1H), 3.90-3.87 (m, 2H), 3.60-3.50 (m, 19H), 3.37-3.24 (m, 2H), 2.91-2.82 (m, 2H), 2.63-2.54 (m, 6H), 2.18-2.13 (m, 1H), 1.99-1.97 (m, 1H)

**LCMS:** RT = 0.818 min, m/z (M + 1) = 932.4;

**HPLC:** RT = 1.487 min.

### (3S)-N-[(3,5-difluorophenyl)methyl]-1-[2-(9-{2-[2-(2-{[2-(2,6-dioxopiperidin-3-yl)-1,3-dioxo-2,3-dihydro-1H-isoindol-5-yl]amino}ethoxy)ethoxy]ethyl}-3,9-diazaspiro[5.5]undecane-3-carbonyl)-1H-indol-5-yl]-3-hydroxy-2-oxopyrrolidine-3-carboxamide ("A49")

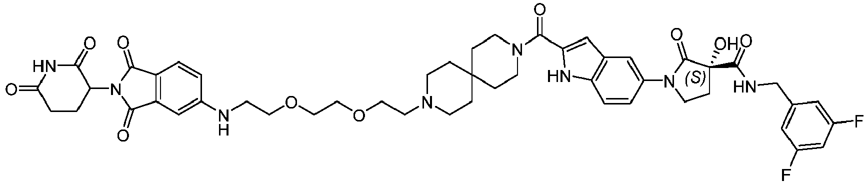

### General procedure for preparation of compound 6b

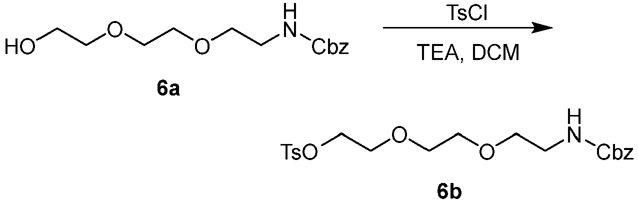

To a solution of compound **6a** (1.00 g, 3.53 mmol) and TEA (1 g, 9.88 mmol, 1.38 mL) in DCM (20 mL) was added 4-methylbenzenesulfonyl chloride (1.01 g, 5.29 mmol) at 20 °C, the mixture was stirred at 40 °C for 10 hrs under N₂. The mixture was concentrated. The residue was purified by column chromatography (SiO₂, Petroleum ether/Ethyl acetate = 5/1 to 1/1), (Petroleum ether/Ethyl acetate = 1:1, Rf = 0.5). Compound **6b** (1.40 g, 3.20 mmol, 91% yield) was obtained as yellow oil and confirmed with next step.

### General procedure for preparation of amine 4

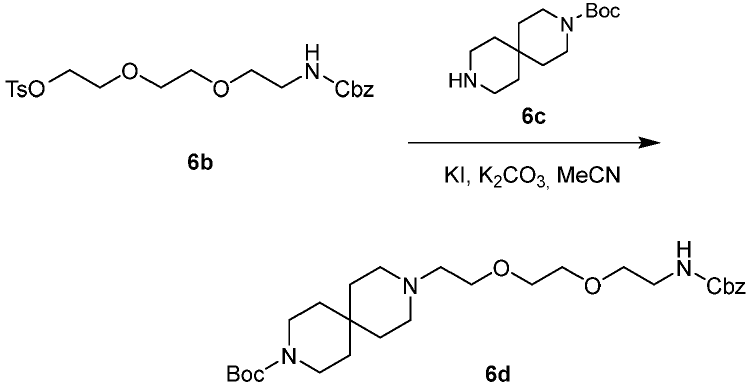

To a solution of compound **6b** (800 mg, 1.83 mmol) and compound **6c** (500 mg, 1.97 mmol) in MeCN (10 mL) was added KI (700 mg, 4.22 mmol) and K₂CO₃ (800 mg, 5.79 mmol), the mixture was stirred at 70 °C under N ₂ for 12 hours. The mixture was cooled to 25 °C, the n poured into water (50 mL), extracted with ethyl acetate (30 mL x 2), the combined organic phase was washed with brine (50 mL x 3), separated and dried with Na₂SO₄, filtered, the filtrate was concentrated under reduced pressure to give brown oil. The oil was purified by reversed phase chromatography (CH₃CN:H₂O (0.1% NH₃.H₂O contained) = 0:1 - 45:1), the fraction was collected and concentrated under reduced pressure to give colourless oil. Compound **6d** (500 mg, 962 µmol, 49% yield) was obtained as yellow oil.

**¹H NMR:** 400 MHz, CDCl₃;

δ 7.42-7.28 (m, 5H), 5.10 (s, 2H), 3.73-3.52 (m, 8H), 3.43-3.27 (m, 6H), 2.61 (t, *J* = 5.4 Hz, 2H), 2.48 (s, 4H), 1.53 (t, *J* = 5.3 Hz, 4H), 1.45 (s, 9H), 1.39 (s, 4H)

### General procedure for preparation of amine 6

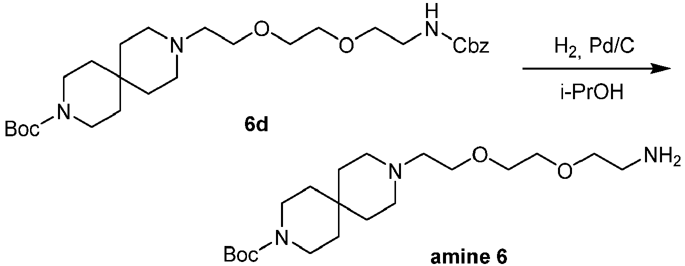

To a solution of compound **6d** (500 mg, 962 µmol) in i-PrOH (20 mL) was added Pd/C (100 mg, 10% purity) under N₂, the suspension under vacuum and purge with H₂ three times, then stirred the mixture under H₂ (15 psi) at 25 °C for 10 h. The mixture was filtered, and the filtrate was concentrated. Compound **amine** 6 (350 mg, 908 µmol, 94% yield) was obtained as yellow oil and confirmed with ¹H-NMR.

**¹H NMR:** 400 MHz, CDCl₃;

δ 3.63-3.58 (m, 6H), 3.55-3.49 (m, 2H), 3.36-3.34 (m, 4H), 2.86 (t, *J=* 5.2 Hz, 2H), 2.59 (t, *J* = 6.0 Hz, 2H), 2.45 (s, 4H), 1.54-1.51 (m, 1H), 1.52 (t, *J* = 5.6 Hz, 3H), 1.44 (s, 8H), 1.42-1.39 (m, 4H)

### General procedure for preparation of compound 1

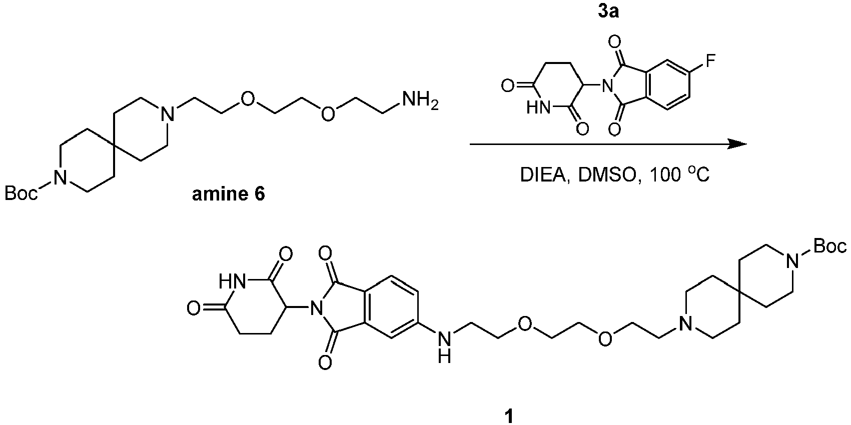

To a solution of compound **3a** (100 mg, 362 µmol) and DIEA (374 mg, 2.90 mmol, 504 µL) in DMSO (1 mL) was added **amine 6** (250 mg, 648 µmol) at 100 °C, and the mixture was stirred at 100 °C for 1 2 hours. The reaction mixture was purified by pre-HPLC (column: Phenomenex luna C18 150 * 25 mm * 10 um; mobile phase: [water (0.225% FA)-ACN]; B%: 15%-45%, 10 min). Compound 1 (90 mg, 127 umol, 35% yield, 91% purity) was obtained as yellow solid.

**LCMS:** RT = 0.636 min, m/z (M + 1) = 642.2

### General procedure for preparation of intermediate 6

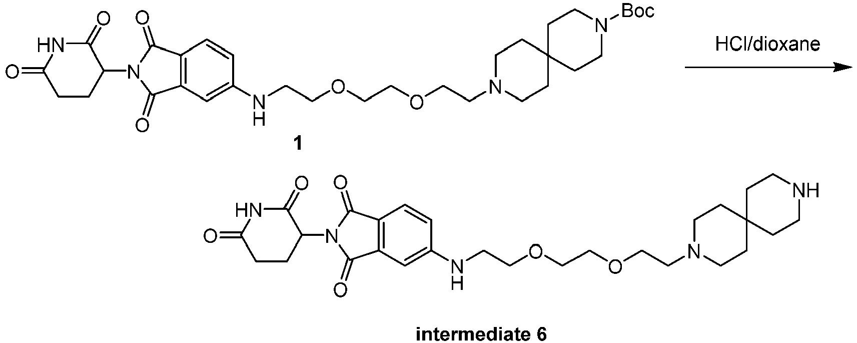

To a solution of compound 1 (80 mg, 106 µmol) in dioxane (2 mL) was added HCl/dioxane (4 M, 1.70 mL) at 0 °C and the mi xture was stirred at 20 °C for 1 hour. The reaction mixture was concent rated. **Intermediate 6** (61 mg, crude, HCl) was obtained as yellow solid and confirmed with next step.

### General procedure for preparation of compound "A49"

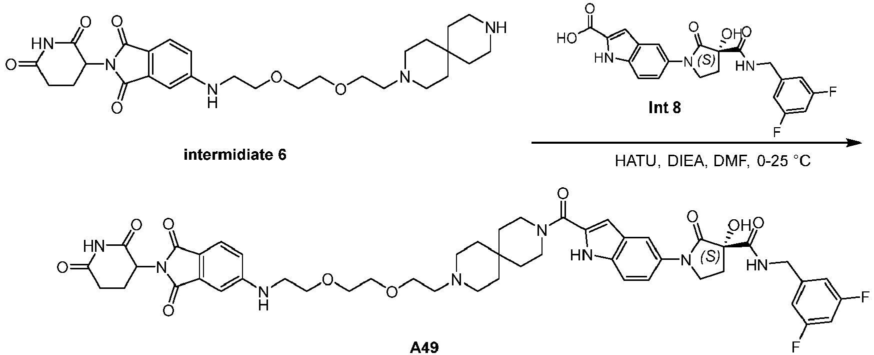

To a solution of **Int 8** (45 mg, 105 µmol) in DMF (1 mL) was successively added DIEA (13.4 mg, 104 µmol, 18.1 µL) and HATU (39.5 mg, 104 µmol) at 0 °C under N ₂, the mixture was stirred at 0 °C for 10 min, then a solution of **intermediate 6** (60 mg, 104 µmol, HCl) in DMF (1 mL) was added into the mixture at 0 °C, it was stirred for 1 hr under N₂ at 0 °C. The reaction mixture was poured into ice water (30 mL) and extracted with EtOAc (20 mL * 3). The combined organic layer was washed with brine (20 mL), dried over Na₂SO₄,filtered and concentrated. The mixture was purified by Prep-HPLC (column: Phenomenex luna C18 150 * 25 mm * 10 um; mobile phase: [water (0.225% FA)-ACN]; B%: 18%-48%, 9 min). **"A49"** (40.4 mg, 38.4 µmol, 37% yield, 95% purity) was obtained as yellow solid.

**¹H NMR:** 400 MHz, DMSO-*d*₆;

δ11.58 (s, 1H), 11.05 (s, 1H), 8.70 (t, *J* = 6.4 Hz, 1H), 8.26 (s, 1H), 7.77 (s, 1H), 7.57-7.52 (m, 2H), 7.41 (d, *J* = 8.8 Hz, 1H), 7.17 (t, *J* = 5.4 Hz, 1H), 7.09-7.00 (m, 4H), 6.90 (d, *J* = 8.8 Hz, 1H), 6.76 (s, 1H), 5.05-5.00 (m, 1H), 4.44-4.39 (m, 1H), 4.29-4.24 (m, 1H), 3.88 (d, *J=* 7.2 Hz, 2H), 3.68-3.59 (m, 14H), 3.38-3.35 (m, 1H), 2.81-2.79 (m, 1H), 2.62-2.57 (m, 5H), 2.46-2.45 (m, 1H), 2.39 (s, 3H), 2.18-2.15 (m, 1H), 1.99-1.96 (m, 1H), 1.46 (s, 8H)

**LCMS:** RT = 0.813 min, m/z (M + 1) = 953.8;

**HPLC:** RT = 1.767 min.

### (3S)-N-[(3,5-difluorophenyl)methyl]-1-{2-[(13-{[2-(2,6-dioxopiperidin-3-yl)-1,3-dioxo-2,3-dihydro-1H-isoindol-5-yl]carbamoyl}tridecyl)carbamoyl]-1H-indol-5-yl}-3-hydroxy-2-oxopyrrolidine-3-carboxamide ("A50")

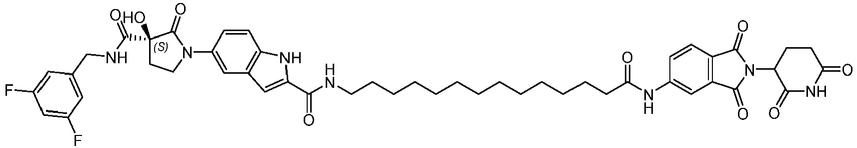

### LC-MS or HPLC Method:

### Method 1:

MS instrument type: SHIMADZU LCMS-2020, Column: Kinetex EVO C18 30*2.1mm, 5um, mobile phase A: 0.0375% TFA in water (v/v), B: 0.01875% TFA in Acetonitrile (v/v), gradient: 0.0 min 0% B→- 0.8 min 95% B→-1.2 min 95% B→-1.21 min 5% B→-1.55 min 5% B, flow rate: 1.5 mL/min, oven temperature: 50 °C; UV detection: 220 nm & 254 nm.

### Method 2:

MS instrument type: SHIMADZU LCMS-2020, Column: Kinetex EVO C18 30*2.1mm, 5um, mobile phase A: 0.0375% TFA in water (v/v), B: 0.01875% TFA in acetonitrile (v/v), gradient: 0.0 min 0% B→-3.0 min 95% B→-3.5 min95% B→-3.51 min 5% B→-4.0 min 5% B, flow rate: 0.8 mL/min, oven temperature: 50 °C; UV detection: 220 nm & 254 nm.

### 4. Experimental for largest scale run:

### General procedure for preparation of compound 1b

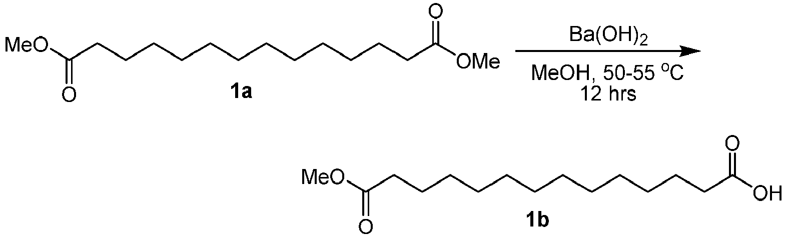

To a solution of compound **1a** (3 g, 10.5 mmol) in MeOH (100 mL) was added Ba(OH)₂ (1.08 g, 6.28 mmol) at 20-25 °C, after addition, t he mixture was stirred at 50-55 °C for 12 hrs. TLC (Petroleum ether: Ethyl acetate = 5:1) showed trace of compound **1a** (Rf = 0.60) was not consumed up completely, a new spot was detected (Rf = 0.10). The reaction solution was filtrated and the filter cake was washed with MeOH (20 mL) and stirred in 1N HCI (50 mL) for 30 mins. Then it was filtrated, the filter cake was concentrated under reduce pressure which was used to next step directly. Compound **1b** (2.30 g, 8.44 mmol, 80.6% yield) was obtained as a white solid, which was confirmed by ¹H NMR.

**¹H NMR:** 400 MHz, CDCl₃

δ 3.69 (d, *J* = 4.0 Hz, 3H), 2.25-2.54 (m, 5H), 1.29 (s, 19H).

### General procedure for preparation of compound 1c

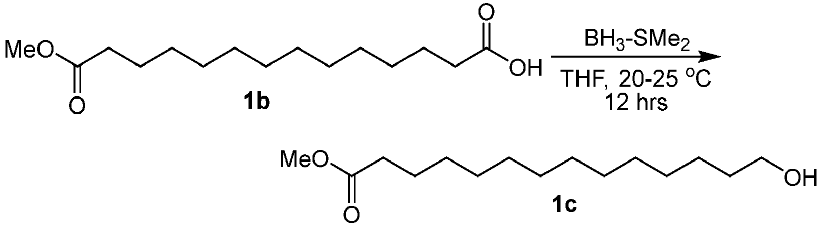

To a solution of compound **1b** (2.30 g, 8.44 mmol) in THF (23 mL) was added BH₃-Me₂S (10 M, 1.01 mL) at 15-20 °C under N ₂, after addition, the mixture was stirred at 20-25 °C for 12 hrs under N ₂. TLC (Petroleum ether: Ethyl acetate = 5:1) showed the compound **1b** (Rf = 0.10) was consumed up, a new spot was detected (Rf = 0.20). The reaction solution was poured into saturated NH₄Cl (50 mL), then it was extracted with ethyl acetate (50 mL*5) and washed with brine (30 mL), dried over Na₂SO₄,filtrated and concentrated under reduce pressure to afford compound **1c** (1.60 g, crude) as a white solid which was used to next step directly.

### General procedure for preparation of compound 1d

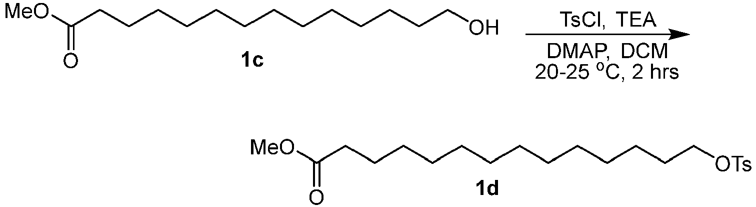

To a solution of compound **1c** (1.60 g, 6.19 mmol) and TsCI (2.40 g, 12.6 mmol) in DCM (50 mL) was added Et₃N (1.92 g, 18.9 mmol) and DMAP (400 mg, 3.27 mmol) at 20-25 °C, after addition ,th e mixture solution was stirred at 20-25 °C for 2 hrs. TLC (Petroleum ethe r: Ethyl acetate = 5:1) showed the compound **1c** (Rf = 0.25) was consumed up, a new spot was detected (Rf = 0.65). The reaction solution was poured into water (200 mL), then it was extracted with ethyl acetate (50 mL*2) and the organic layer was washed with 1 N HCI to pH = 6-7, and washed with brine (50 mL), dried over Na₂SO₄,filtrated and concentrated under reduce pressure to afford the crude product. It was purified by silica gel column chromatography (SiO₂, Petroleum ether: ethyl acetate = 50:1-25:1-10:1), the spot of (Rf = 0.65) was collected. Compound **1d** (2 g, 4.85 mmol, 78.3% yield) was obtained as a white solid, which was confirmed by ¹H NMR.

**¹H NMR:** 400 MHz, CDCl₃

δ 7.81 (d, *J* = 8.4 Hz, 2H), 7.37 (d, *J* = 8.0 Hz, 2H), 4.04 (t, *J* = 6.4 Hz, 2H), 3.69 (s, 3H), 2.47 (s, 3H), 2.32 (t, *J=* 7.6 Hz, 2H), 1.61-1.73 (m, 4H), 1.17 - 1.41 (m, 18H).

### General procedure for preparation of compound 1e

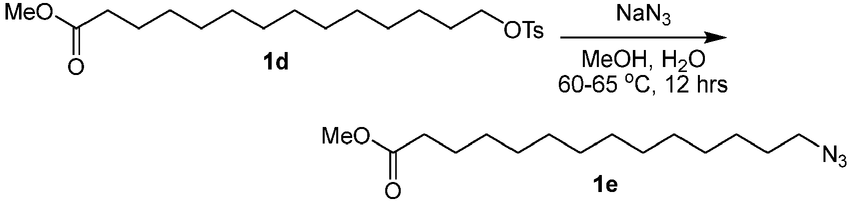

To a solution of compound **1d** (2 g, 4.85 mmol) in MeOH (20 mL) and H₂O (2 mL) was added NaN₃ (470 mg, 7.23 mmol) at 20-25 °C, after addition, the mixture was stirred at 60-65 °C for 12 hrs unde r N₂. The crude ¹H NMR (0.5 mL solution poured into 1 mL water then extracted with 1mL ethyl acetate, then concentrated) showed the compound **1e** was detected. The reaction solution was used to next step directly.

**¹H NMR:** 400 MHz, CDCl₃

δ 3.69 (s, 3H), 3.27 (t, *J* = 7.2 Hz, 2H), 2.32 (t, *J* = 7.6 Hz, 2H), 1.55-1.72 (m, 4H), 1.19-1.44 (m, 18H).

### General procedure for preparation of compound 1f

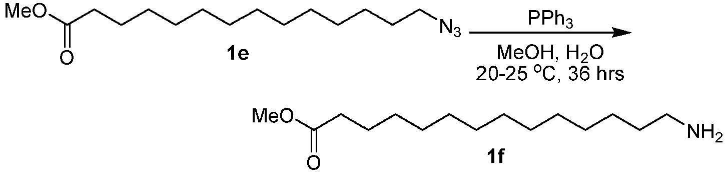

The solution of last step was added PPh₃ (2.41 g, 9.17 mmol) at 20-25 °C, after addition, the mixture solution was stirred at 20-25 °C for 36 hrs. The crude ¹H NMR (0.5 mL reaction solution was poured into 1 mL water then extracted with 1 mL ethyl acetate, then concentrated) showed the compound **1f** was detected. The reaction solution was poured into water (20 mL) and adjust with 1N HCI to pH = 2, then it was extracted with ethyl acetate (20 mL*2), the aqueous phase was adjust with NaHCO₃ solution to pH = 7 and extracted with ethyl acetate (20 mL*2), then it was concentrated under reduce pressure to afford the crude product. Compound **1f** (1 g, 3.88 mmol, 84.7% yield) was obtained as a white solid.

**¹H NMR:** 400 MHz, CDCl₃

δ 3.57 (s, 3H), 2.16-2.29 (m, 4H), 1.44-1.61 (m, 4H), 1.01-1.32 (m, 18H).

### General procedure for preparation of compound 1g

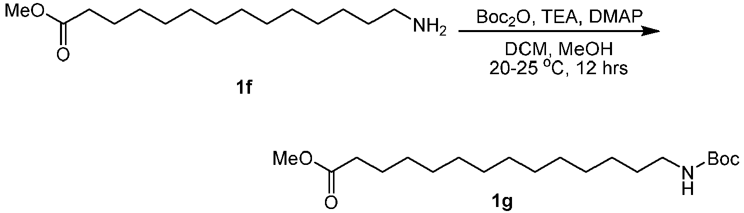

To a solution of compound 1f (1 g, 3.40 mmol) in DCM (10 mL) and MeOH (1 mL) was added Boc₂O (1.11 g, 5.10 mmol), DMAP (84 mg, 687 umol) and Et₃N (1.03 g, 10.2 mmol), then it was stirred at 20-25 °C for 12 hrs. TLC (Petroleum ether: Ethyl acetate = 20:1) showed a new spot was detected (Rf = 0.60), the compound **1f** was consumed up (Rf = 0.10). The reaction solution was poured into water (20 mL) and extracted with ethyl acetate (20 mL*2), then the organic layer was washed with 1 N HCI to pH = 7 and washed with brine (20 mL), dried over Na₂SO₄,filtrated and concentrated under reduce pressure to afford Compound **1g** (900 mg, crude) as a white solid, which was confirmed by ¹H NMR.

**¹H NMR:** 400 MHz, CDCl₃

δ 4.41 (s, 1H), 3.60 (s, 3H), 3.03 (q, *J* = 7.2 Hz, 2H), 2.23 (t, *J* = 7.6 Hz, 2H), 1.50-1.63 (m, 2H), 1.34-1.44 (m, 11H), 1.13-1.29 (m, 18H).

### General procedure for preparation of compound 1h

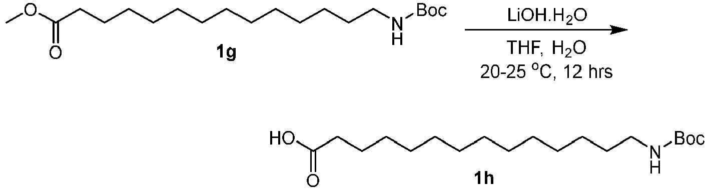

To a solution of compound 1g (900 mg, 2.52 mmol) in THF (10 mL) and H₂O (2 mL) was added LiOH.H₂O (126 mg, 3 mmol) at 20-25 °C, after addition, the mixture was stirred at 20-25 °C for 1 2 hrs. TLC (Petroleum ether: Ethyl acetate = 2:1) showed the compound **1g** was consumed up (Rf = 0.90), a new spot was detected (Rf = 0.30). The reaction solution was poured into water (20 mL) and adjust with 1N HCI to pH = 2, then it was extracted with ethyl acetate (10 mL*2), the organic layer was washed with brine (10 mL),dried over Na₂SO₄,filtrated and concentrated under reduce pressure. The crude was purified by silica gel column chromatography (SiO₂, Petroleum ether: ethyl acetate = 10:1-5:1- 2:1), the spot (Rf = 0.30) was collected to afford Compound **1h** (600 mg, 1.75 mmol, 69.4% yield) as a white solid.

### General procedure for preparation of compound 1m

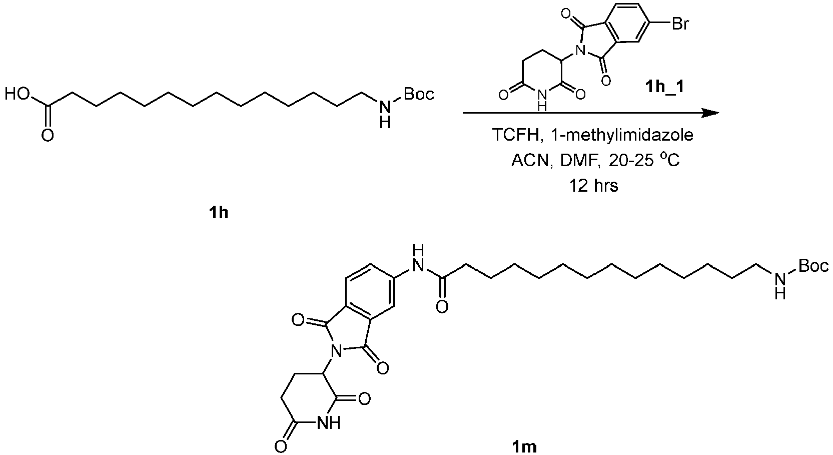

To a solution of compound **1h** (80 mg, 232 umol) and Compound **1h_1** (60 mg, 219 umol) in ACN (4 mL) and DMF (2 mL) was added TCFH (151 mg, 538 umol) and NMI (125 mg, 1.52 mmol) under N₂ at 20-25 °C, after addition, the mixture was stirred at 20-25 °C for 1 2 hrs. The reaction solution was quenched with AcOH (2 mL), then it was poured into water (10 mL) and extracted with ethyl acetate (10 mL*2), the organic layer was washed with brine (10 mL), dried over Na₂SO₄,filtrated and concentrated under reduce pressure. The crude was purified by Pre-HPLC (column: Phenomenex luna C18 150*25mm* 10um; mobile phase: [water (0.1%TFA)-ACN]; B%: 59%-89%, 10 min), then it was concentrated under reduce pressure to afford Compound **1m** (40 mg) as a colorless oil.

**LCMS:** (method 1), RT = 1.125 min, m/z = 543

### General procedure for preparation of compound 1n

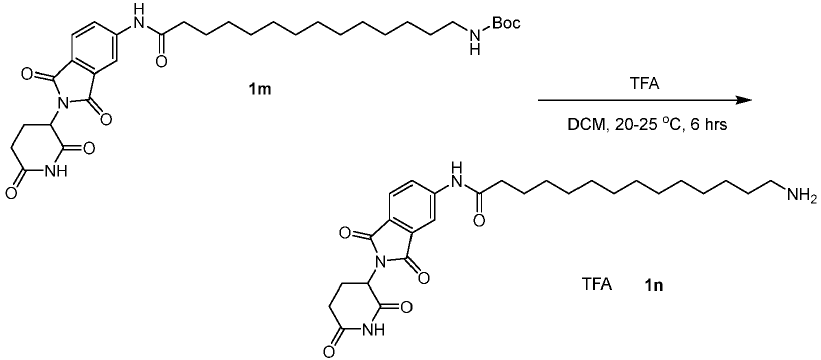

To a solution of compound **1m** (40 mg, 66.8 umol) in DCM (5 mL) was added TFA (76 mg, 666 umol, 10.0 eq) at 20-25 °C, after addition, the mixture was stirred at 20-25 °C for 6 hrs. The LCM S showed the compound **1n** was detected (RT = 0.853 min m/z = 499). The reaction solution was concentrated under reduce pressure to afford compound **1n** (40.0 mg, TFA) as brown oil.

**LCMS:** (method 1), RT = 0.853 min, m/z = 499

### General procedure for preparation of "A50"

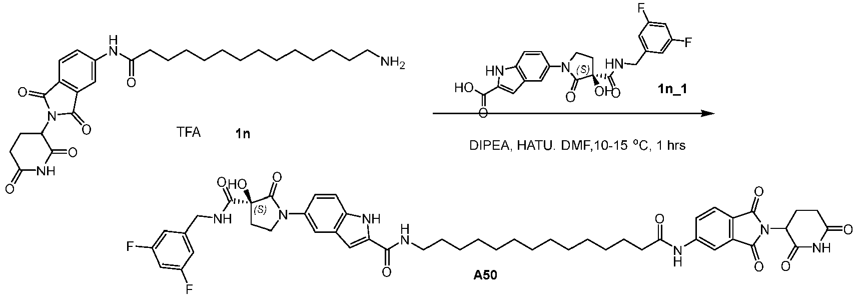

To a solution of compound **1n_1** (25 mg, 58.2 umol) in DMF (3 mL) was added DIPEA (40 mg, 309 umol) and HATU (35 mg, 92.1 umol) at 10-15 °C, after addition, compound **1n** (40 mg, 65.3 umol, TFA) dissolved in DMF (1 mL) was added to the solution at 10-15 °C, then it was stirred at 10-15 °C for 1 hr. The reaction solution was quenched wi th AcOH to pH = 3, then it was poured into water (10 mL), and extracted with ethyl acetate(10 mL*3),the organic layer was washed with brine (10 mL), dried over Na₂SO₄,filtrated and concentrated under reduce pressure. The crude was purified by Pre-HPLC (column: Phenomenex Gemini-NX C18 75*30mm*3um; mobile phase: [water (0.1%TFA)-ACN]; B%: 58%-68%, 7 min), then concentrated under reduce pressure to afford "A50" (27.86 mg, 29.6 umol, 50.9% yield, 96.8% purity) as white solid, which was confirmed by ¹H NMR , LCMS RT = 1.045 min, m/z+1=910), HPLC (, RT = 2.455 min)

**¹H NMR:** 400 MHz, DMSO-*d*₆

δ 11.59 (s, 1H), 11.11 (s, 1H), 10.53 (s, 1H), 8.70 (t, *J* = 6.4 Hz, 1H), 8.46 (t, *J* = 6.4 Hz, 1H), 8.26 (s, 1H), 7.82-7.99 (m, 2H), 7.78 (d, *J* = 1.6 Hz, 1H), 7.52-7.57 (m, 1H), 7.43 (d, *J* = 8.8 Hz, 1H), 6.96-7.15 (m, 4H), 6.71 (s, 1H), 5.05-5.14(m, 1H) 4.39-4.45 (m, 1H), 4.22-4.32 (m, 1H), 3.90 (t, *J* = 6.8 Hz, 2H), 3.22-3.28 (m, 2H), 2.77-2.99 (m, 1H), 2.59-2.70 (m, 2H), 2.38 (t, *J* = 7.6 Hz, 2H), 2.02-2.21 (m, 2H), 1.89-1.93(m, 1H), 1.49-1.74 (m, 4H), 1.22-1.44 (m, 18H).

**LCMS:** (method 1), RT = 1.045 min, m/z = 910

**HPLC:** (method 2), RT = 2.455 min

### 5-[(3S)-3-{[(3,5-difluorophenyl)methyl]carbamoyl}-3-hydroxy-2-oxopyrrolidin-1-yl]-N-{4-[4-(4-{[2-(2,6-dioxopiperidin-3-yl)-1,3-dioxo-2,3-dihydro-1H-isoindol-5-yl]amino}butoxy)butoxy]butyl}-1H-indole-2-carboxamide ("A51")

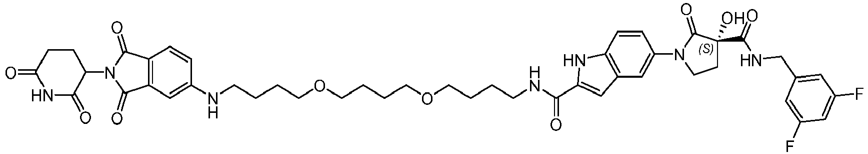

### LC-MS or HPLC Method:

### Method 1:

MS instrument type: SHIMADZU LCMS-2020, Column: Kinetex EVO C18 30*2.1mm, 5um, mobile phase A: 0.0375% TFA in water (v/v), B: 0.01875% TFA in Acetonitrile (v/v), gradient: 0.0 min 0% B→- 0.8 min 95% B→-1.2 min 95% B→-1.21 min 5% B→-1.55 min 5% B, flow rate: 1.5 mL/min, oven temperature: 50 °C; UV detection: 220 nm & 254 nm.

### Method 2:

MS instrument type: SHIMADZU LCMS-2020, Column: Kinetex EVO C18 30*2.1mm, 5um, mobile phase A: 0.0375% TFA in water (v/v), B: 0.01875% TFA in acetonitrile (v/v), gradient: 0.0 min 0% B→-3.0 min 95% B→-3.5 min95% B→-3.51 min 5% B→-4.0 min 5% B, flow rate: 0.8 mL/min, oven temperature: 50 °C; UV detection: 220 nm & 254 nm.

### 4. Experimental for largest scale run:

### General procedure for preparation of compound 7b

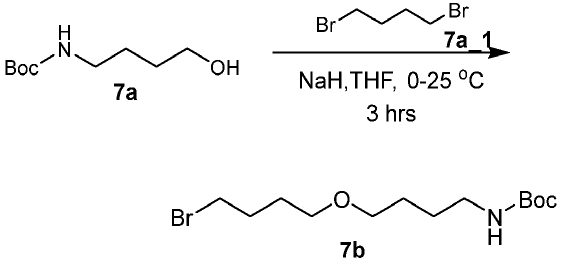

To a solution of compound 7a (1 g, 5.28 mmol) and compound **7a_1** (3.42 g, 15.8 mmol) in THF (10 mL) was added NaH (634 mg, 15.8 mmol, 60% purity) at 0-5 °C under N ₂, then it was stirred at 20-25 °C for 3 hrs under N₂. TLC (Petroleum ether: Ethyl acetate = 5:1) showed compound **7a** was consumed up (Rf = 0.05), a new spot was detected (Rf = 0.40). The reaction solution was poured into water (10 mL), then it was extracted with ethyl acetate (10 mL*2), the combined organic layer was washed with brine (10 mL), dried over Na₂SO₄,filtrated and concentrated under reduce pressure to afford compound 7b (460 mg, 1.42 mmol, 26.8% yield) as a colorless oil, which was confirmed by ¹H NMR.

**¹H NMR:** 400 MHz, CDCl₃

δ 4.58 (s, 1H), 3.29-3.53 (m, 6H), 3.07 (d, *J* = 6.0 Hz, 2H), 1.81-1.95 (m, 2H), 1.61-1.75 (m, 2H), 1.45-1.58 (m, 4H), 1.37 (s, 9H).

### General procedure for preparation of compound 7c

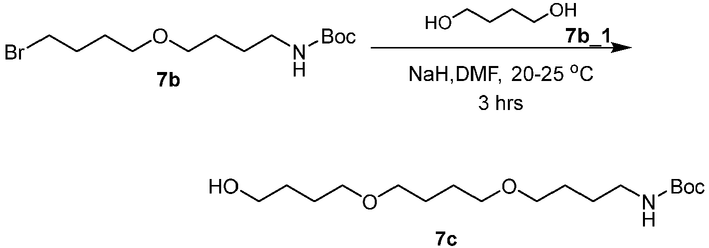

To a solution of compound 7b (350 mg, 1.08 mmol) and compound **7b_1** (486 mg, 5.39 mmol, 476 uL) in DMF (10 mL) was added NaH (560 mg, 14.0 mmol, 60% purity) at 20-25 °C under N ₂ after addition, the mixture was stirred at 20 -25 °C for 1 hr. TLC (Pe troleum ether: Ethyl acetate = 5:1) showed some new spots was detected (Rf = 0.45, 0.10), compound **7b** (Rf = 0.70) was consumed up. The reaction solution was poured into water (100 mL), then it was extracted with ethyl acetate (30 mL*3), the organic layer was washed with brine (30 mL), dried over Na₂SO₄,filtrated and concentrated under reduce pressure. Then it was purified by silica gel column chromatography (SiO₂, Petroleum ether: ethyl acetate = 20:1-10:1-3:1), the spot (Rf = 0.10) was collected. Compound **7c** (230 mg, 689 umol, 63.9% yield) was obtained as a colorless oil, which was confirmed by ¹H NMR.

**¹H NMR:** 400 MHz, CDCl₃

δ 4.65 (s, 1H), 3.54-3.64 (m, 2H), 3.29-3.46 (m, 8H), 3.06 (d, J= 5.6 Hz, 2H), 1.47-1.73 (m, 12H), 1.37 (s, 9H).

### General procedure for preparation of compound 7d

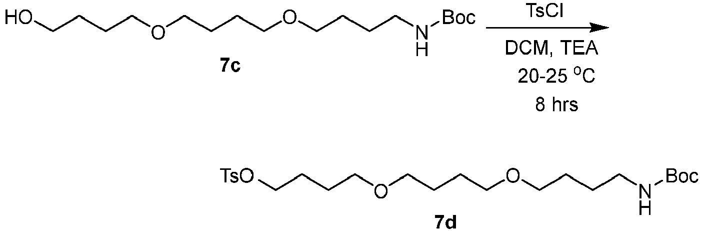

To a solution of compound 7c (230 mg, 689 umol) and TsCI (200 mg, 1.05 mmol) in DCM (5 mL) was added Et₃N (210 mg, 2.08 mmol) at 20-25 °C, after addition, the mixture solution was stirred at 20-25 °C for 8 hrs. TLC (Petroleum ether: Ethyl acetate = 1:1) showed the compound 7c (Rf = 0.10) was consumed up, a new spot (Rf = 0.70) was detected. The reaction solution was poured into water (10 mL), then it was extracted with ethyl acetate (10 mL*2), the combined organic layer was washed with brine (10 mL), dried over Na₂SO₄,filtrated and concentrated under reduce pressure. The crude was purified by silica gel column chromatography (SiO₂, Petroleum ether: ethyl acetate = 20:1-10:1-5:1), the spot (Rf = 0.70) was collected. Compound **7d** (120 mg, 246 umol, 35.6% yield) was obtained as colorless oil, which was confirmed by next step.

### General procedure for preparation of compound 7e

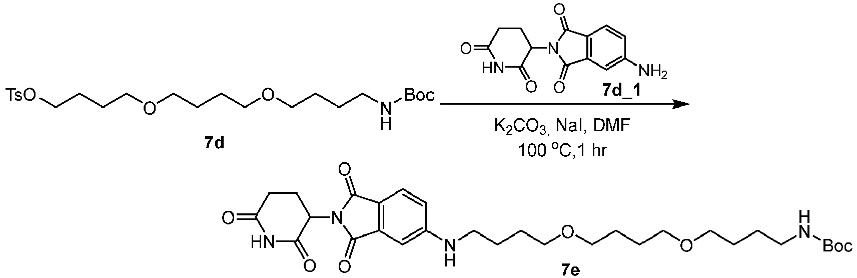

To a solution of compound **7d** (90 mg, 184 umol) and Compound **7d_1** (56 mg, 204 umol) in DMF (5 mL) was added Nal (14 mg, 93.4 umol) and K₂CO₃ (51 mg, 369 umol, 2.00 *eq)* at 20-25 °C, after addition, the mixture solution was stirred at 100 °C for 1 hr. The react ion solution was cooled to 15-20 °C, then it was poured into water (20 mL) and extracted with ethyl acetate (10 mL*2), the combined organic layer washed with brine (10 mL), dried over Na₂SO₄,filtrated and concentrated under reduce pressure to afford the crude product the Crude was purified by Pre-HPLC (column: Phenomenex Gemini-NX C18 75*30mm*3um;mobile phase: [water (0.1% TFA)- ACN]; B%: 52%-62%,7 min), then it was concentrated under reduce pressure to afford compound 7e (40 mg, 80.2 umol, 43.4% yield) as a pale green oil, which was confirmed by LCMS RT = 0.967 min, m/z+1 = 489), **LCMS:** (method 1), RT = 0.967 min, m/z = 489

### General procedure for preparation of compound 7f

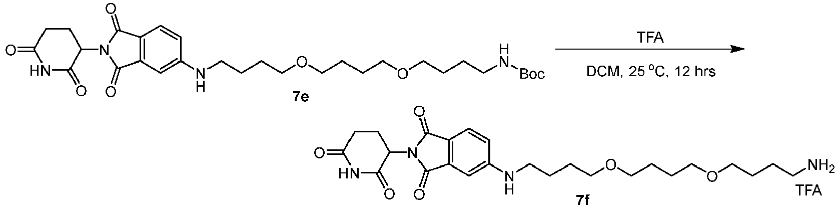

To a solution of compound **7e** (40 mg, 69.6 umol) in DCM (5 mL) was added TFA (154 mg, 1.35 mmol) at 25 °C, then it was stirred at 25 °C for 12 hrs. LCMS showed the MS of compound **7f** (RT = 0.795min, m/z = 489) was detected. The reaction solution was concentrated under reduce pressure to afford compound **7f** (40 mg, 66.3 umol, 95.3% yield, TFA) as a yellow oil, which confirmed by ¹H NMR

**¹H NMR:** 400 MHz, DMSO-*d₆*

δ 7.55 (s, 3H), 7.43 (d, *J* = 8.4 Hz, 1H), 6.86 (d, *J* = 2.0 Hz, 1H), 6.75 (dd, J = 8.4, 2.0 Hz, 1H), 5.02 (dd, *J* = 13.2, 5.4 Hz, 1H), 4.31 (t, *J* = 6.4 Hz, 1H), 3.17-3.34 (m, 10H), 2.81-2.95 (m, 1H), 2.64-2.76 (m, 3H), 1.88-2.01 (m, 1H) 1.30-1.59 (m, 14H).

**LCMS:** (method 1), RT = 0.795 min, m/z+1 = 489

### General procedure for preparation of "A51"

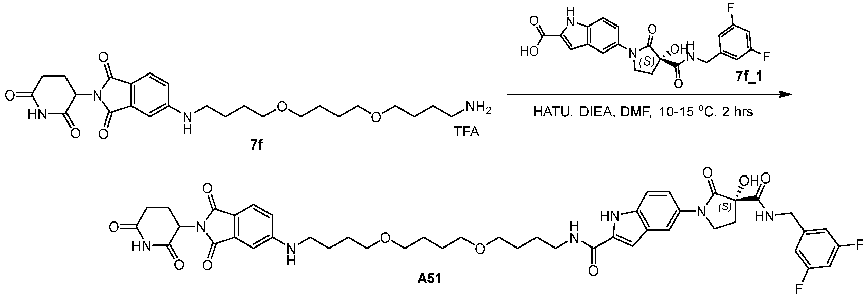

To a solution of compound **7f_1** (30 mg, 69.8 umol) in DMF (3 mL) was added HATU (85 mg, 105 umol, 1.51 eq) and DIEA (45 mg, 348 umol) at 10-15 °C, after addition, the mixture was stirred at 10-15 °C for 30 min, then compound **7f** (40 mg, 66.3 umol, TFA) in DMF (2 mL) was added to the solution at 10-15 °C under N ₂, then it was stirred at 10-15 °C for 1 hrs. LCMS showed the MS of compound **7** was detected (RT = 0.949 min, m/z = 900). the reaction solution was quenched with AcOH (0.5 mL), then it was poured into water(20 mL) and extracted with ethyl acetate(10 mL*2), the combined organic layer was washed with brine (10 mL), dried over Na₂SO₄,filtrated and concentrated under reduce pressure. The crude was purified by Pre-HPLC (column: Phenomenex Gemini-NX C18 75*30mm*3um; mobile phase: [water (0.1%TFA)-ACN]; B%: 48%-58%, 7 min), then it was concentrated under reduce pressure to afford the product. "A51" (44.3 mg, 47.7 umol, 68.3% yield) was obtained as yellow gum, which was confirmed by ¹H NMR, LCMS RT = 0.945 min, m/z = 900),

HPLC RT = 2.292 min).

**¹H NMR:,** 400 MHz, DMSO-*d₆*

δ 11.59 (s, 1H), 8.70 (t, *J* = 6.4 Hz, 1H), 8.70 (t, *J* = 6.4 Hz, 1H), 7.78 (s, 1H), 7.48-7.64, (m, 2H), 7.43 (d, *J* = 8.8 Hz, 1H), 6.92-7.17 (m, 5H), 6.84 (dd, *J* = 8.4, 2.0 Hz, 1H), 5.11 (dd, *J* = 12.8, 5.2 Hz, 2H), 4.25-4.45 (m, 2H), 3.90 (t, *J* = 6.4 Hz, 1H) (overlap H₂O singal), 3.64 (s, 1H), 3.18-3.48 (m, 10H), 2.86-3.07 (m, 1H), 2.56-2.81 (m, 4H) (overlap DMSO singal), 1.92-2.24 (m, 3H), 1.35-1.70 (m, 14H).

**LCMS:** (method 1), RT = 0.945 min m/z = 900

**HPLC:** (method 2), RT = 2.292 min

### 5-[(3S)-3-{[(3,5-difluorophenyl)methyl]carbamoyl}-3-hydroxy-2-oxopyrrolidin-1-yl]-N-(14-{[2-(2,6-dioxopiperidin-3-yl)-1,3-dioxo-2,3-dihydro-1H-isoindol-5-yl]oxy}-3,6,9,12-tetraoxatetradecan-1-yl)-1H-indole-2-carboxamide ("A52")

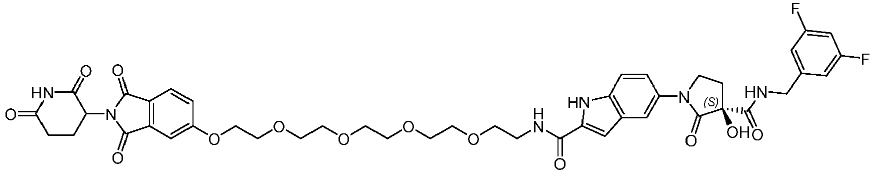

### LC-MS or HPLC Method:

### Method 1:

MS instrument type: SHIMADZU LCMS-2020, Column: Kinetex EVO C18 30*2.1 mm, 5um, mobile phase A: 0.0375% TFA in water (v/v), B: 0.01875% TFA in Acetonitrile (v/v), gradient: 0.0 min 0% B→- 0.8 min 95% B→-1.2 min 95% B→-1.21 min 5% B→-1.55 min 5% B, flow rate: 1.5 mL/min, oven temperature: 50 °C; UV detection: 220 nm & 254 nm.

### Method 2:

MS instrument type: SHIMADZU LCMS-2020, Column: Kinetex EVO C18 30*2.1 mm, 5um, mobile phase A: 0.0375% TFA in water (v/v), B: 0.01875% TFA in acetonitrile (v/v), gradient: 0.0 min 0% B→-3.0 min 95% B→-3.5 min95% B→-3.51 min 5% B→-4.0 min 5% B, flow rate: 0.8 mL/min, oven temperature: 50 °C; UV detection: 220 nm & 254 nm.

### 4. Experimental for largest scale run:

### General procedure for preparation of compound 8-3

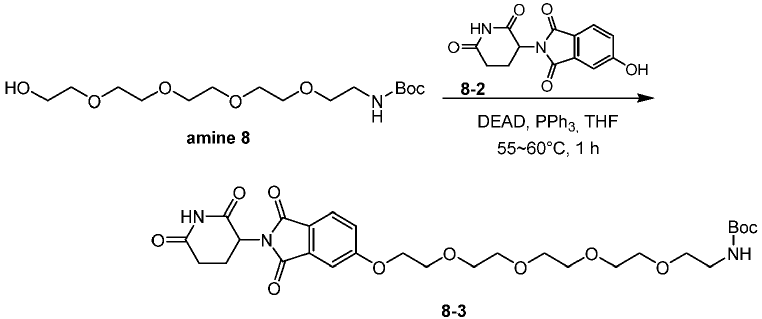

To a solution of compound **8-2** (90 mg, 328 umol), **amine 8** (100 mg, 296 umol) and DEAD (70 mg, 402 umol) in THF (3.00 mL) was added PPh₃ (130 mg, 495 umol) at 0-5 °C. The mixture was stir red at 10~20 °C for 1 h. Then the mixture was stirred at 55-60 °C for 12 h. LCMS showed the MS of compound **8-2** was remained (RT = 0.652 min, m/z (M+1) = 275) and the MS of compound **8-3** was not found. To the mixture was added PPh₃ (172 mg, 656 umol) and DEAD (114 mg, 655 umol) at 55-60 °C and kept stirred for 1 h. LCMS showed the MS of compound **8-2** was remained (RT = 0.643 min, m/z (M+1) = 275) and the MS of compound **8-3** (RT = 0.881 min, m/z (M+23) = 616) was found. TLC (Petroleum ether/Ethyl acetate = 0/1) showed compound **8-2** (Rf = 0.68) was remained and a main new spot (Rf = 0.25) was found. The reaction mixture adjust pH = 6~7 with AcOH and concentrated under reduce pressure. The residue was purified by flash silica gel chromatography (Silica Flash Column, Eluent of 0~100% Ethyl acetate/Petroleum ethergradient). The spot of Rf = 0.25 was collected. Compound 8-3 (110 mg, 182 umol, 55.4% yield, 98.2% purity) was obtained as a yellow gum.

**LCMS:** (method 1), RT = 0.881 min, m/z (M+23) = 616

**LCMS:** (method 1), RT = 0.877 min, m/z (M+1-100) = 494

### General procedure for preparation of compound 8-4

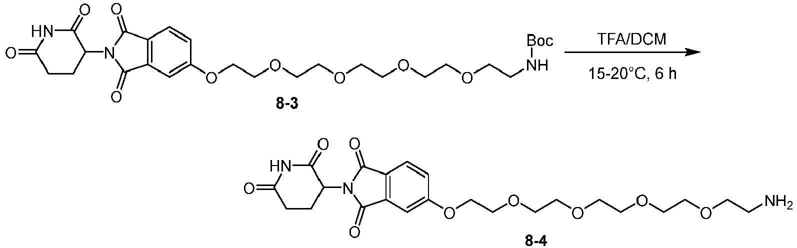

To a solution of compound 8-3 (110 mg, 185 umol) in DCM (5.00 mL) was added TFA (211 mg, 1.85 mmol). The mixture was stirred at 15-20 °C for 6 hr. TLC (Petroleum ether/Ethyl acetate = 0/1) showed compound **8-3** (Rf = 0.25) was consumed and a main new spot (Rf = 0.00) was found. The reaction mixture was concentrated under reduce pressure. Compound **8-4** (98 mg, 155 umol, 83.8% yield, 96.3% purity, TFA salt) was obtained as a yellow gum.

**LCMS:** (method 1), RT = 0.752 min, m/z (M+1) = 494

### General procedure for preparation of "A52"

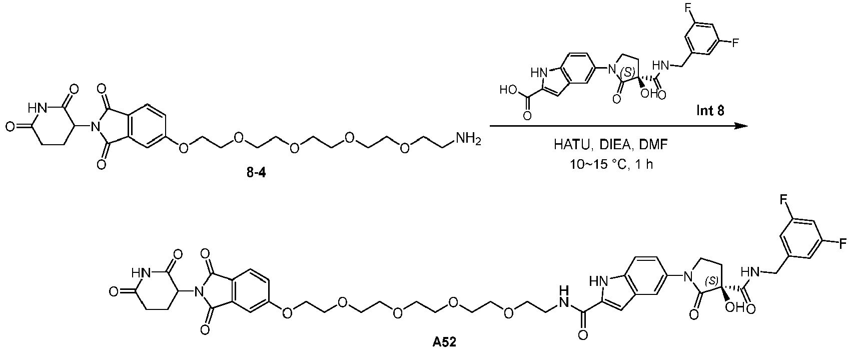

To a solution of **Int 8** (30 mg, 69.8 umol) in DMF (3 mL) was added HATU (40 mg, 105 umol) and DIEA (45 mg, 348 umol) at 10-15 °C, after addtion, compound **8-4** (51 mg, 84.0 umol TFA salt) in DMF (1 mL) was added into the solution, then it was stirred at 10-15 °C for 1 hr. LCMS showed **Int 8** was consumed and a main new peak with desired MS (RT = 0.871 min, m/z (M+1) = 905) was found. The reaction solution was poured into water (5 mL), then it was quenched with AcOH (1.00 mL) and extracted with ethyl acetate (10 mL*2), the organic layer was washed with brine (10 mL), dried over Na₂SO₄,filtrated and concentrated under reduce pressure. The residue was purified by Pre-HPLC (column: Phenomenex Luna C18 150*25mm*10um; mobile phase: [water (0.1 %TFA)-ACN]; B%: 25%-55%,10min), then it was concentrated under reduce pressure to afford the **"A52"** (51.4 mg, 56.8 umol, 81.3% yield, 100% purity), which was confirmed by ¹H-NMR and LCMS as a light yellow gum.

**¹H NMR:** 400 MHz, DMSO-*d*₆

δ 11.62 (s, 1H), 11.11 (s, 1H), 8.70 (t, *J* = 6.4 Hz, 1H), 8.56 (t, *J* = 5.6 Hz, 1H) 7.83 (d, *J* = 8.4 Hz, 1H), 7.78 (d, *J* = 1.6 Hz, 1H), 7.54 (dd, J = 8.8, 2.0 Hz, 1H), 7.41-7.47 (m, 2H), 7.36 (dd, *J* = 8.4, 2.4 Hz, 1H), 7.11-7.16 (m, 1H), 6.97-7.10 (m, 3H), 5.12 (dd, *J* = 12.8, 5.6 Hz, 1H), 4.42 (dd, *J* = 16.0, 6.8 Hz, 1H), 4.22-4.34 (m, 3H), 3.90 (t, *J* = 6.8 Hz, 2H), 3.73-3.78 (m, 2H), 2.83-2.96 (m, 1H), 2.54-2.66 (m, 3H), 2.09-2.19 (m, 1H), 1.99-2.09 (m, 1H). LCMS: (method 2), RT = 2.144 min, m/z (M+1) = 905

### 5-[(3S)-3-{[(3,5-difluorophenyl)methyl]carbamoyl}-3-hydroxy-2-oxopyrrolidin-1-yl]-N-(14-{[2-(2,6-dioxopiperidin-3-yl)-1,3-dioxo-2,3-dihydro-1H-isoindol-4-yl]oxy}-3,6,9,12-tetraoxatetradecan-1-yl)-1H-indole-2-carboxamide ("A53")

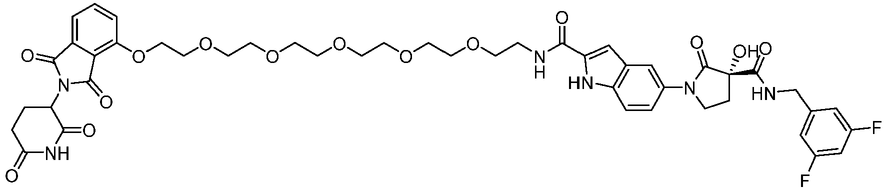

### LC-MS or HPLC Method:

### Method 1:

MS instrument type: SHIMADZU LCMS-2020, Column: Kinetex EVO C18 30*2.1mm, 5um, mobile phase A: 0.0375% TFA in water (v/v), B: 0.01875% TFA in Acetonitrile (v/v), gradient: 0.0 min 0% B→- 0.8 min 95% B→-1.2 min 95% B→-1.21 min 5% B→-1.55 min 5% B, flow rate: 1.5 mL/min, oven temperature: 50 °C; UV detection: 220 nm & 254 nm.

### Method 2:

MS instrument type: SHIMADZU LCMS-2020, Column: Kinetex EVO C18 30*2.1mm, 5um, mobile phase A: 0.0375% TFA in water (v/v), B: 0.01875% TFA in acetonitrile (v/v), gradient: 0.0 min 0% B→-3.0 min 95% B→-3.5 min95% B→-3.51 min 5% B→-4.0 min 5% B, flow rate: 0.8 mL/min, oven temperature: 50 °C; UV detection: 220 nm & 254 nm.

### 4. Experimental for largest scale run:

### General procedure for preparation of amine 13 -

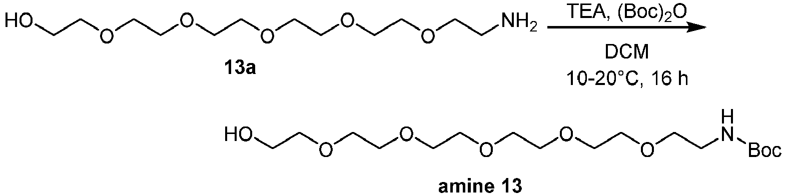

To a solution of compound **13a** (650 mg, 2.31 mmol) and TEA (468 mg, 4.63 mmol) in DCM (6.50 mL) was added dropwise (Boc)₂O (706 mg, 3.23 mmol) at 10-20 °C under N ₂ atmosphere and kept stirred at 10-20 °C for 16 h under N₂ atmosphere. TLC (DCM/Methanol = 10/1) showed compound **13a** (Rf = 0.00) was disappeared and new spot (Rf = 0.52) was found. The reaction mixture was concentrated under reduce pressure. The residue was purified by flash silica gel chromatography (ISCO^{®}; X g SepaFlash^{®} Silica Flash Column, Eluent of 0~2% Methanol/DCM). **Amine 13** (610 mg, 1.60 mmol, 69.2% yield) was obtained as a colorless oil which was confirmed by ¹H NMR.

**¹H NMR:,** 400 MHz, CDCl₃

δ 5.22 (s, 1H), 3.59-3.77 (m, 20 H), 3.55 (t, *J* = 5.2 Hz, 2H), 3.32 (d, *J* = 5.2 Hz, 2H), 2.87-3.07 (m, 1H), 1.45 (s, 9H)

### General procedure for preparation of compound 13-2

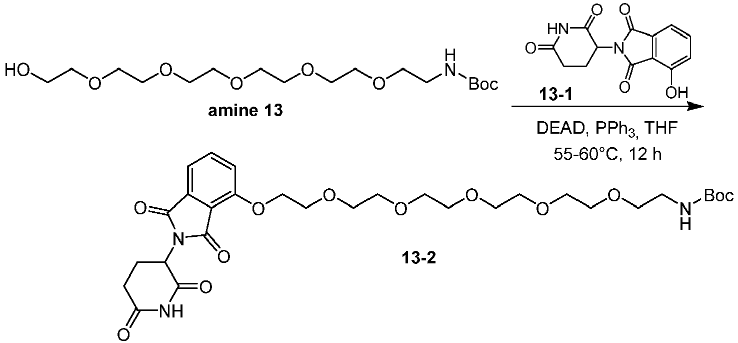

To a solution of **amine 13** (265 mg, 694 umol) and compound **13-1** (200 mg, 729 umol) in THF (10.0 mL) was added PPh₃ (600 mg, 2.29 mmol) at 20-25 °C under N ₂ atmosphere. Then to the mixture was added dropwise DEAD (320 mg, 1.84 mmol) at 55-60 °C under N ₂ atmosphere and kept stirred at 55-60 °C for 12 hours under N ₂ atmosphere. LC-MS (EW18817-71-P1A1) showed the MS of compound **13-2** (RT = 0.867 min, m/z (M-100+1) = 538) was detected. The mixture was concentrated under reduce pressure. The residue was purified by flash silica gel chromatography (SepaFlash^{®} Silica Flash Column, Eluent of 0~100% Ethyl acetate/Petroleum ethergradient). The residue was purified by prep-HPLC (TFA condition, column: Phenomenex Gemini-NX C18 75*30mm*3um; mobile phase: [water (0.1%TFA)-ACN]; B%: 35%-45%,7 min). Compound **13-2** (190 mg, 294 umol, 40.4% yield, 99% purity) was obtained as a yellow gum, which was confirmed by LCMS (BOC cleaved MS: RT = 0.711 mins).

**LCMS:** (method 1), RT = 0.877 min, m/z (M-56+1) = 538 (RT = 0.711 min, m/z (M-100+1) = 494)

### General procedure for preparation of compound 13-3

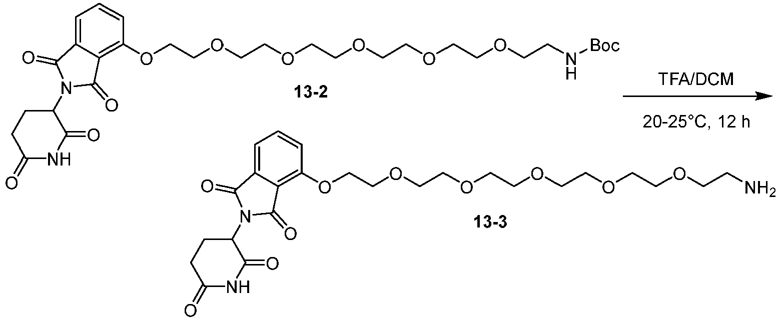

To a solution of compound **13-2** (190 mg, 298 umol) in DCM (5 mL) was added TFA (340 mg, 2.98 mmol) at 20-25 °C and kept stirred for 12 h. TLC (Petroleum ether/Ethyl acetate = 0/1) showed compound **13-2** (Rf = 0.28) was consumed and a main new spot (Rf = 0.00) was formed. The mixture was concentrated under reduce pressure. Compound **13-3** (151 mg, 230 umol, 77.5% yield, 99.6% purity, TFA salt) was obtained as a yellow gum.

### General procedure for preparation of compound "A53"

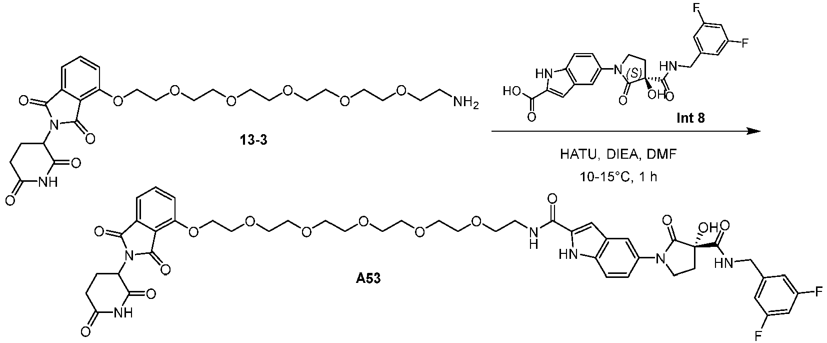

To a solution of **Int 8** (30 mg, 69.8 umol) in DMF (3.00 mL) was added HATU (40 mg, 105 umol) and DIEA (45 mg, 348 umol) at 10-15 °C ,after addtion, compound **13-3** (55 mg, 84.4 umol, TFA salt) in DMF (1.00 mL) was added into the solution, then it was stirred at 10-15 °C for 1 hr. LCMS showed **Int 8** was remained (RT = 0.750 min, M+1 = 538) and a main new peak with the MS of **compound 13** (RT = 0.904 min, M+1 = 949) was found. The reaction solution was poured into water (5.00 mL), then it was quenched with AcOH (1.00 mL) and extracted with ethyl acetate (10.0 mL*2), the organic layer was washed with brine (10.0 mL), dried over Na₂SO₄,filtrated and concentrated under reduce pressure. The crude was purified by Pre-HPLC (column: Phenomenex Gemini-NX C18 75*30mm*3um; mobile phase: [water (0.1%TFA)-ACN]; B%: 38%-48%,7 min), then it was lyophilized to afford **"A53"** (28.32 mg, 29.8 umol, 42.7% yield, 100% purity), which was confirmed by ¹H NMR and LCMS as a yellow gum.

**¹H NMR:** 400 MHz, DMSO-*d*₆

δ 11.62 (s, 1H), 11.10 (s, 1H), 8.70 (t, J= 6.4 Hz, 1H), 8.56 (t, J= 5.6 Hz, 1H), 7.75-7.85 (m, 2H), 7.50-7.58 (m, 2H), 7.41-7.49 (m, 2H), 7.13 (d, *J* = 1.2 Hz, 1H), 7.03-7.11 (m, 1H), 6.97-7.03 (m, 2H), 6.72 (s, 1H), 5.09 (dd, J = 12.8, 5.4 Hz, 1H), 4.37-4.48 (m, 1H), 4.31-4.36 (m, 2H), 4.21-4.31 (m, 1H), 3.90 (t, *J* = 6.8 Hz, 2H), 3.75-3.84 (m, 2H), 3.62 (dd, *J* = 5.6, 3.6 Hz, 2H), 3.41-3.58 (m, 20H), 2.82-2.96 (m, 1H), 2.54-2.66 (m, 3H), 2.15 (dt, J= 13.2, 7.6 Hz, 1H), 1.97-2.07 (m, 1H) (~20 H overlap H₂O signal, ~3H overlap DMSO signal).

**LCMS:** (method 2), RT = 2.101 min, m/z (M+1) = 949

### 5-[(3S)-3-{[(3,5-difluorophenyl)methyl]carbamoyl}-3-hydroxy-2-oxopyrrolidin-1-yl]-N-(14-{[2-(2,6-dioxopiperidin-3-yl)-1,3-dioxo-2,3-dihydro-1H-isoindol-4-yl]oxy}-3,6,9,12-tetraoxatetradecan-1-yl)-1H-indole-2-carboxamide ("A54")

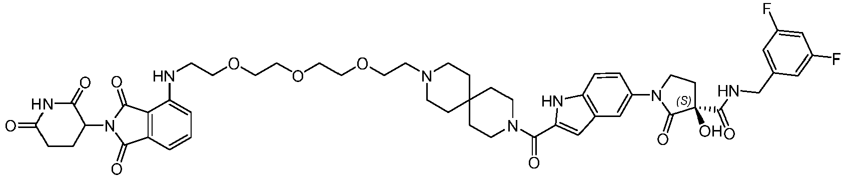

### LC-MS or HPLC Method:

### Method 1:

MS instrument type: SHIMADZU LCMS-2020, Column: Kinetex EVO C18 2.1 x 30mm, 5um, mobile phase A: 0.025% NH₃•H₂O in Water (v/v) , B: Acetonitrile, gradient: 0.0 min 5% B→- 0.8 min 95% B→-1.2 min 95% B→-1.21 min 5% B→-1.55 min 5% B, flow rate: 1.5 mL/min, oven temperature: 50 °C; UV detection: 220 nm & 254 nm.

### Method 2:

MS instrument type: SHIMADZU LCMS-2020, Column: Kinetex EVO C18 30*2.1 mm, 5um, mobile phase A: 0.0375% TFA in water (v/v), B: 0.01875% TFA in Acetonitrile (v/v), gradient: 0.0 min 5% B→- 0.8 min 95% B→-1.2 min 95% B→-1.21 min 5% B→-1.55 min 5% B, flow rate: 1.5 mL/min, oven temperature: 50 °C; UV detection: 220 nm & 254 nm.

### 4. Experimental for largest scale run:

### General procedure for preparation of intermediate 16b

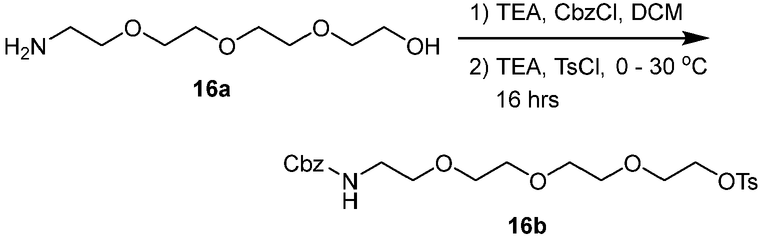

To a solution of compound **16a** (1.10 g, 5.69 mmol) in DCM (10 mL) was added TEA (1.32 g, 13.1 mmol, 1.82 mL) at 25 - 30 °C under N₂, then CbzCl (1.17 g, 6.83 mmol, 100 uL) was added into the mixture at 25 - 30 °C under N ₂, the mixture was stirred for 10 hrs. The mixture was cooled to 0 - 10 °C, TEA (5 g, 49.4 mmol) was added into the mixture at 0 - 10 °C under N₂, then TsCI (5.00 g, 26.2 mmol) was added into the mixture at 0 - 10 °C under N ₂, it was stirred at 25 - 30 °C for 6 hrs under N ₂. 0.5 mL reaction mixture was taken and poured into water (3 mL), extracted with ethyl acetate (1 mL), the organic phase was washed with water (3 mL x 3), then was concentrated under reduced pressure to give colourless oil, LCMS Rt = 0.915 min, m/z (M + 1) = 482.4) showed the MS of compound **16b** was detected, HNMR (EW18785-17-P1A2) showed compound **16b** was formed. The mixture was poured into water (100 mL), extracted with ethyl acetate (50 mL x 2), the organic phase was washed with brine (100 mL x 3), dried with Na₂SO₄,filtered, the filtrate was concentrated under reduced pressure to give brown oil. TLC 1 (Petroleum ether/Ethyl acetate = 1/1, Rf = 0.30). The oil was purified by column chromatography (SiO₂, Petroleum ether/Ethyl acetate = 1/0 - 1/1, Rf = 0.30) to give **compound 16b** (1.80 g, 3.74 mmol, 65.7% yield) as brown oil.

**¹H NMR:** 400 MHz, CDCl₃

δ 7.78 (d, *J* = 8.4 Hz, 2H), 7.26-7.42 (m, 7H), 5.42 (s, 1H), 5.08 (s, 2H), 4.76 (s, 1H), 4.11 (d, *J* = 4.4 Hz, 2H), 3.45-3.74 (m, 13H), 3.37 (d, *J* = 5.2 Hz, 2H), 2.43 (s, 3H), 1.83 (s, 3H)

**LCMS:** (method 1), Rt = 0.915 min, m/z (M + 1) = 482.4

### General procedure for preparation of compound 16d

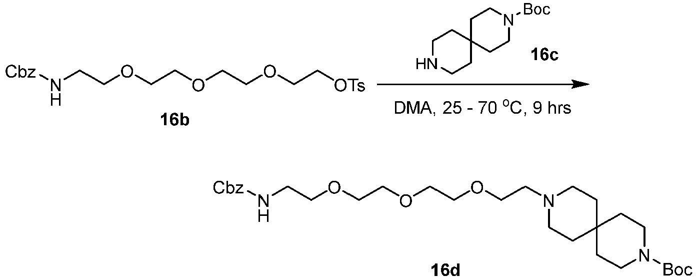

To a solution of compound **16b** (1 g, 2.08 mmol) and compound 16c (600 mg, 2.36 mmol) in DMA (20 mL) was added TEA (2.18 g, 21.5 mmol, 3 mL, 10.4 eq) at 25 °C, the mixture was stirred at 70 °C for 9 hrs. The mixture was cooled to 25 - 30 °C, poured into water (50 mL), extracted with ethyl acetate (20 mL x 3), the organic phase was washed with brine (30 mL x 3), then separated and dried with Na₂SO₄,filtered, the filtrate was concentrated under reduced pressure to give brown oil. The oil was purified by Prep-HPLC (column: Waters Xbridge C18 150*50mm* 10um; mobile phase: [water (10mM NH₄HCO₃)-ACN]; B%: 38% - 68%,11.5min), the fraction was concentrated under reduced pressure to give compound **16d** (500 mg, 886 umol, 42.7% yield) as brown oil.

**¹H NMR:** 400 MHz CDCl₃

δ 7.30 (m, 5H), 5.92 (s, 1H), 5.09 (s, 2H), 3.58 (m, 12H), 3.38 (m, 6H), 2.54 (m, 2H), 2.40 (m, 4H), 1.49 (m, 4H), 1.44 (s, 9H).

**LCMS:** (method 1), Rt = 0.844 min, m/z (M + 1) = 564.5

### General procedure for preparation of amine 16

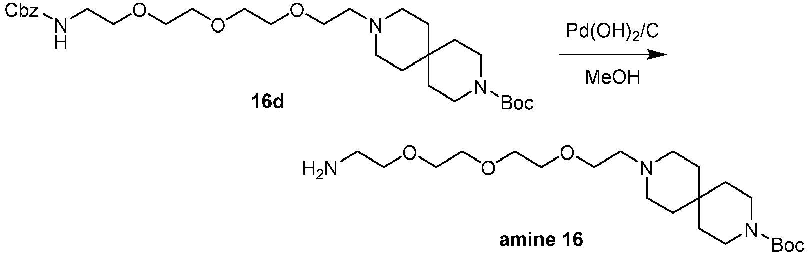

To a solution of Compound **16d** (180 mg, 319 umol) in MeOH (3 mL) was added Pd(OH)₂ (225 mg, 320 umol, 20 % purity). The mixture was stirred at 20 °C for 1 hr under H ₂ (15 psi). The reaction mixture was concentrated under reduced pressure to give **amine 16** (120 mg, 279 umol, 87.4% yield) was obtained as yellow oil.

**¹H NMR:** 400 MHz, CDCl₃

δ 3.71-3.59 (m, 11H), 3.39 (s, 4H), 3.35 (s, 1H), 3.32 (s, 1H), 3.04-2.95 (m, 1H), 2.69-2.62 (m, 2H), 2.56 (s, 4H), 1.57 (s, 4H), 1.45 (s, 13H)

### General procedure for preparation of amine 16

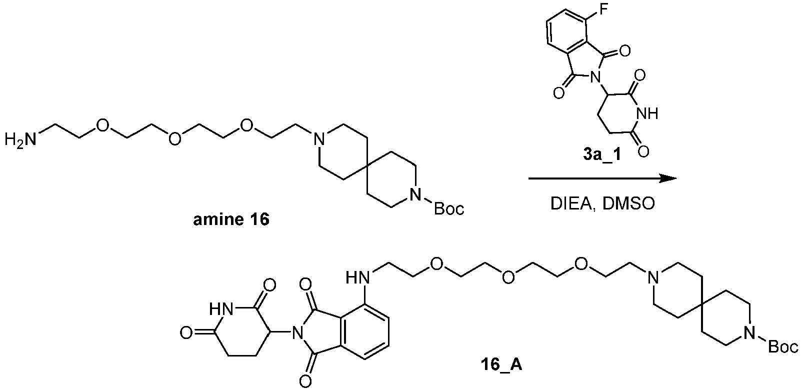

To a solution of Compound **3a_1** (80 mg, 289 mmol) in DMSO (3 mL) was added DIEA (140 mg, 1.08 mmol, 188 uL) and **amine 16** (120 mg, 279 umol). The mixture was stirred at 90 °C for 1 hr. The reaction mixture was acidified to pH = 5 by addition AcOH. The crude product was purified by reversed-phase HPLC (0.1% FA condition) to give Compound **16_A** (50 mg, 72.9 umol, 26.1% yield) was obtained as a red solid.

### General procedure for preparation of Compound 16_B

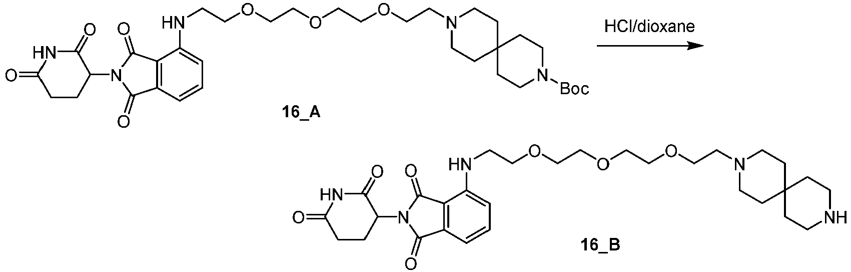

A mixture of **Compound 16_A** (40 mg, 58.3 umol) in HCl/dioxane (4 M, 5 mL) was stirred at 20 °C for 1 hr. The reaction mi xture was concentrated under reduced pressure to give Compound **16_B** (30 mg, 51.2 umol, 87.8% yield) was obtained as yellow oil.

**LCMS:** Rt = 0.539 min, m/z (M + 1) = 586.2

### General procedure for preparation of "A54"

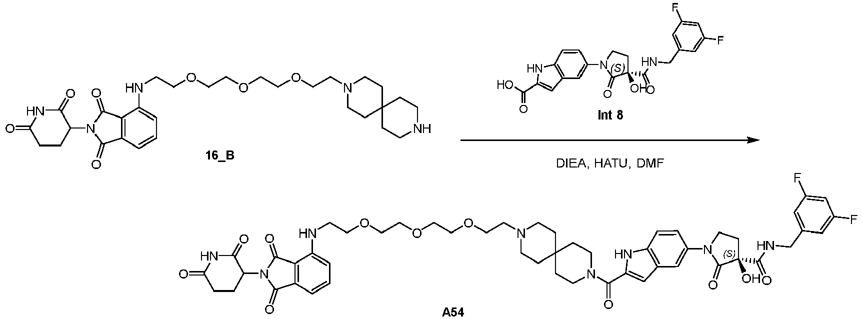

To a solution of **Int 8** (30 mg, 60.8 umol) in DMF (3 mL) was successively added DIEA (24 mg, 185 umol) and HATU (30 mg, 78.9 umol) at 0 °C under N₂, the mixture was stirred at 0 °C for 10 min, then a solution of **Compound 16_B** (30 mg, 51.2 umol, FA) in DMF (3.00 mL) was added into the mixture at 0 °C, it was stirred for 20 min under N₂ at 0 °C. The mixture was poured into icy water (20.0 mL), extracted with ethyl acetate (10.0 mL x 2), the organic phase was separated and washed with brine (20.0 mL x 2), dried over Na₂SO₄,filtered. The filtrate was concentrated under reduced pressure to give brown oil. The crude product was purified by Prep-HPLC (column: Phenomenex Synergi C18 75*30*3um; mobile phase: [water (0.225%FA)-ACN]; B%: 20%-40%, 8min), solvent of the fraction was removed by lyophilization to give **"A54"** (8.38 mg, 7.93 umol, 15.4% yield, 94.2% purity) as yellow solid.

**¹H NMR:** 400 MHz, DMSO-*d*₆;

δ 11.58 (s, 1H), 11.07 (s, 1H), 8.80-8.61 (m, 1H), 8.32 (s, 1H), 7.77 (s, 1H), 7.65-7.51 (m, 2H), 7.41 (d, *J* = 8.4 Hz, 1H), 7.32-7.20 (m, 1H), 7.18-7.06 (m, 1H), 7.06-6.96 (m, 3H), 6.76 (s, 1H), 6.72 (s, 1H), 6.60 (s, 1H), 5.14-4.97 (m, 1H), 4.41 (d, *J* = 6.4 Hz, 1H), 4.26 (d, *J* = 8.0 Hz, 1H), 3.88 (d, *J* = 8.4 Hz, 3H), 3.76-3.61 (m, 8H), 3.55 (d, *J* = 4.4 Hz, 4H), 3.47 (s, 4H), 3.05 (s, 2H), 2.86 (d, *J=* 12.4 Hz, 3H), 2.61 (d, J= 4.8 Hz, 3H), 2.37 (s, 3H), 2.19-2.11 (m, 1H), 2.02 (s, 1H), 1.46 (s, 8H).

**LCMS:** Rt = 0.846 min, m/z (M + 1) = 997.5;

**HPLC:** Rt = 1.675 min.

### (3S)-N-[(3,5-difluorophenyl)methyl]-1-{2-[(1-{[2-(2,6-dioxopiperidin-3-yl)-1,3-dioxo-2,3-dihydro-1H-isoindol-5-yl]carbamoyl}-2,5,8,11,14-pentaoxahexadecan-16-yl)carbamoyl]-1H-indol-5-yl}-3-hydroxy-2-oxopyrrolidine-3-carboxamide ("A55")

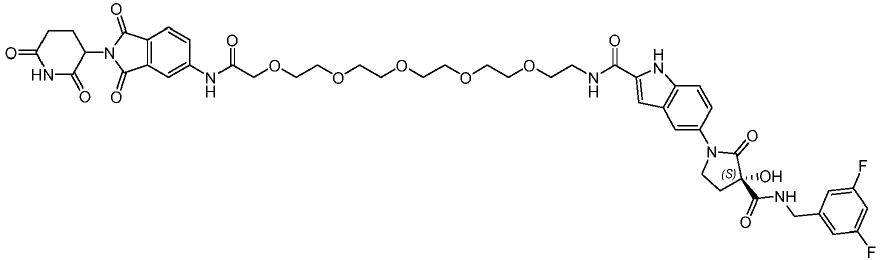

### LC-MS or HPLC Method:

### Method 1:

MS instrument type: SHIMADZU LCMS-2020, Column: Kinetex EVO C18 30*2.1mm, 5um, mobile phase A: 0.0375% TFA in water (v/v), B: 0.01875% TFA in Acetonitrile (v/v), gradient: 0.0 min 0% B→- 0.8 min 95% B→-1.2 min 95% B→-1.21 min 5% B→-1.55 min 5% B, flow rate: 1.5 mL/min, oven temperature: 50 °C; UV detection: 220 nm & 254 nm.

### Method 2:

MS instrument type: SHIMADZU LCMS-2020, Column: Kinetex EVO C18 30*2.1mm, 5um, mobile phase A: 0.0375% TFA in water (v/v), B: 0.01875% TFA in acetonitrile (v/v), gradient: 0.0 min 0% B→-3.0 min 95% B→-3.5 min 95% B→-3.51 min 5% B→-4.0 min 5% B, flow rate: 0.8 mL/min, oven temperature: 50 °C; UV detection: 220 nm & 254 nm.

### 4. Experimental for largest scale run:

### General procedure for preparation of compound 2b

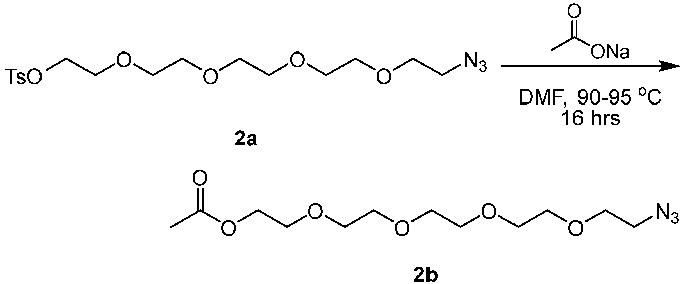

A mixture of compound **2a** (5.00 g, 12.0 mmol), sodium acetate (10.0 g, 122 mmol) in DMF (75 mL) was degassed and purged with N₂ for 3 times, and then the mixture was stirred at 95-100 °C for 16 hrs under N₂ atmosphere. TLC (Petroleum ether/Ethyl acetate = 1/2) showed compound **2a** (Rf = 0.65) was consumed and a main spot (Rf = 0.62) was formed. The reaction mixture was poured into saturated sodium bicarbonate aqueous solution (300 mL), extracted with MTBE (100 mL x 3). The combined organic layers were washed with brine (60 mL x 2), the organic phase was separated and dried with Na₂SO₄,filtered and filtrate was concentrated under reduced pressure to give compound **2b** (4.27 g, crude) as a yellow oil.

### ¹H NMR: 400 MHz, CDCl₃

δ 4.16-4.25 (m, 2H), 3.64-3.70 (m, 16H), 3.38 (t, *J* = 5.2 Hz, 2H), 2.07 (s, 3H).

### General procedure for preparation of compound 2c

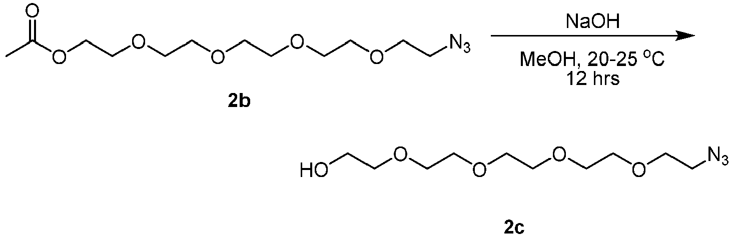

A mixture of compound **2b** (3.66 g, 12.0 mmol), NaOH (2 M, 35 mL) in MeOH (35 mL) was stirred at 20-25 °C for 12 hrs und er N₂ atmosphere. TLC (Petroleum ether/Ethyl acetate = 1/1) indicated compound **2b** (Rf = 0.50) was consumed completely and one new spot formed (Rf = 0.20). The reaction was clean according to TLC. The reaction mixture was diluted with H₂O (200 mL), washed with MTBE (50 x 2 mL). Then to the aqueous layer was saturated with NaCl (s) and extracted with EtOAc (100 mL x 2). The combined organic layer was dried with Na₂SO₄,filtered and filtration was concentrated under reduced pressure to give compound **2c** (1.96 g, 7.44 mmol, 62.1% yield) as a yellow oil.

### ¹H NMR: 400 MHz, CDCl₃

δ 3.69-3.73 (m, 2H), 3.62-3.68 (m, 14H), 3.57-3.61 (m, 2H), 3.33-3.42 (m, 2H).

### General procedure for preparation of compound 2d

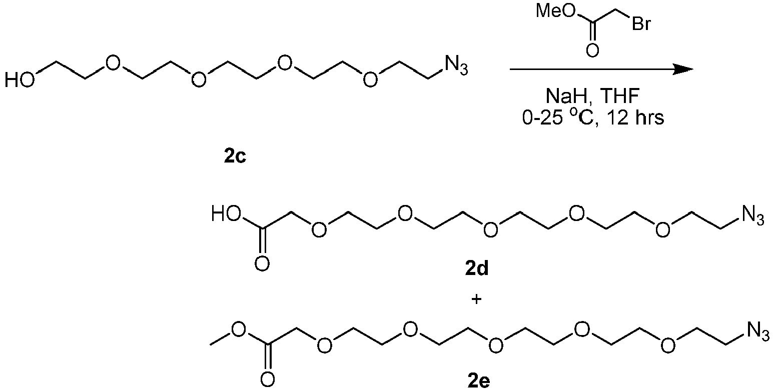

To a solution of NaH (1.83 g, 45.8 mmol, 60% purity) in THF (30 mL) was added compound **2c** (1.96 g, 7.44 mmol) in THF (10 mL) at 0-5 °C under N₂ and kept stirred for 1 h at 20-25 °C under N ₂. To the mixture was added methyl 2-bromoacetate (2.28 g, 14.9 mmol, 1.40 mL) in THF (10 mL) at 0-5 °C under N ₂ and kept stirred for 12 hrs at 20-25 °C under N ₂. TLC (Petroleum ether/Ethyl acetate = 1/1) indicated compound **2c** (R_{f} = 0.50) was consumed completely. The reaction mixture was poured into saturated NH₄Cl (120 mL), extracted with EtOAc (30 mL x 3), the combined organic layers were washed with brine (15 mL), the organic phase was separated and dried with Na₂SO₄,filtered and filtration was concentrated under reduced pressure to give compound **2e** (200 mg, crude) as a brown oil, which checked by 1H NMR. The aqueous layer was adjusted pH = 5-6 with citric acid (s). Then the mixture was extracted with MeCN (thrice, 100 mL, 50 mL, 50 mL). The combined organic layer (compound 2d in MeCN solution) checked by 1H NMR and used to next step directly.

### ¹H NMR: 400 MHz, CDCl₃

δ 3.48-3.63 (m, 20H), 3.25-3.30 (m, 2H).

### General procedure for preparation of compound 2e

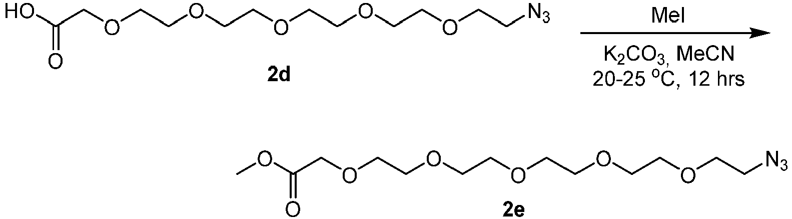

To a solution of compound 2d (2.39 g, 7.44 mmol) in MeCN (200 mL) was added K₂CO₃ (4.11 g, 29.8 mmol) at 20-25 °C under stirred for 1 h at 20-25 °C under N ₂ atmosphere. Then to the mixture was added Mel (10.6 g, 74.4 mmol, 4.63 mL) at 20-25 °C under N ₂ atmosphere. The mixture was stirred at 20-25 °C for 12 hrs under N ₂. 1H NMR showed compound **2d** was disappeared and compound **2e** was found (The reaction mixture was concentrated and directed for 1H NMR). The reaction mixture was concentrated under reduce pressure. The residue was dissolved with EtOAc (200 mL), then the solution was dried over Na₂SO₄,filtered and filtrate was concentrated under reduce pressure to give compound **2e** (890 mg, 2.65 mmol, 35.7% yield) as a brown oil.

### ¹H NMR: 400 MHz, CDCl₃

δ 4.18 (s, 2H), 3.75 (s, 3H), 3.62-3.74 (m, 18H), 3.40 (t, *J=* 5.2 Hz, 2H).

### General procedure for preparation of compound 2f

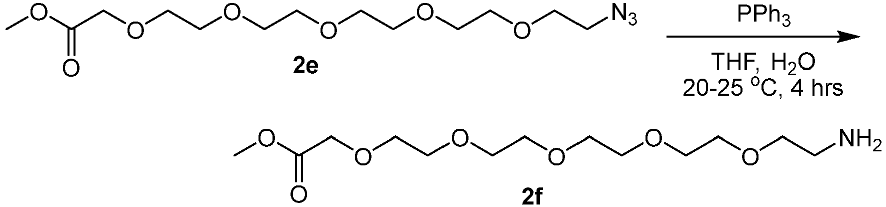

A mixture of compound **2e** (800 mg, 2.39 mmol), PPh₃ (1.12 g, 4.27 mmol) in H₂O (3 mL) and THF (15 mL) was degassed and purged with N₂ for 3 times, and then the mixture was stirred at 20-25 °C for 4 hrs under N₂ atmosphere. TLC (Petroleum ether/Ethyl acetate = 0/1) showed compound **2e** (Rf = 0.60) was consumed (the reactant solution was directed used for next step.

### General procedure for preparation of compound 2g

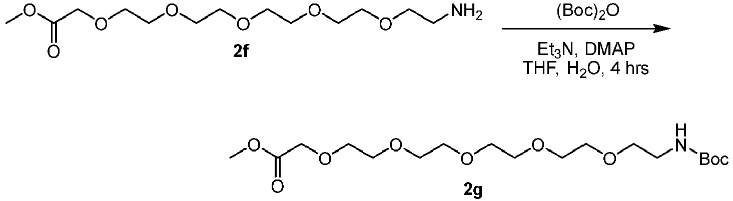

A mixture of compound **2f** (800 mg, 2.59 mmol), TEA (730 mg, 7.21 mmol, 1.00 mL), (Boc)₂O (1.30 g, 5.96 mmol, 1.37 mL), DMAP (145 mg, 1.19 mmol) in H₂O (3 mL) and THF (15 mL) was degassed and purged with N₂ for 3 times, and then the mixture was stirred at 20-25 °C for 12 hrs under N₂ atmosphere. TLC (Petroleum ether/Ethyl acetate = 0/1) showed compound **2g** (Rf = 0.45) was formed. The reaction mixture was concentrated under reduce pressure. The residue was purified with flash silica gel chromatography (Silica Flash Column, Eluent of 0-100% Ethyl acetate/Petroleum ether gradient), the spot (Rf = 0.45) was collected to give compound **2g** (145 mg, 354 umol, 13.7% yield) as a yellow oil.

### ¹H NMR: 400 MHz, CDCl₃

δ 5.06 (s, 1H), 4.17 (s, 2H), 3.75 (s, 3H), 3.59-3.74 (m, 16H), 3.54 (t, *J* = 5.2 Hz, 2H), 3.31 (d, *J* = 4.8 Hz, 2H), 1.44 (s, 9H).

### General procedure for preparation of compound 2h

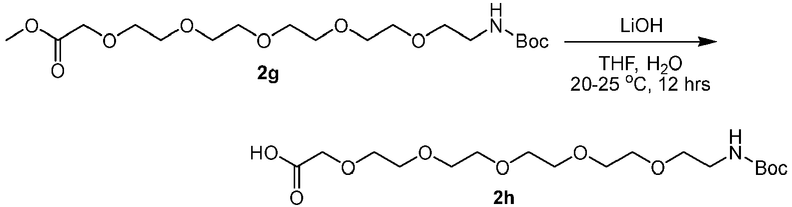

A mixture of compound **2g** (145 mg, 354 umol), LiOH•H₂O (30 mg, 715 umol) in THF (4 mL) and H₂O (1 mL) was stirred at 20-25 °C for 12 hrs under N₂ atmosphere. TLC (Dichloromethane/ Methanol = 10/1) showed compound **2g** (Rf = 0.60) was consumed and a main new spot (Rf = 0.00) was formed. The reaction mixture was diluted with H₂O (30 mL). The mixture was adjusted pH = 3-4 with citric acid (4 M in H₂O) and added NaCl(s) until saturated. Then the mixture was extracted with EtOAc (10 mL x 3), the organic layers were combined and dried over Na₂SO₄,filtered and filtrate was concentrated under reduce pressure to give compound **2h** (137 mg, 346 umol, 97.8% yield) as a yellow oil, which confirmed by 1H NMR.

### ¹H NMR: 400 MHz, DMSO-d₆

δ 12.53 (s, 1H), 6.74 (s, 1H), 4.01 (s, 2H), 3.44-3.62 (m, 16H), 3.35-3.38 (m, 2H), 3.05 (q, *J=* 6.0 Hz, 2H), 1.37 (s, 9H).

### General procedure for preparation of compound 2i

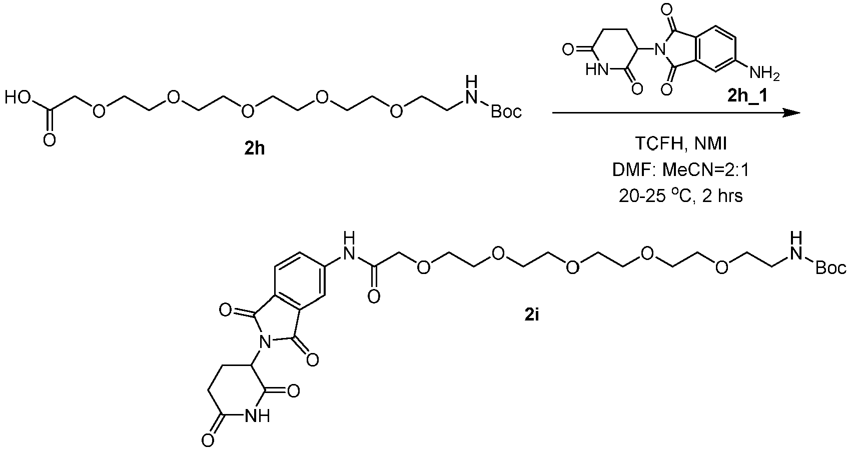

To a solution of compound **2h** (70 mg, 177 umol) and compound **2h_1** (51 mg, 186 umol) in ACN (4 mL) and DMF (2 mL) was added TCFH (121 mg, 431 umol) and NMI (100 mg, 1.22 mmol) under N₂ at 20-25 °C, after addition, the mixture was stirred at 20-25 °C for 2 hrs. LCMS showed the compound **2i** was detected (RT = 0.876 min, m/z (M+1-100 = 551), TLC (Petroleum ether/Ethyl acetate = 0/1) showed compound **2i** was consumed up (Rf = 0.00), a new spot was detected (Rf = 0.10). The reaction solution was quenched with AcOH (1 mL), then it was poured into water (10 mL) and extracted with ethyl acetate (10 mL*2), the organic layer was washed with brine(10 mL), dried over Na₂SO₄,filtrated and concentrated under reduce pressure. The crude was purified by silica gel column chromatography (SiO₂, Petroleum ether/ethyl acetate=5/1-1/1-0/1, the spot Rf = 0.10 was collected) then it was concentrated under reduce pressure to afford compound **2i** (90 mg, 137 umol, 77.4% yield) as a pale yellow oil.

**LCMS:** (method 1), RT = 0.875 min, m/z (M+1-100) = 551

### General procedure for preparation of compound 2j

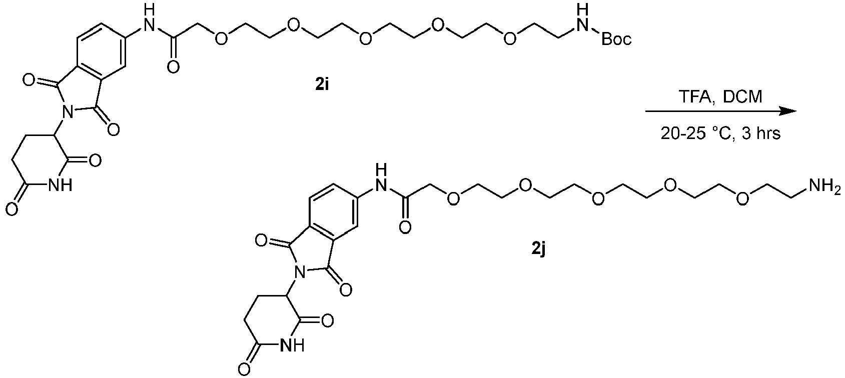

To a solution of compound **2i** (90 mg, 138 umol) in DCM (5 mL) was added TFA (158 mg, 1.39 mmol) at 20-25 °C, after addition , the mixture was stirred at 20-25 °C for 3 hrs. LCMS showed the MS o f compound **2j** was detected (Rt = 0.733 min m/z (M+1) = 551). The reaction was concentrated under reduce pressure to afford compound **2j** (90 mg, crude, TFA) as a yellow oil.

**LCMS:** (method 1), RT=0.733 min, m/z (M+1) = 551

### General procedure for preparation of "A55"

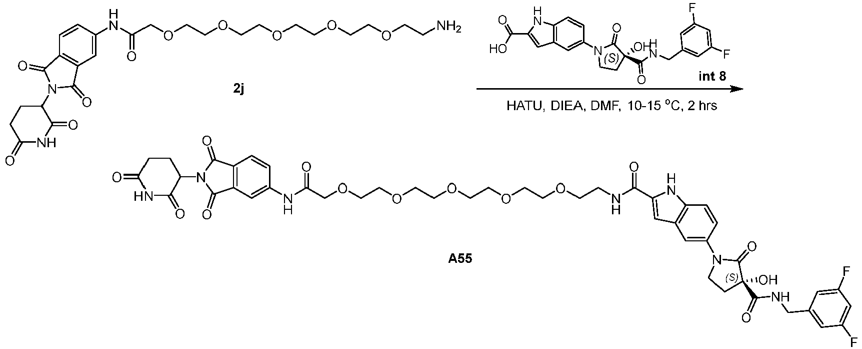

To a solution of compound **int 8** (30 mg, 69.8 umol) in DMF (3 mL) was added HATU (40 mg, 105 umol) and DIEA (45 mg, 348 umol) at 10-15 °C under N₂, after addition, the compound **2j** (55 mg, 82.7 umol TFA) dissolved in DMF (1 mL) was added to the solution at 10-15 °C , then it was stirred at 10-15 °C for 1 hr under N ₂. LCMS showed the mass of compound 2 (RT = 0.890 min, m/z (M+1) = 962) was detected. The reaction solution was quenched with AcOH (1 mL) and poured into water (5 mL), then it was extracted with ethyl acetate (10 mL*2), the organic layer was washed with brine (10 mL), dried over Na₂SO₄,filtrated and concentrated under reduce pressure. The crude was purified by Pre-HPLC (column: Phenomenex Luna C18 150*25 mm*10 um; mobile phase: [water (0.1% TFA)-ACN]; B%: 25%-55%, 10min), then it was concentrated under reduce pressure to afford "A55" (33.96 mg, 33.6 umol, 95.2% purity) as a yellow gum.

**LCMS:** (method 1), RT = 0.885 min, m/z (M+1) = 962

**HPLC:** (method 2), RT = 1.885 min

### ¹H NMR: 400 MHz, DMSO-d₆

δ 11.68 (s, 1H), 11.18 (s, 1H), 10.39 (s, 1H), 8.76 (t, *J* = 6.4 Hz, 1H), 8.61 ( t, *J* = 5.6 Hz, 1H), 8.36 (d, *J* = 1.6 Hz, 1H), 8.09 (dd, *J* = 8.4, 1.6 Hz, 1H), 7.95 (d, *J* = 8.4 Hz, 1H), 7.84 (d, *J* = 1.6 Hz, 1H), 7.60 (dd, *J* = 8.8, 2.0 Hz, 1H), 7.49 (d, *J* = 8.8 Hz, 1H), 7.19 (s, 1H), 7.01-7.15 (m, 3H), 5.19 (dd, J= 12.8, 5.2 Hz, 1H), 4.48 (dd, *J* = 16.0, 6.8 Hz, 1H), 4.33 (dd, *J* = 16.0, 6.0 Hz, 1H), 4.23 (s, 2H), 3.96 (t, *J* = 6.8 Hz, 1H), 3.83-3.43 (m, 24H overlap H₂O signal), 2.89-3.02(m, 1H), 2.64-2.76 (m, 2H), 2.05-2.27 (m, 2H).

### (3S)-N-[(3,5-difluorophenyl)methyl]-1-{2-[4-(14-{[2-(2,6-dioxopiperidin-3-yl)-1,3-dioxo-2,3-dihydro-1H-isoindol-4-yl]amino}-3,6,9,12-tetraoxatetradecan-1-yl)piperazine-1-carbonyl]-1H-indol-5-yl}-3-hydroxy-2-oxopyrrolidine-3-carboxamide ("A56")

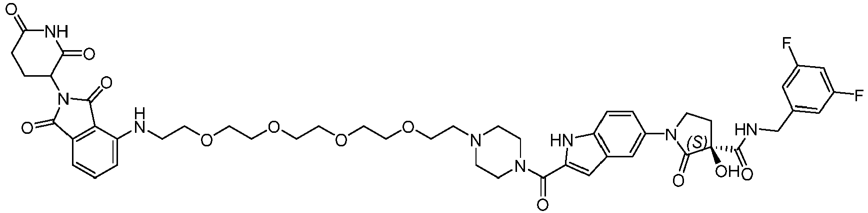

### LC-MS or HPLC Method:

### Method 1:

MS instrument type: SHIMADZU LCMS-2020, Column: Kinetex EVO C18 2.1 x 30mm, 5um, mobile phase A: 0.025% NH₃•H₂O in Water (v/v) , B: Acetonitrile, gradient: 0.0 min 0% B→- 0.8 min 60% B→-1.2 min 60% B→-1.21 min 0% B→-1.55 min 0% B, flow rate: 1.5 mL/min, oven temperature: 40 °C; UV detection: 220 nm & 254 nm.

### Method 2:

MS instrument type: SHIMADZU LCMS-2020, Column: Kinetex EVO C18 30*2.1mm, 5um, mobile phase A: 0.0375% TFA in water (v/v), B: 0.01875% TFA in Acetonitrile (v/v), gradient: 0.0 min 0% B→- 0.8 min 60% B→-1.2 min 60% B→-1.21 min 0% B→-1.55 min 0% B, flow rate: 1.5 mL/min, oven temperature: 50 °C; UV detection: 220 nm & 254 nm. 4.0 min 0% B, flow rate: 0.8 mL/min, oven temperature: 50 °C; UV detection: 220 nm & 254 nm.

### Experimental for largest scale run:

### General procedure for preparation of compound 15a

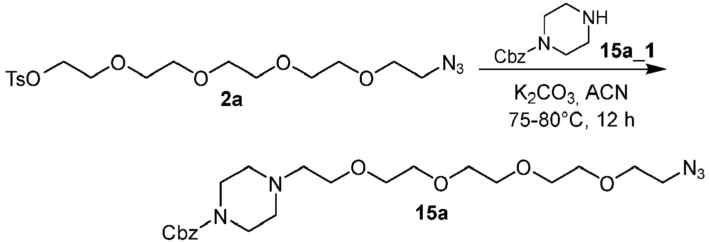

A mixture of compound 2a (5 g, 12 mmol), compound **15a_1** (2.90 g, 13.2 mmol), K₂CO₃ (3.31 g, 24.0 mmol) in ACN (30.0 mL) was degassed and purged with N₂ for 3 times, and then the mixture was stirred at 75-80 °C for 12 hrs under N₂ atmosphere. TLC (Petroleum ether: Ethyl acetate = 1/2) showed compound 2a (Rf = 0.75) was consumed and a main new spot (Rf = 0.05) was found. The reaction mixture was poured into H₂O (140 mL), the mixture was extracted with MTBE (30.0 mL x 3). The organic layer were combined and used to next step.

### General procedure for preparation of compound 15_A

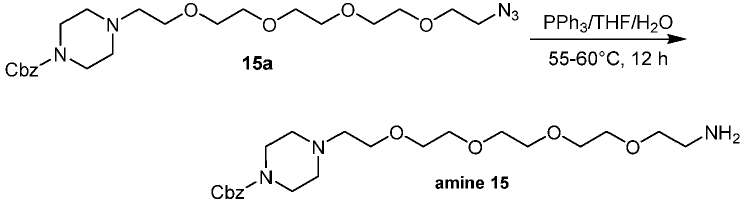

A mixture of compound **15a** (5.58 g, 12 mmol) , PPh₃ (6.30 g, 24.0 mmol) in THF (80 mL) and H₂O (10 mL) was degassed and purged with N₂ for 3 times, and then the mixture was stirred at 55-60 °C for 12 h under N₂ atmosphere. TLC (Petroleum ether: Ethyl acetate = 1/2) showed compound **15a** (Rf = 0.60) was remained. The mixture was kept stirred at 55-60 °C for 16 hrs. LCMS showed compound **15a** was consumed, a main new peaks with MS value of **amine 15** (RT = 0.941 min, M+1 = 440) was detected. The reaction mixture was concentrated under reduce pressure. Half the residue was purified by column chromatography (SiO₂, DCM/MeOH = 1/0 to 10/1) to give **amine 15** (430 mg, 978 umol, 8.16% yield) as a yellow oil which confirmed by 1H NMR and LCMS.

**¹H NMR:** 400 MHz, CDCl₃

δ 7.28-7.41 (m, 5H), 5.13 (s, 2H), 3.59-3.67 (m, 13H) 3.51-3.58 (m, 5H), 2.90 (t, *J* = 5.2 Hz, 2H), 2.61 (t, *J* = 5.6 Hz, 2H), 2.48 (s, 4H), 2.18 (s, 2H) (overlap H₂O signal)

**LCMS:** (method 1), RT = 0.966 min, m/z (M + 1) = 440

### General procedure for preparation of compound 15_A

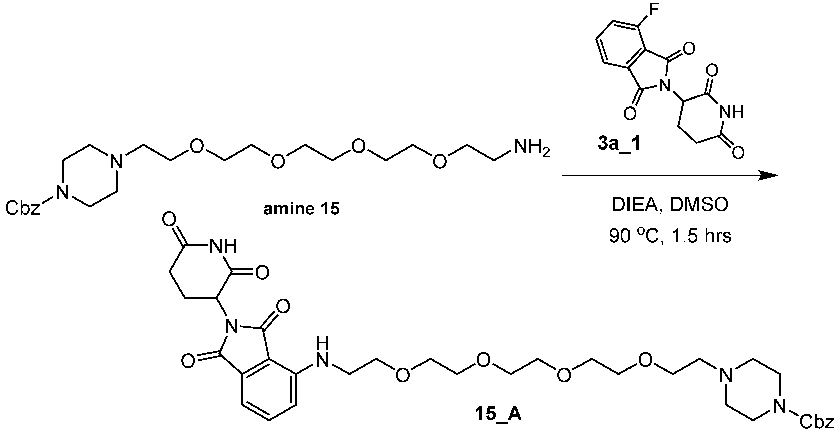

A solution of compound **3a_1** (50 mg, 181 umol), **amine 15** (100 mg, 227 umol) and DIEA (148 mg, 1.15 mmol, 200 uL) in DMSO (3 mL) was stirred at 90 °C under N ₂ for 1.5 hr. Three batches were carried in parallel. The mixture was cooled to 25 - 30 °C, pH value of the m ixture was adjusted to 6 - 7 with AcOH. The mixture was purified by Prep-HPLC (column: Phenomenex Synergi C18 150*25*10um; mobile phase: [water (0.225% FA)-ACN]; B%: 18% - 48%, 10 min), the fraction was concentrated under reduced pressure to give compound ***15_A*** (160 mg, 204 umol, 37.7% yield, 94.9% purity, FA) as yellow oil.

**LCMS:** EW18785-68-P1A, Rt = 0.799 min, m/z (M + 1) = 696.4

### General procedure for preparation of Compound 15-1

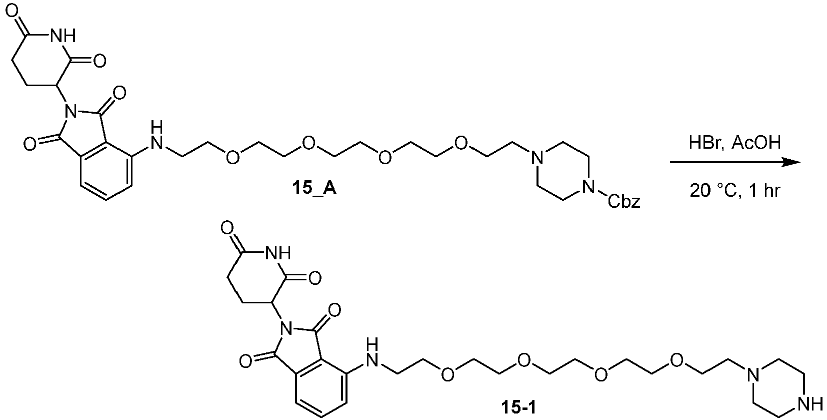

A mixture of Compound ***15_A*** (40 mg, 57.4 umol) in HBr (10 mL) and HOAc (5 mL) was stirred at 20 °C for 1 hr under N ₂ atmosphere. The reaction mixture was acidified to pH = 7 by addition NaHCO₃ (aq), and then was concentrated under reduced pressure to give a residue. The crude product was purified by reversed-phase HPLC (0.1% FA condition) to give compound **15-1** (30 mg, 53.4 umol, 92.9% yield) was obtained as a yellow solid and confirmed by next step.

### LCMS: Rt = 0.543 min, m/z (M + 1) = 562.0

### General procedure for preparation of "A56"

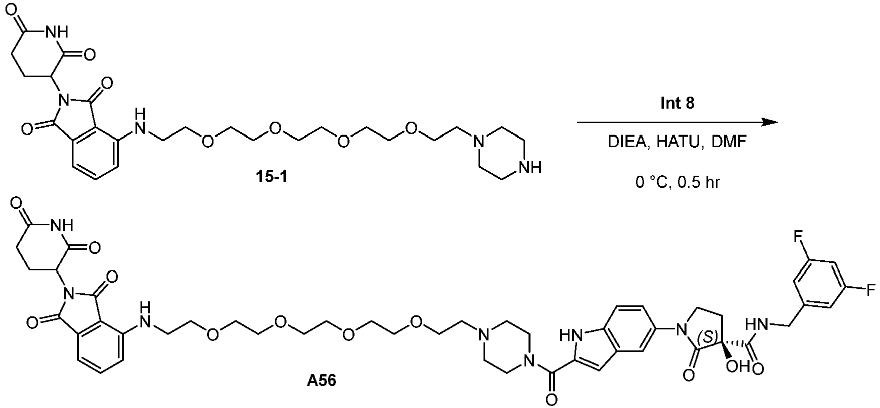

To a solution of **Int 8** (20 mg, 46.5 umol) in DMF (3 mL) was successively added DIEA (20 mg, 154 umol) and HATU (30 mg, 78.9 umol) at 0 °C under N₂, the mixture was stirred at 0 °C for 10 min, then a solution of **Compound 15-1** (30 mg, 53.4 umol, FA) in DMF (3.00 mL) was added into the mixture at 0 °C, it was stirred for 20 min unde r N₂ at 0 °C. The mixture was poured into icy water (20.0 mL), extracted with ethyl acetate (10.0 mL x 2), the organic phase was separated and washed with brine (20.0 mL x 2), dried with Na₂SO₄,filtered, the filtrate was concentrated under reduced pressure to give brown oil. It was purified by Prep-HPLC (column: Phenomenex Synergi C18 150*50*3um; mobile phase: [water (0.225%FA)-ACN]; B%: 20%-50%, 10min), solvent of the fraction was removed by lyophilization to give **"A56"** (10 mg, 10.1 umol, 18.9% yield, 98.3% purity) as yellow solid.

**¹H NMR:** 400 MHz, DMSO-*d*₆;

δ 11.61 (s, 1H), 11.09 (s, 1H), 8.69 (t, *J* = 6.4 Hz, 1H), 8.25 (s, 1H), 7.89-7.72 (m, 1H), 7.60-7.50 (m, 2H), 7.42 (d, *J* = 9.2 Hz, 1H), 7.13 (d, *J* = 8.4 Hz, 1H), 7.09-6.98 (m, 4H), 6.79 (s, 1H), 6.72 (s, 1H), 6.59 (s, 1H), 5.10-5.00 (m, 1H), 4.41 (d, *J* = 6.4, 15.5 Hz, 1H), 4.31-4.23 (m, 1H), 3.89 (s, 3H), 3.72 (s, 5H), 3.60 (t, *J* = 5.6 Hz, 3H), 3.53 (d, *J* = 4.0 Hz, 5H), 3.50 (s, 7H), 3.49-3.46 (m, 6H), 2.64-2.60 (m, 3H), 2.14 (d, *J* = 12.4 Hz, 1H), 2.04 (s, 1H).

**LCMS:** Rt = 0.671 min, m/z (M + 1) = 973.3;

**HPLC:** Rt = 1.637 min.

### (3S)-N-[(3,5-difluorophenyl)methyl]-1-{2-[(16-{[2-(2,6-dioxopiperidin-3-yl)-1,3-dioxo-2,3-dihydro-1H-isoindol-4-yl]carbamoyl}hexadecyl)carbamoyl]-1H-indol-5-yl}-3-hydroxy-2-oxopyrrolidine-3-carboxamide ("A57")

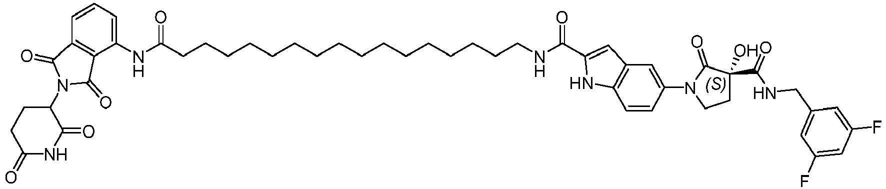

### LC-MS or HPLC Method:

### Method 1:

MS instrument type: SHIMADZU LCMS-2020, Column: Kinetex EVO C18 30*2.1 mm, 5um, mobile phase A: 0.0375% TFA in water (v/v), B: 0.01875% TFA in Acetonitrile (v/v), gradient: 0.0 min 5% B→- 0.8 min 95% B→-1.2 min 95% B→-1.21 min 5% B→-1.55 min 5% B, flow rate: 1.5 mL/min, oven temperature: 50°C; PDA detection: 220 nm & 254 nm.

### Method 2:

MS instrument type: SHIMADZU LCMS-2020, Column: Kinetex EVO C18 30*2.1 mm, 5um, mobile phase A: 0.0375% TFA in water (v/v), B: 0.01875% TFA in Acetonitrile (v/v), gradient: 0.0 min 5% B→- 3.0 min 95% B→-3.5 min 95% B→3.51 min 5% B→4.0 min 5% B, flow rate: 0.8 mL/min, oven temperature: 50°C; PDA detection: 220 nm & 254 nm.

### Method 3:

Instrument type: SHIMADZU LC-20AD, Column: Kinetex C18 LC Column 4.6 x 50mm, 5um, mobile phase A: 0.0375% TFA in water (v/v), B: 0.01875% TFA in Acetonitrile (v/v), gradient: 0.0 min 10% B→2.40 min 80% B→3.7 min 80%B→3.71 min 10%B→4.00 min 10%B, , flow rate: 1.5 mL/min, oven temperature: 50°C; PDA detection: (220 nm&215nm&254nm)

### 4. Experimental for largest scale run:

### General procedure for preparation of compound 10b

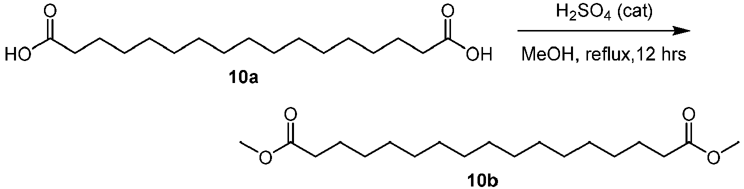

A mixture of compound **10a** (3 g, 9.99 mmol) and H₂SO₄ (8 drops, 98% purity) in MeOH (30 mL) was stirred at 64-65 °C for 12 hours under N₂ atmosphere. 1H NMR showed the compound **10a** was disappeared and compound **10b** was found (The solution was determined by 1H NMR directly). The reaction mixture was cooled to 10-20 °C and kept stirred at 10-20 °C for 30 mins, then filtered and the filter cake was dried under reduced pressure to give compound **10b** (3.02 g, 9.19 mmol, 92.1% yield) as a white solid, which was confirmed by 1H NMR.

**¹H NMR:** 400 MHz, CDCl₃

δ 3.69 (s, 6H), 2.32 (t, *J* = 7.2 Hz, 4H), 1.59-1.69 (m, 4H), 1.22-1.37 (m, 22H).

### General procedure for preparation of compound 10c

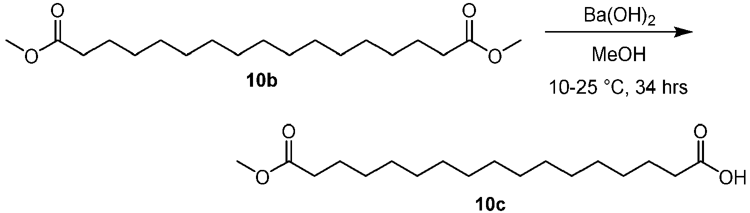

To a suspension of compound **10b** (3.02 g, 9.19 mmol) in MeOH (12.0 mL) was added a solution of Ba(OH)₂ (866 mg, 5.05 mmol) in MeOH (12 mL) at 10-20 °C under N ₂ atmosphere and kept stirred at 15-25 °C for 22 hou rs under N₂ atmosphere. 1H NMR showed the compound **10b** was retained (one drop reaction solution was added to a drop 12 M HCl, then determined by 1H NMR). The reaction mixture was stirred at 15-25 °C for 12 hours under N₂ atmosphere. 1H NMR showed the most compound **10c** was found (one drop reaction solution was added to a drop 12 M HCl, then determined by 1H NMR). The reaction mixture was filtered and the filter cake was suspension in 4 M HCI solution (15 mL) at 15-25 °C and kept stirred at 15-25 °C for 30 mins, then the mixt ure was filtered and filter cake was dried under reduced pressure to give compound **10c** (2.42 g, 7.70 mmol, 83.7% yield) as a white solid, which was confirmed by 1H NMR.

**¹H NMR:** 400 MHz, CDCl₃

δ 3.60 (s, 3H), 2.19-2.33 (m, 4H), 1.48-1.63 (m, 4H), 1.12-1.33 (m, 22H).

### General procedure for preparation of compound 10d

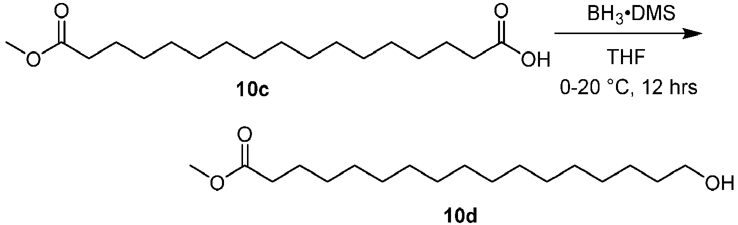

To a suspension of compound **10c** (2.42 g, 7.70 mmol) in THF (25.0 mL) was added dropwise BH₃-Me₂S (10.0 M, 940 uL) at 0-10 °C under N ₂ atmosphere and kept stirred at 10-20 °C for 12 hours under N₂ atmosphere. 1H NMR showed the most compound **10d** was found (one drop reaction solution was added to a drop 12 M HCl, then determined by 1H NMR). The reaction mixture was poured into saturated NH₄Cl solution (25 mL) at 0-10 °C and kept stirred at 10-20 °C for 15 mins, the aqueous phase was extracted with EtOAc (50 mLx 2), the combined organic phase was dried over anhydrous Na₂SO₄, filtered and filtrate was concentrated under reduced pressure. The residue was checked by 1H NMR. TLC 1 (Petroleum ether/Ethyl acetate = 3/1). The residue was purified by silica gel column (Petroleum ether/Ethyl acetate, 20/1 to 3/1), the spot (Rf = 0.32) was collected to give compound **10d** (1.98 g, 6.59 mmol, 85.6% yield) as a white solid, which was confirmed by 1H NMR.

**¹H NMR:** 400 MHz, CDCl₃

δ 3.60 (s, 3H), 3.57 (t, *J* = 7.2 Hz, 2 H), 2.23 (t, *J* = 7.2 Hz, 2H), 1.44-1.61 (m, 4H), 1.13-1.33 (m, 22H).

### General procedure for preparation of compound 10e

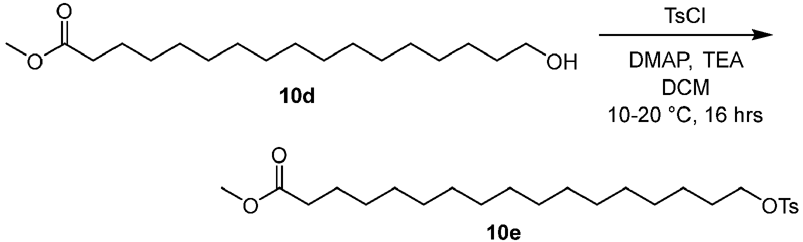

To a solution of compound **10d** (1.98 g, 6.59 mmol) in DCM (20.0 mL) was added TEA (1.33 g, 13.2 mmol, 1.83 mL), DMAP (80 mg, 655 umol) and 4-methylbenzenesulfonyl chloride (1.63 g, 8.57 mmol) at 10-20 °C under N ₂ atmosphere and kept stirred at 10-20 °C for 16 hours under N₂ atmosphere. To the mixture was added water (50.0 mL) at 10-20 °C and the organic phase was dried over anhydrous Na₂SO₄, filtered and filtrated was concentrated under reduced pressure. The residue was purified by silica gel column (Petroleum ether/Ethyl acetate, 1/0 to 3/1), the spot (Rf = 0.83) was collected to give compound **10e** (1.43 g, 3.15 mmol, 47.7% yield) as a white solid.

**¹H NMR:** 400 MHz, CDCl₃;

δ 7.72 (d, *J* = 8.4Hz, 2H), 7.27 (d, *J* = 8.4 Hz, 2H), 3.95 (t, *J* = 6.4 Hz, 2H), 3.60 (s, 3H), 2.38 (s, 3H), 2.23 (t, *J =* 7.6 Hz, 2H), 1.52-1.60 (m, 4H), 1.10-1.28 (m, 24H).

### General procedure for preparation of compound 10f

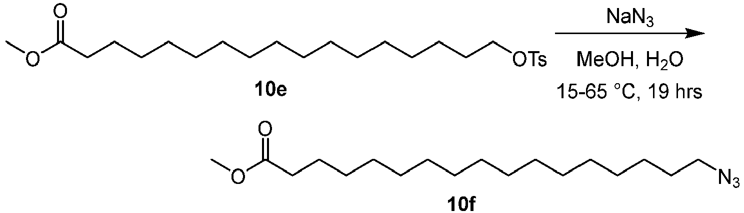

To a solution of methyl compound **10e** (1.43 g, 3.15 mmol) in MeOH (15.0 mL) and H₂O (1.50 mL) was added NaN₃ (310 mg, 4.77 mmol) at 15-25 °C, then the mixture was heated to 64-65 °C and kept st irred at 64-65 °C for 19 hours. 1H NMR showed the compound **10e** was disappeared and compound **10f** was found (To the 0.3 mL of the reaction solution was added saturated NaHCO₃ (5 mL) and DCM (4.00 mL), the organic phase was concentrated under reduced pressure at 25 °C, t hen determined by 1H NMR). The reaction mixture was cooled to 15-25 °C. The reaction mixture was not purified and used for next step directly.

**¹H NMR:** 400 MHz, CDCl₃;

δ 3.69 (s, 3H), 3.28 (t, J= 6.8 Hz, 2H), 2.34 (t, J= 6.8 Hz, 2H), 1.56-1.72 (m, 4H), 1.24-1.43 (m, 22H).

### General procedure for preparation of compound 10g

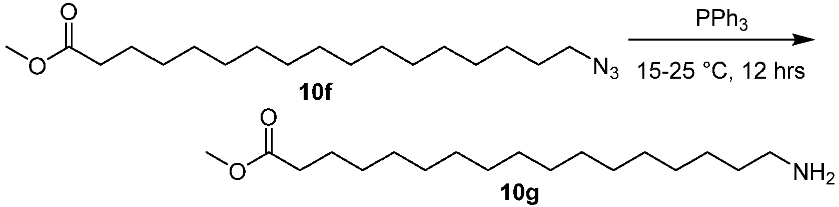

To the mixture of compound **10f** (1.02 g, 3.13 mmol) was added PPh₃ (1.48 g, 5.64 mmol) at 15-25 °C and kept stirred at 15-25 °C for 12 hours. 1H NMR showed the compound **10f** was disappeared and the compound **10g** was found (The reactant solution was determined by 1H NMR directly). To the mixture was added 1 M HCI solution until pH = 1-2 at 15-25 °C, then the mixture was concentrated under redu ced pressure. To the residue was added EtOAc (15.0 mL) at 15-25 °C and kept stirred at 15-25 °C for 15 mins, filtered and filter cake was dried under reduced pressure to give **10g** (1.15 g, TsOH and HCI salt) as a white solid.

**¹H NMR:** 400 MHz, DMSO-*d₆*;

δ 7.70 (s, 3H), 7.48 (d, *J* = 8.0 Hz, 1H), 7.12 (d, *J* = 8.0 Hz, 1H), 3.58 (s, 3H), 2.70-2.83 (m, 2H), 2.29 (t, *J* = 7.6 Hz, 2H), 2.29 (s, 1.5H), 1.45-1.58 (m, 4H), 1.19-1.34 (m, 24H).

### General procedure for preparation of compound 10h

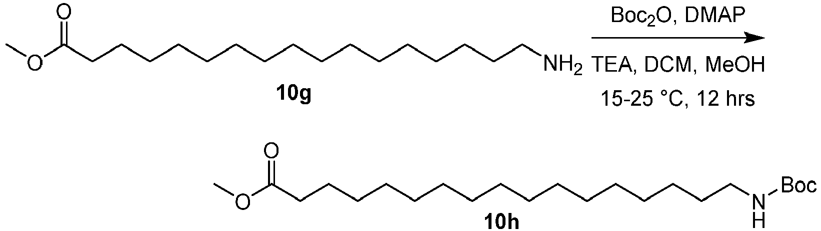

To the mixture of compound **10g** (1.15 g, TsOH and HCI salt) in DCM (10 mL) and MeOH (1 mL) was added Boc₂O (1.03 g, 4.71 mmol, 1.08 mL), DMAP (39 mg, 319 umol) and TEA (794 mg, 7.84 mmol, 1.09 mL) at 15-25 °C and kept stirred at 15-25 °C for 12 hours under N₂ atmosphere. MS showed the MS value of compound **10g** was disappeared. The reaction mixture was concentrated under reduced pressure. The residue was purified by silica gel column (Petroleum ether/Ethyl acetate, 1/0 to 20/1) to give compound **10h** (341 mg, 853 umol, 27.2% yield) as a white solid.

**¹H NMR:** 400 MHz, CDCl₃;

δ 4.42 (s, 1H), 3.60 (s, 3H), 3.03 (q, *J* = 6.4 Hz, 2H), 2.23 (t, *J* = 7.6 Hz, 2H), 1.49-1.61 (m, 4H), 1.37 (s, 9H), 1.11-1.29 (m, 24H).

### General procedure for preparation of compound 10i

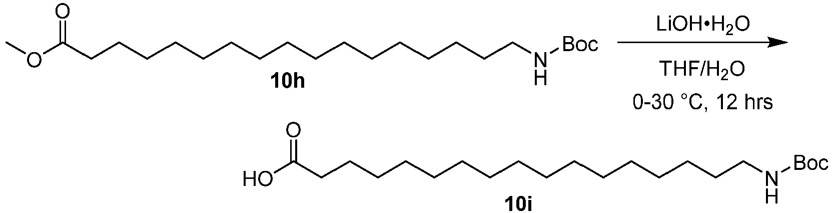

To the mixture of compound **10h** (341 mg, 853 umol) in THF (4 mL) and H₂O (1 mL) was added LiOH•H₂O (46 mg, 1.10 mmol) at 0-10 °C and kept stirred at 20-30 °C for 12 hours. To the mixture w as added water (20.0 mL) and EtOAc (50 mL), then 10% citric acid solution until pH = 3 at 0-10 °C. The organic phase was dried over anhydrous Na₂SO₄, filtered and filtrate was concentrated under reduced pressure, which was confirmed by 1H NMR. The residue was purified by silica gel column (Petroleum ether/Ethyl acetate, 1/0 to 5/1). The spot (Rf = 0.22) was collected to give compound **10i** (228 mg, 591 umol, 69.3% yield) as a white solid.

**¹H NMR:** 400 MHz, CDCl₃

δ 4.54 (s, 1H), 3.00-3.20 (m, 2H), 2.37 (t, *J* = 7.2 Hz, 2H), 1.60-1.70 (m, 4H), 1.46 (s, 9H), 1.21-1.40 (m, 24H).

### General procedure for preparation of compound 10j

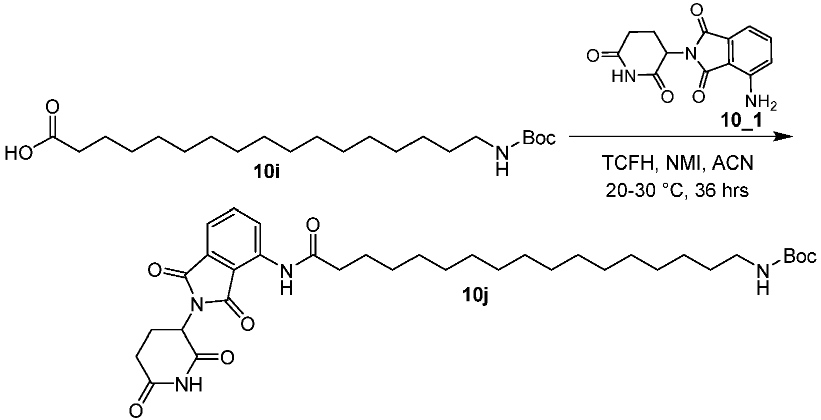

To the solution of compound **10i** (128 mg, 332 umol) and compound **10_1** (118 mg, 432 umol) in ACN (30.0 mL) was added NMI (190 mg, 2.31 mmol, 184 uL) and TCFH (240 mg, 855 umol) at 20-30 °C under N ₂ atmosphere and kept stirred at 20-30 °C for 20 hours under N₂ atmosphere. LCMS showed the MS value of compound **10j** was not found, to the mixthue was added TCFH (240 mg, 855 umol) and NMI (190 mg, 2.31 mmol, 184 uL) at 20-30 °C under N ₂ atmosphere and kept stirred at 20-30 °C for 16 hours under N ₂ atmosphere. LCMS showed the MS value of compound **10j** (RT = 1.208 mins, MS = 641.5) was found. The reaction mixture was poured into water (30 mL) at 0-10 °C, t he aqueous phase was saturated with NaCl solid and extracted with EtOAc (80 mL × 2), the combined organic phase was dried over anhydous Na₂SO₄, filtered and filtrate was concentrated under reduced pressure at 30 °C, which was confirmed by LCMS RT = 1.215 mins, MS = 641.5). The residue was purified by reverse phase (TFA), the collected fraction (RT = 1.215 mins) was concentrated under reduced pressure directly to give compound **10j** (42 mg, 65.5 umol, 19.7% yield, 100% purity) as a white solid.

### LCMS: (method 1), RT = 1.213 mins, m/z (M+1) = 641.5

### General procedure for preparation of compound 10k

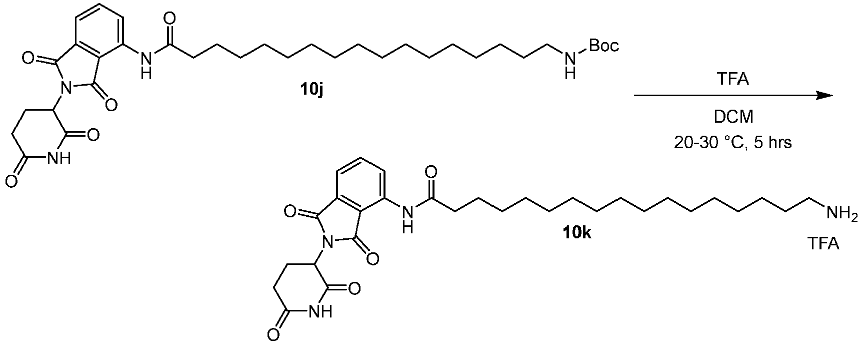

To the solution of compound **10j** (42 mg, 65.5 umol) in DCM (3.00 mL) was added TFA (154 mg, 1.35 mmol, 100 uL) at 20-30 °C u nder N₂ atmosphere and kept stirred at 20-30 °C for 5 hours under N ₂ atmosphere. LCMS showed the MS value of compound **10k** (RT = 0.938 mins, MS = 541.3) was found. The reaction mixture was concentrated under reduced pressure at 30 °C to give compound **10k** (40 mg, 58.8 umol, 89.8% yield, 96.3% purity) as a yellow solid, which was confirmed by LCMS, RT = 0.933 mins, m/z (M+1) = 541.4).

**LCMS:** (method 1), RT = 0.933 mins, m/z (M+1) = 541.4

### General procedure for preparation of "A57"

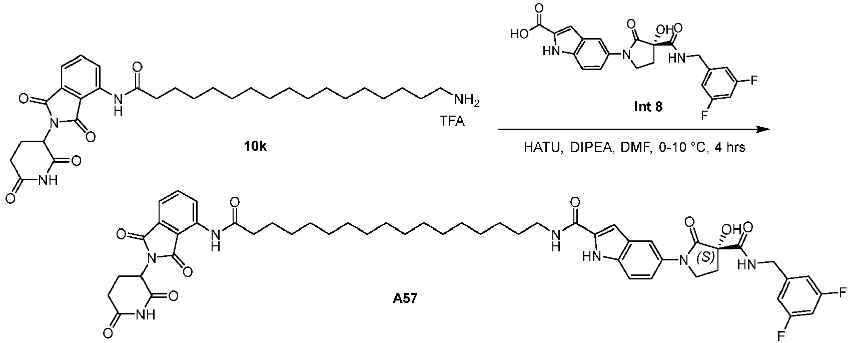

To the solution of compound **int 8** (30 mg, 69.9 umol) and DIPEA (37 mg, 286 umol, 49.9 uL) in DMF (2.00 mL) was added HATU (40 mg, 105 umol) and a solution of compound **10k** (48 mg, 73.3 umol, TFA) in DMF (2.00 mL) at 0-10 °C under N ₂ atmosphere and kept stirred at 0-10 °C for 4 hours under N₂ atmosphere. To the reaction mixture was added glacial acetic acid until pH = 5-6 at 0-5 °C, then the mixture was poured into water (20.0 mL) at 0-5 °C, the aqueous phase was extracted with EtOAc (30.0 mL × 3), the combined organic phase was dried over anhydrous Na₂SO₄, filtered and filtrate was concentrated under reduced pressure, the residue was checked by LCMS. The residue was purified by prep-HPLC (column: Phenomenex luna C18 150*40mm*15um; mobile phase: [water (0.1%TFA)-ACN]; B%: 62%-92%, 11 min), the collected fraction was concentrated under reduced pressure directly. The residue was checked by LCMS (RT = 2.946 mins, MS = 952.5). The residue was further purified by prep-HPLC (column: Phenomenex Gemini-NX C18 75*30mm*3um; mobile phase: [water (0.1%TFA)-ACN]; B%: 70%-80%, 7min), the collected fraction was concentrated under reduced pressure directly to give **"A57"** (7.02 mg, 7.25 umol, 10.4% yield, 98.3% purity) as a brown solid.

**¹H NMR:** 400 MHz, DMSO-*d₆*;

δ 11.58 (s, 1H), 11.15 (s, 1H), 9.68 (s, 1H), 8.69 (t, *J* = 6.0 Hz, 1H), 8.42-8.52 (m, 2H), 7.83 (t, *J* = 7.6 Hz, 1H), 7.78 (d, *J* = 1.6 Hz, 1H), 7.61 (d, *J* = 6.8 Hz, 1H), 7.53 (dd, *J* = 8.8, 2.0 Hz, 1H), 7.43 (d, *J* = 8.8 Hz, 1H), 7.11 (d, *J* = 1.6 Hz, 1H), 6.97-7.09 (m, 3H), 6.72 (s, 1H), 5.14 (dd, *J* = 12.8, 6.4 Hz, 1H), 4.43 (dd, *J* = 15.6, 6.8 Hz, 1H), 4.28 (dd, *J* = 16.4, 6.0 Hz, 1H), 3.87-3.93 (m, 1H) 3.28 (q, *J* = 6.4 Hz, 3H), 3.05 (q, *J* = 6.0 Hz, 1H), 2.83-2.97 (m, 2H), 2.55-2.70 (m, 7H), 2.46 (t, *J* = 7.6 Hz, 2H), 2.13-2.20 (m, 1H), 1.99-2.10 (m, 3H), 1.46-1.67 (m, 5H), 1.15-1.41 (m, 46H).

**¹⁹F NMR:** 400 MHz, DMSO-*d₆*;

δ -110.35.

**LCMS:** (method 2), RT = 2.946 mins, m/z (M+1) = 952.5 **LCMS:** (method 1), RT = 1.139 mins, m/z (M+1) = 952.5

**HPLC:** (method 3), RT = 2.715 min

### 2. Exemplary Testing of the compound

### 1. Cell Seeding

HCT116 cells were sown in 6-well plates. Pipette 1 mL fresh medium to every well and incubate for at least 1 h (37°C, 5% CO ₂, 95% rH).

Harvest and count cells in Vi-Cell. Equinr. 70198475 Adjust cell concentration to 0.35e⁶ cells/mL and add 1 mL of cell suspension (total 0,35e⁶ cells/well). Incubate cells for 24 hours (37 °C, 5 % CO ₂, 95 % rH)

### 2. Compound Treatment

HCT116: 24h: ~50% confluent before treatment

### Treatment with selected compounds

Compounds will be thawed the first time, stock: 10 mM; final concentration: 10 µM & 1µM & 0.1µM

Treatment with DMSO based solution with Digital Dispenser: HCT116 platte 2A1.jpg_meta.xml
→ create new dispensing protocol for compound
→ apply name of test compound and its stock concentration (in mM) for testing
→ 3x 1:10 dilution series
→ final 0.3% DMSO, DMSO normalization for every well
→ shaking after copmpound/DMSO addition
Incubate cells for further 24 hours (37 °C, 5 % CO ₂, 95 % rH)

### 3. Cell Lysis

HCT116: 70-80% confluent before lysis
- after incubation cells were lysed with RIPA lysis buffer including Halt^{™} Protease and Phosphatase Inhibitor
   Cocktail (1x)
   all steps performed on ice or at 4°C
- aspirate supernatant from cells
- wash cells twice with 2.0 mL ice cold PBS buffer
- aspirate supernatant from cells after each washing step
- lyse cells with 100 µL Ripa lysis buffer per well
- scrape cells with cell scraper and transfer lysates to Eppendorf Cups
- incubate for 15 min on ice
- centrifuge 150 min at 13000 x g and 4 °C
- transfer supernatants to fresh Eppendorf cups
- removal of 20 µL lysate for BCA assay
- aliquot and freeze at -20°C

### 4. BCA Assay of protein extracts

Determination of protein concentration of lysates with BCA Protein Assay Kit. BSA supplied with the kit is 2 mg/mL.

Dilute the standard (Stock BSA concentration = 2mg/mL) in a 96 well plate: Samples were run 1:4. Dilute in lysing buffer, lysing buffer alone was run to determine the background from detergents in the assay.

BCA reagent: Dilute Reagent A and Reagent B in a 1:50 ratio. To 9.8 mL A add 200 µL B.

Assay Procedure: Pipette 5 µL/well of standard or samples to 96 wells (see plate layout). Samples were run in duplicate. Blanks = 5 µL/well PBS or 5 µL/well lysing buffer. Add 100 µL/well of the prepared BCA reagent. Tap plate to mix or shake for 1-2 min on the Eppendorf mixer. Incubate at 37°C for 30 min.

Read the OD at 562 nm. Plate was read on EnVision 2104 Multilabel Reader (Perkin Elmer, Equipment Number 70160330) last verification of instrument 17.08.2017 (once a year) (A25/531). AEH181012_BCA.csv

### 5. Data calculation:

In EXCEL the duplicates were averaged and the average background PBS was subtracted for the standard curve.

A trend line was created, the intercept (b) was set at 0. The R2 should be >0.950 for acceptable results. The lysate buffer blank was subtracted from the test values and the averaged, background-corrected O.D. values were used with the equation to calculate mg/ml and then were corrected for the dilution factor for the final mg/mL value. Only results from OD values within the range of the standard curve are acceptable. Values outside of the standard curve range are ignored. BCA AEH00350 Berechnung.xlsx

### 6. Sample Calculation and Preparation

- thaw lysates of AEH00350 on ice
- calculate sample volume for Western Blot loading 10 µg total protein / 13 µL per slot:
- dilute samples with lysis buffer and 10x reducing agent & 4x LDS buffer to 10µg.
- heat them at 70°C for 10 min and 1000 rmp

### 7. WB Analysis

- insert one NuPAGE 4-12% Bis-Tris Midi Gel ( 26 well) and fix in Criterion Cell Device electrophoresis chamber (BIO-RAD, #165-6001)
- fill chamber with 1x NuPAGE MES SDS Running-Buffer
- pipette samples, see Blue Plus 2 Pre-Stained Protein Standard and MagicMark XP per comb:
- 3 µL Magic Marker and 7 µL Seeblue Marker, 13 µL sample per slotlayout.xlsx
- two gels with following layout
- 2D-SDS-Page run: 45 min with const. 200 V (right power supply)
- soak nitrocellulose membrane in Methanol and blot paper in 1x transfer buffer
- preparation of the blot sandwich: 2x blot paper, gel, membrane, 2x blot paper at a Fast Blot B44 (Biometra, #015-200)
- Western Blot run: 1h 35 min with const. 150 mA

### Blocking

- transfer the membrane to Blocking-Buffer and shake for 1 h at RT
- Blocking solution: Odyssey Blocking Buffer

Incubation with primary antibody MetAP2 clone EPR6887 RabMAb Abcam ab134124 (1:5000)
- Primary antibody solution: 0.5x Odyssey Blocking Buffer + 0.5x PBS + 0.1% Tween20
- incubation with primary antibody over night at 4°C with gentle shaking:

Incubation with secondary antibody (Goat anti-rabbit (700nm), Molecular Probes A21076 39542A (1:20000)
- wash membranes 4 times for 10 minutes at room temperature
- Wash solution: 1x PBS + 0.1% Tween20
- Incubate membrane with secondary antibody for 1h on a shaker at room temperature in the dark
- Secondary antibody solution: 0.5x Odyssey Blocking Buffer + 0.5x PBS + 0.1% Tween20 + 0.01%

SDS
- last washing step only in 1x PBS
- scan membranes on Odyssey Imager

**Table 1**

| IC₅₀ of compounds of the formula I according to the invention | | |
|---|---|---|
| Compound No. | IC₅₀ enzyme [µM] | IC₅₀ HUVEC [µM] |
| "A1" | 0.19 | 25 |
| "A2" | 0.25 | 30 |
| "A3" | 0.55 | 30 |
| "A4" | 0.48 | 1.6 |
| "A5" | 0.33 | 5.8 |
| "A6" | 0.37 | 30 |
| "A7" | 0.32 | 7.3 |
| "A8" | 0.33 | 30 |
| "A42" | | 4.6 |
| "A43" | | 7.1 |
| "A44" | | 7 |
| "A45" | | 0.61 |
| "A46" | | >30 |
| "A47" | | 0.24 |
| "A48" | | 0.085 |
| "A49" | | 0.051 |
| "A50" | | 0.18 |
| "A51" | | 0.4 |
| "A52" | | 4.9 |
| "A53" | | 2.8 |
| "A54" | | 0.11 |
| "A55" | | >30 |
| "A56" | | 0.12 |
| "A57" | | >30 |

The following examples relate to medicaments:

### Example A: Injection vials

A solution of 100 g of an active ingredient of the formula I and 5 g of disodium hydrogenphosphate in 3 l of bidistilled water is adjusted to pH 6.5 using 2 N hydrochloric acid, sterile filtered, transferred into injection vials, lyophilised under sterile conditions and sealed under sterile conditions. Each injection vial contains 5 mg of active ingredient.

### Example B: Suppositories

A mixture of 20 g of an active ingredient of the formula I with 100 g of soya lecithin and 1400 g of cocoa butter is melted, poured into moulds and allowed to cool. Each suppository contains 20 mg of active ingredient.

### Example C: Solution

A solution is prepared from 1 g of an active ingredient of the formula I, 9.38 g of NaH₂PO₄ · 2 H₂O, 28.48 g of Na₂HPO₄ · 12 H₂O and 0.1 g of benzalkonium chloride in 940 ml of bidistilled water. The pH is adjusted to 6.8, and the solution is made up to 1 l and sterilised by irradiation. This solution can be used in the form of eye drops.

### Example D: Ointment

500 mg of an active ingredient of the formula I are mixed with 99.5 g of Vaseline under aseptic conditions.

### Example E: Tablets

A mixture of 1 kg of active ingredient of the formula I, 4 kg of lactose, 1.2 kg of potato starch, 0.2 kg of talc and 0.1 kg of magnesium stearate is pressed in a conventional manner to give tablets in such a way that each tablet contains 10 mg of active ingredient.

### Example F: Dragees

Tablets are pressed analogously to Example E and subsequently coated in a conventional manner with a coating of sucrose, potato starch, talc, tragacanth and dye.

### Example G: Capsules

2 kg of active ingredient of the formula I are introduced into hard gelatine capsules in a conventional manner in such a way that each capsule contains 20 mg of the active ingredient.

### Example H: Ampoules

A solution of 1 kg of active ingredient of the formula I in 60 l of bidistilled water is sterile filtered, transferred into ampoules, lyophilised under sterile conditions and sealed under sterile conditions. Each ampoule contains 10 mg of active ingredient.

## Claims

1. Compounds of the formula I
Q¹-Q²-Q³ I
in which
Q¹ denotes 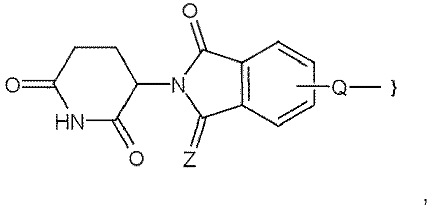 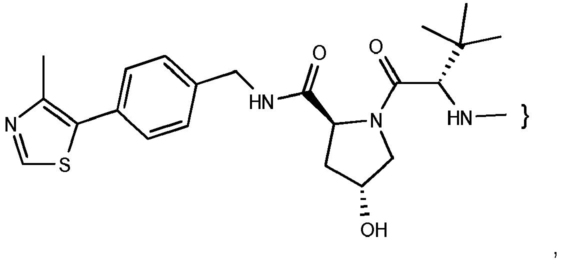 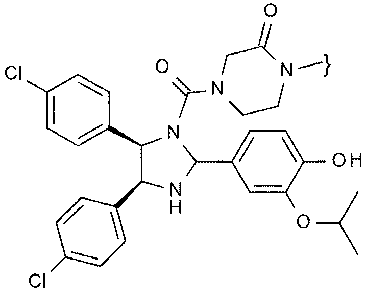
or 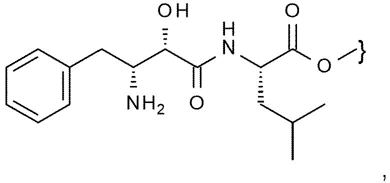
Z denotes O or CH₂,
Q denotes O, NH, NCH₃ or CH₂,
Q² denotes unbranched alkylene having 4-25 C atoms, in which 1-8 non-adjacent CH₂ groups may be replaced by O, CONH and/or NHCO and in which one CH₂ group may be replaced by 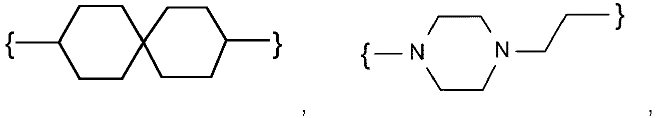 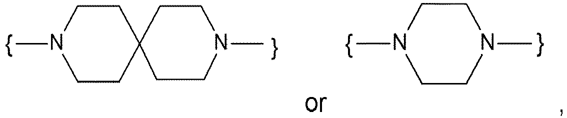
Q³ denotes 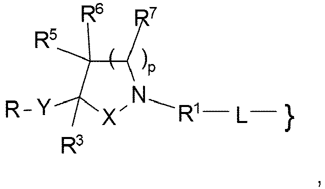
L denotes NR⁴CO, CONR⁴, NH, O, CO, S, SO₂, SO(=NH), NHCONH, SO₂NH or NHSO₂,
R denotes NR²R⁴, Alk, C(=CH₂)[C(R⁴)₂]ₙAr², Het², O[C(R⁴)₂]ₙAr² or OA,
X denotes CO or CH₂,
Y denotes CO or CH₂,
R¹ denotes (CH₂)ₙ, [C(R⁴)₂]ₙAr¹-, (CH₂)ₙHet-, (CH₂)ₙCyc-, [C(R⁴)₂]ₙCONHAr¹-, [C(R⁴)₂]ₙNA-, O[C(R⁴)₂]ₙAr¹- or [C(R⁴)₂]ₙCOO(CH₂)ₙAr¹-, wherein substituent L directly is connected to Ar¹, Het or Cyc,
R² denotes H, [C(R⁴)₂]ₙAr², (CH₂)ₙCOHet¹, (CH₂)ₙCOAr², (CH₂)ₘNA₂, (CH₂)ₙCyc or (CH₂)ₙHet¹,
R³ denotes OH or OCOA,
R⁴ denotes H or alkyl having 1, 2, 3 or 4 C-atoms,
R² and R⁴ together also denote alkylene having 2, 3, 4 or 5 C-atoms, where a CH₂ group may also be replaced by N(CH₂)ₘOH or SO₂,
R⁵, R⁶ each, independently of one another H, F or A,
R⁵ and R⁶ together also denote alkylene having 2, 3, 4 or 5 C-atoms,where a CH₂ group may also be replaced by NCOA or O,
R⁷ denotes H, Hal or A,
Ar¹ denotes phenyl which is unsubstituted or mono-, di- tri-, tetra- or pentasubstituted by Hal, OH, OA, CONH₂, CONHA, CONA₂, NHSO₂A, CONHCyc, NHSO₂Cyc, CONHAr², Het¹, COHet¹ and/or NASO₂A,
Ar² denotes phenyl which is unsubstituted or mono-, di-, tri-, tetra- or pentasubstituted by Hal, A, CONH₂, and/or OAr³,
Ar³ denotes phenyl which is unsubstituted or monosubstituted by NH₂,
Het denotes a mono- or bicyclic saturated, unsaturated or aromatic heterocycle having 1 to 4 N, and/or O and/or S atoms which is unsubstituted or mono-, di- or trisubstituted by Hal, A, OA, CN, NH₂, NHA, NA₂, NO₂, CN, COOH, COOA, (CH₂)ₙCONH₂, (CH₂)ₙCONHA, (CH₂)ₙCONA₂, NHCOA, COA, CHO, Het¹, SO₂A, SO₂NH₂, SO₂NHA, SO₂NA₂, CONHNH₂, CONHAr³, =O and/or Ar³,
Het¹ denotes pyridazinyl, pyrazolyl, pyridyl, piperazinyl, morpholinyl, pyrimidinyl, furyl, thienyl, imidazolyl, pyrrolyl, oxazolyl, oxadiazolyl, isoxazolyl, thiazolyl, triazolyl, tetrazolyl, thiadiazole, piperidin-1-yl, pyrrolidin-1-yl, tetrahydropyranyl, 1,2-oxazinan-2-yl, 1,2,5-oxadiazinan-2-yl, 1,3-oxazinan-3-yl or hexahydro-pyrimidinyl, each of which is unsubstituted or mono-, di- or trisubstituted by A and/or OA,
Het² denotes isoindolyl,
A denotes unbranched or branched alkyl having 1-10 C atoms, in which 1-7 H atoms may be replaced by F, Cl, Br, OH, CHO, COA, COOA, CN, CONA₂, CONHA and/or CONH₂, and/or in which one or two non-adjacent CH and/or CH₂ groups may be replaced by O, or Cyc,
Alk denotes alkenyl having 2, 3, 4, 5 or 6 C atoms
Cyc denotes cyclic alkyl having 3-7 C atoms which is unsubstituted or mono-, di- or trisubstituted by NHCOA, NHSO₂, OH, OA, A, NH₂, NHA, NA₂, COOA, COOH and/or CONHA,
Hal denotes F, Cl, Br or I,
m denotes 1, 2, 3 or 4,
n denotes 0, 1, 2, 3 or 4,
p denotes 1, 2 or 3,
and pharmaceutically acceptable salts, tautomers and stereoisomers thereof, including mixtures thereof in all ratios.

2. Compounds according to claim 1 in which
Het denotes pyrazinyl, pyrazolyl, benzimidazolyl, pyridyl, thienyl, furanyl, indolyl, dihydroindolyl, benzofuranyl, tetrahydropyranyl, dihydroquinolinyl, dihydroisoquinolinyl, tetrahydroquinolinyl, tetrahydroisoquinolinyl, indazolyl, imidazolyl, pyrrolyl, oxazolyl, oxadiazolyl, isoxazolyl, benzothiazolyl, piperidin-1-yl, pyrrolidin-1-yl, 3,4-dihydro-2H-pyrido[3,2-b]-1,4-oxazinyl, 3,4-dihydro-2H-benzo-1,4-oxazinyl, benzofuranyl, azetidinyl, 1H-pyrrolo[2,3-b]-pyridinyl, 2H-chromenyl, 3-azabicylo[3.2.0]hexyl, pyrrolo[2,3-b]pyridinyl, tetrahydrofuranyl, tetrahydro-1,8-naphthyridinyl 2,3-dihydro-benzoisothiazolyl, 1,2,3,4-tetrahydrobenzothiazinyl or hexahydrobenzo-1,3-dioxolyl, each of which is unsubstituted or mono-, di- or trisubstituted by Hal, A, OA, CN, NH₂, NHA, NA₂, NO₂, CN, COOH, COOA, (CH₂)ₙCONH₂, (CH₂)ₙCONHA, (CH₂)ₙCONA₂, NHCOA, COA, CHO, Het¹, SO₂A, SO₂NH₂, SO₂NHA, SO₂NA₂, CONHNH₂, CONHAr³, =O and/or Ar³,
and pharmaceutically acceptable salts, tautomers and stereoisomers thereof, including mixtures thereof in all ratios.

3. Compounds according to Claim 1 or 2, in which
Q¹ denotes 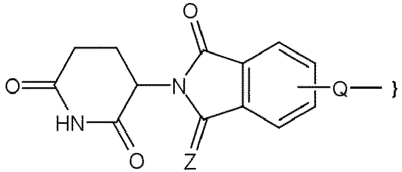
or 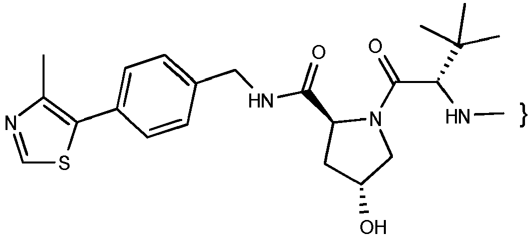 and pharmaceutically acceptable solvates, salts, tautomers and stereoisomers thereof, including mixtures thereof in all ratios.

4. Compounds according to one or more of Claims 1-3, in which
R denotes NR²R⁴,
and pharmaceutically acceptable solvates, salts, tautomers and stereoisomers thereof, including mixtures thereof in all ratios.

5. Compounds according to one or more of Claims 1-4, in which
R¹ denotes (CH₂)ₙ, [C(R⁴)₂]ₙAr¹- or (CH₂)ₙHet-,
and pharmaceutically acceptable solvates, salts, tautomers and stereoisomers thereof, including mixtures thereof in all ratios.

6. Compounds according to one or more of Claims 1-5, in which
R² denotes [C(R⁴)₂]ₙAr², (CH₂)ₙCyc or (CH₂)ₙHet¹,
and pharmaceutically acceptable solvates, salts, tautomers and stereoisomers thereof, including mixtures thereof in all ratios.

7. Compounds according to one or more of Claims 1-6, in which
Het denotes benzimidazolyl or indolyl, each of which is unsubstituted or monosubstituted by Hal,
and pharmaceutically acceptable solvates, salts, tautomers and stereoisomers thereof, including mixtures thereof in all ratios.

8. Compounds according to one or more of Claims 1-7, in which
A denotes unbranched or branched alkyl having 1-6 C atoms, in
which 1-5 H atoms may be replaced by F, Cl and/or OH, and pharmaceutically acceptable solvates, salts, tautomers and stereoisomers thereof, including mixtures thereof in all ratios.

9. Compounds according to one or more of Claims 1-8, in which
Q¹ denotes 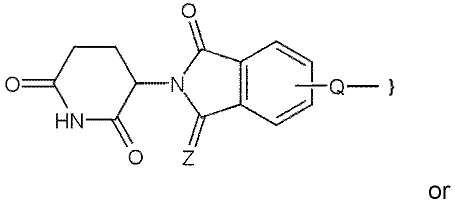 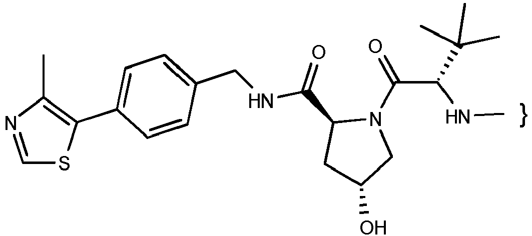
Z denotes O or CH₂,
Q denotes O, NH, NCH₃ or CH₂,
Q² denotes unbranched alkylene having 4-25 C atoms, in which 1-8 non-adjacent CH₂ groups may be replaced by O, CONH and/or NHCO and in which one CH₂ group may be replaced by 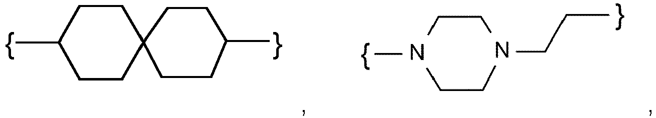 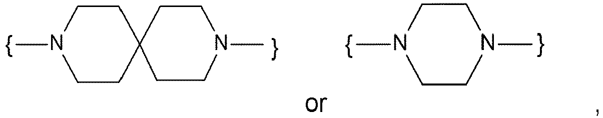
Q³ denotes 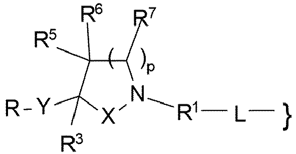
L denotes NR⁴CO, CONR⁴, NH, O, CO, S, SO₂, SO(=NH), NHCONH, SO₂NH or NHSO₂,
R denotes NR²R⁴,
X denotes CO or CH₂,
Y denotes CO or CH₂,
R¹ denotes (CH₂)ₙ, [C(R⁴)₂]ₙAr¹- or (CH₂)ₙHet-, wherein substituent L directly is connected to Ar¹, Het or Cyc,
R² denotes [C(R⁴)₂]ₙAr², (CH₂)ₙCyc or (CH₂)ₙHet¹,
R³ denotes OH,
R⁴ denotes H or alkyl having 1, 2, 3 or 4 C-atoms,
R⁵, R⁶ denote H,
R⁷ denotes H, Hal or A,
Ar¹ denotes phenyl,
Ar² denotes phenyl which is unsubstituted or mono-, di-, tri- or tetra-substituted by Hal,
Het denotes pyrazinyl, pyrazolyl, benzimidazolyl, pyridyl, thienyl, furanyl, indolyl, dihydroindolyl, benzofuranyl, tetrahydropyranyl, dihydroquinolinyl, dihydroisoquinolinyl, tetrahydroquinolinyl, tetra-hydroisoquinolinyl, indazolyl, imidazolyl, pyrrolyl, oxazolyl, oxadiazolyl, isoxazolyl, benzothiazolyl, piperidin-1-yl, pyrrolidin-1-yl, 3,4-dihydro-2H-pyrido[3,2-b]-1,4-oxazinyl, 3,4-dihydro-2H-benzo-1,4-oxazinyl, benzofuranyl, azetidinyl, 1H-pyrrolo[2,3-b]-pyridinyl, 2H-chromenyl, 3-azabicylo[3.2.0]hexyl, pyrrolo[2,3-b]pyridinyl, tetrahydrofuranyl, tetrahydro-1,8-naphthyridinyl 2,3-dihydro-benzoisothiazolyl, 1,2,3,4-tetrahydrobenzothiazinyl or hexahydrobenzo-1,3-dioxolyl, each of which is unsubstituted or mono-, di- or trisubstituted by Hal, A, OA, CN, NH₂, NHA, NA₂, NO₂, CN, COOH, COOA, (CH₂)ₙCONH₂, (CH₂)ₙCONHA, (CH₂)ₙCONA₂, NHCOA, COA, CHO, Het¹, SO₂A, SO₂NH₂, SO₂NHA, SO₂NA₂, CONHNH₂, CONHAr³, =O and/or Ar³,
Het¹ denotes pyridyl, furyl, thienyl, imidazolyl or pyrrolyl,
A denotes unbranched or branched alkyl having 1-6 C atoms, in which 1-5 H atoms may be replaced by F, Cl and/or OH,
Cyc denotes cyclic alkyl having 3-7 C atoms,
Hal denotes F, Cl, Br or I,
n denotes 0, 1, 2, 3 or 4,
p denotes 1, 2 or 3,
and pharmaceutically acceptable solvates, salts, tautomers and stereoisomers thereof, including mixtures thereof in all ratios.

10. Compounds according to Claim 1, selected from the group
| No. | chemical structure |
|---|---|
| "A1" | 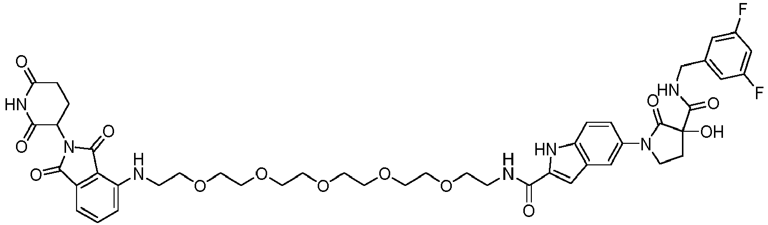 |
| "A2" | 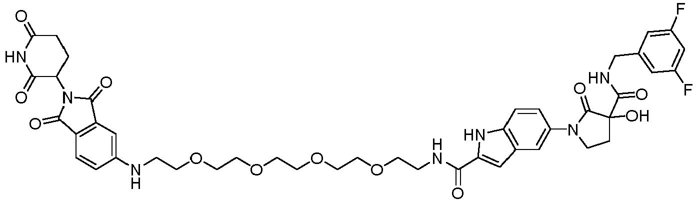 |
| "A3" | 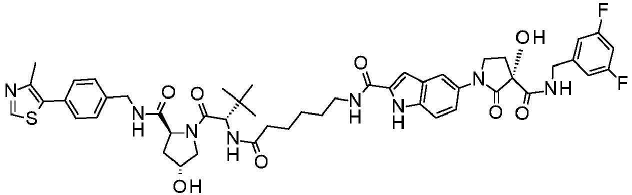 |
| "A4" | 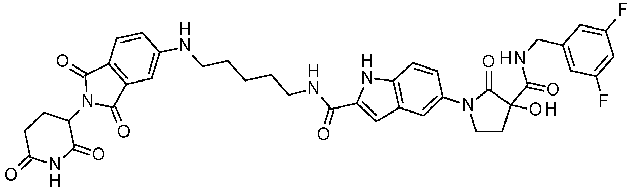 |
| "A5" | 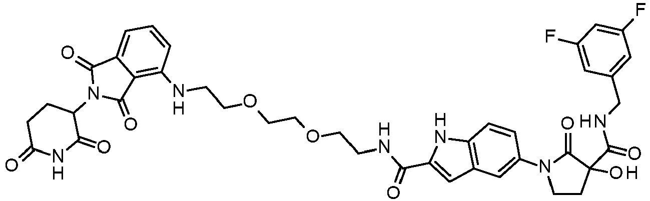 |
| "A6" | 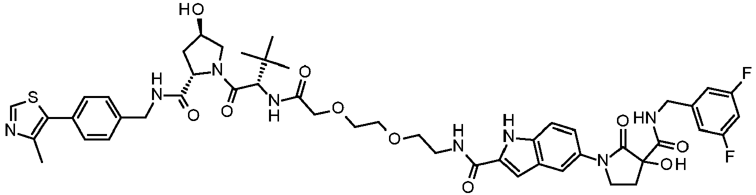 |
| "A7" | 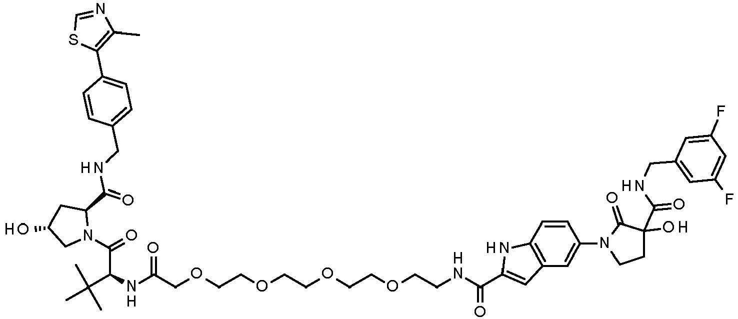 |
| "A8" | 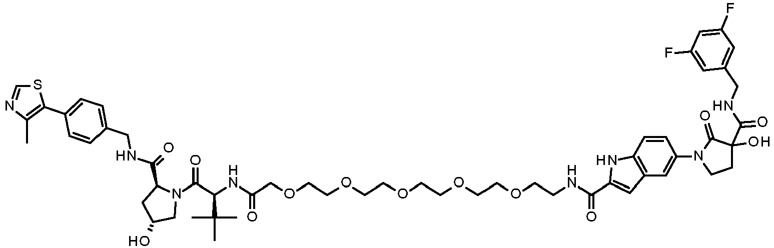 |
| "A9" | 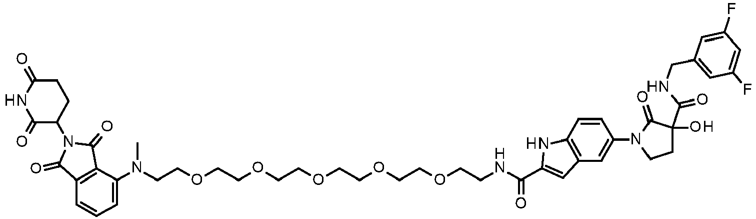 |
| "A10" | 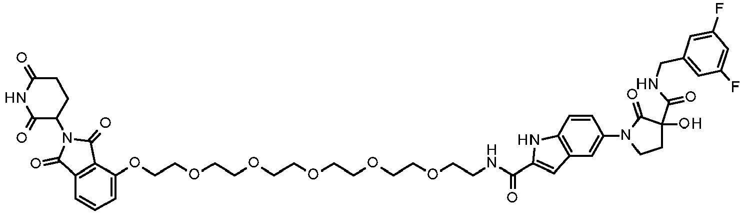 |
| "A11" | 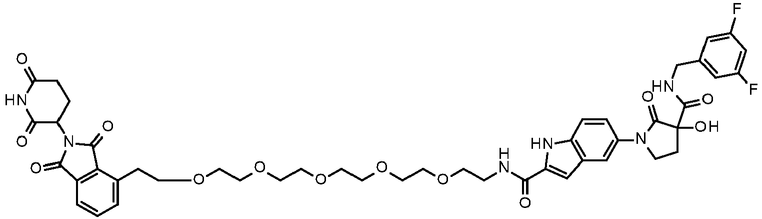 |
| "A12" | 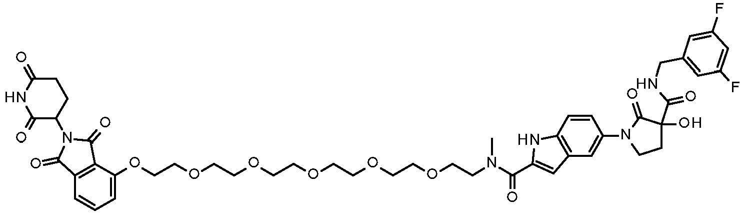 |
| "A13" | 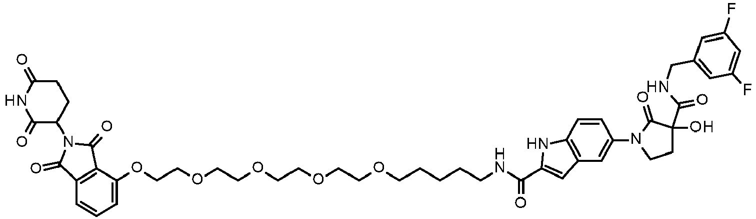 |
| "A14" | 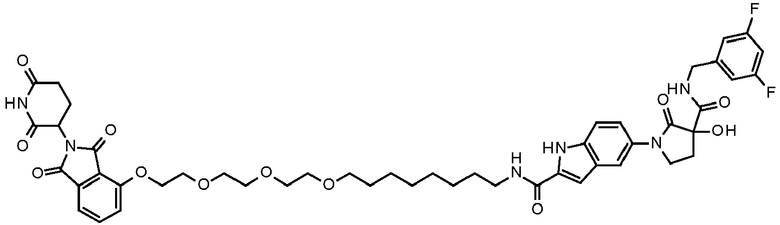 |
| "A15" | 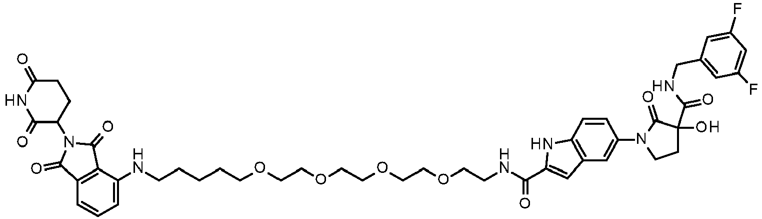 |
| "A16" | 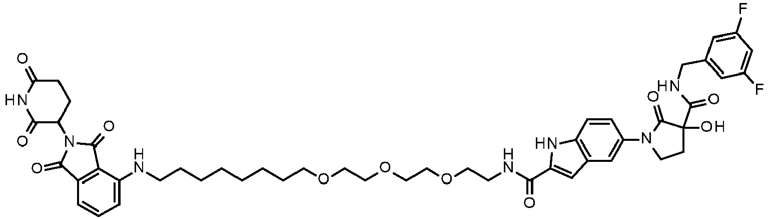 |
| "A17" | 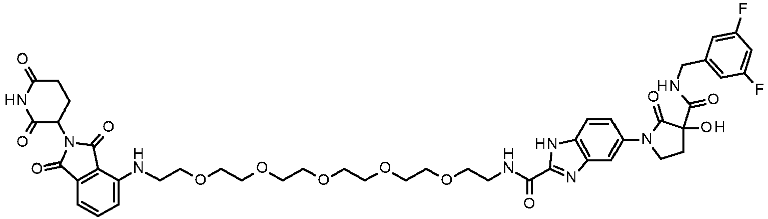 |
| "A18" | 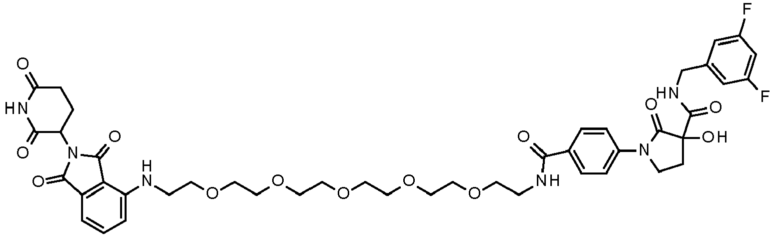 |
| "A19" | 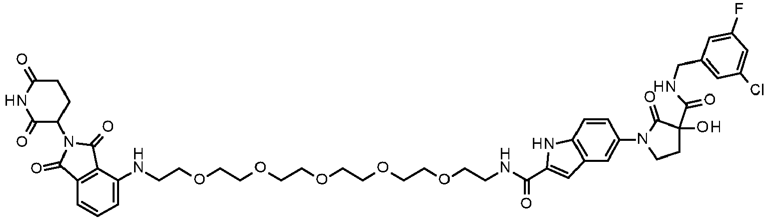 |
| "A20" | 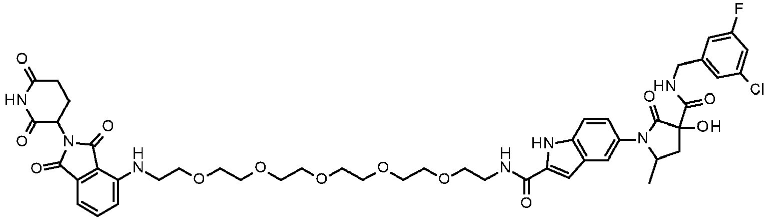 |
| "A21" | 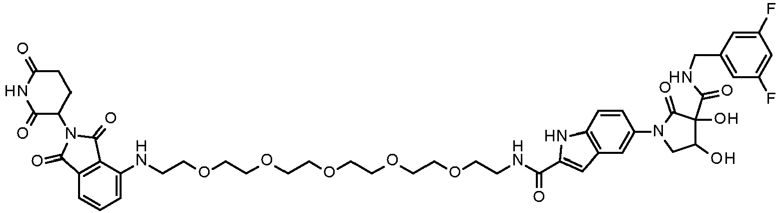 |
| "A22" | 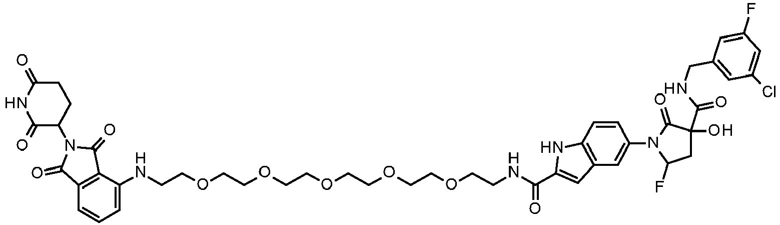 |
| "A23" | 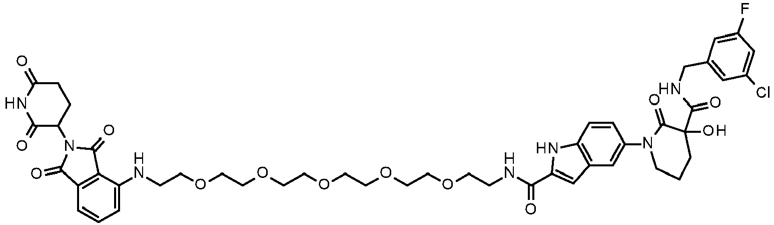 |
| "A24" | 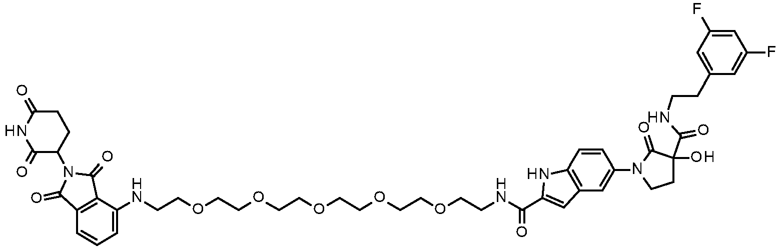 |
| "A25" | 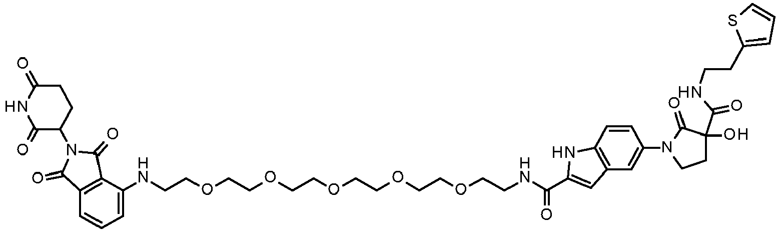 |
| "A26" | 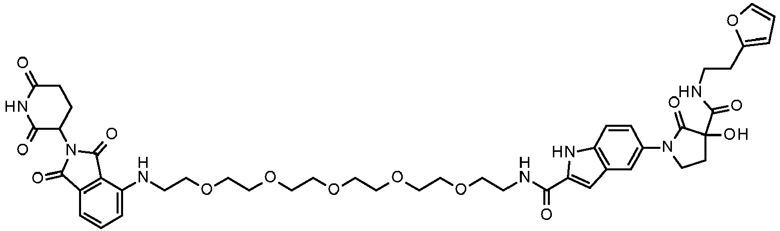 |
| "A27" | 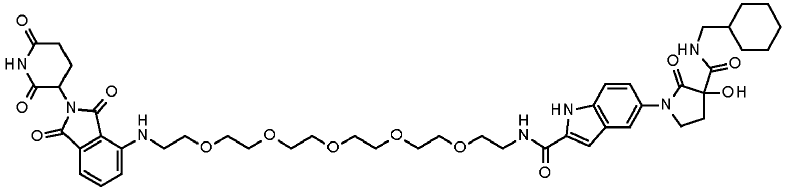 |
| "A28" | 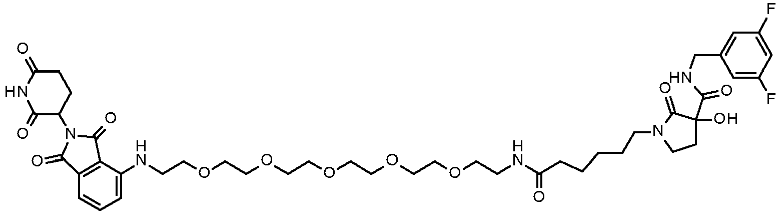 |
| "A29" | 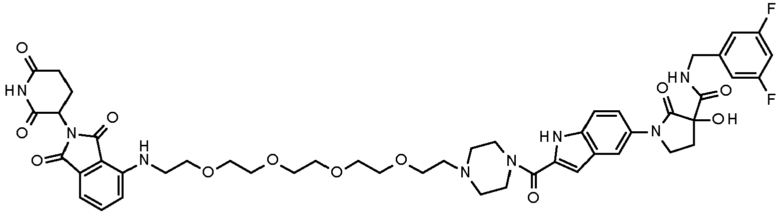 |
| "A30" | 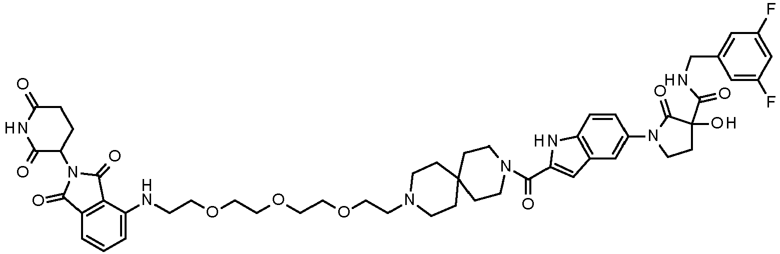 |
| "A31" | 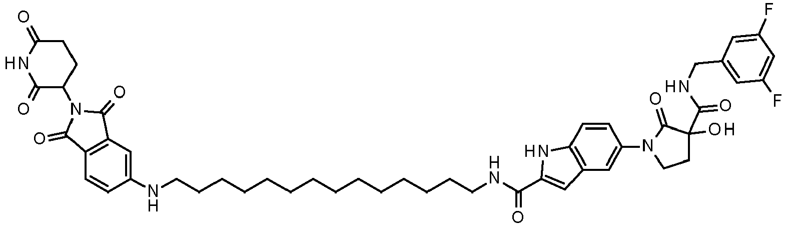 |
| "A32" | 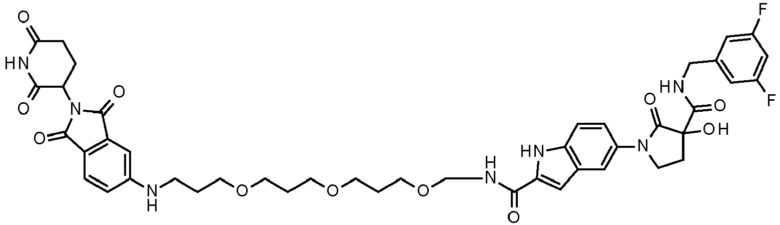 |
| "A33" | 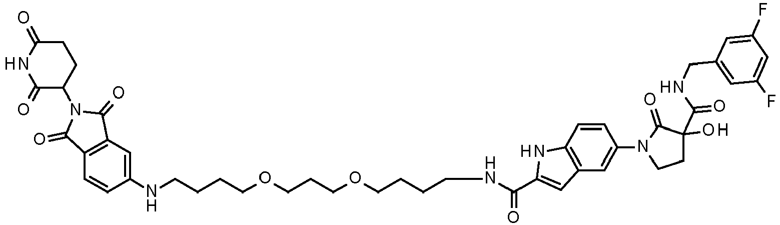 |
| "A34" | 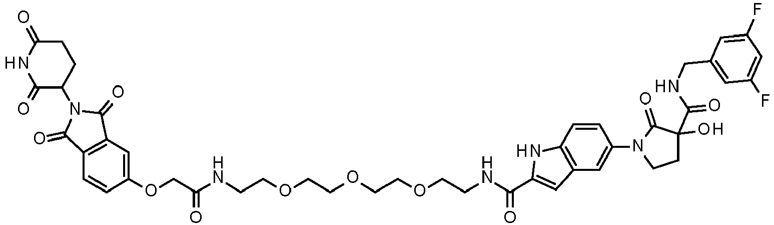 |
| "A35" | 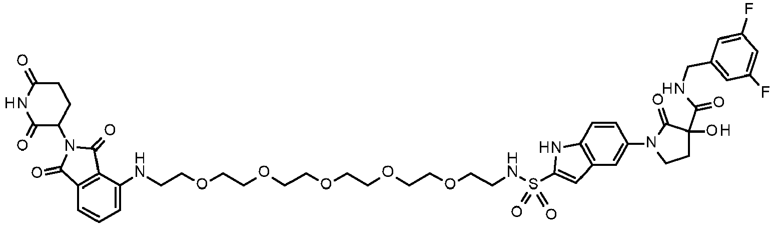 |
| "A36" | 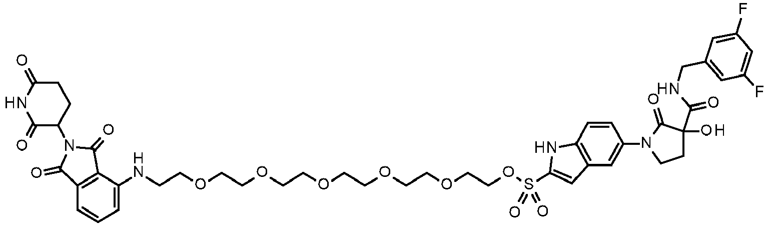 |
| "A37" | 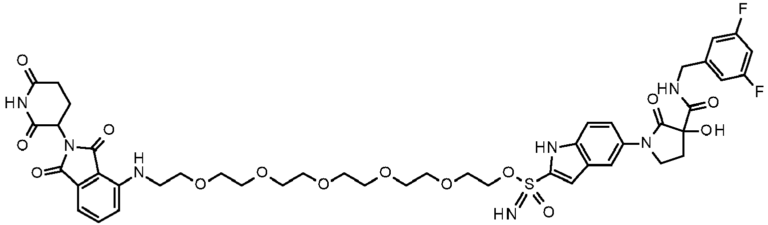 |
| "A38" | 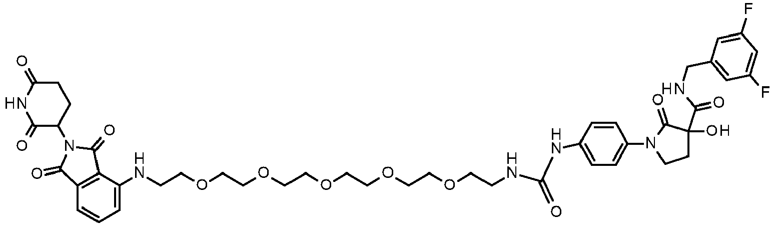 |
| "A39" | 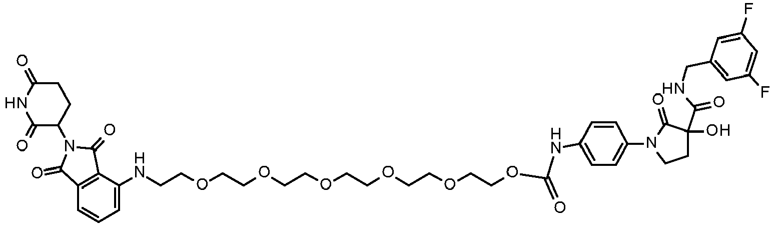 |
| "A40" | 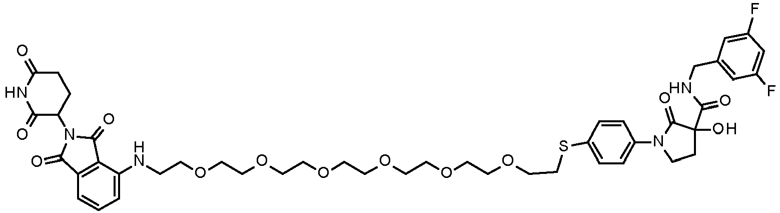 |
| "A41" | 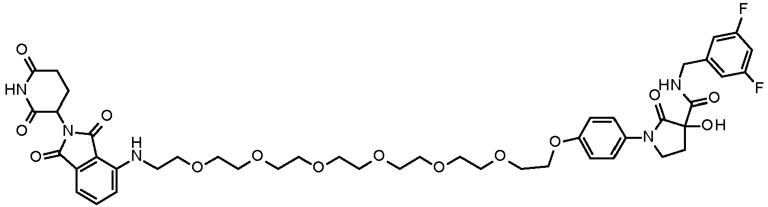 |
| "A42" | 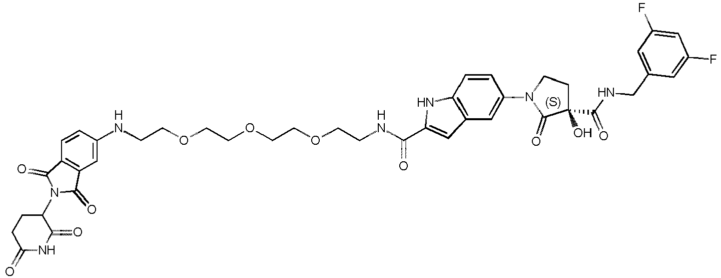 |
| "A43" | 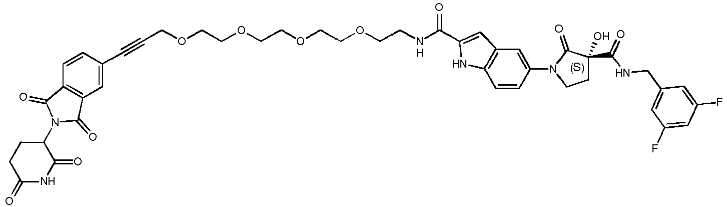 |
| "A44" | 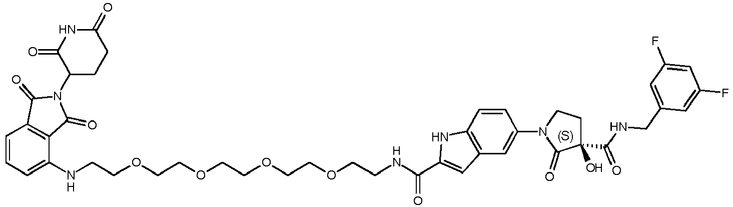 |
| "A45" | 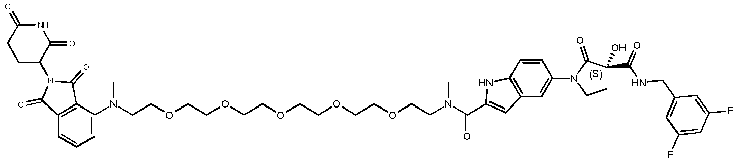 |
| "A46" | 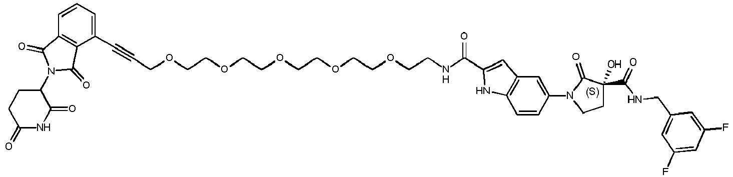 |
| "A47" | 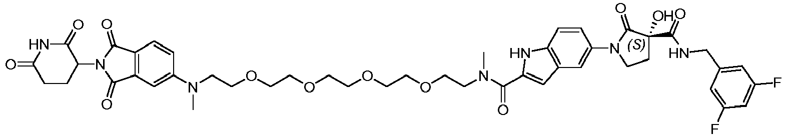 |
| "A48" | 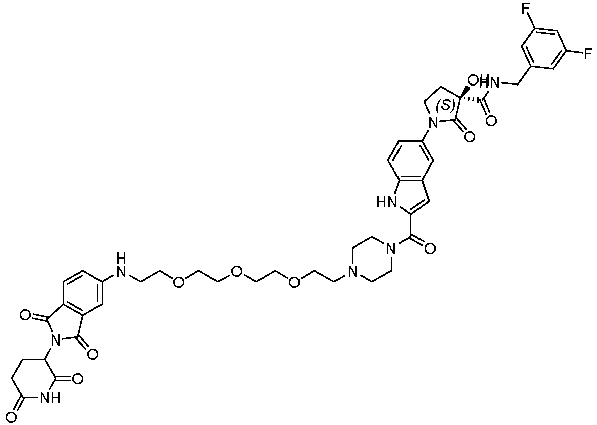 |
| "A49" | 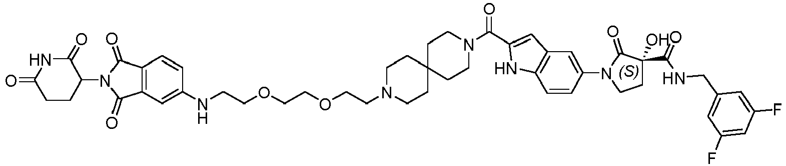 |
| "A50" | 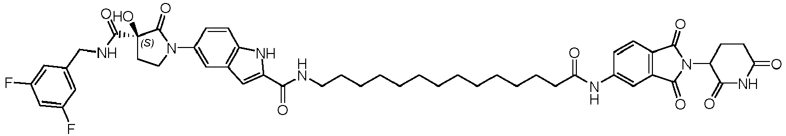 |
| "A51" | 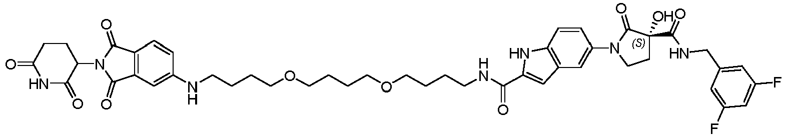 |
| "A52" | 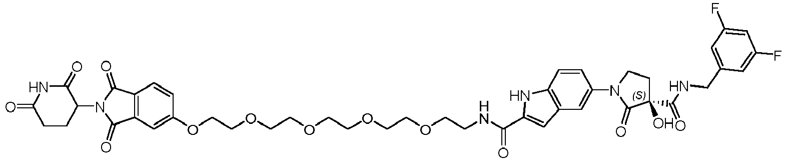 |
| "A53" | 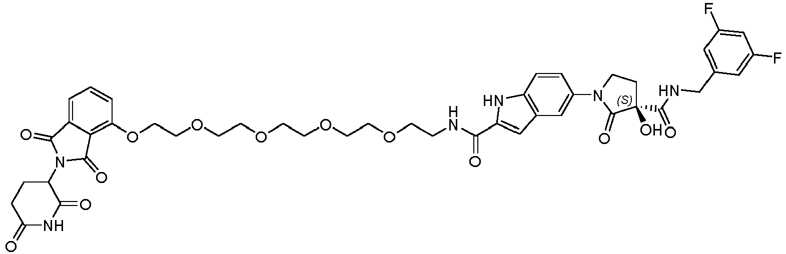 |
| "A54" | 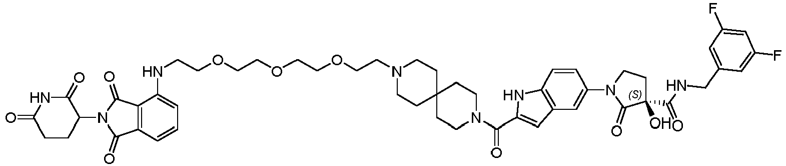 |
| "A55" | 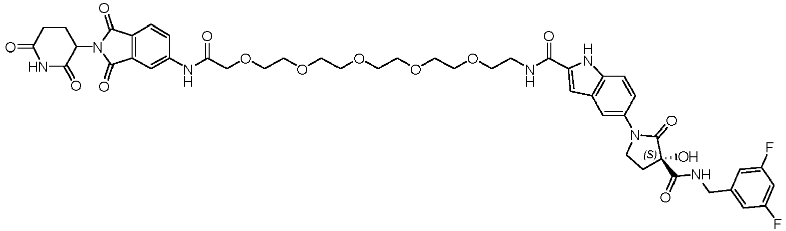 |
| "A56" | 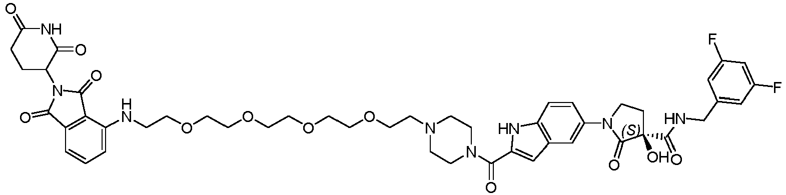 |
| "A57" | 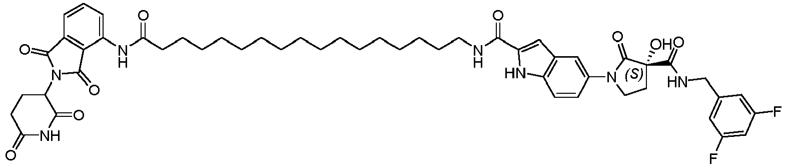 |
and pharmaceutically acceptable solvates, salts, tautomers and stereoisomers thereof, including mixtures thereof in all ratios.

11. Process for the preparation of compounds of the formula I according to Claims 1-10 and pharmaceutically acceptable salts, solvates, tautomers and stereoisomers thereof,
wherein L denotes CONR⁴,
**characterised in that** a compound of formula II 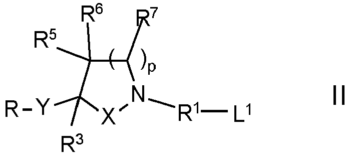
in which X, R, R¹, R³, R⁵, R⁶, R⁷ and p have the meanings indicated in Claim 1,
and L¹ denotes Cl, Br, I or a free or reactively functionally modified OH group,
is reacted with a compound of the formula III
Q¹-Q²-NH₂ III
in which Q¹ and Q² have the meanings indicated in Claim 1, and/or
a base or acid of the formula I is converted into one of its salts.

12. Medicaments comprising at least one compound of the formula I according to claim 1 and/or pharmaceutically acceptable salts, solvates, tautomers and stereoisomers thereof, including mixtures thereof in all ratios, and optionally an pharmaceutically acceptable carrier, excipient or vehicle.

13. Compounds of the formula I according to claim 1 and pharmaceutically acceptable salts, solvates, tautomers and stereoisomers thereof, including mixtures thereof in all ratios, for use in the treatment and/or prevention of tumours, tumour metastases, proliferative diseases of the mesangial cells, haemangioma, proliferative retinopathy, rheumatoid arthritis, atherosclerotic neovascularisation, psoriasis, ocular neovascularisation, osteoporosis, diabetes and obesity, lymphoid leukaemia, lymphoma, malaria and prostate hypertrophy.

14. Compounds for use according to Claim 13, where the tumour disease is selected from the group
of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx, of the lung, of the skin, monocytic leukaemia, lung adenocarcinoma, small-cell lung carcinoma, pancreatic cancer, glioblastoma, breast carcinoma, acute myeloid leukaemia, chronic myeloid leukaemia, acute lymphatic leukaemia, chronic lymphatic leukaemia, Hodgkin's lymphoma, non-Hodgkin's lymphoma.

15. Medicaments comprising at least one compound of the formula I according to claim 1 and/or pharmaceutically acceptable salts, solvates and stereoisomers thereof, including mixtures thereof in all ratios, and at least one further medicament active ingredient.

16. Set (kit) consisting of separate packs of
(a) an effective amount of a compound of the formula I according to claim 1 and/or pharmaceutically acceptable salts, solvates, salts and stereoisomers thereof, including mixtures thereof in all ratios, and
(b) an effective amount of a further medicament active ingredient.

## Patentansprüche

1. Verbindungen der Formel I
Q¹-Q²-Q³ I
worin
Q¹ 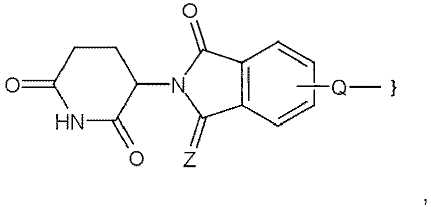 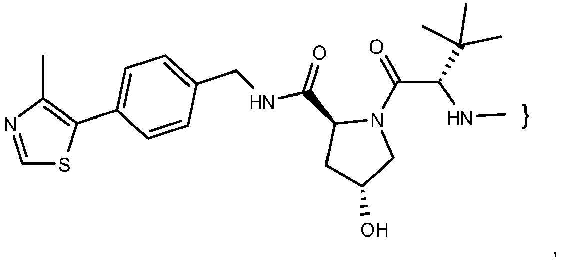 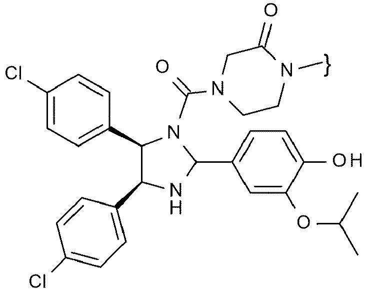 oder 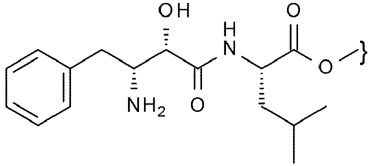 bedeutet,
Z O oder CH₂ bedeutet,
Q O, NH, NCH₃ oder CH₂ bedeutet,
Q² unverzweigtes Alkylen mit 4-25 C-Atomen bedeutet, worin 1-8 nicht benachbarte CH₂-Gruppen durch O, CONH und/oder NHCO ersetzt sein können und worin eine CH₂-Gruppe durch 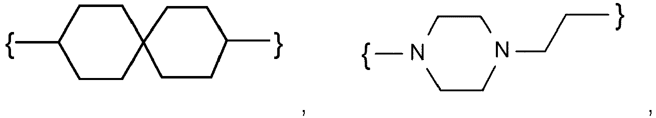 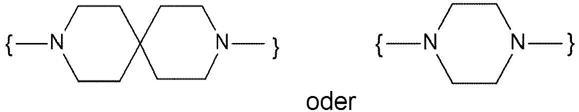
ersetzt sein kann,
Q³ 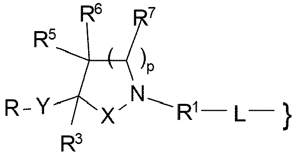 bedeutet,
L NR⁴CO, CONR⁴, NH, O, CO, S, SO₂, SO(=NH), NHCONH, SO₂NH oder NHSO₂ bedeutet,
R NR²R⁴, Alk, C(=CH₂)[C(R⁴)₂]ₙAr², Het², O[C(R⁴)₂]ₙAr² oder OA bedeutet,
X CO oder CH₂ bedeutet,
Y CO oder CH₂ bedeutet,
R¹ (CH₂)ₙ, [C(R⁴)₂]ₙAr¹-, (CH₂)ₙHet-, (CH₂)ₙCyc-, [C(R⁴)₂]ₙCONHAr¹-, [C(R⁴)₂]ₙNA-, O[C(R⁴)₂]ₙAr¹- oder [C(R⁴)₂]ₙCOO(CH₂)ₙAr¹-bedeutet, bei denen der Substituent L direkt mit Ar¹, Het oder Cyc verbunden ist,
R² H, [C(R⁴)₂]ₙAr², (CH₂)ₙCOHet¹, (CH₂)ₙCOAr², (CH₂)ₘNA₂, (CH₂)ₙCyc oder (CH₂)ₙHet¹ bedeutet,
R³ OH oder OCOA bedeutet,
R⁴ H oder Alkyl mit 1, 2, 3 oder 4 C-Atomen bedeutet,
R² und R⁴ zusammen auch Alkylen mit 2, 3, 4 oder 5 C-Atomen bedeuten, wobei eine CH₂-Gruppe auch durch N(CH₂)ₘOH oder SO₂ ersetzt sein kann,
R⁵, R⁶ jeweils unabhängig voneinander H, F oder A bedeuten,
R⁵ und R⁶ zusammen auch Alkylen mit 2, 3, 4 oder 5 C-Atomen bedeuten, wobei eine CH₂-Gruppe auch durch NCOA oder O ersetzt sein kann,
R⁷ H, Hal oder A bedeutet,
Ar¹ Phenyl bedeutet, das unsubstituiert oder ein-, zwei-, drei-, vier- oder fünffach durch Hal, OH, OA, CONH₂, CONHA, CONA₂, NHSO₂A, CONHCyc, NHSO₂Cyc, CONHAr², Het¹, COHet¹ und/oder NASO₂A substituiert ist,
Ar² Phenyl bedeutet, das unsubstituiert oder ein-, zwei-, drei-, vier- oder fünffach durch Hal, A, CONH₂ und/oder OAr₃ substituiert ist,
Ar³ Phenyl bedeutet, das unsubstituiert oder einfach durch NH₂ substituiert ist,
Het einen mono- oder bicyclischen gesättigten, ungesättigten oder aromatischen Heterocyclus mit 1 bis 4 N- und/oder O- und/oder S-Atomen bedeutet, der unsubstituiert oder ein-, zwei- oder dreifach durch Hal, A, OA, CN, NH₂, NHA, NA₂, NO₂, CN, COOH, COOA, (CH₂)ₙCONH₂, (CH₂)ₙCONHA, (CH₂)ₙCONA₂, NHCOA, COA, CHO, Het¹, SO₂A, SO₂NH₂, SO₂NHA, SO₂NA₂, CONHNH₂, CONHAr³, =O und/oder Ar³ substituiert ist,
Het¹ Pyridazinyl, Pyrazolyl, Pyridyl, Piperazinyl, Morpholinyl, Pyrimidinyl, Furyl, Thienyl, Imidazolyl, Pyrrolyl, Oxazolyl, Oxadiazolyl, Isoxazolyl, Thiazolyl, Triazolyl, Tetrazolyl, Thiadiazol, Piperidin-1-yl, Pyrrolidin-1-yl, Tetrahydropyranyl, 1,2-Oxazinan-2-yl, 1,2,5-Oxadiazinan-2-yl, 1,3-Oxazinan-3-yl oder Hexahydropyrimidinyl bedeutet, das jeweils unsubstituiert oder ein-, zwei- oder dreifach durch A und/oder OA substituiert ist,
Het² Isoindolyl bedeutet,
A unverzweigtes oder verzweigtes Alkyl mit 1-10 C-Atomen, worin 1-7 H-Atome durch F, Cl, Br, OH, CHO, COA, COOA, CN, CONA₂, CONHA und/oder CONH₂ ersetzt sein können, und/oder worin eine oder zwei nicht benachbarte CH- und/oder CH₂-Gruppen durch O ersetzt sein können, oder Cyc bedeutet,
Alk Alkenyl mit 2, 3, 4, 5 oder 6 C-Atomen bedeutet,
Cyc cyclisches Alkyl mit 3-7 C-Atomen bedeutet, das unsubstituiert oder ein-, zwei- oder dreifach durch NHCOA, NHSO₂, OH, OA, A, NH₂, NHA, NA₂, COOA, COOH und/oder CONHA substituiert ist,
Hal F, Cl, Br oder I bedeutet,
m 1, 2, 3 oder 4 bedeutet,
n 0, 1, 2, 3 oder 4 bedeutet,
p 1, 2 oder 3 bedeutet,
und pharmazeutisch unbedenkliche Salze, Tautomere und Stereoisomere davon, einschließlich deren Mischungen in allen Verhältnissen.

2. Verbindungen nach Anspruch 1, worin
Het Pyrazinyl, Pyrazolyl, Benzimidazolyl, Pyridyl, Thienyl, Furanyl, Indolyl, Dihydroindolyl, Benzofuranyl, Tetrahydropyranyl, Dihydrochinolinyl, Dihydroisochinolinyl, Tetrahydrochinolinyl, Tetrahydroisochinolinyl, Indazolyl, Imidazolyl, Pyrrolyl, Oxazolyl, Oxadiazolyl, Isoxazolyl, Benzothiazolyl, Piperidin-1-yl, Pyrrolidin-1-yl, 3,4-Dihydro-2H-pyrido[3,2-b]-1,4-oxazinyl, 3,4-Dihydro-2H-benzo-1,4-oxazinyl, Benzofuranyl, Azetidinyl, 1H-Pyrrolo[2,3-b]-pyridinyl, 2H-Chromenyl, 3-Azabicylo[3.2.0]hexyl, Pyrrolo[2,3-b]-pyridinyl, Tetrahydrofuranyl, Tetrahydro-1,8-naphthyridinyl, 2,3-Dihydro-benzoisothiazolyl, 1,2,3,4-Tetrahydrobenzothiazinyl oder Hexahydrobenzo-1,3-dioxolyl bedeutet, das jeweils unsubstituiert oder ein-, zwei- oder dreifach durch Hal, A, OA, CN, NH₂, NHA, NA2, NO₂, CN, COOH, COOA, (CH₂)ₙCONH₂, (CH₂)ₙCONHA, (CH₂)ₙCONA₂, NHCOA, COA, CHO, Het¹, SO₂A, SO₂NH₂, SO₂NHA, SO₂NA₂, CONHNH₂, CONHAr³, =O und/oder Ar³ substituiert ist,
und pharmazeutisch unbedenkliche Salze, Tautomere und Stereoisomere davon, einschließlich deren Mischungen in allen Verhältnissen.

3. Verbindungen nach Anspruch 1 oder 2, worin
Q¹ 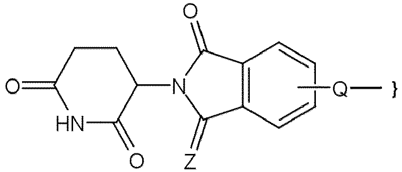 oder 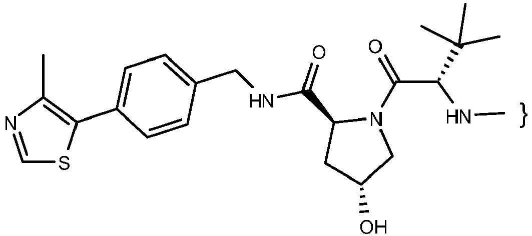 bedeutet, und pharmazeutisch unbedenkliche Solvate, Salze, Tautomere und Stereoisomere davon, einschließlich deren Mischungen in allen Verhältnissen.

4. Verbindungen nach einem oder mehreren der Ansprüche 1-3, worin
R NR²R⁴ bedeutet,
und pharmazeutisch unbedenkliche Solvate, Salze, Tautomere und Stereoisomere davon, einschließlich deren Mischungen in allen Verhältnissen.

5. Verbindungen nach einem oder mehreren der Ansprüche 1-4, worin
R¹ (CH₂)ₙ, [C(R⁴)₂]ₙAr¹ - oder (CH₂)ₙHet- bedeutet,
und pharmazeutisch unbedenkliche Solvate, Salze, Tautomere und Stereoisomere davon, einschließlich deren Mischungen in allen Verhältnissen.

6. Verbindungen nach einem oder mehreren der Ansprüche 1-5, worin
R² [C(R⁴)₂]ₙAr², (CH₂)ₙCyc oder (CH₂)ₙHet¹ bedeutet,
und pharmazeutisch unbedenkliche Solvate, Salze, Tautomere und Stereoisomere davon, einschließlich deren Mischungen in allen Verhältnissen.

7. Verbindungen nach einem oder mehreren der Ansprüche 1-6, worin
Het Benzimidazolyl oder Indolyl bedeutet, das jeweils unsubstituiert oder einfach durch Hal substituiert ist,
und pharmazeutisch unbedenkliche Solvate, Salze, Tautomere und Stereoisomere davon, einschließlich deren Mischungen in allen Verhältnissen.

8. Verbindungen nach einem oder mehreren der Ansprüche 1-7, worin
A unverzweigtes oder verzweigtes Alkyl mit 1-6 C-Atomen bedeutet, worin 1-5 H-Atome durch F, Cl und/oder OH ersetzt sein können,
und pharmazeutisch unbedenkliche Solvate, Salze, Tautomere und Stereoisomere davon, einschließlich deren Mischungen in allen Verhältnissen.

9. Verbindungen nach einem oder mehreren der Ansprüche 1-8, worin Q¹ 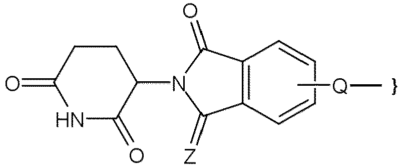 oder 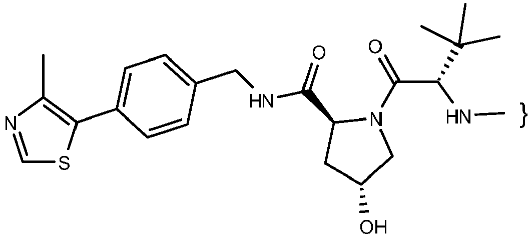 bedeutet,
Z O oder CH₂ bedeutet,
Q O, NH, NCH₃ oder CH₂ bedeutet,
Q² unverzweigtes Alkylen mit 4-25 C-Atomen bedeutet, worin 1-8 nicht benachbarte CH₂-Gruppen durch O, CONH und/oder NHCO ersetzt sein können und worin eine CH₂-Gruppe durch 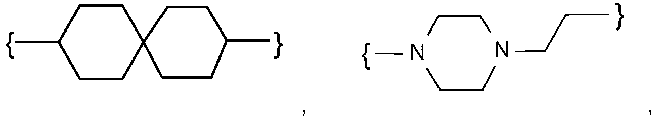 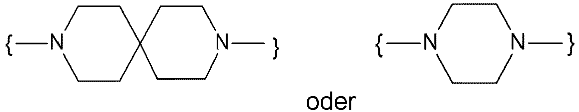
ersetzt sein kann, Q³ 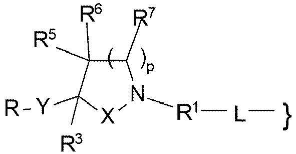 bedeutet,
L NR⁴CO, CONR⁴, NH, O, CO, S, SO₂, SO(=NH), NHCONH, SO₂NH oder NHSO₂ bedeutet,
R NR²R⁴ bedeutet,
X CO oder CH₂ bedeutet,
Y CO oder CH₂ bedeutet,
R¹ (CH₂)ₙ, [C(R⁴)₂]ₙAr¹ - oder (CH₂)ₙHet- bedeutet, bei denen der Substituent L direkt mit Ar¹, Het oder Cyc verbunden ist,
R² [C(R⁴)₂]ₙAr², (CH₂)ₙCyc oder (CH₂)ₙHet¹ bedeutet,
R³ OH bedeutet,
R⁴ H oder Alkyl mit 1, 2, 3 oder 4 C-Atomen bedeutet,
R⁵, R⁶ H bedeuten,
R⁷ H, Hal oder A bedeutet,
Ar¹ Phenyl bedeutet,
Ar² Phenyl bedeutet, das unsubstituiert oder ein-, zwei-, drei- oder vierfach durch Hal substituiert ist,
Het Pyrazinyl, Pyrazolyl, Benzimidazolyl, Pyridyl, Thienyl, Furanyl, Indolyl, Dihydroindolyl, Benzofuranyl, Tetrahydropyranyl, Dihydrochinolinyl, Dihydroisochinolinyl, Tetrahydrochinolinyl, Tetrahydroisochinolinyl, Indazolyl, Imidazolyl, Pyrrolyl, Oxazolyl, Oxadiazolyl, Isoxazolyl, Benzothiazolyl, Piperidin-1-yl, Pyrrolidin-1-yl, 3,4-Dihydro-2H-pyrido[3,2-b]-1,4-oxazinyl, 3,4-Dihydro-2H-benzo-1,4-oxazinyl, Benzofuranyl, Azetidinyl, 1H-Pyrrolo[2,3-b]-pyridinyl, 2H-Chromenyl, 3-Azabicylo[3.2.0]hexyl, Pyrrolo[2,3-b]-pyridinyl, Tetrahydrofuranyl, Tetrahydro-1,8-naphthyridinyl, 2,3-Dihydro-benzoisothiazolyl, 1,2,3,4-Tetrahydrobenzothiazinyl oder Hexahydrobenzo-1,3-dioxolyl bedeutet, das jeweils unsubstituiert oder ein-, zwei- oder dreifach durch Hal, A, OA, CN, NH₂, NHA, NA2, NO₂, CN, COOH, COOA, (CH₂)ₙCONH₂, (CH₂)ₙCONHA, (CH₂)ₙCONA₂, NHCOA, COA, CHO, Het¹, SO₂A, SO₂NH₂, SO₂NHA, SO₂NA₂, CONHNH₂, CONHAr³, =O und/oder Ar³ substituiert ist,
Het¹ Pyridyl, Furyl, Thienyl, Imidazolyl oder Pyrrolyl bedeutet,
A unverzweigtes oder verzweigtes Alkyl mit 1-6 C-Atomen bedeutet, worin 1-5 H-Atome durch F, Cl und/oder OH ersetzt sein können,
Cyc cyclisches Alkyl mit 3-7 C-Atomen bedeutet,
Hal F, Cl, Br oder I bedeutet,
n 0, 1, 2, 3 oder 4 bedeutet,
p 1, 2 oder 3 bedeutet,
und pharmazeutisch unbedenkliche Solvate, Salze, Tautomere und Stereoisomere davon, einschließlich deren Mischungen in allen Verhältnissen.

10. Verbindungen nach Anspruch 1, ausgewählt aus der Gruppe
| Nr. | chemische Struktur |
|---|---|
| "A1 " | 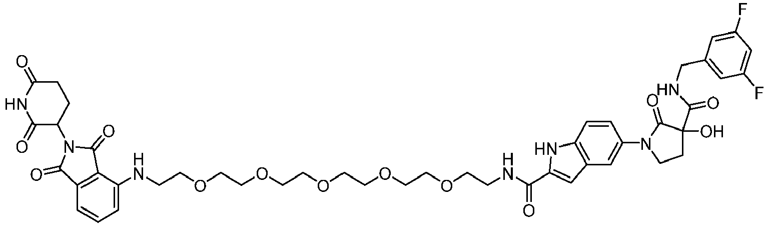 |
| "A2" | 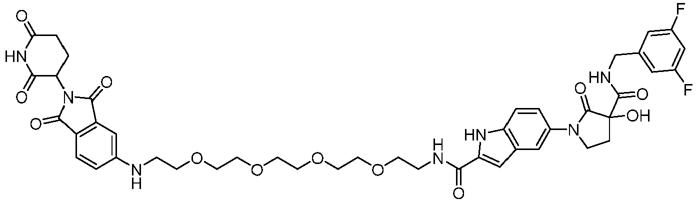 |
| "A3" | 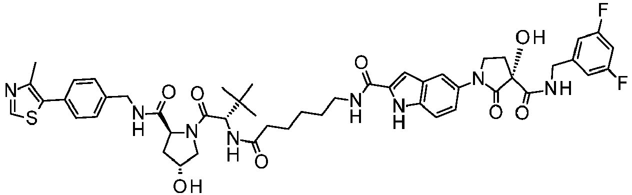 |
| "A4" | 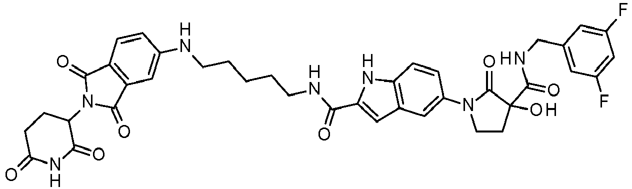 |
| "A5" | 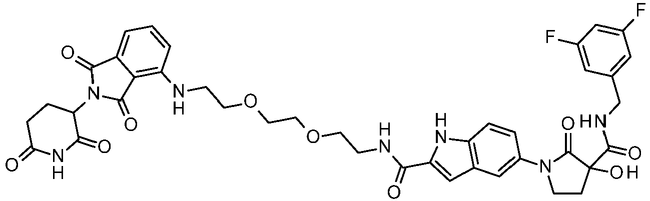 |
| "A6" | 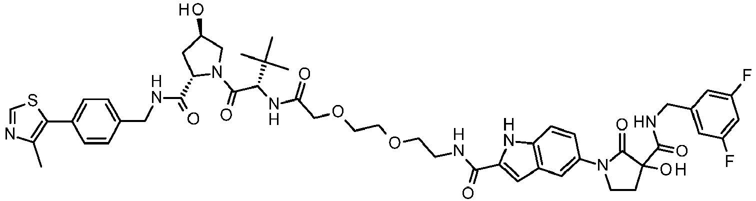 |
| "A7" | 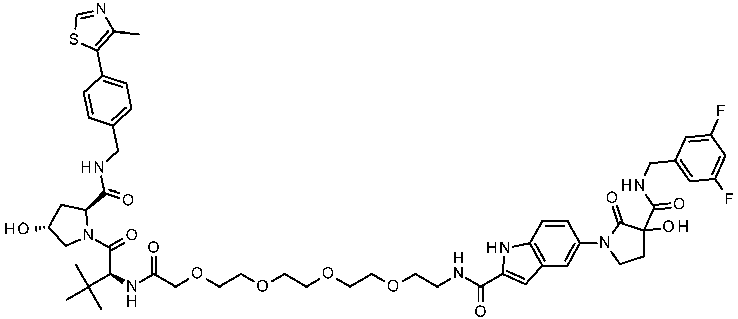 |
| "A8" | 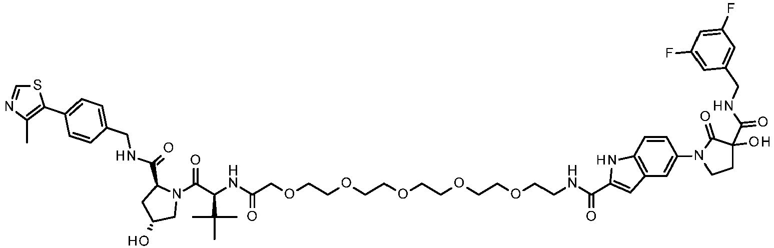 |
| "A9" | 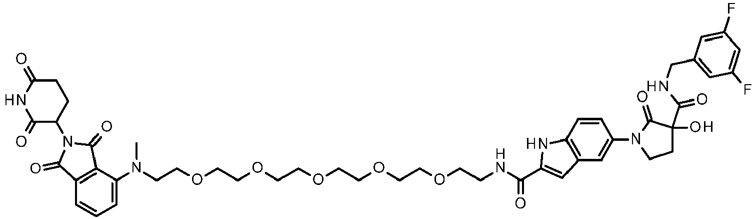 |
| "A10" | 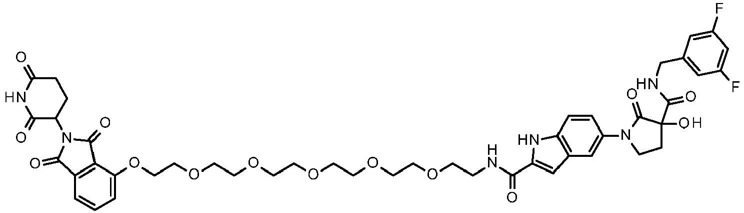 |
| "A11" | 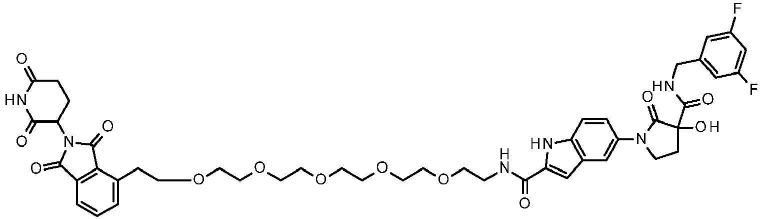 |
| "A12" | 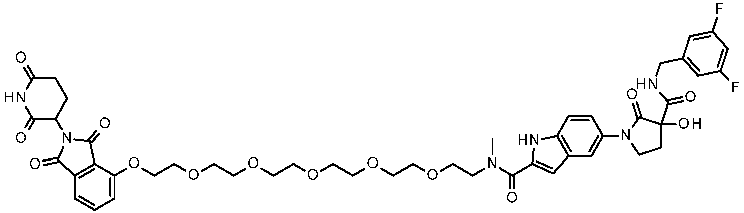 |
| "A13" | 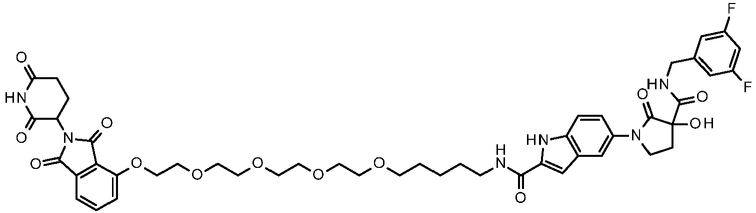 |
| "A14" | 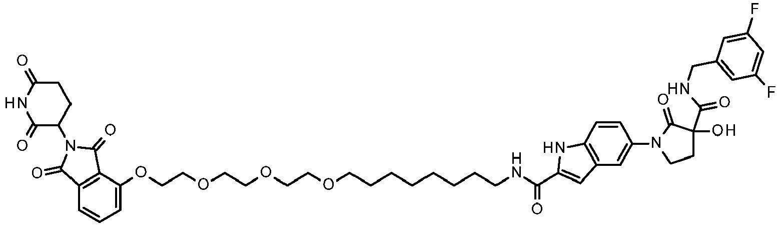 |
| "A15" | 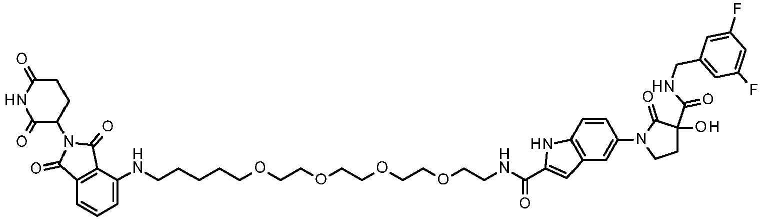 |
| "A16" | 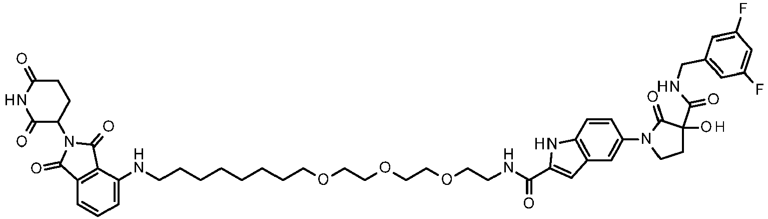 |
| "A17" | 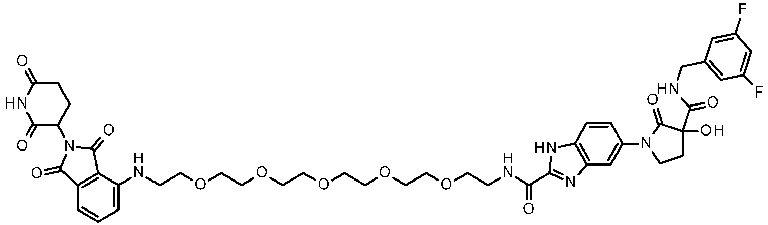 |
| "A18" | 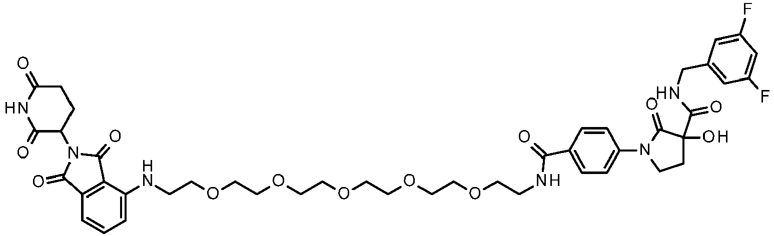 |
| "A19" | 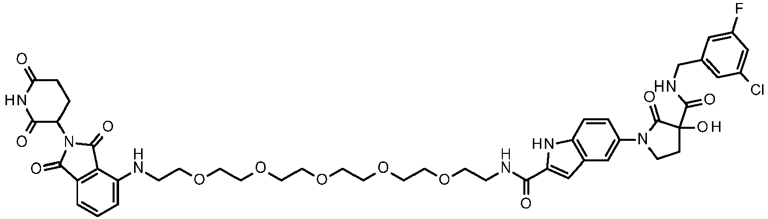 |
| "A20" | 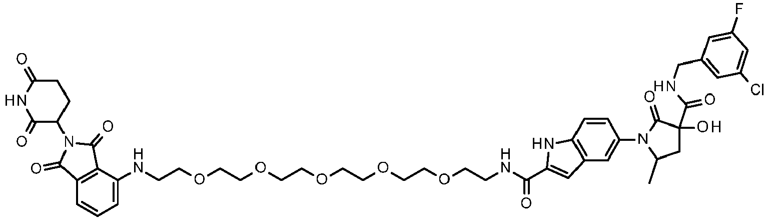 |
| "A21" | 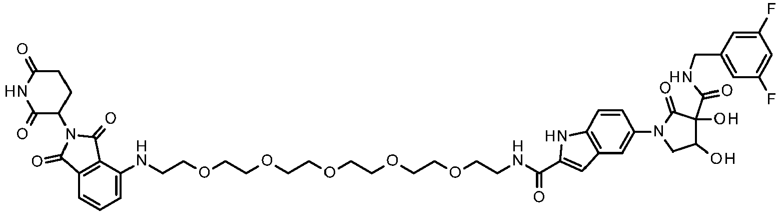 |
| "A22" | 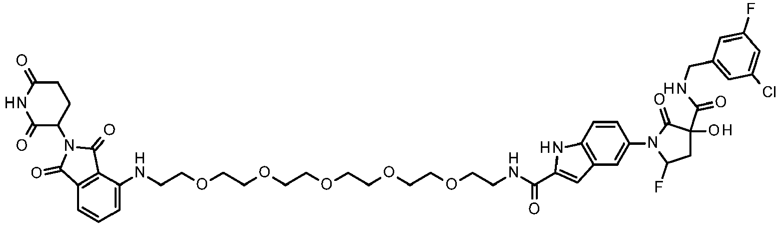 |
| "A23" | 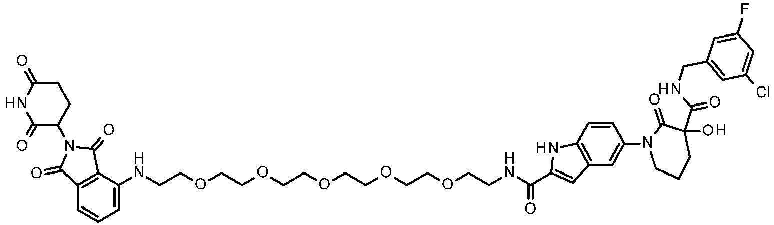 |
| "A24" | 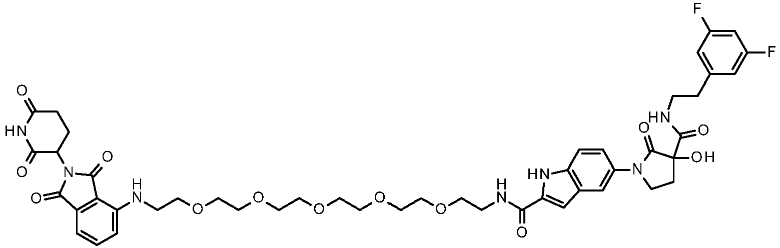 |
| "A25" | 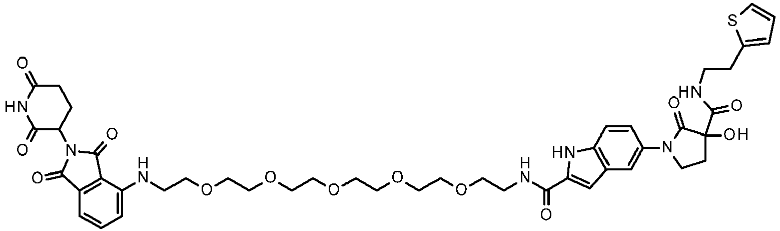 |
| "A26" | 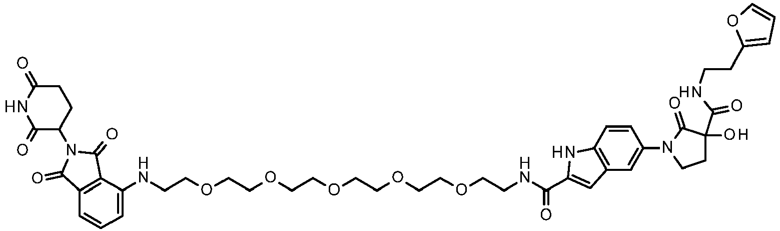 |
| "A27" | 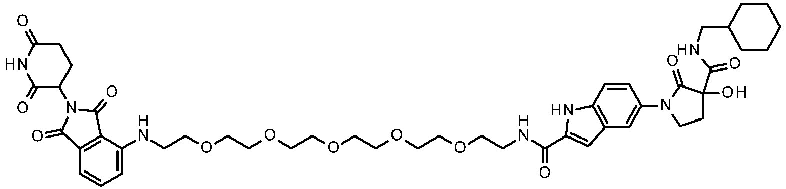 |
| "A28" | 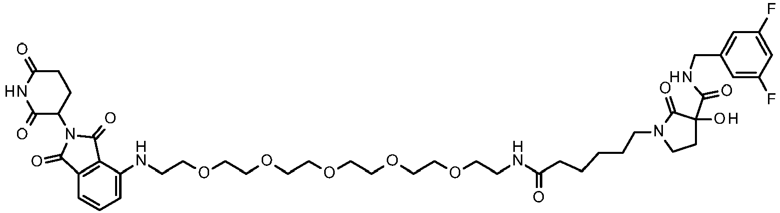 |
| "A29" | 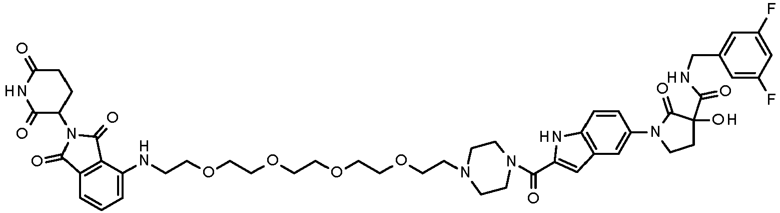 |
| "A30" | 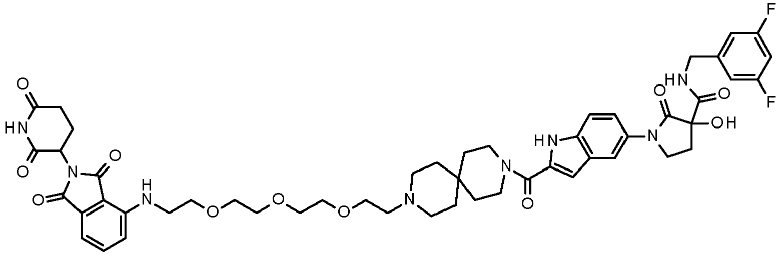 |
| "A31 " | 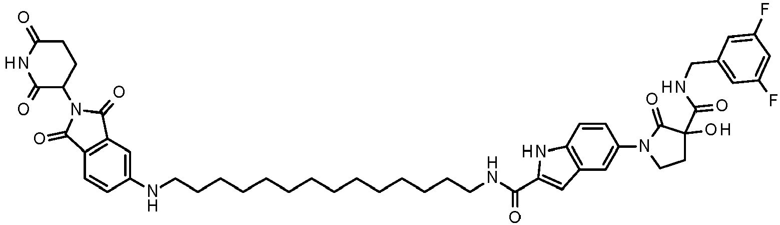 |
| "A32" | 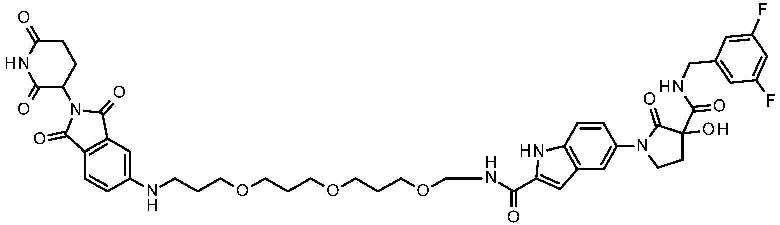 |
| "A33" | 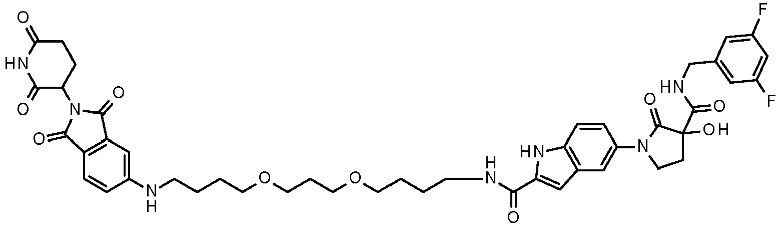 |
| "A34" | 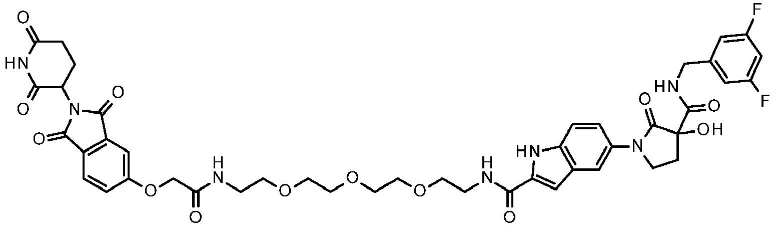 |
| "A35" | 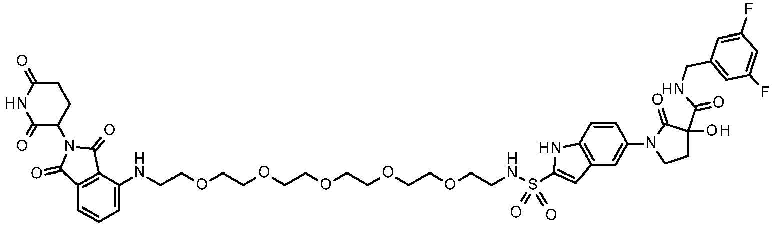 |
| "A36" | 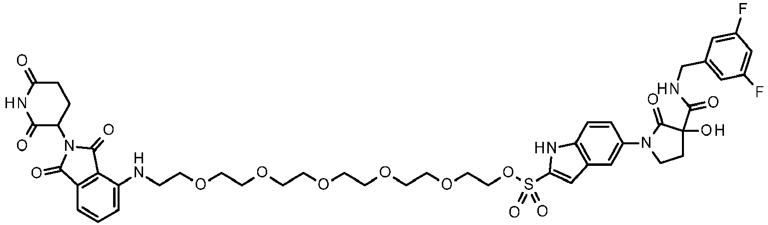 |
| "A37" | 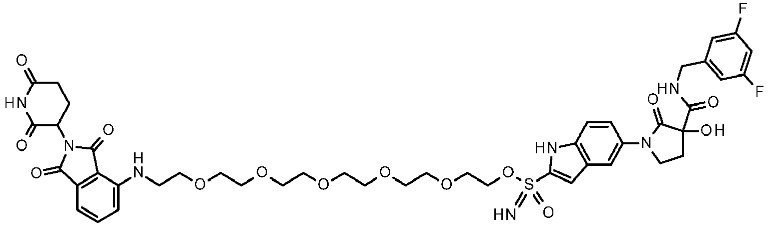 |
| "A38" | 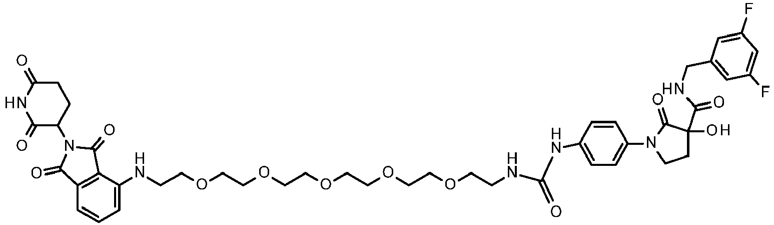 |
| "A39" | 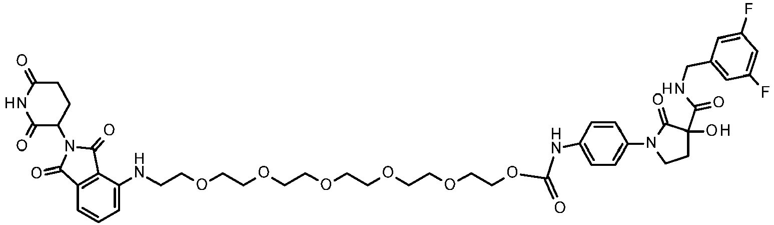 |
| "A40" | 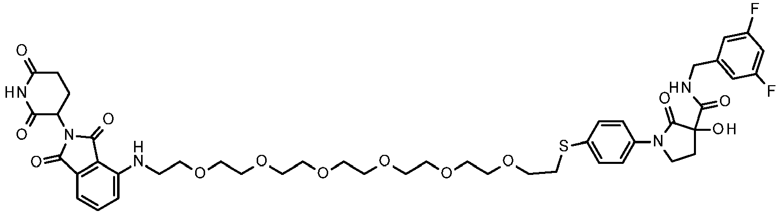 |
| "A41 " | 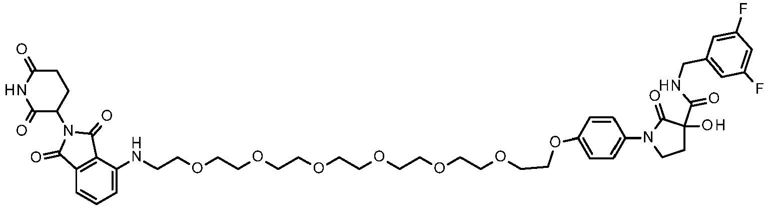 |
| "A42" | 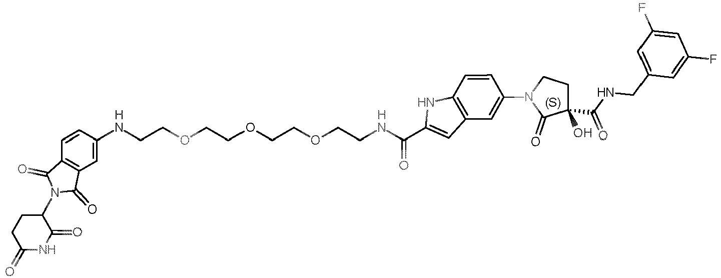 |
| "A43" | 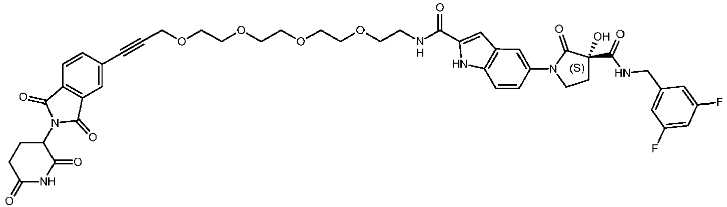 |
| "A44" | 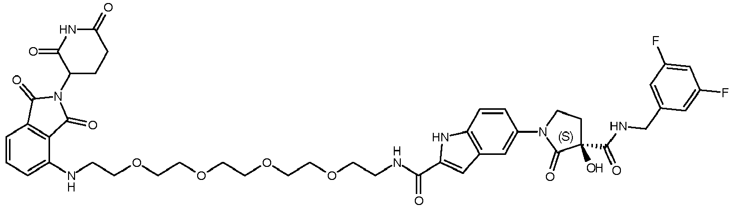 |
| "A45" | 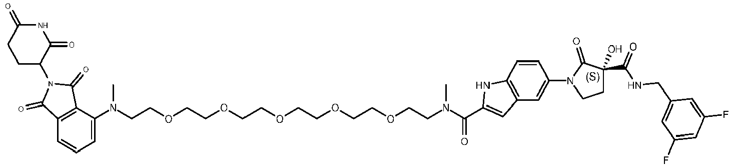 |
| "A46" | 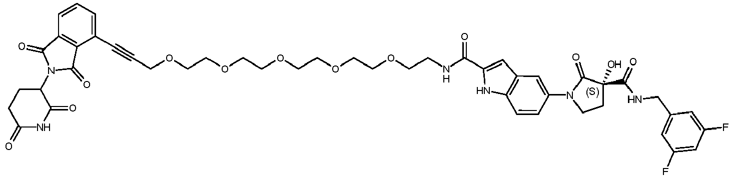 |
| "A47" | 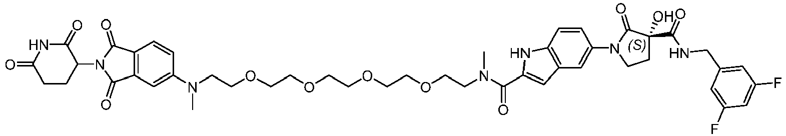 |
| "A48" | 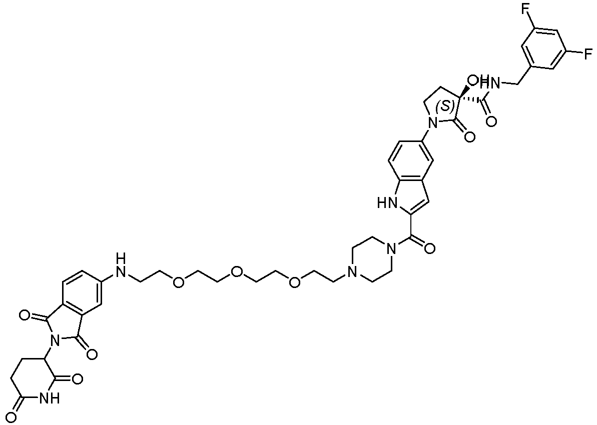 |
| "A49" | 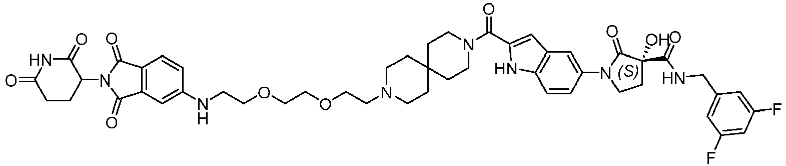 |
| "A50" | 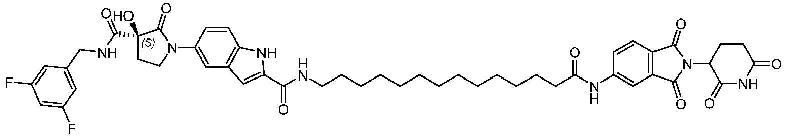 |
| "A51" | 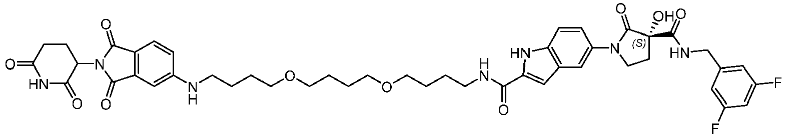 |
| "A52" | 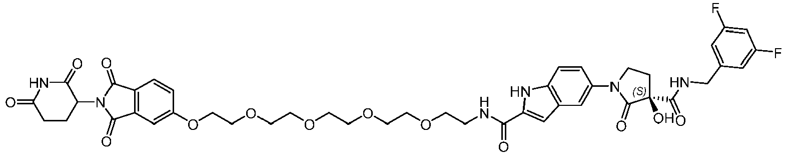 |
| "A53" | 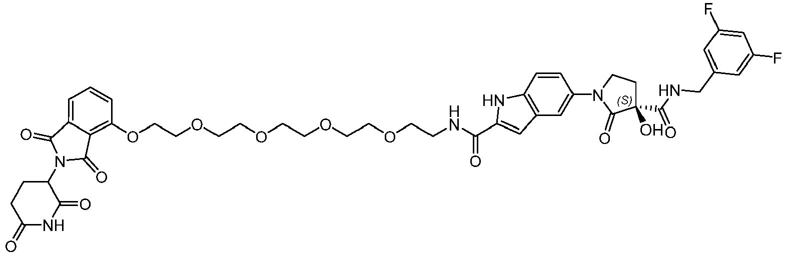 |
| "A54" | 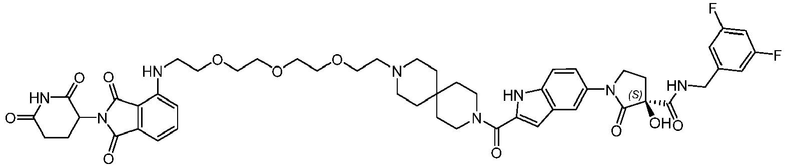 |
| "A55" | 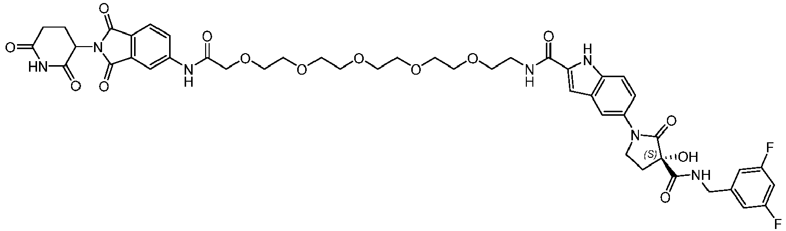 |
| "A56" | 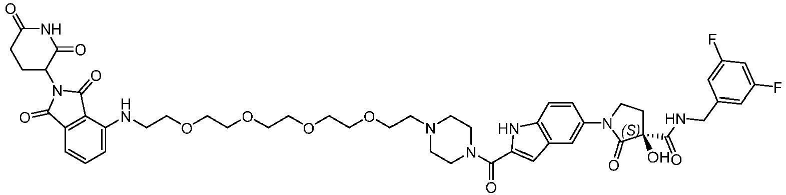 |
| "A57" | 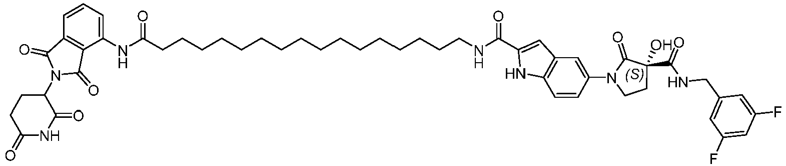 |
und pharmazeutisch unbedenkliche Solvate, Salze, Tautomere und Stereoisomere davon, einschließlich deren Mischungen in allen Verhältnissen.

11. Verfahren zur Herstellung von Verbindungen der Formel I nach den Ansprüchen 1-10 und pharmazeutisch unbedenklichen Salzen, Solvaten, Tautomeren und Stereoisomeren davon,
bei der L CONR⁴ bedeutet,
**dadurch gekennzeichnet, dass** man eine Verbindung der Formel II 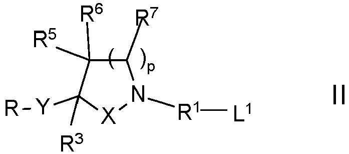
worin X, R, R¹, R³, R⁵, R⁶, R⁷ und p die in Anspruch 1 angegebenen Bedeutungen besitzen,
und L¹ Cl, Br, I oder eine freie oder reaktionsfähig funktionell abgewandelte OH-Gruppe bedeutet,
mit einer Verbindung der Formel III
Q¹-Q²-NH₂ III
umsetzt, worin Q¹ und Q² die in Anspruch 1 angegebenen Bedeutungen besitzen,
und/oder
eine Base oder Säure der Formel I in eines ihrer Salze umwandelt.

12. Arzneimittel enthaltend mindestens eine Verbindung der Formel I nach Anspruch 1 und/oder pharmazeutisch unbedenkliche Salze, Solvate, Tautomere und Stereoisomere davon, einschließlich deren Mischungen in allen Verhältnissen, sowie gegebenenfalls ein(en) pharmazeutisch unbedenklichen/s Trägerstoff, Exzipienten oder Vehikel.

13. Verbindungen der Formel I nach Anspruch 1 und pharmazeutisch unbedenkliche Salze, Solvate, Tautomere und Stereoisomere davon, einschließlich deren Mischungen in allen Verhältnissen, zur Verwendung bei der Behandlung und/oder Prävention von Tumoren, Tumormetastasen, proliferativen Erkrankungen der Mesangiumzellen, Hämangiom, proliferativer Retinopathie, rheumatoider Arthritis, atherosklerotischer Neovaskularisation, Psoriasis, okularer Neovaskularisation, Osteoporose, Diabetes und Fettleibigkeit, lymphoider Leukämie, Lymphom, Malaria und Prostatahypertrophie.

14. Verbindungen zur Verwendung nach Anspruch 13, wobei die Tumorerkrankung ausgewählt ist aus der Gruppe
des Plattenepithels, der Blase, des Magens, der Nieren, von Kopf und Hals, der Speiseröhre, des Gebährmutterhalses, der Schilddrüse, des Darms, der Leber, des Gehirns, der Prostata, des Urogenitaltraktes, des lymphatischen Systems, des Kehlkopfes, der Lunge, der Haut, Monozytenleukämie, Lungenadenokarzinom, kleinzelliges Lungenkarzinom, Bauchspeicheldrüsenkrebs, Glioblastom, Brustkarzinom, akute myelotische Leukämie, chronische myelotische Leukämie, akute lymphatische Leukämie, Hodgkin-Lymphom, Non-Hodgkin-Lymphom.

15. Arzneimittel enthaltend mindestens eine Verbindung der Formel I nach Anspruch 1 und/oder pharmazeutisch unbedenkliche Salze, Solvate und Stereoisomere davon, einschließlich deren Mischungen in allen Verhältnissen, und mindestens einen weiteren Arzneimittelwirkstoff.

16. Set (Kit) bestehend aus getrennten Packungen von
(a) einer wirksamen Menge einer Verbindung der Formel I nach Anspruch 1 und/oder pharmazeutisch unbedenklicher Salze, Solvate und Stereoisomere davon, einschließlich deren Mischungen in allen Verhältnissen,
und
(b) einer wirksamen Menge eines weiteren Arzneimittelwirkstoffs.

## Revendications

1. Composés de formule I
Q¹-Q²-Q³ I
dans lesquels
Q¹ désigne 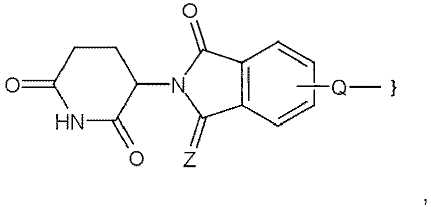 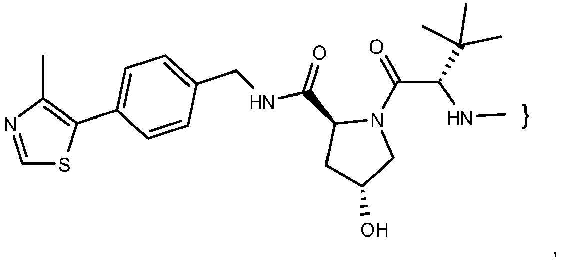 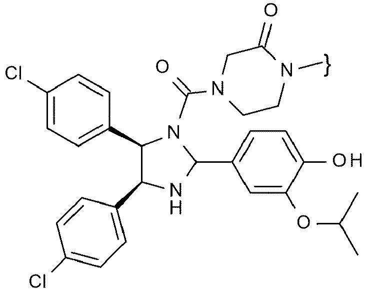
ou 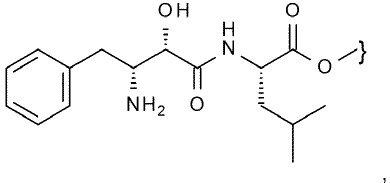
Z désigne O ou CH₂,
Q désigne O, NH, NCH₃ ou CH₂,
Q² désigne alkylène non ramifié ayant 4-25 atomes de C, où 1-8 groupements CH₂ non adjacents peuvent être remplacés par O, CONH et/ou NHCO et où un groupement CH₂ peut être remplacé par 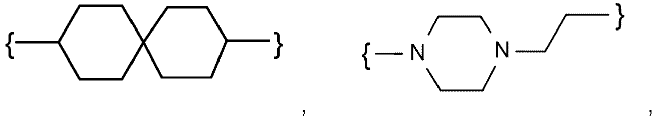 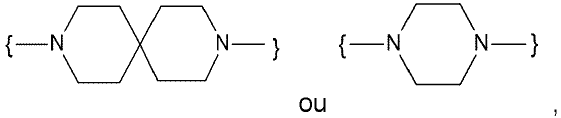
Q³ désigne 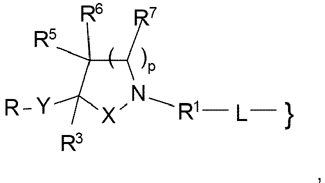
L désigne NR⁴CO, CONR⁴, NH, O, CO, S, SO₂, SO(=NH), NHCONH, SO₂NH ou NHSO₂,
R désigne NR²R⁴, Alk, C(=CH₂)[C(R⁴)₂]ₙAr², Het², O[C(R⁴)₂]ₙAr² ou OA,
X désigne CO ou CH₂,
Y désigne CO ou CH₂,
R¹ désigne (CH₂)ₙ, [C(R⁴)₂]ₙAr¹-, (CH₂)ₙHet-, (CH₂)ₙCyc-, [C(R⁴)₂]ₙCONHAr¹-, [C(R⁴)₂]ₙNA-, O[C(R⁴)₂]ₙAr¹- ou [C(R⁴)₂]ₙCOO(CH₂)ₙAr¹-, où le substituant L est relié directement à Ar¹, Het ou Cyc,
R² désigne H, [C(R⁴)₂]ₙAr², (CH₂)ₙCOHet¹, (CH₂)ₙCOAr², (CH2)mNA2, (CH2)ₙCyc ou (CH₂)ₙHet¹,
R³ désigne OH ou OCOA,
R⁴ désigne H ou alkyle ayant 1, 2, 3 ou 4 atomes de C,
R² et R⁴ désignent également ensemble alkylène ayant 2, 3, 4 ou 5 atomes de C, où un groupement CH₂ peut également être remplacé par N(CH₂)ₘOH ou SO₂,
R⁵, R⁶ désignent chacun, indépendamment l'un de l'autre, H, F ou A,
R⁵ et R⁶ désignent également ensemble alkylène ayant 2, 3, 4 ou 5 atomes de C, où un groupement CH₂ peut également être remplacé par NCOA ou O,
R⁷ désigne H, Hal ou A,
Ar¹ désigne phényle qui est non substitué ou mono-, di- tri-, tétra- ou pentasubstitué par Hal, OH, OA, CONH₂, CONHA, CONA₂, NHSO₂A, CONHCyc, NHSO₂Cyc, CONHAr², Het¹, COHet¹ et/ou NASO₂A,
Ar² désigne phényle qui est non substitué ou mono-, di-, tri-, tétra- ou pentasubstitué par Hal, A, CONH₂, et/ou OAr³,
Ar³ désigne phényle qui est non substitué ou monosubstitué par NH₂,
Het désigne un hétérocycle mono- ou bicyclique saturé, insaturé ou aromatique ayant de 1 à 4 atomes de N, et/ou O et/ou S qui est non substitué ou mono-, di- ou trisubstitué par Hal, A, OA, CN, NH2, NHA, NA2, NO₂, CN, COOH, COOA, (CH₂)ₙCONH₂, (CH₂)ₙCONHA, (CH₂)ₙCONA₂, NHCOA, COA, CHO, Het¹, SO₂A, SO₂NH₂, SO₂NHA, SO₂NA₂, CONHNH₂, CONHAr³, =O et/ou Ar³,
Het¹ désigne pyridazinyle, pyrazolyle, pyridyle, pipérazinyle, morpholinyle, pyrimidinyle, furyle, thiényle, imidazolyle, pyrrolyle, oxazolyle, oxadiazolyle, isoxazolyle, thiazolyle, triazolyle, tétrazolyle, thiadiazole, pipéridin-1-yle, pyrrolidin-1-yle, tétrahydropyranyle, 1,2-oxazinan-2-yle, 1,2,5-oxadiazinan-2-yle, 1,3-oxazinan-3-yle ou hexahydropyrimidinyle, chacun d'entre eux étant non substitué ou mono-, di- ou trisubstitué par A et/ou OA,
Het² désigne isoindolyle,
A désigne alkyle non ramifié ou ramifié ayant 1-10 atomes de C, où 1-7 atomes de H peuvent être remplacés par F, Cl, Br, OH, CHO, COA, COOA, CN, CONA₂, CONHA et/ou CONH₂, et/ou où un ou deux groupements CH et/ou CH₂ non adjacents peuvent être remplacés par O, ou Cyc,
Alk désigne alcényle ayant 2, 3, 4, 5 ou 6 atomes de C,
Cyc désigne alkyle cyclique ayant 3-7 atomes de C qui est non substitué ou mono-, di- ou trisubstitué par NHCOA, NHSO₂,OH, OA, A, NH₂, NHA, NA₂, COOA, COOH et/ou CONHA,
Hal désigne F, Cl, Br ou I,
m désigne 1, 2, 3 ou 4,
n désigne 0, 1, 2, 3 ou 4,
p désigne 1, 2 ou 3,
ainsi que les sels, tautomères et stéréoisomères pharmaceutiquement acceptables de ceux-ci, y compris des mélanges de ceux-ci en toutes proportions.

2. Composés selon la revendication 1
dans lesquels
Het désigne pyrazinyle, pyrazolyle, benzimidazolyle, pyridyle, thiényle, furanyle, indolyle, dihydroindolyle, benzofuranyle, tétrahydropyranyle, dihydroquinoléinyle, dihydroisoquinoléinyle, tétrahydroquinoléinyle, tétrahydroisoquinoléinyle, indazolyle, imidazolyle, pyrrolyle, oxazolyle, oxadiazolyle, isoxazolyle, benzothiazolyle, pipéridin-1-yle, pyrrolidin-1-yle, 3,4-dihydro-2H-pyrido[3,2-b]-1,4-oxazinyle, 3,4-dihydro-2H-benzo-1,4-oxazinyle, benzofuranyle, azétidinyle, 1H-pyrrolo[2,3-b]pyridinyle, 2H-chroményle, 3-azabicylo[3.2.0]hexyle, pyrrolo[2,3-b]pyridinyle, tétrahydrofuranyle, tétrahydro-1,8-naphtyridinyle, 2,3-dihydro-benzoisothiazolyle, 1,2,3,4-tétrahydrobenzothiazinyle ou hexahydrobenzo-1,3-dioxolyle, chacun d'entre eux étant non substitué ou mono-, di- ou trisubstitué par Hal, A, OA, CN, NH₂, NHA, NA₂, NO₂, CN, COOH, COOA, (CH₂)ₙCONH₂, (CH₂)ₙCONHA, (CH₂)ₙCONA₂, NHCOA, COA, CHO, Het¹, SO₂A,SO₂NH₂, SO₂NHA, SO₂NA₂, CONHNH₂, CONHAr³, =O et/ou Ar³,
ainsi que les sels, tautomères et stéréoisomères pharmaceutiquement acceptables de ceux-ci, y compris des mélanges de ceux-ci en toutes proportions.

3. Composés selon la revendication 1 ou 2, dans lesquels
Q¹ désigne 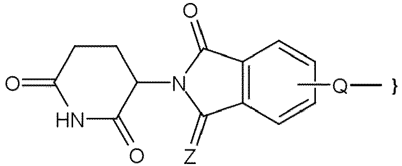
ou 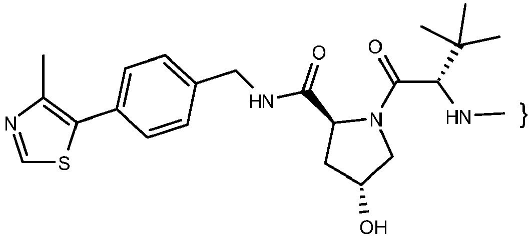 ainsi que les solvates, sels, tautomères et stéréoisomères pharmaceutiquement acceptables de ceux-ci, y compris des mélanges de ceux-ci en toutes proportions.

4. Composés selon l'une ou plusieurs parmi les revendications 1-3, dans lesquels
R désigne NR²R⁴,
ainsi que les solvates, sels, tautomères et stéréoisomères pharmaceutiquement acceptables de ceux-ci, y compris des mélanges de ceux-ci en toutes proportions.

5. Composés selon l'une ou plusieurs parmi les revendications 1-4, dans lesquels
R¹ désigne (CH₂)ₙ, [C(R⁴)₂]ₙAr¹- ou (CH₂)ₙHet-,
ainsi que les solvates, sels, tautomères et stéréoisomères pharmaceutiquement acceptables de ceux-ci, y compris des mélanges de ceux-ci en toutes proportions.

6. Composés selon l'une ou plusieurs parmi les revendications 1-5, dans lesquels
R² désigne [C(R⁴)₂]ₙAr², (CH2)ₙCyc ou (CH₂)ₙHet¹,
ainsi que les solvates, sels, tautomères et stéréoisomères pharmaceutiquement acceptables de ceux-ci, y compris des mélanges de ceux-ci en toutes proportions.

7. Composés selon l'une ou plusieurs parmi les revendications 1-6, dans lesquels
Het désigne benzimidazolyle ou indolyle, chacun d'entre eux étant non substitué ou monosubstitué par Hal,
ainsi que les solvates, sels, tautomères et stéréoisomères pharmaceutiquement acceptables de ceux-ci, y compris des mélanges de ceux-ci en toutes proportions.

8. Composés selon l'une ou plusieurs parmi les revendications 1-7, dans lesquels
A désigne alkyle non ramifié ou ramifié ayant 1-6 atomes de C, où 1-5 atomes de H peuvent être remplacés par F, Cl et/ou OH,
ainsi que les solvates, sels, tautomères et stéréoisomères pharmaceutiquement acceptables de ceux-ci, y compris des mélanges de ceux-ci en toutes proportions.

9. Composés selon l'une ou plusieurs parmi les revendications 1-8, dans lesquels
Q¹ désigne 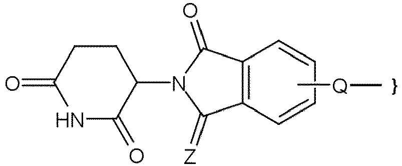
ou 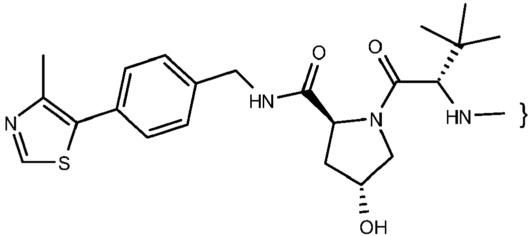
Z désigne O ou CH₂,
Q désigne O, NH, NCH₃ ou CH₂,
Q² désigne alkylène non ramifié ayant 4-25 atomes de C, où 1-8 groupements CH₂ non adjacents peuvent être remplacés par O, CONH et/ou NHCO et où un groupement CH₂ peut être remplacé par 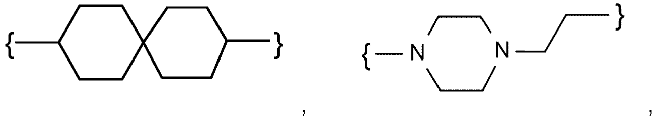 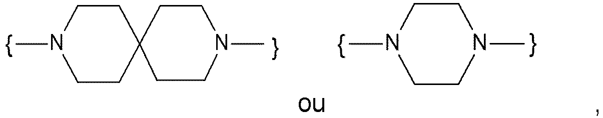
Q³ désigne 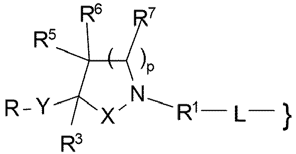
L , désigne NR⁴CO, CONR⁴, NH, O, CO, S, SO₂, SO(=NH), NHCONH, SO₂NH ou NHSO₂,
R désigne NR²R⁴,
X désigne CO ou CH₂,
Y désigne CO ou CH₂,
R¹ désigne (CH₂)ₙ, [C(R⁴)₂]ₙAr¹- ou (CH₂)ₙHet-, où le substituant L est relié directement à Ar¹, Het ou Cyc,
R² désigne [C(R⁴)₂]ₙAr², (CH2)ₙCyc ou (CH₂)ₙHet¹,
R³ désigne OH,
R⁴ désigne H ou alkyle ayant 1, 2, 3 ou 4 atomes de C,
R⁵, R⁶ désignent H,
R⁷ désigne H, Hal ou A,
Ar¹ désigne phényle,
Ar² désigne phényle qui est non substitué ou mono-, di-, tri- ou tétrasubstitué par Hal,
Het désigne pyrazinyle, pyrazolyle, benzimidazolyle, pyridyle, thiényle, furanyle, indolyle, dihydroindolyle, benzofuranyle, tétrahydropyranyle, dihydroquinoléinyle, dihydroisoquinoléinyle, tétrahydroquinoléinyle, tétrahydroisoquinoléinyle, indazolyle, imidazolyle, pyrrolyle, oxazolyle, oxadiazolyle, isoxazolyle, benzothiazolyle, pipéridin-1-yle, pyrrolidin-1-yle, 3,4-dihydro-2H-pyrido[3,2b]-1,4-oxazinyle, 3,4-dihydro-2H-benzo-1,4-oxazinyle, benzofuranyle, azétidinyle, 1H-pyrrolo[2,3-b]pyridinyle, 2H-chroményle, 3-azabicylo[3.2.0]hexyle, pyrrolo[2,3-b]pyridinyle, tétrahydrofuranyle, tétrahydro-1,8-naphtyridinyle, 2,3-dihydro-benzoisothia-zolyle, 1,2,3,4-tétrahydrobenzothiazinyle ou hexahydrobenzo-1,3-dioxolyle, chacun d'entre eux étant non substitué ou mono-, di- ou trisubstitué par Hal, A, OA, CN, NH₂, NHA, NA₂, NO₂, CN, COOH, COOA, (CH₂)ₙCONH₂, (CH₂)ₙCONHA, (CH₂)ₙCONA₂, NHCOA, COA, CHO, Het¹, SO₂A,SO₂NH₂, SO₂NHA, SO₂NA₂, CONHNH₂, CONHAr³, =O et/ou Ar³,
Het¹ désigne pyridyle, furyle, thiényle, imidazolyle ou pyrrolyle,
A désigne alkyle non ramifié ou ramifié ayant 1-6 atomes de C, où 1-5 atomes de H peuvent être remplacés par F, Cl et/ou OH,
Cyc désigne alkyle cyclique ayant 3-7 atomes de C,
Hal désigne F, Cl, Br ou I,
n désigne 0, 1, 2, 3 ou 4,
p désigne 1, 2 ou 3,
ainsi que les solvates, sels, tautomères et stéréoisomères pharmaceutiquement acceptables de ceux-ci, y compris des mélanges de ceux-ci en toutes proportions.

10. Composés selon la revendication 1, choisis dans le groupe constitué par
| n° | structure chimique |
|---|---|
| «A1 » | 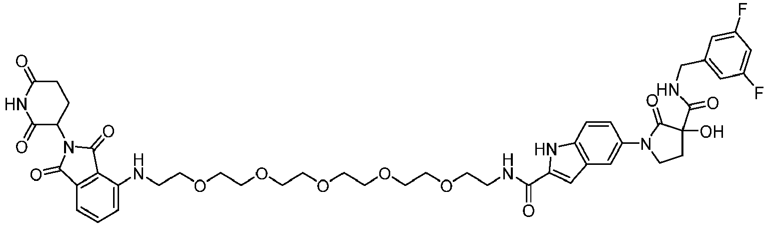 |
| « A2 » | 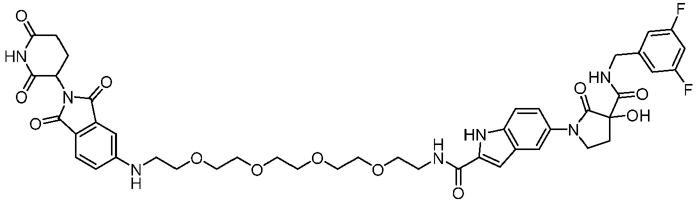 |
| « A3 » | 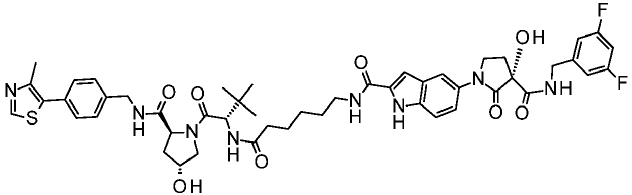 |
| « A4 » | 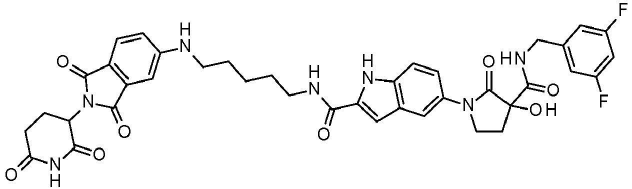 |
| « A5 » | 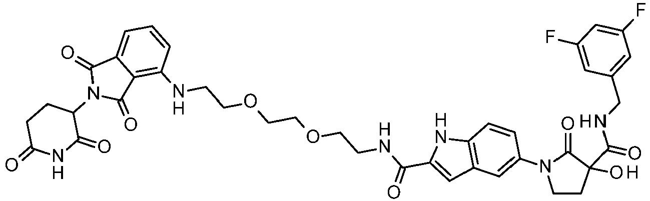 |
| « A6 » | 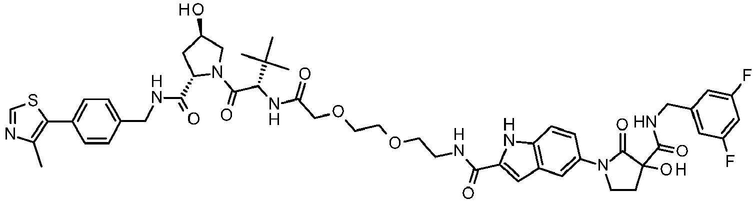 |
| « A7 » | 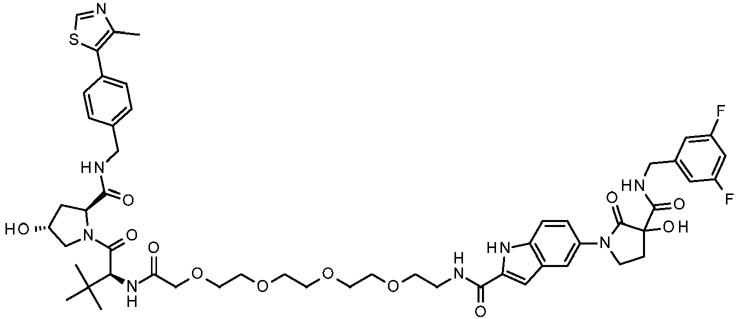 |
| « A8 » | 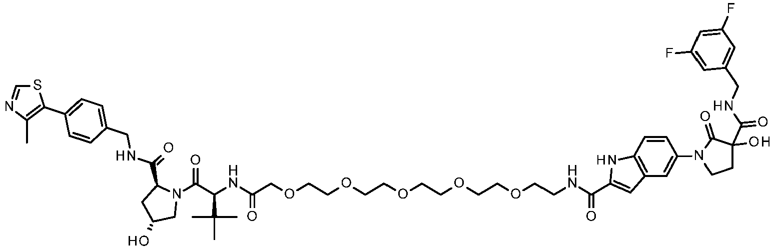 |
| « A9 » | 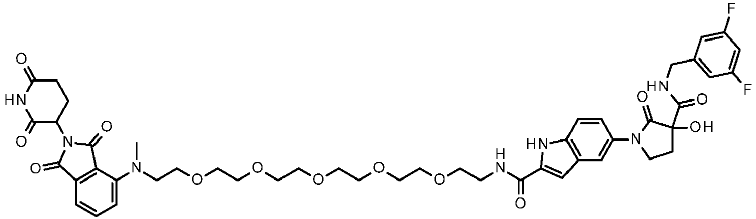 |
| « A10 » | 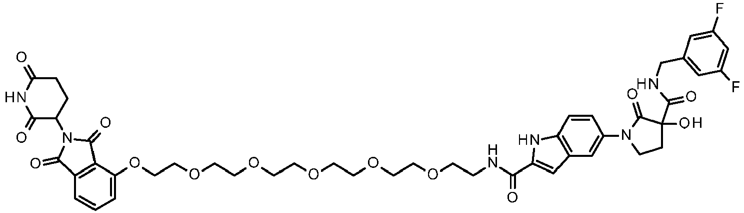 |
| « A11 » | 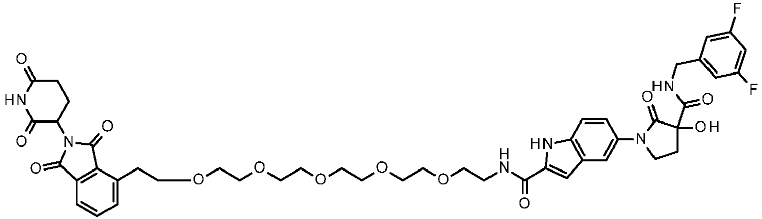 |
| « A12 » | 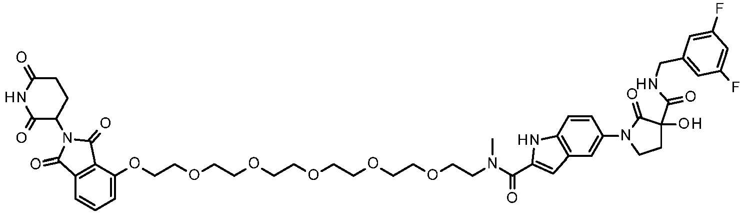 |
| « A13 » | 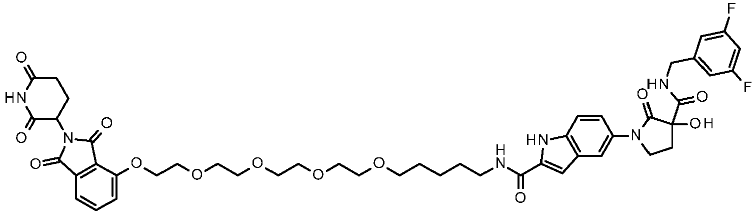 |
| « A14 » | 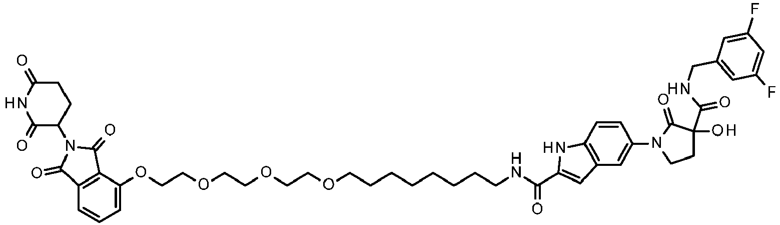 |
| « A15 » | 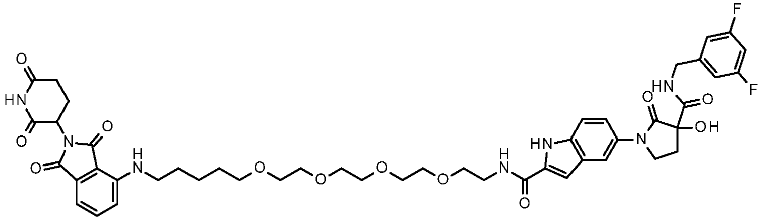 |
| « A16 » | 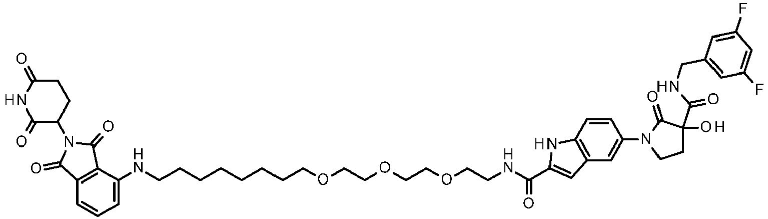 |
| « A17 » | 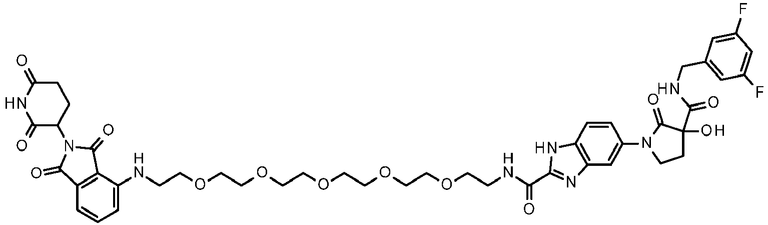 |
| « A18 » | 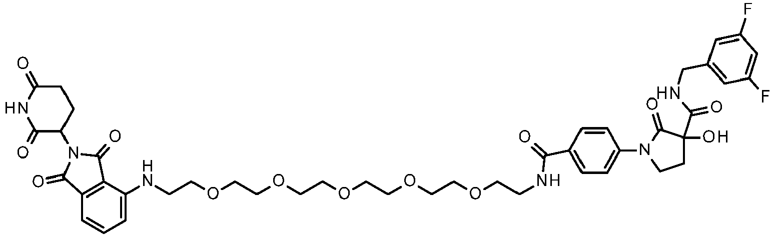 |
| « A19 » | 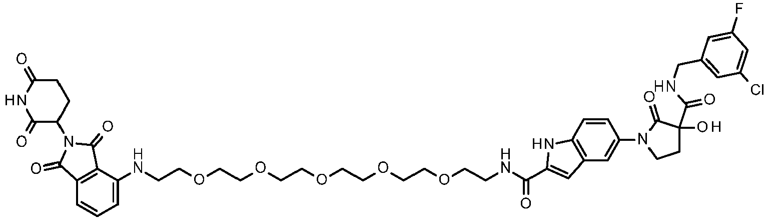 |
| « A20 » | 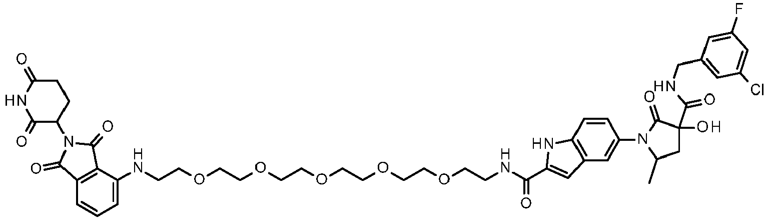 |
| « A21 » | 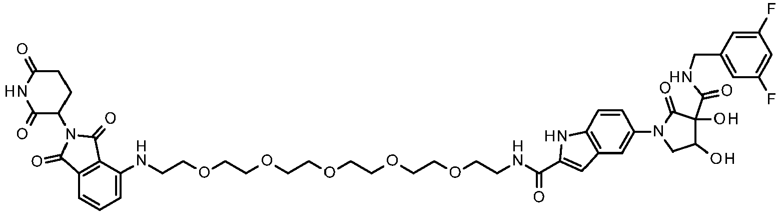 |
| « A22 » | 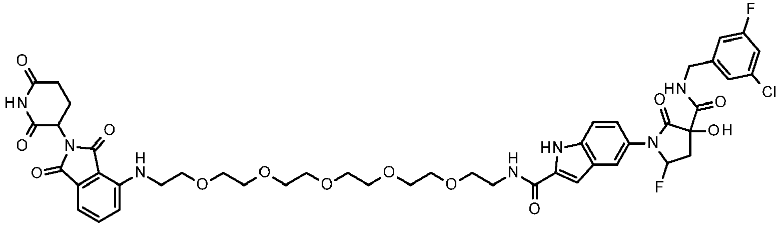 |
| « A23 » | 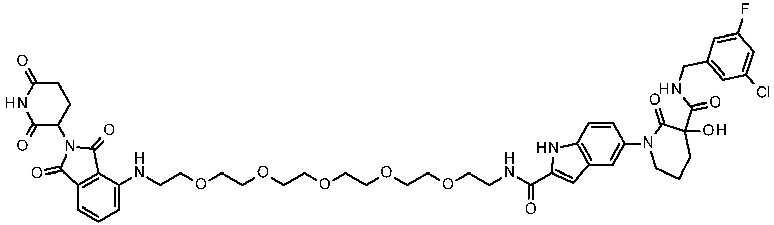 |
| « A24 » | 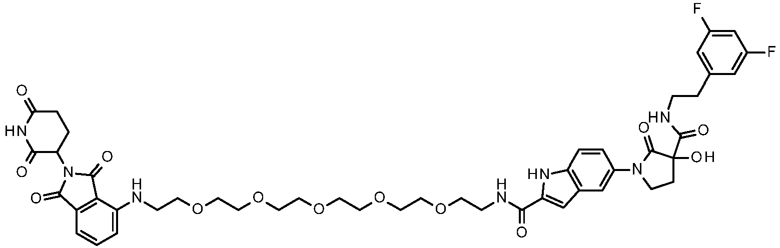 |
| « A25 » | 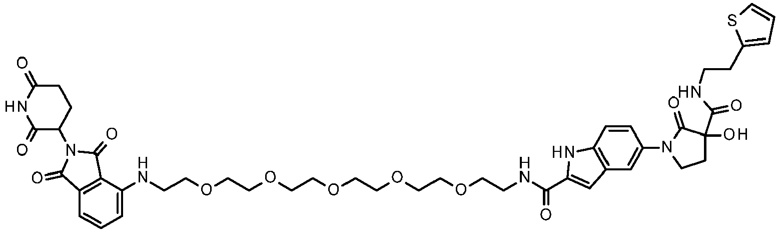 |
| « A26 » | 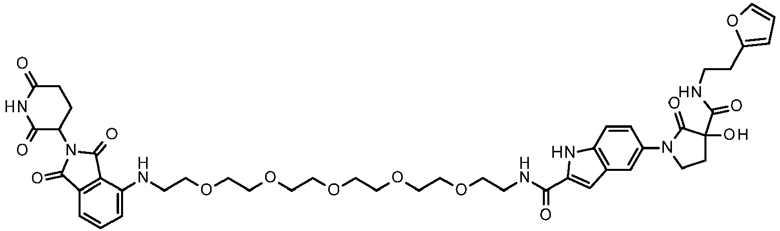 |
| « A27 » | 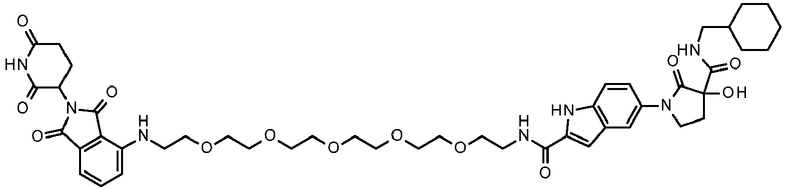 |
| « A28 » | 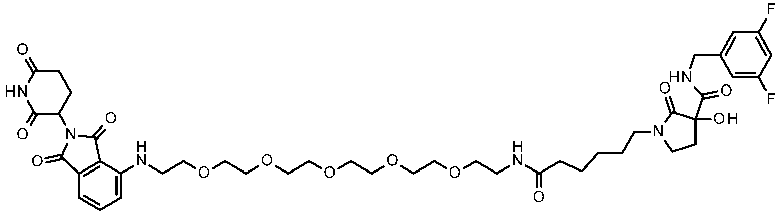 |
| « A29 » | 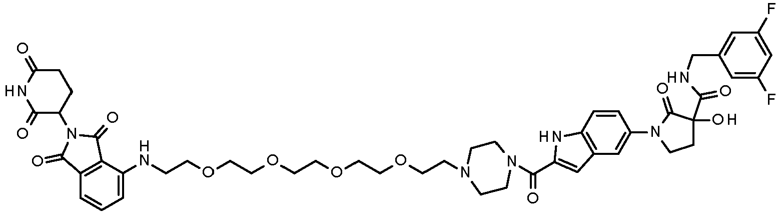 |
| « A30 » | 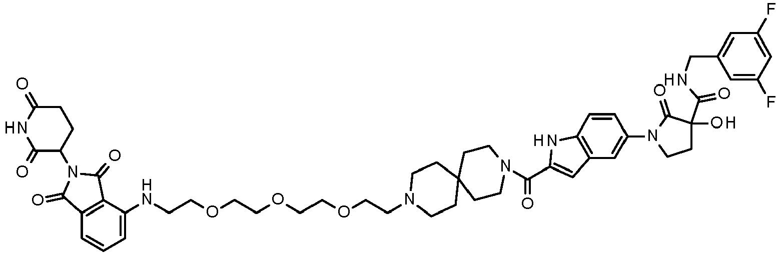 |
| « A31 » | 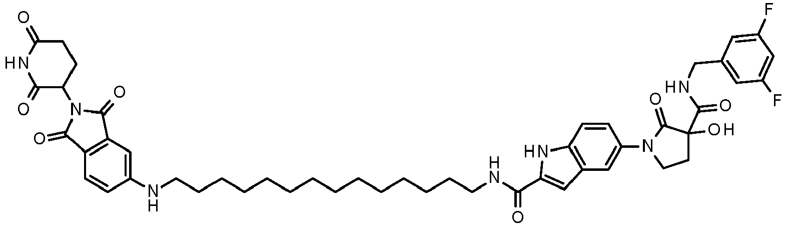 |
| « A32 » | 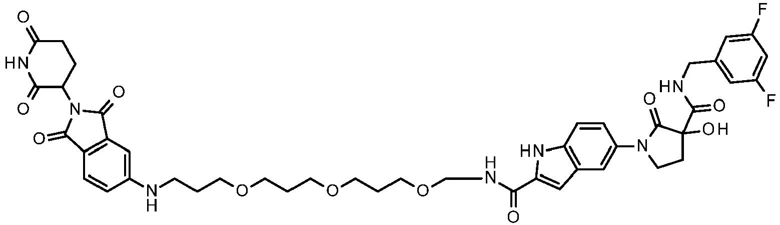 |
| « A33 » | 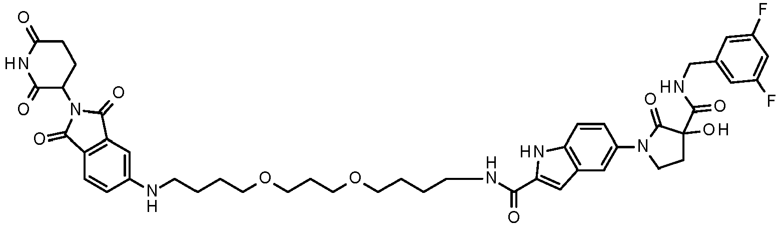 |
| « A34 » | 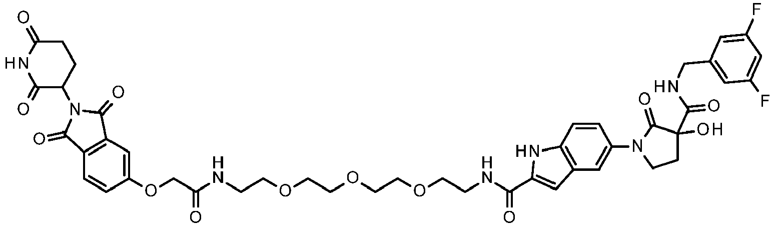 |
| « A35 » | 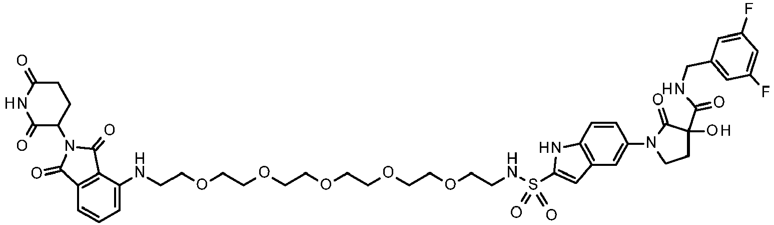 |
| « A36 » | 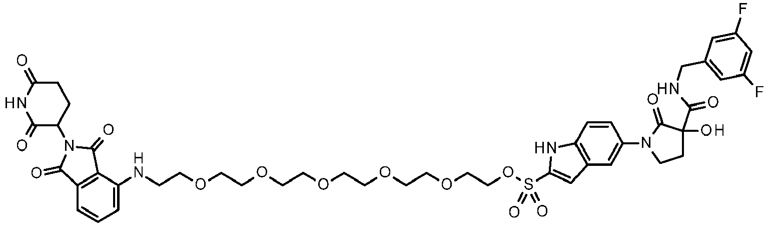 |
| « A37 » | 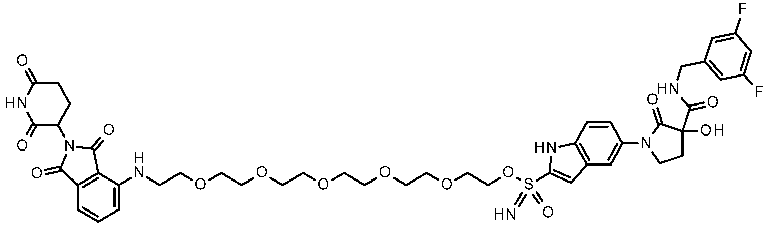 |
| « A38 » | 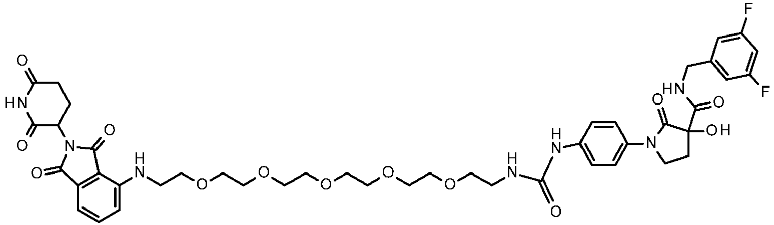 |
| « A39 » | 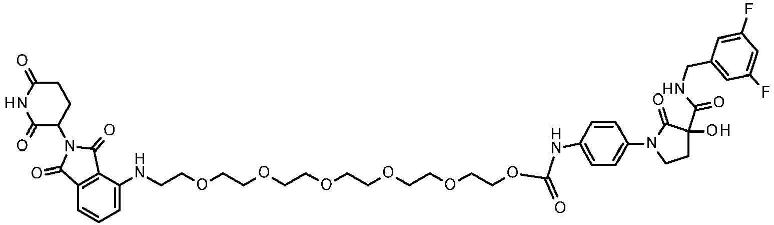 |
| « A40 » | 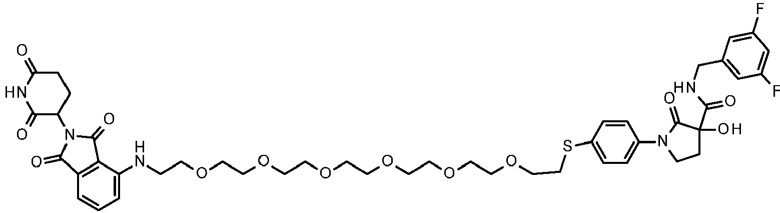 |
| « A41 » | 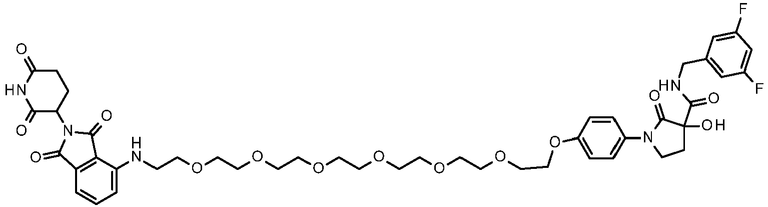 |
| « A42 » | 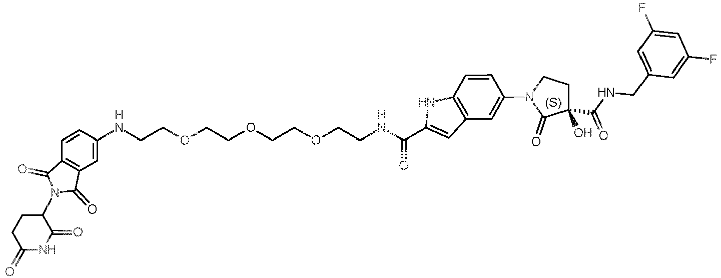 |
| « A43 » | 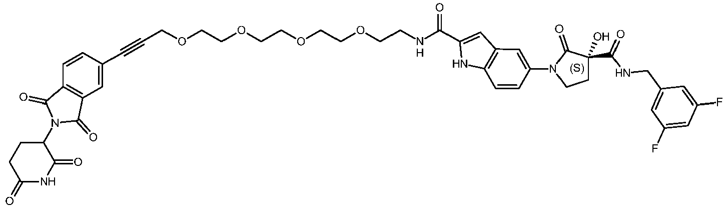 |
| « A44 » | 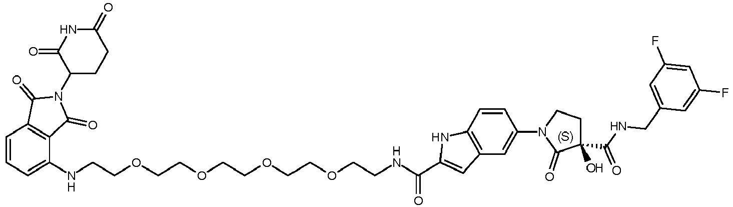 |
| « A45 » | 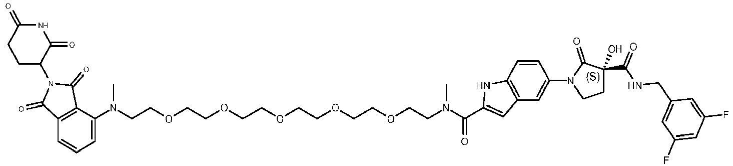 |
| « A46 » | 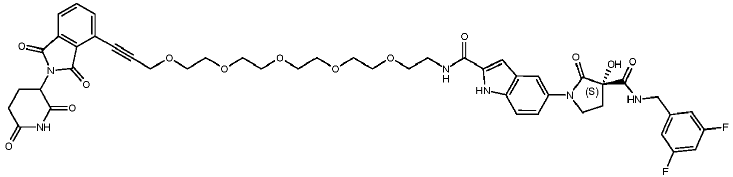 |
| « A47 » | 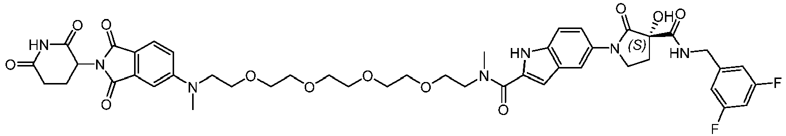 |
| « A48 » | 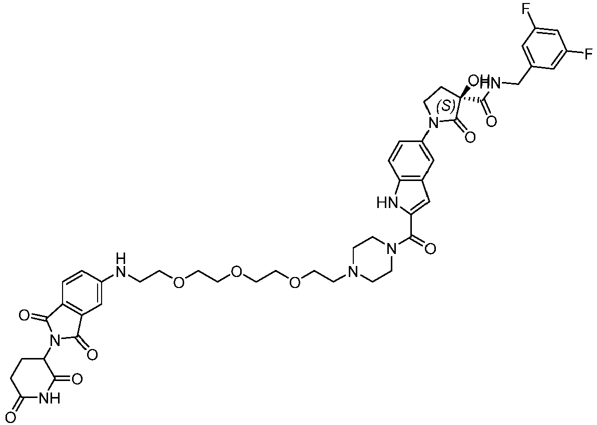 |
| « A49» | 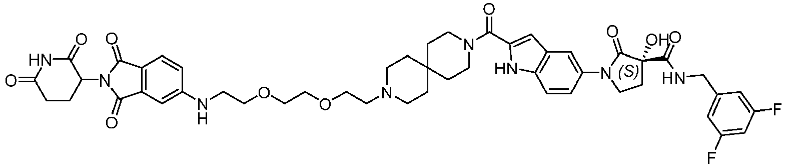 |
| « A50 » | 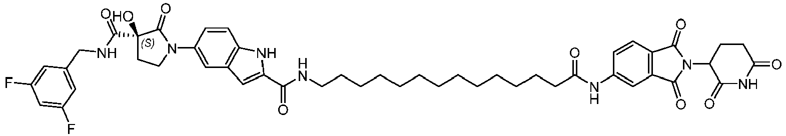 |
| « A51 » | 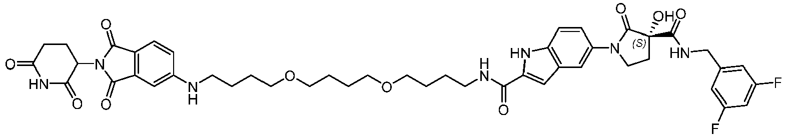 |
| « A52 » | 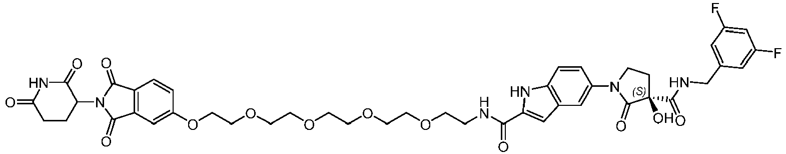 |
| « A53 » | 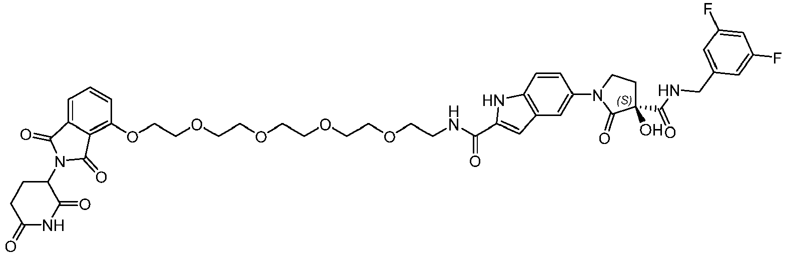 |
| « A54 » | 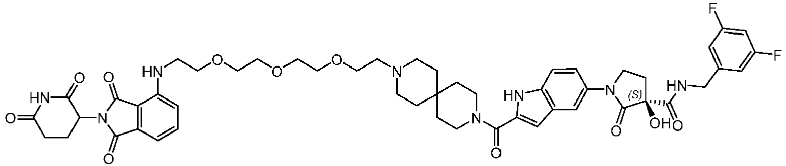 |
| « A55 » | 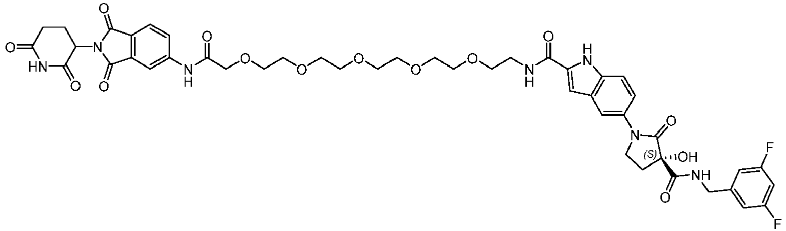 |
| « A56 » | 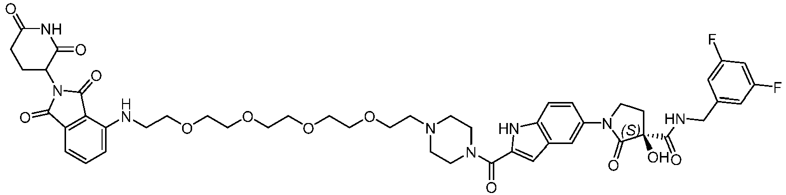 |
| « A57 » | 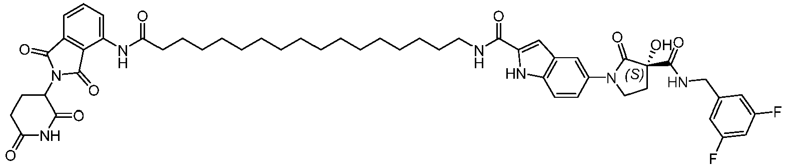 |
ainsi que les solvates, sels, tautomères et stéréoisomères pharmaceutiquement acceptables de ceux-ci, y compris des mélanges de ceux-ci en toutes proportions.

11. Procédé de préparation de composés de formule I selon les revendications 1-10 et de sels, solvates, tautomères et stéréoisomères pharmaceutiquement acceptables de ceux-ci,
dans lesquels L désigne CONR⁴,
**caractérisé en ce qu'**un composé de formule II 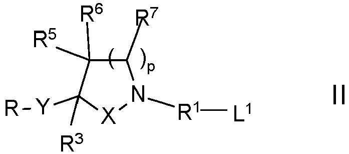
dans lequel X, R, R¹, R³, R⁵, R⁶, R⁷ et p revêtent les significations indiquées selon la revendication 1,
et L¹ désigne Cl, Br, I ou un groupement OH libre ou modifié de manière réactive et fonctionnelle,
est réagi avec un composé de formule III
Q¹-Q²-NH₂ III
dans lequel Q¹ et Q² revêtent les significations indiquées selon la revendication 1,
et/ou
une base ou un acide de formule I est converti(e) en l'un de ses sels.

12. Médicaments comprenant au moins un composé de formule I selon la revendication 1 et/ou des sels, solvates, tautomères et stéréoisomères pharmaceutiquement acceptables de celui-ci, y compris des mélanges de ceux-ci en toutes proportions, et éventuellement un support, un excipient ou un véhicule pharmaceutiquement acceptable.

13. Composés de formule I selon la revendication 1 et les sels, solvates, tautomères et stéréoisomères pharmaceutiquement acceptables de ceux-ci, y compris des mélanges de ceux-ci en toutes proportions, pour une utilisation dans le traitement et/ou la prévention de tumeurs, de métastases tumorales, de maladies prolifératives des cellules mésangiales, de l'hémangiome, de la rétinopathie proliférative, de la polyarthrite rhumatoïde, de la néovascularisation athérosclérotique, du psoriasis, de la néovascularisation oculaire, de l'ostéoporose, du diabète et de l'obésité, de la leucémie lymphoïde, du lymphome, du paludisme et de l'hypertrophie de la prostate.

14. Composés pour une utilisation selon la revendication 13, la maladie tumorale étant choisie dans le groupe de l'épithélium squameux, de la vessie, de l'estomac, des reins, de la tête et du cou, de l'oesophage, du col de l'utérus, de la thyroïde, des intestins, du foie, du cerveau, de la prostate, du tractus urogénital, du système lymphatique, du larynx, du poumon, de la peau, la leucémie monocytique, l'adénocarcinome du poumon, le carcinome du poumon à petites cellules, le cancer du pancréas, le glioblastome, le carcinome du sein, la leucémie myéloïde aiguë, la leucémie myéloïde chronique, la leucémie lymphoïde aiguë, la leucémie lymphoïde chronique, le lymphome hodgkinien, le lymphome non hodgkinien.

15. Médicaments comprenant au moins un composé de formule I selon la revendication 1 et/ou des sels, solvates et stéréoisomères pharmaceutiquement acceptables de celui-ci, y compris des mélanges de ceux-ci en toutes proportions, et au moins un autre ingrédient actif médicamenteux.

16. Ensemble (kit) constitué de conditionnements séparés
(a) d'une quantité efficace d'un composé de formule I selon la revendication 1 et/ou de sels, solvates et stéréoisomères pharmaceutiquement acceptables de celui-ci, y compris des mélanges de ceux-ci en toutes proportions,
et
(b) d'une quantité efficace d'un autre ingrédient actif médicamenteux.
